# EUROPEAN PATENT APPLICATION

(11) **EP 1 683 865 A2**
(43) Date of publication of application: **26.07.2006**
(21) Application number: 05111398.3
(22) Date of filing: 28.01.2002
(51) Int. Cl.: C12N 15/12, C07K 14/47, C12Q 1/68, C07K 16/18, G01N 33/53, C12N 15/52, C07K 14/435

(54) **Mammalian proteins and in particular CD200**

(30) Priority: 02.02.2001 US 266359 P; 21.02.2001 US 270564 P; 13.07.2001 US 305058 P
(62) Divisional of application: 02718810.1
(71) Applicant: Eli Lilly & Company, Indianapolis, IN 46285 (US)
(72) Inventor: Bhatt, Ramesh Rajani, Carmel, INC 46033 (US); Perkins, Douglas Raymond, New Palestine, IN 46163 (US); Rowlinson, Scott William, Zionsville, IN 46077 (US); Smith, Rosamund Carol, Greenfield, IN 46140 (US); Su, Eric Wen, Carmel, IN 46032 (US); Wang, He, Carmel, IN 46032 (US); Zhi, Yu, Indianapolis, IN 46202 (US); Calley, John Nels, Indianapolis, IN 46280 (US); Heuer, Josef Georg, Indianapolis, IN 46214 (US); Keleher, Gerald Patrick, Indianapolis, IN 46206 (US); Lancaster, Joanne Sloan, Indianapolis, IN 46288 (US); Li, Qingqin, Flemington, NJ 08822 (US); Lu, Deshun, Carmel, IN 46033 (US); Mills, Bradley Jay, Fountaintown, IN 46130 (US); Mishra, Santosh Kumar, 598315 Singapore (SG)
(74) Representative: Ingham, Stephen H.

(57) **Abstract**

Isolated nucleic acid molecules encoding polypeptides from a human, reagents related thereto (including purified polypeptides specific antibodies) are provided.
Methods of using said reagents and diagnostic kits are also provided.

## Description

### FIELD OF THE INVENTION

The present invention generally relates to compositions related to proteins. In particular, it provides purified genes, polynucleotide sequences, proteins, polypeptides, antibodies, binding compositions, and related reagents useful, e.g., in the diagnosis, treatment, and prevention of cell proliferative, autoimmune/inflammatory, cardiovascular, neurological, and developmental disorders, and in the assessment of the effects of exogenous compounds on the expression of nucleic acid and amino acid sequences of such proteins.

### BACKGROUND OF THE INVENTION

Protein transport and secretion are essential for cellular function. Protein transport is mediated by a signal peptide located at the amino terminus of the protein to be transported or secreted. Proteins targeted to the ER may either proceed through the secretory pathway or remain in any of the secretory organelles such as the ER, Golgi apparatus, or lysosomes. Proteins that transit through the secretory pathway are either secreted into the extracellular space or retained in the plasma membrane. Proteins that are retained in the plasma membrane contain one or more transmembrane domains, each comprised of about 20 hydrophobic amino acid residues. Secreted proteins are generally synthesized as inactive precursors that are activated by post-translational processing events during transit through the secretory pathway. Such events include glycosylation, proteolysis, and removal of the signal peptide by a signal peptidase. Examples of secreted proteins with amino terminal signal peptides are discussed below and include proteins with important roles in cell-to-cell signaling. Such proteins include transmembrane receptors and cell surface markers, extracellular matrix molecules, cytokines, hormones, growth and differentiation factors, enzymes, neuropeptides, and vasomediators (reviewed in Alberts, et al. (1994) Molecular Biology of The Cell, Garland Publishing, New York, NY, pp. 557-560, 582-592.). The discovery of new secreted proteins and the polynucleotides encoding them satisfies a need in the art by providing new compositions which are useful in the diagnosis, prevention, and treatment of cell proliferative, autoimmune/inflammatory, cardiovascular, neurological, and developmental disorders, and in the assessment of the effects of exogenous compounds on the expression of nucleic acid and amino acid sequences of secreted proteins.

### SUMMARY OF THE INVENTION

The present invention is based in part upon the discovery of LP (LP194, LP263a, LP263b, LP264, LP265, LP283, LP286, LP284, LP282, LP273, LP277, LP287, LP209, LP209b, LP209c, LP209d, LP293, LP294, or LP295) proteins and/or polypeptides. The invention provides substantially pure, isolated, and/or recombinant LP protein or peptide (LP194, LP263a, LP263b, LP264, LP265, LP283, LP286, LP284, LP282, LP273, LP277, LP287, LP209, LP209b, LP209c, LP209d, LP293, LP294, or LP295) exhibiting identity over a length of at least about 12 contiguous amino acids to a corresponding sequence of SEQ ID NO: Y; a natural sequence LP (LP194, LP263a, LP263b, LP264, LP265, LP283, LP286, LP284, LP282, LP273, LP277, LP287, LP209, LP209b, LP209c, LP209d, LP293, LP294, or LP295) of SEQ ID NO: Y; a fusion protein comprising LP (LP194, LP263a, LP263b, LP264, LP265, LP283, LP286, LP284, LP282, LP273, LP277, LP287, LP209, LP209b, LP209c, LP209d, LP293, LP294, or LP295) sequence. In preferred embodiments, the portion is at least about 20, 25, 30, 35, 40, 45, 50, 55, 60, 65, 70, 75, 80, 85, 90, 95, or 100 contiguous amino acid residues in length. In other embodiments, the LP (LP194, LP263a, LP263b, LP264, LP265, LP283, LP286, LP284, LP282, LP273, LP277, LP287, LP209, LP209b, LP209c, LP209d, LP293, LP294, or LP295): LP194 comprises a mature sequence of Table 1; LP263a and LP263b comprises a mature sequence of Table 2; LP264 comprises a mature sequence of Table 3; LP265 comprises a mature sequence of Table 4; LP283 comprises a mature sequence of Table 5; LP286 comprises a mature sequence of Table 6; LP284 comprises a mature sequence of Table 7; LP282 comprises a mature sequence of Table 8; LP273 comprises a mature sequence of Table 9; LP277 comprises a mature sequence of Table 10; LP287 comprises a mature sequence of Table 11; LP209 comprises a mature sequence of Table 12; LP209b comprises a mature sequence of Table 13; LP209c comprises a mature sequence of Table 14; and LP209d comprises a mature sequence of Table 15; LP293 comprises a mature sequence of Table 16; LP294 comprises a mature sequence of Table 17; and LP295 comprises a mature sequence of Table 18; protein or peptide: is from a warm blooded animal selected from a mammal, including a primate; comprises at least one polypeptide segment of SEQ ID NO:Y exhibits a plurality of portions exhibiting the identity; is a natural allelic variant of the LP (LP194, LP263a, LP263b, LP264, LP265, LP283, LP286, LP284, LP282, LP273, LP277, LP287, LP209, LP209b, LP209c, LP209d, LP293, LP294, or LP295) has a length at least about 30 amino acids; exhibits at least two non-overlapping epitopes which are specific for a mammalian LP(LP194, LP263a, LP263b, LP264, LP265, LP283, LP286, LP284, LP282, LP273, LP277, LP287, LP209, LP209b, LP209c, LP209d, LP293, LP294, or LP295) exhibits identity over a length of at least about 20 amino acids to LP (LP194, LP263a, LP263b, LP264, LP265, LP283, LP286, LP284, LP282, LP273, LP277, LP287, LP209, LP209b, LP209c, LP209d, LP293, LP294, or LP295) exhibits at least two non-overlapping epitopes which are specific for a LP (LP194, LP263a, LP263b, LP264, LP265, LP283, LP286, LP284, LP282, LP273, LP277, LP287, LP209, LP209b, LP209c, LP209d, LP293, LP294, or LP295) exhibits identity over a length of at least about 25 amino acids to a primate LP (LP194, LP263a, LP263b, LP264, LP265, LP283, LP286, LP284, LP282, LP273, LP277, LP287, LP209, LP209b, LP209c, LP209d, LP293, LP294, or LP295) is glycosylated; is a synthetic polypeptide; is attached to a solid substrate; is conjugated to another chemical moiety; is a 5-fold or less substitution from natural LP (LP194, LP263a, LP263b, LP264, LP265, LP283, LP286, LP284, LP282, LP273, LP277, LP287, LP209, LP209b, LP209c, LP209d, LP293, LP294, or LP295) sequence; or is a deletion or insertion variant from a natural LP (LP194, LP263a, LP263b, LP264, LP265, LP283, LP286, LP284, LP282, LP273, LP277, LP287, LP209, LP209b, LP209c, LP209d, LP293, LP294, or LP295) sequence. Various preferred embodiments include a composition comprising: a sterile LP (LP194, LP263a, LP263b, LP264, LP265, LP283, LP286, LP284, LP282, LP273, LP277, LP287, LP209, LP209b, LP209c, LP209d, LP293, LP294, or LP295) protein or peptide and a carrier, wherein the carrier is: an aqueous compound, including water, saline, and/or buffer; and/or formulated for oral, rectal, nasal, topical, or parenteral administration. The invention further provides a fusion protein, comprising: mature protein comprising sequence of Table 1, 2, 3, 4, 5, 6, 7, 8, 9, 10, 11, 12, 13, 14, 15, 16, 17, or 18 a detection or purification tag, including a FLAG, His6, or Ig sequence; or sequence of another LP (LP194, LP263a, LP263b, LP264, LP265, LP283, LP286, LP284, LP282, LP273, LP277, LP287, LP209, LP209b, LP209c, LP209d, LP293, LP294, or LP295) protein or peptide. These reagents also make available a kit comprising such an LP (LP194, LP263a, LP263b, LP264, LP265, LP283, LP286, LP284, LP282, LP273, LP277, LP287, LP209, LP209b, LP209c, LP209d, LP293, LP294, or LP295) protein or polypeptide, and: a compartment comprising the protein or polypeptide; and/or instructions for use or disposal of reagents in the kit. Providing an antigen, the invention further provides a binding compound comprising an antigen binding portion from an antibody, which specifically binds to a natural LP (LP194, LP263a, LP263b, LP264, LP265, LP283, LP286, LP284, LP282, LP273, LP277, LP287, LP209, LP209b, LP209c, LP209d, LP293, LP294, or LP295) protein or polypeptide, wherein: the protein or polypeptide is a primate protein; the binding compound is an Fv, Fab, or Fab2 fragment; the binding compound is conjugated to another chemical moiety; or the antibody: is raised against a peptide sequence of a mature polypeptide comprising sequence of Table 1, 2, 3, 4, 5, 6, 7, 8, 9, 10, 11, 12, 13, 14, 15, 16, 17, or 18 is raised against a mature LP (LP194, LP263a, LP263b, LP264, LP265, LP283, LP286, LP284, LP282, LP273, LP277, LP287, LP209, LP209b, LP209c, LP209d, LP293, LP294, or LP295) is immunoselected; is a polyclonal antibody; binds to a denatured LP, LP1, LP2, LP3, LP4, LP5, LP6, LP7, LP8, LP9, LP10, LP11, LP12, LP13, LP14, LP15, LP16, LP17, LP18 or LP19 exhibits a Kd to antigen of at least 30 µM; is attached to a solid substrate, including a bead or plastic membrane; is in a sterile composition; or is detectably labeled, including, for example, a radioactive, enzymatic, structural, or fluorescent label. Preferred kits include those containing the binding compound, and: a compartment comprising the binding compound; and/or instructions for use or disposal of reagents in the kit. Many of the kits will be used for making a qualitative or quantitative analysis. Other preferred compositions will be those comprising: a sterile binding compound, or the binding compound and a carrier, wherein the carrier is: an aqueous compound, including water, saline, and/or buffer; and/or formulated for oral, rectal, nasal, topical, or parenteral administration. The present invention further provides an isolated or recombinant LP nucleic acid encoding a protein or peptide or fusion protein described above, wherein: the LP protein and/or polypeptide is from a mammal, including a primate; or the LP nucleic acid: encodes an antigenic peptide sequence from an LP (LP194, LP263a, LP263b, LP264, LP265, LP283, LP286, LP284, LP282, LP273, LP277, LP287, LP209, LP209b, LP209c, LP209d, LP293, LP294, or LP295) of Table 1, 2, 3, 4, 5, 6, 7, 8, 9, 10, 11, 12, 13, 14, 15, 16, 17, or 18 encodes a plurality of antigenic peptide sequences from an LP(LP194, LP263a, LP263b, LP264, LP265, LP283, LP286, LP284, LP282, LP273, LP277, LP287, LP209, LP209b, LP209c, LP209d, LP293, LP294, or LP295) of Table 1, 2, 3, 4, 5, 6, 7, 8, 9, 10, 11, 12, 13, 14, 15, 16, 17, or 18 exhibits identity to a natural cDNA encoding the segment; is an expression vector; further comprises an origin of replication; is from a natural source; comprises a detectable label; comprises synthetic nucleotide sequence; is less than 6 kb, preferably less than 3 kb; is from a mammal, including a primate; comprises a natural full length coding sequence; is a hybridization probe for a gene encoding an LP family protein; or is a PCR primer, PCR product, or mutagenesis primer. In certain embodiments, the invention provides a cell or tissue comprising such a recombinant LP nucleic acid. Preferred cells include: a prokaryotic cell; a eukaryotic cell; a bacterial cell; a yeast cell; an insect cell; a mammalian cell; a mouse cell; a primate cell; or a human cell. Other kit embodiments include a kit comprising the described LP nucleic acid, and: a compartment comprising the LP nucleic acid; a compartment further comprising an LP (LP194, LP263a, LP263b, LP264, LP265, LP283, LP286, LP284, LP282, LP273, LP277, LP287, LP209, LP209b, LP209c, LP209d, LP293, LP294, or LP295) protein or polypeptide; and/or instructions for use or disposal of reagents in the kit. In many versions, the kit is capable of making a qualitative or quantitative analysis. Other LP nucleic acid embodiments include those which: hybridize under wash conditions of at least 42°C, 45°C, 47°C, 50°C, 55°C, 60°C, 65°C, or 70°C and less than about 500 mM, 450 mM, 400 mM, 350 mM, 300 mM, 250 mM, 200 mM, 100 mM, to an LP of SEQ ID NO: X that exhibit identity over a stretch of at least about 30, 32, 34, 36, 38, 39, 40, 42, 44, 46, 48, 49, 50, 52, 54, 56, 58, 59, 75, or at least about 150 contiguous nucleotides to an LP (LP194, LP263a, LP263b, LP264, LP265, LP283, LP286, LP284, LP282, LP273, LP277, LP287, LP209, LP209b, LP209c, LP209d, LP293, LP294, or LP295). In other embodiments: the wash conditions are at 55° C and/or 300 mM salt; 60° C and/or 150 mM salt; the identity is over a stretch is at least 55 or 75 nucleotides. In other embodiments, the invention provides a method of modulating physiology or development of a cell or tissue culture cells comprising introducing into such cell an agonist or antagonist of an LP (LP194, LP263a, LP263b, LP264, LP265, LP283, LP286, LP284, LP282, LP273, LP277, LP287, LP209, LP209b, LP209c, LP209d, LP293, LP294, or LP295).

### DETAILED DESCRIPTION OF THE PREFERRED EMBODIMENTS

### I. General

It is to be understood that this invention is not limited to the particular compositions, methods, and techniques described herein, as such compositions, methods, and techniques may, of course, vary. It is also to be understood that the terminology used herein is for the purpose of describing particular embodiments only, and is not intended to limit the scope of the present invention which is only limited by the appended claims.

As used herein, including the appended claims, singular forms of words such as "a," "an," and "the" include, e.g., their corresponding plural referents unless the context clearly dictates otherwise. Thus, for example, reference to "an organism" includes, e.g., one or more different organisms, reference to "a cell" includes, e.g., one or more of such cells, and reference to "a method" include, e.g., reference to equivalent steps and methods known to a person of ordinary skill in the art, and so forth.

Unless otherwise defined, all technical and scientific terms used herein have the same meaning as commonly understood by a person of ordinary skill in the art to which this invention belongs. Although methods and materials similar or equivalent to those described herein can be used to practice or test the present invention, suitable methods and materials are described below. All publications, patent applications, patents, and other references discussed herein are provided solely for their disclosure before the filing date of the present application. Nothing herein is to be construed as an admission that the invention is not entitled to antedate any such disclosure by virtue of its prior invention. All publications, patent applications, patents, and other references mentioned herein are incorporated by reference in their entirety for the teachings for which they are cited (as the context clearly dictates), including all figures, drawings, pictures, graphs, hyperlinks, and other form of browser-executable code.

Polynucleotide sequences encoding an LP of the present invention are analyzed with respect to the tissue sources from which they were derived. Various cDNA library/tissue information described herein is found in the cDNA library/tissues of the LIFESEQ GOLD™ database (Incyte Genomics, Palo Alto CA.) which corresponding information is incorporated herein by reference. Generally, in the LIFESEQ GOLD™ database a cDNA sequence is derived from a cDNA library constructed from a primate, (e.g., a human tissue). Each tissue is generally classified into an organ/tissue category (such as, e.g., cardiovascular system; connective tissue; digestive system; embryonic structures; endocrine system; exocrine glands; genitalia, female; genitalia, male; germ cells; hemic and immune system; liver; musculoskeletal system; nervous system; pancreas; respiratory system; sense organs; skin; stomatognathic system; unclassified/mixed; or urinary tract). Typically, the number of libraries in each category is counted and divided by the total number of libraries across all categories. Results using the LIFESEQ GOLD™ database reflect the tissue-specific expression of cDNA encoding an LP of the present invention.

Additionally, each LP sequence of the invention is also searched via BLAST against the UniGene database. The UniGene database contains a non-redundant set of gene-oriented clusters. Each UniGene cluster theoretically contains sequences that represent a unique gene, as well as related information such as the tissue types in which the gene has been expressed and map location.

Particularly interesting portions, segments, or fragments of LP's of the present invention are discovered based on an analysis of hydrophobicity plots calculated via the "GREASE" application, which is a computer program implementation based on the Kyte-Doolittle algorithm (J. Mol. Biol. (1982) 157:105-132) that calculates a hydropathic index for each amino acid position in a polypeptide via a moving average of relative hydrophobicity. A hydrophilicity plot is determined based on a hydrophilicity scale derived from HPLC peptide retention times (see, e.g., Parker, et al., 1986 Biochemistry 25:5425-5431). Another hydrophobicity index is calculated based on the method of Cowan and Whittaker (Peptide Research 3:75-80; 1990). Antigenic features of LPs are calculated based on antigenicity plots (such as, e.g., via algorithms of: Welling, et al. 1985 FEBS Lett. 188:215-218; the Hopp and Woods Antigenicity Prediction (Hopp & Woods, 1981 Proc. Natl. Acad. Sci., 78, 3824); the Parker Antigenicity Prediction (Parker, et al. 1986 Biochemistry, 25, 5425); the Protrusion Index (Thornton) Antigenicity Prediction (Thornton, et al. 1986 EMBO J., 5, 409); and the Welling Antigenicity Prediction (Welling, et al. 1985 FEBS Letters.188, 215)). Particularly interesting secondary LP structural features (e.g., such as a helix, a strand, or a coil) are discovered based on an application which is a computer implementation program based on the Predator (Frishman, and Argos, (1997) Proteins, 27, 329-335; and Frishman, D. and Argos, P. (1996) Prot. Eng., 9, 133-142); GOR IV (Methods in Enzymology 1996 RF. Doolittle Ed., vol. 266, 540-553 Garnier J, Gibrat J-F, Robson B); and Simpa96 (Levin, et al., J FEBS Lett 1986 Sep 15;205(2):303-308) algorithms. One of skill in the art can use such programs to discover such secondary structural features without undue experimentation given the sequences supplied herein.

### FEATURES OF LP NO: 1 (LP194)

It has been discovered that LP194 sequence (SEQ ID NO: 1) is expressed primarily in the following number of LIFESEQ GOLD™ database tissue and cDNA libraries: Cardiovascular System 23/68 (the numerator represents the number of libraries positively expressing LP 194 sequence and the denominator represents the total number of libraries examined. So, for example, in the LIFESEQ GOLD™ database of Cardiovascular System libraries, twenty-three libraries contained LP194 sequence out of sixty-eight cardiovascular libraries examined); Connective Tissue 6/47; Digestive System 30/148; Embryonic Structures 4/21; Endocrine System 10/53; Exocrine Glands 12/64; Genitalia, Female 26/106; Genitalia, Male 19/114; Hemic and Immune System 17/159; Liver 2/35; Musculoskeletal System 6/47; Nervous System 54/198; Pancreas 3/24; Respiratory System 18/93; Sense Organs 1/8; Skin 4/15; and Urinary Tract 15/64. Additionally, it has been discovered that LP194 sequence is expressed in the following UniGene database cDNA libraries: Aorta, Brain, Breast, CNS, Colon, Ear, Foreskin, Gall bladder, Heart, Kidney, Lung, Ovary, Parathyroid, Placenta, Prostate, Skin, Spleen, Tonsil, Uterus, Whole embryo: brain, normal breast, connective tissue, lung, and ovary.
Table 1: Primate, e.g., human, LP194 polynucleotide sequence (SEQ ID NO: 1) and corresponding polypeptide (SEQ ID NO:2). The ORF for LP194 is 168-851 bp (with the start (ATG) and stop codons (TAG) identified in bold lettering and underlined in case numbering is misidentified one skilled in the art could determine the open reading frame without undue experimentation).

LP194 Nucleotide Sequence (2475 bp)(ORF = 168-851 bp):

ATGAGGGTAGCAGGTGCTGCAAAGTTGGTGGTAGCTGTGGCAGTGTTTTTACTGACATTTTATGTTATTTCT
CAAGTATTTGAAATAAAAATGGATGCAAGTTTAGGAAATCTATTTGCAAGATCAGCATTGGACACAGCTGCA
CGTTCTACAAAGCCTCCCAGATATAAGTGTGGGATCTCAAAAGCTTGCCCTGAGAAGCATTTTGCTTTTAAA
ATGGCAAGTGGAGCAGCCAACGTGGTGGACCCAAAATCTGCCTGGAAGATAATGTTTTAATGAGTGGTGTT
AAGAATAATGTTGGAAGAGGGATCAATGTTGCCTTGGCAAATGGAAAAACAGGAGAAGTATTAGACACTAAA
TATTTTGACATGTGGGGAGGAGATGTGGCACCATTTATTGAGTTTCTGAAGGCCATACAAGATGGAACAATA
GTTTTAATGGGAACATACGATGATGGAGCAACCAAACTCAATGATGAGGCACGGCGGCTCATTGCTGATTTG
GGGAGCACATCTATTACTAATCTTGGTTTTAGAGACAACTGGGTCTTCTGTGGTGGGAAGGGCATTAAGACA
AAAAGCCCTTTTGAACAGCACATAAAGAACAATAAGGATACAAACAAATATGAAGGATGGCCTGAAGTTGTA
GAAATGGAAGGATGCATCCCCCAGAAGCAAGACTAA

LP194 Full-Length sequence (227aa):
The underlined portion is a predicted signal sequence (Met-1 to Val-26). A predicted SP cleavage site is
between Val-26 and Phe-27 indicated as follows: 1 MRVAGAAKLVVAVAVFLLTFYVISQV^FE 28

MRVAGAAKLVVAVAVFLLTFYVISQVFEIKMDASLGNLFARSALDTAARSTKPPRYKCGISKACPEKHFAFK
MASGAANVVGPKICLEDNVLMSGVKNNVGRGINVALANGKTGEVLDTKYFDMWGGDVAPFIEFLKAIQDGTI
VLMGTYDDGATKLNDEARRLIADLGSTSITNLGFRDNWVFCGGKGIKTKSPFEQHIKNNKDTNKYEGWPEVV
EMEGCIPQKQD*

An LP194 Mature sequence (201aa):
A predicted mature LP194 sequence is as follows:

FEIKMDASLGNLFARSALDTAARSTKPPRYKCGISKACPEKHFAFKMASGAANVVGPKICLEDNVLMSGVK
NNVGRGINVALANGKTGEVLDTKYFDMWGGDVAPFIEFLKAIQDGTIVLMGTYDDGATKLNDEARRLIADL
GSTSITNLGFRDNWVFCGGKGIKTKSPFEQHIKNNKDTNKYEGWPEVVEMEGCIPQKQD*

Particularly interesting portions or fragments of LP194 polypeptide include, e.g., a predicted transmembrane-like region from Met-1 to Ser-24; a predicted signal sequence from Met-1 to Val-26; another predicted signal sequence from Met-1 to Ala-40. A portion from the end of the predicted transmembrane region to the beginning of a predicted hydrophobic sequence (Ser-24 to Ser-42); also particularly interesting are LP194 fragments Ser-42 to Val-81; Val-81 to Asn-90; Val-91 to Gly-103; Lys-120 to Ile-133; Glu-134 to Met-147; Gly-148 to Thr-155; Lys-157 to Lys-168; Gly-169 to Val-183; Phe-184 to Asn-202; and Asn-203 to Asp-227, each of which was discovered based on an analysis of a hydrophobicity plot. Further interesting sections of LP194 are the portions of the polypeptide from Arg-49 to Gly-59; Val-96 to Arg-102; Pro-53 to Lys-67; Lys-117 to Trp-125; Thr-149 to Ala-154; Lys-188 to Ser-194; His-199 to Gly-211; and Pro-223 to Asp-227; which were discovered based on an analysis of antigenicity plots. Further, particularly interesting LP 194 secondary structures (e.g., such as a helix, a strand, or a coil) are the following helical structures of LP194: Lys-8 to Leu-17; Ile-23 to Phe-27; Asn-37 to Ala-48; Phe-132 to Ile-139; and Tyr-155 to Asp-167. Particularly interesting coil structures are Ser-50 to Arg-55; Gly-59 to Glu-66; Ser-75 to Lys-84; Glu-88 to Asn-90; Ser-94 to Gly-103; Asn-109 to Glu-115; Asp-123 to Val-129; Gln-140 to Gly-142; Thr-149 to Ala-155; Leu-168 to Ser-172; Leu-176 to Asp-180; Gly-186 to Phe-196; Asn-202 to Pro-213; and Glu-219 to Asp-227. Particularly interesting strands are Ile-85 to Leu-87; Val-191 to Met-93; Ile-104 to Leu-108; Val-116 to Leu-117; Tyr-121 to Phe-122; Thr-143 to Gly-148; Ile-173 to Thr-174; Asn-181 to Cys-185; and Glu-214 to Val-216. Further encompassed by the invention are contiguous amino acid residue combinations of any of the predicted secondary structures described above. For example, one coil-strand-coil motif of LP194 combines the Leu-176 to Asp-180 coil; with the Asn-181 to Cys-185 strand; and the Gly-186 to Phe-196 coil to form a fragment of contiguous amino acid residues from Leu-176 to Phe-196. Other combinations of contiguous amino acids are contemplated as can be easily determined. LP194 exhibits similarity to the human cytokine family member EF-7 protein (WO9956778-A1) and human cytokine family member 2-19 polynucleotide (W09947658-A1. Although no canonical cytokine-like motif was found when examining LP194, it is possible that Lp194 is an atypical cytokine and thus, may be important for immune system regulation.

### FEATURES OF LP NO: 2 & 3 (LP263a & LP263b)

Construction of a normalized human brain cDNA Library was used to discover LP263 a (SEQ ID NO: 3) AND LP263b (SEQ ID NO: 5). The construction was based generally on the methods of Ko (1990) Nucleic Acids Res. 18(19):5705-11 and Scares, et al (1994) Proc. Natl. Acad. Sci.91:9228-9232. Tissues from twelve brain subregions were obtained from Harvard Medical School Tissue Bank (Hypothalamus, Thalamus, Amygdyla, Sensory Cortex, Motor Cortex, Hippocampus, Cerebellum, Pons and Locus Coeruleus, Caudate/Putamen/Nucleus Accumbens, Entero-Cortex and Anterior Hippocampus, Prefrontal cortex, Anterior Cingulate Cortex) and mRNAs (made from each tissue) were mixed in equal amounts. The resulting cDNA generated from the mixed mRNA was used for PCR amplification and subsequent normalization. Normalization was evaluated by checking the ratio among high, medium, and low abundance control genes. Subsequently, a brain cDNA library was then constructed using the normalized cDNA. LP263 cDNA has two alternatively spliced forms (Table 2) designated LP263a (which is 229 amino acids; SEQ ID NO: 4) and LP263b (which is 179 amino acids; SEQ ID NO: 6).
Table 2: Primate, e.g., human, LP263a and LP263b (SEQ ID NO: 3/4 and 5/6). Nucleotide may be A, C, T, or G at positions 502,663, and 725 of the polynucleotide coding for LP263a; Nucleotide may be A, C, T, or G at positions 269,351, and 428 of the polynucleotide coding for LP263b. The ORF for LP263a is 52-741bp. The ORF for LP263b is 52-591bp. (The start and stop codons are underlined and in bold typeface.)

LP263a DNA sequence: (763bp)(ORF=52-741):

ATGGCTCTGTCCAGCCTGATCGTCATCCTGCTGGTGGTGTTCGCCCTCGTCCTGCACGGGCAGAATAAGAA
GTATAAGAACTGCAGCACAGGAAAGGGGATCTCCACCATGGAGGAGTCTGTGACCCTGGACAACGGAGGAT
TTGCTGCCCTGGAGCTCAGCAGCCGCCACCTCAATGTCAAGAGCACCTTCTCCAAGAAGAACGGGACCAGG
TCCCCACCCCGGCCTAGCCCCGGCGGCCTGCACTACTCAGACGAGGACATCTGCAACAAGTACAACGGCGC
CGTGCTGACCGAGAGCGTGAGCCTCAAGGAGAAGTCGGCAGATGCATCAGAATCTGAGGCCACGGACTCTG
ACTACGAGGACGCGCTGCCCAAGCACTCCTTCGTGAACCACTACATGAGCGACCCCACCTACTACAACTCA
TGGAAGCGCAGGGCCCAGGGCCCGNGCACCTGCGCCGCACAGGTACGAGGCGGTGGCGGGCTCCGAGGCGG
GCGCGCAGCTGCACCCGGTCATCACCACGCAGAGCGCGGGCGGCGTCTACACCCCCGCTGGCCCCGGCGCG
CGAACTCCGCTCACCGGCTTCTCCTCCTTCGTGTGAGCAAAGCNCCGCGCCTCCCTCAGGGCGGAACGGAG
GCAACTTTCCGGAGTCTATTTTTGTTAAGACAATNCAACTCCAATAACTGA

LP263a Full-Length sequence (229aa):

>g6065887GS+1_ORF1 (SEQ ID NO: 4). The underlined portion is a predicted signal sequence (Met-1 to
Ala-15). Misc. feature of LP263a is that X at position 151 (GPXTC) can be Ser (S), Cys (C), Gly (G), or Arg
(R). Misc. feature of LP263a is that X at position 225 (RQXNSN) can be Ser (S), Cys (C), Phe (F), or Tyr (Y).

MALSSLIVILLVVFALVLHGQNKKYKNCSTGKGISTMEESVTLDNGGFAALELSSRHLNVKSTFSKKNGTR
SPPRPSPGGLHYSDEDICNKYNGAVLTESVSLKEKSADASESEATDSDYEDALPKHSFVNHYMSDPTYYNS
WKRRAQGPXTCAAQVRGGGGLRGGRAAAPGHHHAERGRRLHPRWPRRANSAHRLLLLLRVSKAPRLPQGGTE
ATFRSLFLLRQXNSNN*

A Mature LP263a sequence (214aa):

Misc. feature of LP263a is that X at position 136 (GPXTC) can be Ser (S), Cys (C), Gly (G), or Arg (R). Misc.
feature of LP263a is that X at position 210 (RQXNSN) can be Ser (S), Cys (C), Phe (F), or Tyr (Y). A
predicted mature LP263 protein is as follows:

LVLHGQNKKYKNCSTGKGISTMEESVTLDNGGFAALELSSRHLNVKSTFSKKNGTRSPPRPSPGGLHYSDE
DICNKYNGAVLTESVSLKEKSADASESEATDSDYEDALPKHSFVNHYMSDPTYYNSWKRRAQGPXTCAAQV
RGGGGLRGGRAAAPGHHHAERGRRLHPRWPRRANSAHRLLLLRVSKAPRLPQGGTEATFRSLFLLRQXNSN
N*

LP263b DNA sequence (769bp) ORF= 52-591:
ATGGCTCTGTCCAGCCTGATCGTCATCCTGCTGGTGGTGTTCGCCCTCGTCCTGCACGGGCAGAATAAGAA
GTATAAGAACTGCAGCACAGGAAAGGGGATCTCCACCATGGAGGAGTCTGTGACCCTGGACAACGGAGGAT
TTGCTGCCCTGGAGCTCAGCAGCCGCCACCTCAATGTCAAGAGCACCTTCTCCAAGAAGAACGGGACCAGG
TCCCNACCCCGGCCTAGCCCCGGCGGCCTGCACTACTCAGACGAGGACATCTGCAACAAGTACAACGGCGC
CGTGCTGACCGAGAGNGTGAGCCTCAAGGAGAAGTCGGCAGATGCATCAGAATCTGAGGCCACGGACTCTG
ACTACGAGGACGCGCTGCCCANGCACTCCTTCGTGAACCACTACATGAGCGACCCCACCTACTACAACTCA
TGGAAGCGCAGGGCCCAGGGCCGCGCACCTGCGCCGCACAGTGTAGCTATCCTCCTGACGAGCAACCCGTC
TGCGTACCTAAGTATGGCTCCCCGTGGGTCAGCGTCCTGGTAG
LP263b Full-Length sequence (179aa):
>600852946set2_ORF1 (SEQ ID NO: 6). The underlined portion is a predicted signal sequence (Met-1 to
Ala-15). Misc. feature of LP263b is that X at position 73 (RSXPR) can be Leu (L), Pro (P), Gln (Q), or Arg
(R). Misc. feature of LP263a is that X at position 100 (TEXVS) can be Ser (S), or Arg (R). Misc. feature of
LP263a is that X at position 126 (LPXHS) can be Arg (R), Lys (K), Met (M), or Thr (T).

MALSSLIVILLVVFALVLHGQNKKYKNCSTGKGISTMEESVTLDNGGFAALELSSRHLNVKSTFSKKNGTR
SXPRPSPGGLHYSDEDICNKYNGAVLTEXVSLKEKSADASESEATDSDYEDALPXHSFVNHYMSDPTYYNS
WKRRAQGRAPAPHSVAILLTSNPSAYLSMAPRGSASW*

LP263b Mature sequence (164aa):

Misc. feature of LP263b is that X at position 58 (RSXPR) can be Leu (L), Pro (P), Gln (Q), or Arg (R). Misc.
feature of LP263a is that X at position 85 (TEXVS) can be Ser (S), or Arg (R). Misc. feature of LP263a is that
X at position 111 (LPXHS) can be Arg (R), Lys (K), Met (M), or Thr (T). A predicted mature LP263 sequence
is as follows:

LVLHGQNKKYKNCSTGKGISTMEESVTLDNGGFAALELSSRHLNVKSTFSKKNGTRSXPRPSPGGLHYSDED
ICNKYNGAVLTEXVSLKEKSADASESEATDSDYEDALPXHSFVNHYMSDPTYYNSWKRRAQGRAPAPHSVAI
LLTSNPSAYLSMAPRGSASW*

It has been discovered that LP263 sequences (SEQ ID NO: 3 and SEQ ID NO: 5) are expressed primarily in the following number of LIFESEQ GOLD™ database tissue and cDNA libraries: Digestive System 2/148; Endocrine System 1/53; Exocrine Glands 2/64; Genitalia, Female 3/106; Genitalia, Male 5/114; Hemic and Immune System 1/159; Nervous System 3/198; Respiratory System 7/93; and Stomatognathic System 1/10. Particularly interesting portions or fragments of LP263a (SEQ ID NO: 4) polypeptide include, e.g., a predicted signal sequence from Met-1 to Ala-15; a predicted transmembrane-like region from Ser-4 to Asp-27; also particularly interesting are LP263a fragments Leu-6 to Leu-18; His-19 to Gly-31; Lys-32 to Met-37; Ser-40 to Asp-44; Asn-45 to Leu-53; Arg-56 to Tyr-83; Thr-63 to Gly79; Asp-85 to Asn-93; Val-101 to Ser-111; Glu-114 to Pro-125; Met-134 to Pro-150, Pro-137 to Gln-148; and Glu-177 to Asn-191, each of which was discovered based on an analysis of a hydrophobicity plot. Additional interesting sections of LP263a are the portions of the polypeptide from Met-1 to Val-12; Val-13 to Asn-27; Thr-30 to Ser-40; Thr-42 to Ala-50; Leu-51 to Val-60; Thr-70 to Leu-81; Glu-86 to Asn-93; Val-110 to Glu-112; Thr-116 to Val-130; Asn-131 to Asn-141; Gln-148 to Cys-153; Gln-156 to Gly-165; Ala-170 to Arg-188; Ala-190 to Arg-206; and Lys-207 to Lys-219. These fragments were discovered based on analysis of antigenicity plots. Further, particularly interesting LP 194 secondary structures (e.g., such as a helix, a strand, or a coil) are the following helical structures of LP263a: Ser-192 to Leu-196; and Thr-215 to Arg-223. Particularly interesting coil structures are His-19 to Lys-24; Ser-29 to Ile-34; Asp-44 to Gly-47; Arg-56 to Lys-58; Ser-65 to Asp-85; Lys-91 to Gly-93; Lys-106 to Asp-119; Leu-124 to Ser-128; Met-134 to Thr-138; Gln-148 to Thr-152; Gly-159 to His-173; Leu-182 to Pro-187; Lys-203 to Thr-212 and Lys-, Met-, Thr, or Arg-225 to Asn-229. Particularly interesting strands are Val-96 to Leu-97; Phe-129 to Val-130; and Ala-155 to Val-157. Further encompassed by the invention are contiguous amino acid residue combinations of any of the predicted secondary structures described above. For example, one coil-strand-coil motif of LP263a combines the Gln-148 to Thr-152 coil; with the Ala-155 to Val-157 strand; and the Gly-159 to His-173 coil to form a fragment of contiguous amino acid residues from Gln-148 to His-173. Other combinations of contiguous amino acids are contemplated as can be easily determined.

Particularly interesting portions or fragments of LP263b (SEQ ID NO: 6) polypeptide include, e.g., a predicted signal sequence from Met-1 to Ala-15; a transmembrane-like region from Met-1 to Asn-27; also particularly interesting are LP263b fragments Met-1 to Tyr-25; Lys-26 to Ser-35; Thr-36 to His-57; Leu-58 to Ser-65; Lys-66 to Ser-72; Gly-79 to Asp-85; Glu-86 to Arg- or Ser-100; Ser-102 to Asp-119; Asp-122 to Met-134; Ser-135 to Trp-143; Lys-26 to Thr-36; Met-37 to Glu-52; Ser-65 to Ser-72; Ser-84 to Asn-93; ser-102 to Asp-117; Asp-119 to Asp-136; and Lys-144 to Pro-154; discovered based on an analysis of hydropathicity and hydrophobicity plots. Additional interesting sections of LP263a are the portions of the polypeptide from Met-1 to Val-12; Val-13 to Asn-27; Gly-20 to Gly-33; Cys-28 to Ser-40; Thr-42 to Ala-50; Leu-51 to Val-60; Thr-63 to Gly-79; Gly-79 to Ile-88; Thr-70 to Leu-81; Glu-86 to Asn-93; Val-110 to Glu-112; Thr-116 to Val-130; Tyr-133 to Gly-149; Asn-131 to Asn-141; Ser-142 to Arg-150; Pro-152 to Arg-158; Ile-159 to Pro-165; and Tyr-168 to Arg-174. These fragments were discovered based on analysis of antigenicity plots. Further, particularly interesting LP 194 secondary structures (e.g., such as a helix, a strand, or a coil) are the following helical structures of LP263b: Ser-142 to Arg-145. Particularly interesting coil structures are His-19 to Lys-24; Asp-44 to Gly-47; Arg-56 to His-57; Ser-65 to Asp-85; Lys-91 to Gly-94; Lys-106 to Ser-128; Tyr-133 to Thr-138; Gly-149 to His-155; Ser-163 to Ser-166; and Ala-172 to Trp-179. Particularly interesting strands are Phe-129 to Val-130; Val-157 to Leu-161; and Tyr-168 to Met-171. Further encompassed by the invention are contiguous amino acid residue combinations of any of the predicted secondary structures described above. For example, one coil-strand-coil-strand-coil motif of LP263b combines the Gly-149 to His-155 coil; with the Val-157 to Leu-161 strand; the Ser-163 to Ser-166 coil, the Tyr-168 to Met-171 strand, and the Ala-172 to Trp-179 coil to form a fragment of contiguous amino acid residues from Gly-149 to Trp-179. Other combinations of contiguous amino acids are contemplated as can be easily determined.

### FEATURES OF LP NO: 4 (LP264)

Construction of a normalized human brain cDNA Library (as described above) was used to discover LP264.
Table 3: Primate, e.g., human, LP264 polynucleotide sequence (SEQ ID NO: 7). Nucleotide may be A, C, T, or G at position 229 of the polynucleotide coding for LP264. An ORF for LP264 is 29-427bp (with the start and stop codons identified in bold lettering). The putative start and stop codons are underlined and in bold.)

LP264 DNA sequence (616bp)(ORF 29-427bp):
ATGTTCTTCACATTAATACTGTCATTTCAGCTACTGAGTTATTTTTCTCCTCTTTTTTTTTTTTTTTGGC
AGCAAAGCCGTTGCCTGGCCTTGAAGAGAAACCACCAGGTTGTTTGGTACTTTCAGAAGGGGGTCTTACCT
CTCACACAACTTACCTTCACCCCACACCTCTCCCCTGTGCAGACGTGACTGTGCACCANGGCAGAGCTCTC
ACCACAACAAAAGGGAAACAGGTGGGCCCGGGACGCTCTCAAAAGCAGCTCTCCTCTCCGAATCTCTTGCG
AGACAAGTGTGTTAAAGCGCAATTAGAAGGGCAAGAAATCTCCCTGTGGCGCCCTGCGGATTATCGCAGCT
ATATCCCGCGGCGCCGAGCCGGCGCCAAGGGGTGTCGTCGTTAG

LP 264 Full length Protein sequence (132aa):
Underlined portion is a predicted signal sequence (Met-1 to Phe-16).
X (underlined) at amino acid residue position 67 of the full length LP264 protein (SEQ ID NO: 8) can be His
(H) or Gln (Q).
MFFTLILSFQLLSYFFSSFFFFLAAKPLPGLEEKPPGCLVLSEGGLTSHTTYLHPTPLPCADVTVHXGRALT
TTKGKQVGPGRSQKQLSSPNLLRDKCVKAQLEGQEISLWRPADYRSYIPRRRAGAKGCRR*

A Mature LP 264 Protein (116aa):
X (underlined), which is still either His or Gln (Q), is now at amino acid residue position 51 of this example of
a mature LP264 protein in which the predicted signal sequence Met-1 to Phe-16 has been removed.
SSFFFFLAAKPLPGLEEKPPGCLVLSEGGLTSHTTYLHPTPLPCADVTVHXGRALTTTKGKQVGPGRSQKQ
LSSPNLLRDKCVKAQLEGQEISLWRPADYRSYIPRRRAGAKGCRR

Particularly interesting portions or fragments of the full length LP264 polypeptide include, e.g., a predicted signal sequence from Met-1 to Ser-17; a predicted transmembrane-like region from Thr-4 to Pro-29; also particularly interesting are LP264 fragments Lys-26 to Ser-48; His-49 to Val-65; Ala-70 to Glu-85; Asp-96 to Glu-107; Lys-26 to Val-40; Lys-41 to Thr-50; Thr-51 to Val-63; Ala-70 to Arg-83; Ser-84 to Arg-95; and Lys-97 to Gln-107, whose discovery was based on an analysis of hydrophobicity and hydrophilicity plots. Additional interesting sections of LP264 are the portions of the polypeptide from Met-1 to Phe-9; Lys-12 to Phe-22; Gly-30 to Thr-47; Lys-77 to Ser-84; Glu-104 to Trp-111; Arg-117 to Arg-123; Lys-15 to Pro-20; Thr-58 to Gly-71; Pro-97 to Arg-116; Ser-32 to Pro-41; and Thr-57 to Gln-71. These fragments were discovered based on analysis of antigenicity plots. Further, particularly interesting LP 264 secondary structures (e.g., such as a helix, a strand, or a coil) are the following LP264 helical structures: Thr-4 to Phe-9; Ser-8 to Phe-9; and Asp-96 to Ala-101. Particularly interesting coil structures are Ala-25 to Gly-37; Glu-41 to Ser-48; His-54 to Cys-60; Gln-67 to Arg-69; Thr-73 to Ser-84; Ser-89 to Pro-91; Glu-104 to Gln-106; Arg-112 to Ser-118; and Cys-130 to Arg-132. Particularly interesting strands are Leu-39 to Leu-41; and Val-63 to His-66. Further encompassed by the invention are contiguous amino acid residue combinations of any of the predicted secondary structures described above. For example, one coil-strand-coil motif of LP264 combines the Ala-25 to Gly-37 coil; with the Leu-39 to Leu-41 strand; and the Glu-43 to Ser-48 coil to form a fragment of contiguous amino acid residues from Ala-25 to Ser-48. Other combinations of contiguous amino acids are contemplated as can be easily determined.

### FEATURES OF LP NO: 5 (LP265)

Construction of a normalized human brain cDNA Library (as described above) was used to discover LP265 polynucleotide sequence (SEQ ID NO: 9).
Table 4: Primate, e.g., human, LP265 polynucleotide sequence (SEQ ID NO: 9). Nucleotide may be A, C, T, or G at position 419. The ORF for LP265 is 87-395bp (with the start and stop codons identified in bold lettering).

LP265 DNA sequence (660bp)(ORF 87-395):
The "N" at position 419 can be A, T, C, or G.

ATGCCCTTAATTGGAATACTTATCGTGCCCATGTCCAAACCTGCGGGCTCTTTTTCTTACCGCGCTTTGC
TCTTGGCCCCTCTCTTTGCCCTTGTGCCGTCTGTCTGCCCTTCACAACTCGTTTTCTTTCTGTCTCCGTGAC
TCGTTAGTTCTCCTCTGTGAATTTTCCTTTGTGGGATTATACGCGGTAATTTACTCCTGCCTTCTTCTGATG
TTGATTGCCTGGATTAGCTCTGGTCTTCCTGCTCTTGCTATGCTCGAATCTTGTAGCTTTCGAGTGATGCAT
TTGCGTGAGTTGCGAAGTTGA

LP265 Full-length Sequence (102aa):
(SEQ ID NO: 10) The underlined portion is a predicted signal sequence
(Met-1 to Ser-29).

MPLIGILIVPMSKPAGLFFLTALCSWPLSLPLCRLSALHNSFSFCLRDSLVLLCEFSFVGLYAVIYSCLLLM
LIAWISSGLPALAMLESCSFRVMHLRELRS*

A Mature LP265 Sequence (73aa):

A predicted mature LP265 is as follows:

LPLCRLSALHNSFSFCLRDSLVLLCEFSFVGLYAVIYSCLLLMLIAWISSGLPALAMLESCSFRVMHLREL
RS*

Particularly interesting portions or fragments of the full length LP265 polypeptide (SEQ ID NO: 10) include, e.g., a predicted signal sequence from Met-1 to Ser-29; a predicted transmembrane-like region from Ile-8 to Ser-36; also particularly interesting are LP265 fragments Met-1 to Ser-25; Trp-26 to Arg-34; Ser-36 to Leu-46; Arg-47 to Val-59; Gly-60 to Val-64; Ile-65 to Ile-74; and Ala-75 to Ser-102, whose discovery was based on an analysis of hydrophobicity and hydrophilicity plots. Additional interesting sections of LP265 are the portions of the polypeptide from Ile-4 to Pro-14; Gly-16 to Lys-30; Pro-31 to Asn-40; Ser-41 to Arg-47; Asp-48 to Val-51; Leu-52 to Leu-61; Tyr-62 to Met-72; Leu-73 to Leu-81 and Ala-85 to Met-95. These fragments were discovered based on analysis of antigenicity plots. A further interesting portion of LP265 is the segment FVGLYAVIYSC that is identified based on PROSITE analysis as a potential lipoprotein lipid attachment site. Further, particularly interesting LP 265 secondary structures (e.g., such as a helix, a strand, or a coil) are the following LP265 helical structures: Arg-34 to Ser-36; Ala-63 to Ile-77; Leu-84 to Leu-87; and His-96 to Glu-99. Particularly interesting coil structures are Met-1 to Leu-3; Pro-10 to Gly-16; Ser-25 to Pro-31; His-39 to Ser-41; Ser-79 to Pro-82; and Arg-101 to Ser-102. Particularly interesting strands are Gly-5 to Val-9. Further encompassed by the invention are contiguous amino acid residue combinations of any of the predicted secondary structures described above. For example, one coil-helix-coil motif of LP265 combines the Ser-25 to Pro-31 coil; with the Arg-34 to Ser-36 helix; and the His-39 to Ser-41 coil to form an interesting fragment of contiguous amino acid residues from Ser-25 to Ser-41. Other combinations of contiguous amino acids are contemplated as can be easily determined.

### FEATURES OF LP NO: 6 (LP283)

It has been discovered that LP283 sequences (SEQ ID NO: 11) are expressed primarily in the following number of LIFESEQ GOLD™ database tissue and cDNA libraries: Cardiovascular System 1/68; Genitalia, Female 1/106; Genitalia, Male 1/114; Nervous System 1/198; Respiratory System 3/93; Urinary Tract 1/64. As LP283 is expressed in cardiovascular system (specifically in heart) and respiratory system (specifically in lung), LP283 compositions are useful in the treatment, for example, of defects in or wounds to organs including, but not limited to, the heart and lung. Sequence encoding LP283 has been localized to human chromosome region 6p21.1-21.33. Moreover, the following diseases, conditions, syndromes, disorders, or pathological states have also been mapped to this region of the human chromosome: psoriasis (Balendran, et al. 1999 J Invest Dermatol 113(3):322-328), autosomal recessive polycystic kidney disease (Besbas, et al., 1998 Turk J Pediatr 40(2):245-7, and Mucher, et al., 1998 Genomics 48(1):40-45), autosomal recessive retinitispigmentosa (Banerjee, et al., 1998 Genomics 48(2):171-177), Hamartoma of the breast (Dal Cin, et al., 1997 Genes Chromosomes Cancer (1):90-92), juvenile myoclonic epilepsy (Liu, et al., 1995 Am J Hum Genet 57(2):368-381), and hypotrichosis simplex of the scalp (Betz, et al., 2000 Am J Hum Genet 66(6):1979-1983). Accordingly, an isolated and/or recombinant DNA molecule of LP283 meets the statutory utility requirement of 35 U.S.C. 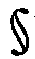 101 since it can be used to hybridize near one or more of the above stated diseases and thus serve as a marker for a such a disease gene. Additionally, compositions comprising LP283 polypeptides or polynucleotides (fragments thereof), LP283 agonists or antagonists, and/or binding compositions (e.g., LP283 antibodies) will also be useful for diagnosis, prognosis, treatment, amelioration, and/or intervention of a disease, condition, or state associated with such an above referenced disease.
Table 5: Primate, e.g., human, LP283 polynucleotide sequence (SEQ ID NO: 11). The ORF for LP283 is 157-2850 bp (with the start (ATG) and stop codons (TAG) identified in bold lettering in case numbering is misidentified one skilled in the art could determine the open reading frame). The two underlined AG's in 5' UTR may be splicing sites.

LP283 DNA sequence (3287bp) (ORF = 157-2850):
ATGGGCTCGGGGCGCGTACCCGGGCTCTGCCTGCTTGTCCTGCTGGTCCACGCCCGCGCCGCCCAGTACAG
CAAAGCCGCGCAAGATGTGGATGAGTGTGTGGAGGGGACTGACAACTGCCACATCGATGCTATCTGCCAGA
ACACCCCGAGGTCATACAAGTGCATCTGCAAGTCTGGCTACACAGGGGACGGCAAACACTGCAAAGACGTG
GATGAGTGCGAGCGAGAGGATAATGCAGGTTGTGTGCATGACTGTGTCAACATCCCTGGCAATTACCGGTG
TACCTGCTATGATGGATTCCACCTGGCACATGACGGACACAACTGTCTGGATGTGGACGAGTGTGCCGAGG
GCAACGGCGGCTGTCAGCAGAGCTGTGTCAACATGATGGGCAGCTATGAGTGCCACTGCCGGGAAGGCTTC
TTCCTCAGCGACAACCAGCATACCTGTATCCAGCGGCCAGAAGAAGGAATGAATTGCATGAACAAGAACCA
CGGCTGTGCCCACATTTGCCGGGAGACACCCAAGGGGGGTATTGCCTGTGAATGCCGTCCTGGCTTTGAGC
TTACCAAGAACCAACGGGACTGTAAATGTGAGATAATTGGGATGGCAGTGACATGCAACTATGGTAACGGC
GGCTGCCAGCACACGTGTGATGACACAGAGCAGGGTCCCCGGTGCGGCTGCCATATCAAGTTTGTGCTCCA
TACCGACGGGAAGACATGCATCGAGACCTGTGCTGTCAACAACGGGGGCTGTGACAGTAAGTGCCATGATG
CAGCGACTGGTGTCCACTGCACCTGCCCTGTGGGCTTCATGCTGCAGCCAGACAGGAAGACGTGCAAAGAT
ATAGATGAGTGCCGCTTAAACAACGGGGGCTGTGACCATATTTGCCGCAACACAGTGGGCAGCTTCGAATG
CAGTTGCAAGAAAGGCTATAAGCTTCTCATCAATGAGAGGAACTGCCAGGATATAGACGAGTGTTCCTTTG
ATCGAACCTGTGACCACATATGTGTCAACACACCAGGAAGCTTCCAGTGTCTCTGCCATCGTGGCTACCTG
TTGTATGGTATCACCCACTGTGGGGATGTGGATGAATGCAGCATCAACCGGGGAGGTTGCCGCTTTGGCTG
CATCAACACTCCTGGCAGCTACCAGTGTACCTGCCCAGCAGGCCAGGGTCGGCTGCACTGGAATGGCAAAG
ATTGCACAGAGCCACTGAAGTGTCAGGGCAGTCCTGGGGCCTCGAAAGCCATGCTCAGCTGCAACCGGTCT
GGCAAGAAGGACACCTGTGCCCTGACCTGTCCCTCCAGGGCCCGATTTTTGCCAGAGTCTGAGAATGGCTT
CACGGTGAGCTGTGGGACCCCCAGCCCCAGGGCTGCTCCAGCCCGAGCTGGCCACAATGGAACAGCACCA
ACTCCAACCACTGCCATGAGGCTGCAGTGCTGTCCATTAAACAACGGGCCTCCTTCAAGATCAAGGATGCC
AAATGCCGTTTGCACCTGCGAAACAAAGGCAAAACAGAGGAGGCTGGCAGAATCACAGGGCCAGGTGGTGC
CCCCTGCTCTGAATGCCAGGTCACCTTCATCCACCTTAAGTGTGACTCCTCTCGGAAGGGCAAGGGCCGAC
GGGCCCGGACCCCTCCAGGCAAAGAGGTCACAAGGCTCACCCTGGAACTGGAGGCAGAGCAACTCTTTCTC
CTCCCTGATACACACGGCCATCCACCACCAGCCAGCTGTGGGCTGCCCTGCCTCCGACAGCGAATGGAACG
GCGGCTGAAAGGATCCCTGAAGATGCTCAGAAAGTCCATCAACCAGGACCGCTTCCTGCTGCGCCTGGCAG
GCCTTGATTATGAGCTGGCCCACAAGCCGGGCCTGGTAGCCGGGGAGCGAGCAGAGCCGATGGAGTCCTGT
AGGCCCGGGCAGCACCGTGCTGGGACCAAGTGTGTCCAGTGCTCCCCAGGGCACTACTACAACACCAGCAT
CCACCGCTGTATTCGCTGTGCCATGGGCTCCTATCAGCCCGACTTCCGTCAGAACTTCTGCAGCCGCTGTC
CAGGAAACACAAGCACAGACTTTGATGGCTCTACCAGTGTGGCCCAATGCAAGAATCGTCAGTGTGGTGGG
GAGCTGGGTGAGTTCACTGGCTATATTGAGTCCCCCAACTACCCGGGCAACTACCCAGCTGGTGTGGAGTG
CATCTGGAACATCAACCCCCCACCCAAGCGCAAGATCCTTATCGTGGTACCAGAGATCTTCCTGCCATCTG
AGGATGAGTGTGGGGACGTCCTCGTCATGAGAAAGAACTCATCCCCATCCTCCATTACCACTTATGAGACC
TGCCAGACCTACGAGCGTCCCATTGCCTTCACTGCCCGTTCCAGGAAGCTCTGGATCAACTTCAAGACAAG
CGAGGCCAACAGCGCCCGTGGCTTCCAGATTCCCTATGTTACCTATGATGAGGACTATGAGCAGCTGGTAG
AAGACATTGTGCGAGATGGCCGGCTCTATGCCTCTGAAAACCACCAGGAGATTTTAAAGGACAAGAAGCTC
ATCAAGGCCTTCTTTGAGGTGCTAGCCCACCCCCAGAACTACTTCAAGTACACAGAGAAACACAAGGAGAT
GCTGCCAAAATCCTTCATCAAGCTGCTCCGCTCCAAAGTTTCCAGCTTCCTGAGGCCCTACAAATAG

LP283 Full-Length sequence (897aa):
>g6065887GS+1_ORF1. The underlined portion is a predicted signal
sequence (Met-1 to Ala-20)

MGSGRVPGLCLLVLLVHARAAQYSKAAQDVDECVEGTDNCHIDAICQNTPRSYKCICKSGYTGDGKHCKDV
DECEREDNAGCVHDCVNIPGNYRCTCYDGFHLAHDGHNCLDVDECAEGNGGCQQSCVNMMGSYECHCREGF
FLSDNQHTCIQRPEEGMNCMNKNHGCAHICRETPKGGIACECRPGFELTKNQRDCKCEIIGMAVTCNYGNG
GCQHTCDDTEQGPRCGCHIKFVLHTDGKTCIETCAVNNGGCDSKCHDAATGVHCTCPVGFMLQPDRKTCKD
IDECRLNNGGCDHICRNTVGSFECSCKKGYKLLINERNCQDIDECSFDRTCDHICVNTPGSFQCLCHRGYL
LYGITHCGDVDECSINRGGCRFGCINTPGSYQCTCPAGQGRLHWNGKDCTEPLKCQGSPGASKAMLSCNRS
GKKDTCALTCPSRARFLPESENGFTVSCGTPSPRAAPARAGHNGNSTNSNHCHEAAVLSIKQRASFKIKDA

KCRLHLRNKGKTEEAGRITGPGGAPCSECQVTFIHLKCDSSRKGKGRRARTPPGKEVTRLTLELEAEQLFL
LPDTHGHPPPASCGLPCLRQRMERRLKGSLKMLRKSINQDRFLLRLAGLDYELAHKPGLVAGERAEPMESC
RPGQHRAGTKCVQCSPGHYYNTSIHRCIRCAMGSYQPDFRQNFCSRCPGNTSTDFDGSTSVAQCKNRQCGG
ELGEFTGYIESPNYPGNYPAGVECIWNINPPPKRKILIVVPEIFLPSEDECGDVLVMRKNSSPSSITTYET
CQTYERPIAFTARSRKLWINFKTSEANSARGFQIPYVTYDEDYEQLVEDIVRDGRLYASENHQEILKDKKL
IKAFFEVLAHPQNYFKYTEKHKEMLPKSFIKLLRSKVSSFLRPYK*

LP283 Mature sequence (877aa):

A predicted mature LP283 sequence is as follows:

AQYSKAAQDVDECVEGTDNCHIDAICQNTPRSYKCICKSGYTGDGKHCKDVDECEREDNAGCVHDCVNIPG
NYRCTCYDGFHLAHDGHNCLDVDECAEGNGGCQQSCVNMMGSYECHCREGFFLSDNQHTCIQRPEEGMNCM
NKNHGCAHICRETPKGGIACECRPGFELTKNQRDCKCEIIGMAVTCNYGNGGCQHTCDDTEQGPRCGCHIK
FVLHTDGKTCIETCAVNNGGCDSKCHDAATGVHCTCPVGFMLQPDRKTCKDIDECRLNNGGCDHICRNTVG
SFECSCKKGYKLLINERNCQDIDECSFDRTCDHICVNTPGSFQCLCHRGYLLYGITHCGDVDECSINRGGC
RFGCINTPGSYQCTCPAGQGRLHWNGKDCTEPLKCQGSPGASKAMLSCNRSGKKDTCALTCPSRARFLPES
ENGFTVSCGTPSPRAAPARAGHNGNSTNSNHCHEAAVLSIKQRASFKIKDAKCRLHLRNKGKTEEAGRITG
PGGAPCSECQVTFIHLKCDSSRKGKGRRARTPPGKEVTRLTLELEAEQLFLLPDTHGHPPPASCGLPCLRQ
RMERRLKGSLKMLRKSINQDRFLLRLAGLDYELAHKPGLVAGERAEPMESCRPGQHRAGTKCVQCSPGHYY
NTSIHRCIRCAMGSYQPDFRQNFCSRCPGNTSTDFDGSTSVAQCKNRQCGGELGEFTGYIESPNYPGNYPA
GVECIWNINPPPKRKILIVVPEIFLPSEDECGDVLVMRKNSSPSSITTYETCQTYERPIAFTARSRKLWIN
FKTSEANSARGFQIPYVTYDEDYEQLVEDIVRDGRLYASENHQEILKDKKLIKAFFEVLAHPQNYFKYTEK
HKEMLPKSFIKLLRSKVSSFLRPYK*

Particularly interesting portions or fragments of the full length LP283 polypeptide (SEQ ID NO: 12) include, e.g., a predicted signal sequence from Met-1 to Ala-20; a predicted transmembrane-like region from Gly-4 to Ala-21; also particularly interesting are LP283 fragments Ser-24 to Asp-38; Asp-38 to Arg-51; Ser-52 to Asp-64; His-67 to Asp-78; Ala-80 to Tyr-93; Arg-139 to lys-164; Gly-167 to Asp-182; Cys-228 to Cys-247; His-297 to Asn-322; Phe-331 to Pro-383; Gly-401 to Lys-428; Phe-442 to Ser-458; His-468 to Asn-505; Aly-507 to Lys-542; Gln-565 to Leu-583; Leu-586 to Ser-597; Met-600 to Leu-611; Glu-637 to Val-651; Ser-654 to Tyr-674; Asp-677 to Asp-693; Ser-721 to Gly-762; Asp-763 to Glu-780; Ser-795 to Tyr-817; Val-832 to His-843; and Ile-846 to Tyr-869, whose discovery was based on an analysis of hydrophobicity and hydrophilicity plots. Additional interesting sections of LP283 are the portions of the polypeptide from Met-1 to Lys-11; Lys-11 to Val-30; Asp-31 to Cys-40; Val-34 to His-41; Ile-42 to Pro-50; Gln-47 to Tyr-53; Tyr-53 to Gly-63; Lys-58 to Val-71; Asp-64 to Asp-72; Cys-86 to Asp-99; Pro-90 to Asp-99; Gly-100 to Lys-111; Glu-115 to Glu-135; Cys-116 to Gln-125; Met-131 to Glu-140; Ser-145 to Ile-152; His-149 to Asn-165; Pro-155 to Cys-168; His-170 to Tyr-191; Arg-173 to Gly-179; Pro-186 to Cys-199; Lys-198 to His-217; Asn-209 to Gly-229; Asp-221 to Ile-232; His-237 to Cys-243; Lys-241 to Asp-255; Asn-251 to Asp-260; His-266 to Pro-277; Gln-276 to Cys-295; Asp-284 to Lys-311; Cys-308 to Tyr-314; Leu-316 to Arg-333; Asn-319 to Glu-328; Ser-330 to Asp-336; Asp-336 to Cys-348; Val-365 to Gln-394; Gly-363 to Cys-375; Thr-382 to Gln-387; Pro-391 to Glu-406; Cys-423 to Thr-431; Pro-437 to Gly-449; Pro-457 to Pro-463; Lys-443 to Ser-458; His-468 to His-477; Phe-492 to Gly-507; Leu-503 to Gly-519; Ala-512 to Phe-530; Lys-534 to Lys-552; Asp-571 to Ser-580; Met-636 to Thr-648; Lys-627 to Pro-641; Ser-654 to His-664; Ala-670 to Arg-685; Arg-685 to Ser-697; Asn-660 to Asp-677; Phe-678 to Val-700; Gln-702 to Glu-711; Asn-705 to Gly-726; Ser-721 to Gly-731; Trp-736 to Lys-745; Tyr-728 to Pro-741; Lys-745 to Leu-765; Met-767 to Ile-776; Glu-780 to Ala-790; Pro-773 to Thr-792; Thr-792 to Asn-801; Lys-803 to Gln-814; Ser-809 to Thr-819; Asp-830 to Ile-846; and Leu-860 to Leu-877. These fragments were discovered based on analysis of antigenicity plots. Additionally interesting portions of LP283 are the segments identified based on PROSITE analysis as potential aspartic acid and asparagine hydroxylation sites: Cys-46 to Cys-57; Cys-86 to Cys-97; Cys-127 to Cys-138; Cys-299 to Cys-310; Cys-339 to Cys-350; and Cys-379 to Cys-390. Additionally interesting portions of LP283 are the segments identified based on PROSITE analysis as calcium-binding, EGF-like domains: Asp-29 to Cys-55; ASp-70 to Cys-95; Asp-112 to Cys-136; Asp-284 to Cys-308; Asp-325 to Cys-348; and Asp-364 to Cys-388. Additionally interesting portions of LP283 are the segments identified based on ExPASy analysis as EGF-like domains: Cys-33 to Cys-68; Cys-74 to Cys-110; Cys-116 to Cys-151; Cys-161 to Cys-197; Cys-208 to Cys-243; Cys-247 to Cys-282; Cys-288 to Cys-323; Cys-329 to Cys-362; and Cys-368 to Cys-404. A common feature of EGF-like domains are that they are typically found in the extracellular domain of membrane-bound or secreted proteins. The EGF domain includes six cysteine residues which have been shown (in EGF) to be involved in disulfide bonds. The main structure is a two-stranded beta-sheet followed by a loop to a C-terminal short two-stranded sheet. Subdomains between the conserved cysteines strongly vary in length. An additionally interesting portion of LP283 is a segment identified based on ExPASy analysis as an EB-like domain: Gly-91 to Cys-110. An additionally interesting portion of LP283 is a segment identified based on ExPASy analysis as a Keratin B2-like domain: Cys-95 to Cys-247. An additionally interesting portion of LP283 is a segment identified based on ExPASy analysis as a CUB-like domain: Cys-708 to Tyr-817. An additionally interesting portion of LP283 is a segment identified based on ExPASy analysis as a TIL-like domain: Cys-219 to Cys-288.

Further, particularly interesting LP 283 secondary structures (e.g., such as a helix, a strand, or a coil) are the following LP283 coil structures: Met-1 to Pro-6; Glu-35 to Cys-40; Asn-48 to Ser-52; Ser 59 to Asp-70; Arg-76 to Cys-82; Asn 88 to Asn 92; Tyr-98 to Phe-101; His-105 to Cys-110; Glu-118 to Cys-123; Met-131 to Tyr-134; Cys-138 to Gly-141; Ser-145 to His-149; Gln-153 to Asn-160; Asn-163 to Gly-167; Arg-173 to Gly-179; Cys-184 to Phe-188; Thr-191 to Lys-198; Tyr-210 to Cys-215; Cys-219 to Gly-229; Thr-238 to Lys-241; Asn-250 to Asp-255; His-259 to Bly-264; Cys-269 to Val-271; Gln-276 to Asp-286; Asn-291 to Asp-296; Asn-301 to Ser-305; Cys-310 to Tyr-314; Glu-320 to Asp-336; Thr-342 to Ser-345; His-351 to Tyr-354; Thr-360 to Val-365; Ile-370 to Cys-375; Asn-381 to Ser-385; Cys-390 to Gln-394; Asn-400 to Pro-407; Gln-411 to Ser-417; Asn-424 to Asp-430; Thr-435 to Gly-449; Cys-454 to Asn-466; Asn-505 to Thr-509; Thr-516 to Glu-525; Asp-536 to Gly-543;Thr-548 to Lys-552; Pro-570 to Gly-582; Gln-607; Gly-616 to Gly-626; Glu-631 to Thr-648; Ser-653 to Ile-663; Gly-672 to Arg-679; Arg-685 to Thr-698; Asn-705 to Glu-714; Ser-721 to Gly-731; Ile-738 to Lys-743; Phe-754 to Gly-762; Asn-770 to Ser-775; Cys-782 to Pro-788; Thr-804 to Gly-812; Asp-834 to Arg-836; Ser-840 to Glu-841; Ala-861 to Gln-864; and Pro-895 to Gly-897. Particularly interesting helix structures are His-17 to Ala-27; Glu-560 to Gln-565; Arg-587 to Leu-594; Leu-598 to Ser-604; Tyr-824 to Arg-833; Gln-844 to Phe-857; and Ser-871 to Leu-876. Particularly interesting strands are Lys-54- Cys-57; Cys-95 to Cys-97; Leu-111 to Val-113; Ile-180 to Cys-182; Ile-201 to Cys-208; His-231 to Leu-236; Cys-243 to Glu-245; His-266 to Cys-267; Cys-288 to Arg-289; His-297 to Cys-299; Leu-316 to Ile-318; His-337 to Val-340; Gln-347 to Leu-349; Leu-355 to Tyr-357; Tyr-386 to Cys-388; Ala-419 to Leu-421; Thr-451 to Ser-453; Gln-527 to Leu 533; Lys-649 to Gln-652; Val-700 to Ala-701; Tyr-718 to Ile-719; Glu-733 to Trp-736; Lys-745 to Val-749; Val-764 to Arg-768; Phe-791; and Ile-815 to Val-818.. Further encompassed by the invention are contiguous amino acid residue combinations of any of the predicted secondary structures described above. For example, one strand-coil-helix motif of LP283 combines the Ile-815 to Val-818 strand; with the Tyr-820 to Asp-821 coil; and the Tyr-824 to Arg-833 helix to form an interesting fragment of contiguous amino acid residues from Ile-815 to Arg-833. Other combinations of contiguous amino acids are contemplated as can be easily determined.

### FEATURES OF LP NO: 7 (LP286)

LP286 is a member of a family of alternatively spliced LP's which include LP188 and LP284 (LP188 has been previously disclosed in USSN 60/255850 filed on 15-DEC-2000). It has been discovered that LP286 sequences (SEQ ID NO: 13) are expressed primarily in the following number of LIFESEQ GOLD™ database tissue and cDNA libraries: Cardiovascular System 2/68; Connective Tissue 1 /47; Digestive System (small intestine, colon, and stomach) 16/148; Exocrine Glands 2/64; Genitalia, Female 3/106; Genitalia, Male 9/114; Hemic and Immune System 3/159; Musculoskeletal System 2/47; Nervous System (brain and spinal cord) 26/198; Pancreas 1/24; Respiratory System 3/93; and Sense Organs (eye) 1/8. As LP286 is abundantly expressed in nervous system (specifically in brain and spinal cord) and digestive system (specifically in small intestine, colon and stomach), LP286 compositions are useful in the treatment, for example, of defects in or wounds to organs including, but not limited to, brain, spinal cord, small intestine, colon and stomach. According to its expression in the nervous system, compositions comprising LP286 polypeptides or polynucleotides (including fragments thereof), and/or antibodies are useful for diagnosis, treatment and intervention of diseases, disorders, and/or conditions including, but not limited to, cancers, Alzheimer's Disease, Parkinson's Disease, Huntington's Disease, Tourette Syndrome, meningitis, encephalitis, demyelinating diseases, peripheral neuropathies, neoplasias, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses, autism, and altered behaviors, including, for example, disorders in feeding, sleep patterns, balance, and perception.

Sequence encoding LP286 polypeptide has been localized to chromosome 19. Moreover, the following diseases, conditions, syndromes, disorders, or pathological states have also been mapped to this region of the human chromosome: glioma (Smith, et al., 2000 Genes Chromosomes Cancer 2000 (1):16-25), asthma, heart defects, and eye development (Hamshere, et al., 2000 Genomics 2000 63(3):425-9), alcoholism (Valdes et al., 1999 Genet Epidemiol Suppl 1:S367-72), and Wilms' tumor (McDonald, et al., 1998 Cancer Res. 58(7):1387-90). Accordingly, an isolated and/or recombinant DNA molecule of LP286 meets the statutory utility requirement of 35 U.S.C. 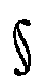 101 since it can be used to hybridize near one or more of the above stated diseases and thus serve as a marker for a such a disease gene. Additionally, compositions comprising LP286 polypeptides or polynucleotides (fragments thereof), LP286 agonists or antagonists, and/or binding compositions (e.g., LP286 antibodies) will also be useful for diagnosis, prognosis, treatment, amelioration, and/or intervention of a disease, condition, or state associated with such an above referenced disease. Additionally, LP286 polypeptide exhibits sequence similarity to LGI1 (Leucine-rich gene-Glioma Inactivated Protein) g9939002, g4826816. The rearrangement of the LGI1 gene has been detected in the A172 glioblastoma cell line and several glioblastoma tumors. These rearrangements lead to a complete absence of LGI1 expression in glioblastoma cells (Chernova, et al., 1998 Oncogene 17(22):2873-81). Consequently, it has been suggested that LGI1 is a candidate tumor suppressor gene involved in the progression of glial tumors. Similarly, it is suggested here that compositions comprising LP286 (e.g., polypeptides, polynucleotides, and/or binding agents) are useful for diagnosis, prognosis, treatment, amelioration, and/or intervention of a cancer, such as, for example, especially a glioblastoma.
Table 6: Primate, e.g., human, LP286 polynucleotide sequence (SEQ ID NO: 13). The ORF for LP286 is 532-2073 bp (with the start (ATG) and stop codons (TAG) identified in bold lettering in case numbering is misidentified one skilled in the art could determine the open reading frame).

LP286 DNA sequence (2610bp) (ORF = 532-2073):
ATGGGAGGGGCAGGCATTCTGCTGCTGCTGCTGGCTGGGGCGGGGGTGGTGGTGGCCTGGAGACCCCCAAA
GGGAAAGTGTCCCCTGCGCTGCTCCTGCTCTAAAGACAGCGCCCTGTGTGAGGGCTCCCCGGACCTGCCCG
TCAGCTTCTCTCCGACCCTGCTGTCACTCTCACTCGTCAGGACGGAGTCACCCAGCTGAAGGCCGGCAGC
TTCCTGAGAATTCCGTCTCTGCACCTGCTCTTCATCGAGGACAATGAGATTGGCTCCATCTCTAAGAATGC
CCTCAGAGGACTTCGCTCGCTTACACACCTAAGCCTGGCCAATAACCATCTGGAGACCCTCCCCAGATTCC
TGTTCCGAGGCCTGGACACCCTTACTCACGTGGACCTCCGCGGGAACCCGTTCCAGTGTGACTGCCGCGTC
CTCTGGCTCCTGCAGTGGATGCCCACCGTGAATGCCAGCGTGGGGACCGGCGCCTGTGCGGGCCCCGCCTC
CCTGAGCCACATGCAGCTCCACCACCTCGACCCCAAGACTTTCAAGTGCAGAGCCATAGAGCTGTCCTGGT
TCCAGACGGTGGGGGAGTCGGCACTGAGCGTAGAGCCCTTCTCCTACCAAGGGGAGCCTCACATTGTGCTG
GCACAGCCCTTCGCCGGCCGCTGCCTGATTCTCTCCTGGGACTACAGCCTGCAGCGCTTCCGGCCCGAGGA
AGAGCTGCCCGCGGCCTCCGTGGTGTCCTGCAAGCCACTGGTGCTGGGCCCGAGCCTCTTCGTGCTGGCTG
CCCGCCTGTGGGGGGGCTCACAGCTGTGGGCCCGGCCCAGTCCCGGCCTGCGCCTGGCCCCAACGCAGACC
CTGGCCCCGCGGCGGCTGCTGCGGCCCAATGACGCCGAGCTCCTGTGGCTGGAAGGGCAACCCTGCTTCGT
GGTGGCCGATGCCTCCAAGGCGGGCAGCACCACGCTGCTGTGCCGCGACGGGCCCGGCTTTTACCCGCACC
AGAGCCTGCACGCCTGGCACCGGGACACGGACGCTGAGGCCCTGGAGCTGGACGGCCGGCCCCACCTGCTG
CTGGCCTCGGCTTCCCAGCGGCCCGTGCTCTTCCACTGGACCGGTGGCCGCTTCGAGAGACGCACAGACAT
CCCCGAGGCCGAGGATGTCTATGCCACACGCCACTTCCAGGCTGGTGGGGACGTGTTCCTGTGCCTCACAC
GCTACATTGGGGACTCCATGGTCATGCGCTGGGACGGCTCCATGTTTCGTCTGCTGCAGCAACTTCCCTCG
CGCGGTGCCCACGTCTTCCAGCCACTGCTCATCGCCAGGGACCAGCTGGCCATCCTAGGCAGCGACTTCGC
CTTCAGCCAGGTCCTCCGCCTTGAGCCTGACAAGGGGCTCCTGGAGCCACTGCAGGAGCTGGGGCCTCCGG
CCCTGGTGGCCCCCCGTGCCTTTGCCCACATCACTATGGCCGGCAGACGCTTCCTCTTTGCTGCTTGCTTT
AAGGGCCCCACACAGATCTACCAGCATCACGAGATCGACCTCAGTGCCTGA

LP286 Full-Length Sequence (513aa):
>ds37732+1_ORF2. (SEQ ID NO: 14) The underlined portion is a predicted signal sequence (Met-1 to Ala-
19). A predicted SP cleavage site is between Ala-19 and Trp-20 indicated as follows: 1
MGGAGILLLLLAGAGVVVA^WR 21

MGGAGILLLLLAGAGVVVAWRPPKGKCPLRCSCSKDSALCEGSPDLPVSFSPTLLSLSLVRTGVTQLKAGSF
LRIPSLHLLFIEDNEIGSISKNALRGLRSLTHLSLANNHLETLPRFLFRGLDTLTHVDLRGNPFQCDCRVLW
LLQWMPTVNASVGTGACAGPASLSHMQLHHLDPKTFKCRAIELSWFQTVGESALSVEPFSYQGEPHIVLAQP
FAGRCLILSWDYSLQRFRPEEELPAASVVSCKPLVLGPSLFVLAARLWGGSQLWARPSPGLRLAPTQTLAPR
RLLRPNDAELLWLEGQPCFVVADASKAGSTTLLCRDGPGFYPHQSLHAWHRDTDAEALELDGRPHLLLASAS
QRPVLFHWTGGRFERRTDIPEAEDVYATRHFQAGGDVFLCLTRYIGDSMVMRWDGSMFRLLQQLPSRGAHVF
QPLLIARDQLAILGSDFAFSQVLRLEPDKGLLEPLQELGPPALVAPRAFAHITMAGRRFLFAACFKGPTQIY
QHHEIDLSA*

A Mature LP286 Sequence (494aa):
An exon of LP286 is absent from the splice form of LP188. Blast genomic DNA analysis of LP188 demonstrates that the extra sequence in LP188 is a distinct exon. A predicted mature LP286 is as follows:

WRPPKGKCPLRCSCSKDSALCEGSPDLPVSFSPTLLSLSLVRTGVTQLKAGSFLRIPSLHLLFIEDNEIGS
ISKNALRGLRSLTHLSLANNHLETLPRFLFRGLDTLTHVDLRGNPFQCDCRVLWLLQWMPTVNASVGTGAC
AGPASLSHMQLHHLDPKTFKCRAIELSWFQTVGESALSVEPFSYQGEPHIVLAQPFAGRCLILSWDYSLQR
FRPEEELPAASVVSCKPLVLGPSLFVLAARLWGGSQLWARPSPGLRLAPTQTLAPRRLLRPNDAELLWLEG
QPCFVVADASKAGSTTLLCRDGPGFYPHQSLHAWHRDTDAEALELDGRPHLLLASASQRPVLFHWTGGRFE

RRTDIPEAEDVYATRHFQAGGDVFLCLTRYIGDSMVMRWDGSMFRLLQQLPSRGAHVFQPLLIARDQLAIL
GSDFAFSQVLRLEPDKGLLEPLQELGPPALVAPRAFAHITMAGRRFLFAACFKGPTQIYQHHEIDLSA*

Particularly interesting portions or fragments of the full length LP286 polypeptide (SEQ ID NO: 14) include, e.g., a predicted signal sequence from Met-1 to Ala-19; another analysis yields a predicted signal sequence from Met-1 to Cys-40. Additionally interesting segments of LP286 are two transmembrane-like regions: Ala-4 to Pro-23, and Ser-246 to Gly-266; also particularly interesting are LP286 fragments Arg-30 to Ser-49; Phe-50 to Val-60; Arg-61 to Gly-70; Ser-71 to Ile-83; Glu-84 to Ser-101; Asp-124 to Cys-140; Arg-141 to Pro-150; Thr-151 to His-169; His-174 to Ala-184; Sln-191 to His-210; Tyr-228 to Lys-248; Pro-249 to Trp-264; Ala-310 to Leu-320; Cys-322 to Leu-334; Ala-336 to Pro-352; Gly-370 to His-390; Phe-497 to His-507; Lys-26 to Gly-42; Pro-47 to Asp-85; Leu-107 to Leu-119; Phe-136 to Thr-151; Ala-160 to Met-170; Lys-181 to Leu-198; Ser-199 to His-210; Asp-227 to Pro-249; Leu-252 to Trp-270; Leu-285 to Asp-295; Ala-310 to Leu-321; Ser-333 to Thr-341; Leu-346 to Glu-374; Arg-389 to Cys-400; Gly-415 to Gly-428; Ala-438 to Ser-447; Phe-449 to Glu-465; and Pro-466 to Ala-480, whose discovery was based on an analysis of hydrophobicity and hydrophilicity plots. Additional interesting sections of LP286 are the portions of the polypeptide from Gly-42 to Phe-50; Pro-76 to Ala-95; Gly-98 to Leu-115; Gly-122 to Leu131; Arg-132 to Arg-141; Val-142 to Ala-165; Leu-167 to Lys-181; Arg-183 to Ala-197; Leu-198 to Gln-215; Phe-217 to Pro-235; Ala-280 to Ala-296; Glu-297 to Val-308; Thr-319 to Phe-328; Glu-332 to Leu-346; Glu-347 to Pro-363; Val-364 to Glu-383; Gly-394 to Gln-423; Ala-482 to Pro-500; Arg-21 to Leu-29; Ser-32 to Pro-44; Val-129 to Val-142; Leu-172 to Lys-181; Pro-202 to Pro-209; Trp-226 to Glu-238; Gly-266 to Leu-277; Pro-287 to Leu-298; Ala-312 to Thr-318; Cys-322 to Asp-342; Thr-369 to Glu-381; Gln-422 to Val-431; Leu-455 to Leu-463; Arg-21 to Cys-31; Arg-30 to Glu-41; Ser-37 to Leu-46; Ala-271 to Pro-281; Leu-285 to Ala-296; Trp-337 to Arg-351; Gly-371 to Tyr-386; Leu-424 to Ala-429; and Leu-455 to Leu-464. These fragments were discovered based on analysis of antigenicity plots. An additionally interesting portion of LP286 is a segment discovered based on a PROSITE analysis as membrane lipoprotein lipid attachment-like site: Thr-151 to Cys-161. In prokaryotes, such membrane lipoprotein sites are synthesized with a precursor signal peptide that is cleaved by a specific lipoprotein signal peptidase (signal peptidase II). The peptidase recognizes a conserved sequence and cuts upstream of a cysteine residue to which a glyceride-fatty acid lipid is attached resulting in a post-translational modification (Hayashi & Wu (1990) J. Bioenerg. Biomembr 22:451-471). Additionally interesting portions of LP286 are segments discovered based on a PROSITE analysis as Leucine Rich Repeat-like domains (LRR): Thr-53 to Pro-76; Ser-77 to Arg-100; Ser-101 to Asp-124; and Thr-125 to Gln-147. LRRs are short sequence motifs present in a number of proteins with diverse functions and cellular locations. LRRs are usually involved in protein-protein interactions. Each Leucine Rich Repeat is composed of a beta-alpha unit, which typically form elongated non-globular structures. Leucine Rich Repeats are often flanked by cysteine rich domains (Kobe & Deisenhofer 1994 Trends Biochem Sci 19:415-421). An additionally interesting portion of LP286 is a segment discovered based on a PROSITE analysis as a Leucine Rich Repeat C-terminal-like domain (LRRCT): Asn-134 to Arg-183. The LRRCT domain is often found at the C-terminus of tandem leucine rich repeats (Kobe & Deisenhofer 1994 Trends Biochem Sci 19:415-421). Further, particularly interesting LP286 secondary structures (e.g., such as a helix, a strand, or a coil) are the following LP286 coil structures: Met-1 to Gly-3;Gly-13 to Gly-15; Pro-22 to Pro-28; Ser-32 to Ser-37; Glu-41 to Val-48; Ser-51 to Pro-52; Lue-67 to Ser-71; Pro-76 to Leu-78; Glu-84 to Ile-88; Asn-109; Gly-122 to Thr-125; Leu-131 to Cys-138; Trp-148 to Pro-164; Leu-175 to Thr-179; Thr-192 to Ala-197; Glu-201 to Pro-209; Pro-216 to Arg-220; Phe-233 to Ala-242; Cys-247 to Pro-254; Trp-264 to Gln-268; Ala-271 to Leu-277; Ala-280 to Thr-282; Arg-292 to Asp-295; Glu-302 to Pro-305; Ala-312 to Ser-317; Arg-323 to Ser-333; Asp-349 to Pro-352; Ser-360 to Arg-362; Thr-369 to Pro-380; Gln-392 to Asp-396; Ile-405 to Ser-408; Asp-414 to Ser-416; Leu-424 to Val-431; Gly-446 to Asp-448; Glu-458 to Lys-461; Leu-470 to Val-476; Cys-496 to Thr-501; and His-506 to Ala-513. Particularly interesting strands are Val-17 to Trp-20; Leu-81 to Ile-83; Thr-127 to Thr-128; Cys-221 to Leu-224; Phe-307 to Ala-310; Thr-318 to Cys-322; Val-364 to His-367; Val-397 to Leu-401; and Val-410 to Arg-412. Particularly interesting helix structures are Ala-95 to Thr-103; Leu-115 to Phe-120; Ala-260 to Leu-263; Ala-343 to Glu-347; Glu-383 to Ala-387; Met-417 to Gln-422; Pro-434 to Leu-435; Ala-450 to Arg-456 and Arg-479 to Ala-482. Further encompassed by the invention are contiguous amino acid residue combinations of any of the predicted secondary structures described above. For example, one coil-strand-coil motif of LP286 combines the Ala-312 to Ser-317 coil; with the Thr-318 to Cys-322 strand; and the Arg-323 to Ser-333 coil to form an interesting fragment of contiguous amino acid residues from Ala-312 to Ser-333. Other combinations of contiguous amino acids are contemplated as can be easily determined.

### FEATURES OF LP NO: 8 (LP284)

LP284, which was cloned from a neuroganglion tumor, is a member of a family of alternatively spliced LP's that include LP188 and LP284 (LP188 has been previously disclosed in USSN 60/255850 filed on 15-DEC-2000). It has been discovered that LP284 sequences (SEQ ID NO: 15) are expressed primarily in the following number of LIFESEQ GOLD™ database tissue and cDNA libraries: Genitalia, Male (seminal vesicle and testis) 2/114; Hemic and Immune System 1/159; Musculoskeletal System (skeletal muscle) 1/47; and Nervous System (brain and ganglion) 16/198. As LP284 is abundantly expressed in the nervous system (specifically in brain and ganglion), the musculoskeletal system (specifically in skeletal muscle) and the male reproductive system (specifically in seminal vesicle and testis) LP284 compositions are useful in the treatment, for example, of defects in or wounds to organs including, but not limited to, nerve, muscle, and organs including, but not limited to brain, ganglion, skeletal muscle, seminal vesicle and testis. According to its expression in the nervous system, compositions comprising LP284 polypeptides or polynucleotides (including fragments thereof), and/or antibodies are useful for diagnosis, treatment and intervention of diseases, disorders, and/or conditions including, but not limited to, cancer, Alzheimer's Disease, Parkinson's Disease, Huntington's Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder learning disabilities, ALS, psychoses, autism, and altered behaviors, including disorders in feeding, sleep patterns, balance, and perception. Sequence encoding LP284 polypeptide has been localized to chromosome 19. Moreover, the following diseases, conditions, syndromes, disorders, or pathological states have also been mapped to this region of the human chromosome: glioma (Smith, et al., 2000 Genes Chromosomes Cancer 2000 (1):16-25), asthma, heart defects, and eye development (Hamshere, et al., 2000 Genomics 2000 63(3):425-9), alcoholism (Valdes et al., 1999 Genet Epidemiol Suppl 1:S367-72), and Wilms' tumor (McDonald, et al., 1998 Cancer Res. 58(7):1387-90). Accordingly, an isolated and/or recombinant DNA molecule of LP284 meets the statutory utility requirement of 35 U.S.C. 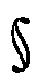 101 since it can be used to hybridize near one or more of the above stated diseases and thus serve as a marker for a such a disease gene. Additionally, compositions comprising LP284 polypeptides or polynucleotides (fragments thereof), LP284 agonists or antagonists, and/or binding compositions (e.g., LP284 antibodies) will also be useful for diagnosis, prognosis, treatment, amelioration, and/or intervention of a disease, condition, or state associated with such an above referenced disease. Additionally, LP284 polypeptide exhibits sequence similarity to LGI1 (Leucine-rich gene-Glioma Inactivated Gene) g9938002. The rearrangement of the LGI1 gene has been detected in the A172 glioblastoma cell line and several glioblastoma tumors. These rearrangements lead to a complete absence of LGI1 expression in glioblastoma cells (Chernova, et al., 1998 Oncogene 17(22):2873-81). Consequently, it has been suggested that LGI1 is a candidate tumor suppressor gene involved in the progression of glial tumors. Similarly, it is suggested here that compositions comprising LP286 (e.g., polypeptides, polynucleotides, and/or binding agents) are useful for diagnosis, prognosis, treatment, amelioration, and/or intervention of a cancer, such as, for example, especially a glioblastoma.
Table 7: Primate, e.g., human, LP284 polynucleotide sequence (SEQ ID NO: 15). The ORF for LP284 is 158-943 bp (with the start (ATG) and stop codons (TAG) identified in bold lettering in case numbering is misidentified one skilled in the art could determine the open reading frame without undue experimentation).

LP284 DNA sequence: (983bp) (ORF 158 = 943):
>ds37412 The underlined "AATAAA" is the polyadenylation signal.

ATGGGAGGGGCAGGCATTCTGCTGCTGCTGGCTGGGGCGGGGGTGGTGGCCTGGAGACCCCCAAA
GGGAAAGTGTCCCCTGCGCTGCTCCTGCTCTAAAGACAGCGCCCTGTGTGAGGGCTCCCCGGACCTGCCCG
TCAGCTTCTCTCCGACCCTGCTGTCACTCTCACTCGTCAGGACGGGAGTCACCCAGCTGAAGGCCGGCAGC
TTCCTGAGAATTCCGTCTCTGCACCTGCTCCTCTTCACCTCCAACTCCTTCTCCGTGATTGAGGACGATGC
ATTTGCGGGCCTGTCCCACCTGCAGTACCTCTTCATCGAGGACAATGAGATTGGCTCCATCTCTAAGAATG
CCCTCAGAGGACTTCGCTCGCTTACACACCTAAGCCTGGCCAATAACCATCTGGAGACCCTCCCCAGATTC
CTGTTCCGAGGCCTGGACACCCTTACTCACGTGGACCTCCGCGGGAACCCGTTCCAGTGTGACTGCCGCGT
CCTCTGGCTCCTGCAGTGGATGCCCACCGTGAATGCCAGCGTGGGGACCGGCGCCTGTGCGGGCCCCGCCT
CCCTGAGCCACATGCAGCTCCACCACCTCGACCCCAAGACTTTCAAGTGCAGAGCCATAGGTGGGGGGCTT
TCCCGATGGGGTGGGAGGCGGGAGATCTGGGGGAAAGGCTGCCAGGGCCAAGAGGCTCGTCTCACTCCCTG
CCCTGCCATTTCCCGGAGTGGGAAGACCCTGAGCAAGCAGCACTGCCTTCCTGAGCCCCAGTTTTCTCATC
TGTAA

LP284 Full-Length Sequence (261aa):
>ds37412+3_ORF1 (SEQ ID NO: 16)  The underlined portion is a predicted
signal sequence (Met-1 to Ala-19).  A predicted SP cleavage site is
between Ala-19 and Trp-20 indicated as follows: 1
MGGAGILLLLLAGAGVVVA^WR 21
MGGAGILLLLLAGAGVVVAWRPPKGKCPLRCSCSKDSALCEGSPDLPVSFSPTLLSLSLVRTGVTQLKAGS
FLRIPSLHLLLFTSNSFSVIEDDAFAGLSHLQYLFIEDNEIGSISKNALRGLRSLTHLSLANNHLETLPRF
LFRGLDTLTHVDLRGNPFQCDCRVLWLLQWMPTVNASVGTGACAGPASLSHMQLHHLDPKTFKCRAIGGGL
SRWGGRREIWGKGCQGQEARLTPCPAISRSGKTLSKQHCLPEPQFSHL*

LP284 Mature Sequence (242aa):
A predicted mature LP284 sequence is as follows:

WRPPKGKCPLRCSCSKDSALCEGSPDLPVSFSPTLLSLSLVRTGVTQLKAGSFLRIPSLHLLLFTSNSFSV
IEDDAFAGLSHLQYLFIEDNEIGSISKNALRGLRSLTHLSLANNHLETLPRFLFRGLDTLTHVDLRGNPFQ
CDCRVLWLLQWMPTVNASVGTGACAGPASLSHMQLHHLDPKTFKCRAIGGGLSRWGGRREIWGKGCQGQEA
RLTPCPAISRSGKTLSKQHCLPEPQFSHL*

Particularly interesting portions or fragments of the full length LP284 polypeptide (SEQ ID NO: 16) include, e.g., a predicted signal sequence from Met-1 to Ala-19; another analysis yields a predicted signal sequence from Met-1 to Cys-40. An additionally interesting segment of LP284 is a transmembrane-like region: Ala-4 to Pro-23; also particularly interesting are LP284 fragments Lys-26 to Gly-42; Ser-56 to Lys-68; Gly-70 to Thr-84; Ser-87 to Gly-98; Leu-131 to Leu-143; Phe-159 to Thr-175; Asp-200 to Ser-214; Gly-217 to Gln-228; Leu-7 to Trp-20; Arg-21 to Ser-49; Phe-50 to Val-60; Arg-61 to Gly-70; Ser-71 to Thr-84; Ser-85 to Gly-98; leu-99 to Ile-107; Glu-108 to Ser-125; Asp-148 to Cys-164; Thr-175 to His-193; His-198 to Trp-223; Gly-224 to Cys-237; Pro-238 to Pro-256; Lys-26 to Leu-46; Phe-50 to Lys-68; Gly-70 to Ser-85; Ser-87 to Glu-108; Glu-111 to Ala-132; Phe-142 to Gly-157; Phe-159 to Met-173; Pro-174 to Gln-195; Asp-200 to Ser-214; and Gly-217 to Ala-232, whose discovery was based on an analysis of hydrophobicity, hydropathicity, and hydrophilicity plots. Additional interesting sections of LP284 are the portions of the polypeptide from Glu-41 to Leu-55; Ser-85 to Ala-95; Leu-105 to Leu-120; Arg-121 to Pro-140; Leu-147 to Leu-155; Gly-157 to Val-166; Leu-167 to Gly-187; Ser-192 to Phe-204; Lys-205 to Gly-229; Gln-230 to Ser-243; Thr-246 to Pro-256; Arg-21 to Ser-32; Leu-29 to Gly-42; Lys-35 to Phe-50; His-152 to Val-166; Met-194 to Cys-206; Gly-210 to Trp-223; Ser-214 to Gly-226; Gly-217 to Ala-232; Gly-224 to Pro-236; Pro-238 to Gln-250; Ile-240 to Ser-259; Ser-243 to Pro-256; Cys-252 to Leu-261; Arg-21 to Asp-36; Ser-32 to Leu-46; His-198 to Ala-208; Leu-213 to Lys-225; Ile-222 to Leu-234; and Ala-239 to Leu-253. These fragments were discovered based on analysis of antigenicity plots. An additionally interesting portion of LP284 is a segment discovered based on a PROSITE analysis is a membrane lipoprotein lipid attachment-like site: Thr-175 to Cys-186. In prokaryotes, such membrane lipoprotein sites are synthesized with a precursor signal peptide that is cleaved by a specific lipoprotein signal peptidase (signal peptidase II). The peptidase recognizes a conserved sequence and cuts upstream of a cysteine residue to which a glyceride-fatty acid lipid is attached resulting in a post-translational modification (Hayashi & Wu (1990) J. Bioenerg. Biomembr 22:451-471). An additionally interesting portion of LP284 is a segment discovered based on a PROSITE analysis as an ATP/GTP binding site motif-like A sequence or P-loop: Ala-239 to Thr-246. Sequence comparisons and crystallographic data have shown that an appreciable proportion of proteins that bind ATP or GTP share a number of more or less conserved sequence motifs. The best conserved of these motifs is a glycine-rich region, which typically forms a flexible loop between a beta-strand and an alpha-helix. This loop interacts with one of the phosphate groups of the nucleotide. This sequence motif is generally referred to as the 'A' consensus sequence (Walker, et al, (1982) EMBO J. 1:945-951) or the 'P-loop' (Saraste, et al., (1990) Trends Biochem. Sci. 15:430-4345). Additionally interesting portions of LP284 are segments discovered based on a PROSITE analysis as a Leucine Rich Repeat-like domains (LRR): Thr-53 to Pro-76; Ser-77 to Ser-100; His-101 to Arg-124; and Ser-125 to Asp-148; and Thr-149 to Gln-171. LRRs are short sequence motifs present in a number of proteins with diverse functions and cellular locations. LRRs are usually involved in protein-protein interactions. Each Leucine Rich Repeat is composed of a beta-alpha unit, which typically form elongated non-globular structures. Leucine Rich Repeats are often flanked by cysteine rich domains (Kobe & Deisenhofer 1994 Trends Biochem Sci 19:415-421). An additionally interesting portion of LP284 is a segment discovered based on a PROSITE analysis as a Leucine Rich Repeat C-terminal-like domain (LRRCT): Pro-201 to Arg-207. The LRRCT domain is often found at the C-terminus of tandem leucine rich repeats (Kobe & Deisenhofer 1994 Trends Biochem Sci 19:415-421). Further, particularly interesting LP284 secondary structures (e.g., such as a helix, a strand, or a coil) are the following LP284 helical structures: His-101 to Gln-103; Ala-119 to Thr-127; and Leu-139 to Phe-144. Particularly interesting coil structures are Met-1 to Gly-3; Gly-12 to Gly-14; Pro-22 to Pro-28; Ser-32 to Ser-37; Glu-41 to Val-48; Ser-51 to Pro-52; Thr-62 to Gly-63; Leu-66 to Ser-71; Pro-76 to Leu-78; Thr-84 to Ser-87; Glu-108 to Ile-112; Gly-146 to Tyr-149; Leu-155 to Cys-162; Trp-172 to Asn-177; Gly-181 to Pro-188; Leu-199 to Thr-203; Gly-210 to Arg-219; Gly-224 to Lys-245; and Lys-249 to Leu-261. Particularly interesting strands are Val-16 to Trp-20; Leu-80 to Phe-83; Phe-204 to Ala-208; and Arg-220 to Trp-223. Further encompassed by the invention are contiguous amino acid residue combinations of any of the predicted secondary structures described above. For example, one coil-strand-coil motif of LP284 combines the Gly-210 to Arg-219 coil; with the Arg-220 to Trp-223 strand; and the Gly-224 to Lys-245 coil to form an interesting fragment of contiguous amino acid residues from Gly-210 to Lys-245. Other combinations of contiguous amino acids are contemplated as can be easily determined.

### FEATURES OF LP NO: 9 (LP282)

LP282 (HGF-AIh2) is an alternatively spliced version of LP136 (HGF-AIh1), which was previously disclosed in provisional application USSN 60/188,881 and was filed 13-MAR-2000. Both LP136 and LP282 exhibit sequence similarity to the hepatocyte growth factor activator inhibitor (HGF-AI) protein, which is a serine protease inhibitor that was discovered from the conditioned medium of a cancer cell line (Shimomura, et al., 1997 J Biol. Chem. 272(10): 6370-6) W27368. Hepatocyte growth factor (HGF) is activated in response to tissue injury and is believed to promote healing in a variety of damaged tissues. HGF is a cell product that is secreted extracellularly as an inactive single chain precursor, which associates with an extracellular matrix where it is subsequently converted to a biologically active heterodimer through the action of a liver-produced serine protease that is designated HGF activator (HGF-A) (Shimomura, et al., 1995 Eur J Biochem., 1;229(1):257-261). HGF activator circulates as an inactive zymogen in the blood where it is converted to an active form by limited proteolysis. A Kunitz-type serine protease inhibitor (HAI-1), isolated from the conditioned medium of a human stomach carcinoma cell line (MKN45), is believed to participate in regulating the action of HGF via inhibitory effects on HGF activator (Shimomura, et al., 1997 J Biol. Chem., 272(10):6370-6). A second Kunitz-type HGF activator inhibitor (HAI-2), also isolated from the MKN45 carcinoma cell line, shares certain properties with HAI-1. Molecular cloning revealed that HAI-2 is derived from a 252 amino acid precursor that contains two Kunitz-type domains and a hydrophobic COOH-terminal region (Kawaguchi et al., 1997J Biol. Chem., 272(44):27558-64). HAI-1 expression levels are decreased in the white matter of Alzheimer brains (Yamada et al., 1998 Exp. Neurol., 153(1):60-4) and in adenocarcinoma tissue (Kataoke et al., 1998 Cancer Lett, 128(2):219-27) suggesting that HAI and related proteins (such as LP136 and LP282) may be useful in treating neural conditions diseases, syndromes, or conditions associated with cell proliferation such as, for example, wound healing and cancer (Shimomura, et al., 1997 J Biol. Chem., 272(10): 6370-6).
It has been discovered that LP282 sequences (SEQ ID NO: 17) are expressed primarily in the following number of LIFESEQ GOLD™ database tissue and cDNA libraries: Connective Tissue 2/47; Digestive System 1/148; Embryonic structures 1/21; Endrocrine System 2/53; Genitalia, Female 2/106; Genitalia, Male 1/114; Germ Cells 1/5; Nervous System (brain) 5/198; Respiratory System (lung) 5/93; and Sense Organs 1/8. Since LP282 is mainly expressed in the respiratory system (specifically, e.g., in the lung) and the nervous system (specifically, e.g., in brain) LP282 compositions are useful in the treatment, for example, of defects in or wounds to organs including, but not limited to the nervous system (e.g., brain) and the respiratory system (such as, e.g., the lung). According to its expression in the nervous system, compositions comprising LP282 polypeptides or polynucleotides (including fragments thereof), and/or antibodies are useful for diagnosis, treatment and intervention of diseases, disorders, and/or conditions including, but not limited to, cancer, Alzheimer's Disease, Parkinson's Disease, Huntington's Disease, Tourette Syndrome, meningitis, encephalitis, demyelinationg diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder learning disabilities, ALS, psychoses, autism, and altered behaviors, including disorders in feeding, sleep patterns, balance, and perception. LP282 expression has also been observed in smooth muscle cells in the small intestine and in pancreatic islets; peri-portal hepatocytes, as well as in the testis and smooth muscle of the uterus. Accordingly, compositions comprising LP282 polypeptides, polynucleotides, its agonists/antagonists and/or antibodies are also useful for the treatment of defects in or wounds to tissues including, but not limited to small intestine, pancreas, liver, testis, and uterus. LP282 polypeptide has been shown to promote growth of the following cultured cells: GT1-7: hypothalamic cells; GnRH producing cell lines; HDF: human dermal fibroblasts; Hep G2: human hepatoma cell line; and M07E: megakaryoblastic cell line (Megakaryocyte: <hematology> Giant polyploid cell of bone marrow that gives rise to 3-4,000 platelets.). Sequence encoding LP282 polypeptide has been localized to chromosome 6q25.1. Moreover, the following diseases, conditions, syndromes, disorders, or pathological states have also been mapped to this region of the human chromosome: autosomal recessive polycystic kidney disease (ARPKD) autism (Martinez & Grantham, 1995 Dis Mon Nov;41 (11):693-765; Parkinson disease, juvenile, type 2, (Jones, et al., 1998 Am. J. Hum. Genet. 63: 80-87); Diabetes mellitus, insulin-dependent (Concannon, et al., 1998 Nat. Genet. 19: 292-296, Luo, et al., 1995 Am. J. Hum. Genet. 57: 911-919); Retinal cone dystrophy-1 (Went, et al., 1992 J. Med. Genet. 29: 295-298); Breast cancer; Estrogen resistance (Becherini, et al., 2000 Hum. Molec. Genet. 9: 2043-2050; Chaidarun & Alexander, 1998 Molec. Endocr. 12: 1355-1366, Gosden, et al., 1986 Cytogenet. Cell Genet. 43: 218-220). Accordingly, an isolated and/or recombinant DNA molecule of LP277 (or fragment thereof) meets the statutory utility requirement of 35 U.S.C. 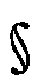 101 as it can be used to hybridize near one or more of the above stated diseases and thus serve as a marker for a such a disease gene.
Table 8: Primate, e.g., human, LP282 polynucleotide sequence (SEQ ID NO: 17) and corresponding polypeptide (SEQ ID NO: 18). The ORF for LP282 is 27-1433 bp (with the start (ATG) and stop codons (TAG) identified in bold lettering and underlined in case numbering is misidentified one skilled in the art could determine the open reading frame without undue experimentation).

LP282 DNA sequence (1555bp) (ORF = 27-1433):
ATGGCCTCCGTCGCCCAGGAGAGCGCGGGCTCGCAGCGCCGGCTACCGCCGCGTCACGGGGCGCTGCGCGG
GCTGCTACTGCTCTGCCTGTGGCTGCCAAGCGGCCGTGCGGCCTTGCCGCCCGCGGCGCCGCTGTCCGAAC
TGCACGCGCAGCTGTCGGGCGTGGAGCAGCTGCTGGAGGAGTTCCGCCGGCAACTGCAGCAGGAGCGGCCT
CAGGAGGAGCTGGAGCTGGAGCTGCGTGCGGGCGGCGGCCCCCAGGAGGACTGCCCGGGCCCGGGCAGCGG
CGGCTACAGCGCAATGCCTGACGCCATCATCCGCACCAAGGACTCCCTGGCGGCGGGTGCCAGCTTCCTGC
GGGCGCCGGCGGCCGTGCGGGGCTGGCGGCAATGCGTGGCGGCCTGCTGCTCCGAGCCGCGCTGCTCCGTG

GCCGTGGTGGAGCTGCCCCGGCGCCCCGCGCCCCCGGCAGCCGTGCTCGGCTGCTACCTCTTCAACTGCAC
GGCGCGCGGCCGCAACGTCTGCAAGTTCGCGCTGCACAGCGGCTACAGCAGCTACAGCCTCAGCCGCGCGC
CGGACGGCGCCGCCCTGGCCACCGCGCGCGCCTCGCCCCGGCAGGAAAAGGATGCGCCTCCACTTAGCAAG
GCTGGGCAGGATGTGGTTCTGCATCTGCCCACAGACGGGGTGGTTCTAGACGGCCGCGAGAGCACAGATGA
CCACGCCATCGTCCAGTATGAGTGGGCACTGCTGCAGGGGGACCCGTCAGTGGACATGAAGGTGCCTCAAT
CAGGAACCCTGAAGCTGTCCCACCTACAGGAGGGAACCTACACCTTCCAGCTGACCGTGACGGACACTGCC
GGGCAGAGAAGCTCTGACAACGTGTCAGTGACAGTGCTTCGCGCAGCCTACTCCACAGGAGGATGTTTGCA
CACTTGCTCACGCTACCACTTCTTCTGTGACGATGGCTGCTGCATTGACATCACGCTCGCCTGCGATGGAG
TGCAGCAGTGTCCTGATGGGTCTGATGAAGACTTCTGCCAGAATCTGGGCCTGGACCGCAAGATGGTAACC
CACACGGCAGCTAGTCCTGCCCTGCCAAGAACCACAGGGCCGAGTGAAGATGCAGGGGGTGATTCCTTGGT
GGAAAAGTCTCAGAAAGCCACTGCCCCAAACAAGCCACCTGCATTATCAAACACAGAGAAGAGGAATCATT
CCGCCTTTTGGGGACCAGAGAGTCAAATCATTCCTGTGATGCCAGGTGCAGTGCTACCCCTGGCGCTGGGT
TTGGCTATCACTGCTCTGCTGCTTCTCATGGTTGCATGCCGACTACGACTGGTGAAACAGAAACTGAAAAA
AGCTCGTCCCATTACATCTGAGGAATCGGACTACCTCATAAATGGGATGTATCTATAG

LP282 Full-Length Sequence (468aa):
>ds37486+3_ORF1 (SEQ ID NO: 18) The underlined portion is a predicted signal sequence (Met-1 to Ala-
37). A predicted SP cleavage site is between Ala-37 and Ala-38 indicated as follows: 1
MASVAQESAGSQRRLPPRHGALRGLLLLCLWLPSGRA^AL 39

MASVAQESAGSQRRLPPRHGALRGLLLLCLWLPSGRAALPPAAPLSELHAQLSGVEQLLEEFRRQLQQERP
QEELELELRAGGGPQEDCPGPGSGGYSAMPDAIIRTKDSLAAGASFLRAPAAVRGWRQCVAACCSEPRCSV
AVVELPRRPAPPAAVLGCYLFNCTARGRNVCKFALHSGYSSYSLSRAPDGAALATARASPRQEKDAPPLSK
AGQDVVLHLPTDGVVLDGRESTDDHAIVQYEWALLQGDPSVDMKVPQSGTLKLSHLQEGTYTFQLTVTDTA
GQRSSDNVSVTVLRAAYSTGGCLHTCSRYHFFCDDGCCIDITLACDGVQQCPDGSDEDFCQNLGLDRKMVT
HTAASPALPRTTGPSEDAGGDSLVEKSQKATAPNKPPALSNTEKRNHSAFWGPESQIIPVMPGAVLPLALG
LAITALLLLMVACRLRLVKQKLKKARPITSEESDYLINGMYL*

LP282 Mature Sequence (431aa):
A predicted mature LP282 protein is as follows:

ALPPAAPLSELHAQLSGVEQLLEEFRRQLQQERPQEELELELRAGGGPQEDCPGPGSGGYSAMPDAIIRTK
DSLAAGASFLRAPAAVRGWRQCVAACCSEPRCSVAVVELPRRPAPPAAVLGCYLFNCTARGRNVCKFALHS
GYSSYSLSRAPDGAALATARASPRQEKDAPPLSKAGQDVVLHLPTDGVVLDGRESTDDHAIVQYEWALLQG
DPSVDMKVPQSGTLKLSHLQEGTYTFQLTVTDTAGQRSSDNVSVTVLRAAYSTGGCLHTCSRYHFFCDDGC
CIDITLACDGVQQCPDGSDEDFCQNLGLDRKMVTHTAASPALPRTTGPSEDAGGDSLVEKSQKATAPNKPP
ALSNTEKRNHSAFWGPESQIIPVMPGAVLPLALGLAITALLLLMVACRLRLVKQKLKKARPITSEESDYLI
NGMYL*

Blast genomic DNA of LP188 demonstrates that the extra sequence in LP188 is a distinct exon. LP282 exhibits sequence identity with Kunitz type 1 Murine Serine Protease Inhibitor g8394351

Particularly interesting portions or fragments of the full length LP282 polypeptide (SEQ ID NO: 18) include, e.g., a predicted signal sequence from Met-1 to Ala-37. Additionally interesting segments of LP282 are two transmembrane-like regions: Gly-20 to Ala-37 and Ile-413 to Leu-441; also particularly interesting are LP282 fragments Glu-7 to ala-21; Leu-22 to Pro-33; Leu-59 to Glu-78; Arg-80 to Ser-98; Leu-111 to Ala-123; Pro-154 to Cys-165; Arg-199 to Gln-216; Pro-223 to Ala-246; Val-280 to Ser-302; Ala-328 to His-356; Arg-365 to Pro-392; Ile-413 to Leu-441 and Cys-439 to Leu-462, Glu-7 to Ala-21; Leu-22 to Pro-33; glu-1 to Glu-76; Ala-81 to Ala-103; Arg-106 to Ser-116; Ala-122 to Ala-143; Ser-179 to Ala-194; Ala-196 to Leu-211; Thr-224 to Gln-242; Gln-249 to Lys-265; Vval-280 to Thr-295; Ala-328 to Asn-346; Arg-365 to Leu-378; Glu-380 to Pro-392; Leu-394 to Phe-405; Val-420 to Val-444; and Glu-446 to Ile-463, whose discovery was based on an analysis of hydrophobicity, hydropathicity, and hydrophilicity plots. Additional interesting sections of LP282 are the portions of the polypeptide from Arg-14 to Leu-28; Ala-37 to Gly-54; Leu-77 to Arg-106; Ser-110 to Val-124; Gln-129 to Ala-143; Leu-158 to Asn-171; Val-172 to Ser-183; Arg-188 to Asp-207; Ala-208 to Gly-26; His-314 to Val-332; Gln-333 to Asp-350; Arg-351 to Leu-363; Glu-380 to Ala-393; Phe-405 to Leu-421; Arg-442 to Pro-453; Gly-20 to Ala-37; Gly-84 to Ser-98; Ala-194 to Lys-213; Val-228 to Ala-239; Phe-276 to Thr-295; Val-332 to Gln-345; Leu-363 to Ala-385; Pro-388 to Lys-399; Lys-449 to Ser-459 and Thr-455 to Asn-464. These fragments were discovered based on analysis of antigenicity plots. An additionally interesting portion of LP282 is a segment discovered based on a PROSITE analysis is a membrane lipoprotein lipid attachment-like site: Ile-429 to Cys-439. In prokaryotes, such membrane lipoprotein sites are synthesized with a precursor signal peptide that is cleaved by a specific lipoprotein signal peptidase (signal peptidase II). The peptidase recognizes a conserved sequence and cuts upstream of a cysteine residue to which a glyceride-fatty acid lipid is attached resulting in a post-translational modification (Hayashi & Wu (1990) J. Bioenerg. Biomembr 22:451-471). An additionally interesting portion of LP282 is a segment discovered based on a PROSITE analysis as an LDL-receptor class A (LDLRA) domain-like signature and profile: Cys-322 to Cys344; and a low-density lipoprotein receptor-like domain (LDL): His-308 to Asn-346. Low-density lipoprotein (LDL) receptors are the major cholesterol-carrying lipoproteins of plasma. Seven successive cysteine-rich repeats of about 40 amino acids are present in the N-terminal of this multidomain membrane protein (Yamamoto, et al., 1984 Cell 39:27-38.). Similar domains have been found in other extracellular and membrane proteins (Daly, et al. 1995 Proc. Natl. Acad. Sci. U.S.A. 92:6334-6338). In LDL-receptors the class A domains form the binding site for LDL and calcium. The acidic residues between the fourth and sixth cysteines are important for high-affinity binding of positively charged sequences in LDLR's ligands (Mahley R.W. 1988 Science 240:622-630). The repeat has been shown to consist of a beta-hairpin structure followed by a series of beta turns. The binding of calcium seems to induce no significant conformational change (Yamamoto, et al., 1984 Cell 39:27-38.). An additionally interesting portion of LP282 is a segment discovered based on a PROSITE analysis as a PKD-like domain (Bycroft, et al., 1999 EMBO J; 18:297-305): Pro-209 to Leu-297. The PKD domain was first identified in the polycystic kidney disease protein (PKD1) and is predicted to contain an Ig-like fold (Cell 1995 81:289-2981). Further, particularly interesting LP282 secondary structures (e.g., such as a helix, a strand, or a coil) are the following LP282 helical structures: Val-4 to Glu-7; Ser-46-to Ala-50; Val-55to Gln-68; Gln-72 to Leu-77; Leu-111 to Ala-112; Ala-115 to Leu-118; Ala-123 to Gln-129; Ala-194 to Arg-199; and Leu-427 to Lys-450. Particularly interesting coil structures are Ala-9 to Gly-20 Pro-33 to Ala-43; Ala-81 to Met-100; Ala-133 to Cys-140; Pro-148 to Ala-155; Asn-164 to Asn-171; His-178 to Ser-182; Ser-187 to Asp-191; Pro-202 to Glu-216; Leu-222 to Gly-226; Gly-231 to His-238; Gly-250 to Ser-253; Pro-259 to Thr-263; Leu-269 to Gly-272; Asp-282 to Asn-291; Ala-300 to Ser-311; Asp-318 to Cys-321; Cys-329 to Asp-340; Leu-347 to Lys-352; Thr-357 to Asp-376; Ala-385 to Ala-404; Gly-407 to Ser-410; Met-416 to Met-419; and Arg-452 to Ser-459. Particularly interesting strands are Leu-28 to Leu-30; Vla-142 to Val-145; Val-172 to Leu-177; Val-218 to His-221; Tyr-274 to Val-280; Ser-293 to Arg-298; Tyr-313 to Phe-316; Ile-323 to Leu-327; and Val-354 to Thr-355. Further encompassed by the invention are contiguous amino acid residue combinations of any of the predicted secondary structures described above. For example, one coil-strand-coil-strand-coil-strand-coil-strand-coil motif of LP282 combines the Leu-269 to Gly-272 coil; with the Tyr-274 to Val-280 strand; the Asp-282 to Asn-291 coil, the Ser-293 to Arg-298 strand; the Ala-300 to Ser-311 coil, the Tyr-313 to Phe-316 strand, the Asp-318 to Cys-321 coil; the Ile-323 to Leu-327 strand, and the Cys-329 to Asp-340 coil to form an interesting fragment of contiguous amino acid residues from Leu-269 to Asp-340. Other combinations of contiguous amino acids are contemplated as can be easily determined.

### FEATURES OF LP NO: 10 (LP273)

The molecular cloning of genes generated as a result of chromosomal translocations provides a powerful tool to identify novel proteins, such as, for example, novel oncogenes and novel pathogenic mechanisms of neoplasias. Recently, using modern molecular techniques (e.g., long-distance inverse polymerase-chain reaction (Willis, et al. 1998 Blood 91(6):1873-1881)), a new gene (BCL9) was discovered that had been generated as the result of a chromosome translocation (q21; q23) (Hertz & Jensen 1985 Ann Genet 28(4):228-30). Similar methods were taken to show that genes discovered in solid human neoplasias were also the result of complex chromosomal translocationa (q33;q22;q12) (Halal, et al. 1989 Am J Med. Genet 32(3):376-379; Peter, et al. 1997 Oncogene 14(10):1159-1164). Using similar molecular biological techniques, Applicants have discovered that a new protein (LP273; SEQ ID NO: 20) is derived from the result of an in-frame translocation between human chromosome 1 and human chromosome 2. The resulting novel protein, designated LP273, is a variant of LP268. LP273 comprises an N-terminal portion (from about Met-1 to about Cys-191) that exhibits amino acid sequence identity to the N-terminal portion of LP268 ― except for a two amino acid residue difference at amino residues 65 and 114 (I-65 of LP268 → M-65 of LP273 and R-114 of LP268 → S-114 of LP273; see Table 9 below). LP273 also comprises a C-terminal portion (from about Arg-193 to about Ser-310) that exhibits amino acid sequence similarity to a "membrane protein" having GenBank Accession No. g6467175. A Lys residue in LP273 (at about amino acid Lys-192) appears to link the N- and C-terminal portions of this fused protein. As described above, the N-terminal portion of LP273 that is similar to LP268 exhibits amino acid sequence similarity to a carboxypeptidase B molecule designated thrombin-activatable fibrinolysis inhibitor (TAFI). TAFI is a member of the carboxypeptidase B family that shows amino acid sequence similarity to both pancreatic carboxypeptidase A and B (Eaton, et al. 1991 J. Biol. Chem. 266(32):21833-21838). TAFI has been shown to play a role in blood coagulation (such as, e.g., prolonging clot lysis by removing C-terminal Lys residues in partially degraded fibrin and by inhibiting fibrinolysis (Marx, et al., 2000 Thromb Haemost (2):297-303)). Inhibition of TAFI (or activated TAFI) appears to have an opposite effect (e.g., resulting in enhanced fibrinolysis and benefiting tPA-induced thrombolysis). Taken as a whole, these data suggest that modulating TAFI influences thrombolysis (Klement et al., 1999 Blood 94(8), 2735-2743). Testing a protein for carboxypeptidase activity is routine and would not require undue experimentation given the teachings supplied herein (e.g., as to the LP273 sequence) and given teachings in the art for methods of determining whether a suspected protein has carboxypeptidase activity (see, e.g., Skidgel, R A., "Assays for Arginine/Lysine Carboxypeptidase" 1991 Methods in Neurosciences (6)373-385; Thomas, et al., "An Improved Spectrophotometic Assay for Human Plasma Carboxypeptidase N" 1980 Analytical Biochemistry (108) 348-353; and Thomas, et al., "Comparative Studies on Human Carboxypeptidase B and N" 1979 Archives of Biochemistry and Biophysics (197) 487-492). The C-terminal portion of LP273 is similar to the g6467175 "membrane protein." The g6467175 molecule was identified as a putative endoplasmic reticulum membrane protein as the result of a *large-scale in vitro* translation analysis of a full-length human cDNA bank (Iwamuro, et al. 1999 J Biochem (Tokyo) 126(1):48-53). The same cDNA bank study indicated that g6467175 fails to assume a mature three-dimensional conformation in the absence of its translocation target. Consequently, improperly folded g6467175 molecules are degraded via an ubiquitin-proteasome pathway.

Applicants have discovered here that an immediate impact of the formation of LP273 from the translocation between chromosome 1 and 2 is the loss of a putative membrane domain from the g6467175's contribution to LP273. Thus, the translocation producing LP273 generates a unique molecule without a membrane domain and that is processed as a secreted molecule having its own signal peptide sequence. Similar fusion-like proteins ―resulting from chromosomal translocations― have been shown to cause human diseases (see, e.g., Testa, J. R. 1990 Cell Growth Differ 1(2): 97-101). For example, the chimeric fusion protein Bcr-Abl is an oncogene that has been implicated in the pathogenesis of chronic myelogenous leukemia (CML). Furthermore, translocated Philadelphia chromosome (Ph1)-positive has been shown to cause acute lymphocytic leukemia (ALL) (Gishizky, et al., 1996 Cytokines Mol Ther (4):251-61; Nowell & Hungerford 1960 Science 132: 1497). Since the discovery of the Philadelphia chromosome, many other chromosomal translocations have been implicated in the genesis of human neoplasias. For instance, small intestinal T-cell lymphoma is associated with the chromosomal translocation q26;p13 (Rojas-Atencio, et al., Invest Clin 1999 Sep;40(3):179-89; Carbonnel, et al. 1994 Cancer 73(4): 1286-91). Although instances of primary T-cell lymphoma of the small intestine are rare, this is an aggressive disease with a poor prognostic outcome. More specifically, translocations involving human chromosomes 1 and 2 have been reported in subjects exhibiting psychomotor retardation or multiple congenital anomaly syndromes (Hertz & Jensen 1985 Ann Genet 28(4):228-30; Halal, et al., Am J Med. Genet 32(3):376-379; Chitayat, et al., Am J Ed Genet 1989 Jan;32(1):36-41). Moreover, translocations involving chromosomes 1 and 2 have also been reported in breast cancer patients (Rojas-Atencio, et al., 1999 Invest Clin 40(3):179-89) and it has been associated with an intestinal carcinoma (Hertz & Jensen 1985 Ann Genet 28(4):228-30; Halal, et al., Am J Med. Genet 32(3):376-379). The sequence encoding LP273 itself was isolated from tissue of a cancer patient with a family history of cell proliferative conditions (such as, e.g., cancer, including colon cancer, lung cancer, stomach cancer, and liver cancer). Accordingly, the findings of an association between chromosomal translocations and human disease, between cancer and translocations of human chromosomes 1 and 2, and the fact that LP273 sequence was isolated from diseased tissue in a patient with cancer, suggest that the chromosomal translocation that generates LP273 is involved in the pathogenesis of cell proliferative conditions (such as, e.g., certain cancers, including colon cancer, lung cancer, stomach cancer, and liver cancer). Consequently, compositions comprising LP273 polypeptides (fragments thereof), polynucleotides (fragments thereof), and/or antibodies, activators and inhibitors of LP273 will be useful for the diagnosis and/or treatment of proliferative states or conditions in tissues in which it is expressed (such as, e.g., cancers including, but not limited to, colon cancer, lung cancer, stomach cancer, and liver cancer).

It has been discovered that LP273 sequences (SEQ ID NO: 19) are expressed primarily in the following number of LIFESEQ GOLD™ database tissue and cDNA libraries: Cardiovascular System 5/68; Connective Tissue 3/47; Digestive System 10/148; Embryonic structures 2/21; Endocrine System 2/53; Exocrine System 8/64; Genitalia, Female 12/106; Genitalia, Male 5/114; Hemic and Immune System 17/159; Liver 2/35; Musculoskeletal System 4/47; Nervous System 20/198; Pancreas 1/24; Respiratory System 6/93; and Skin 2/15. Sequence encoding the N-terminal portion of LP273 (approximately, bp 1-592 of LP273 DNA sequence (ds35878; Table 9 below)) has been located to human chromosome 2, while sequence encoding the C-terminal portion of LP273 (approximately, bp 593-1434 of LP273 DNA sequence (ds35878; Table 9 below)) has been located to human chromosome 1.
Table 9: Primate, e.g., human, LP273 polynucleotide sequence (SEQ ID NO: 19) and corresponding polypeptide (SEQ ID NO: 20). The ORF for LP273 is 19-951 bp (with the start (ATG) and stop codons (TAG) identified in bold lettering in case numbering is misidentified one skilled in the art could determine the open reading frame without undue experimentation).

LP273 DNA sequence (1501bp)(ORF = 19-951):

ATGAAGCCTCTGCTTGAAACCCTTTATCTTTTGGGGATGCTGGTTCCTGGAGGGCTGGGATATGATAGATCC
TTAGCCCAACACAGACAAGAGATTGTGGACAAGTCAGTGAGTCCATGGAGCCTGGAGACGTATTCCTATAAC
ATATACCACCCCATGGGAGAGATCTATGAGTGGATGAGAGAGATCAGTGAGAAGTACAAGGAAGTGGTGACA
CAGCATTTCCTAGGAGTGACCTATGAGACCCACCCCATGTATTATCTGAAGATCAGCCAACCATCTGGTAAT
CCCAAGAAAATCATTTGGATGGACTGTGGAATTCACGCCAGAGAATGGATTGCTCCTGCTTTTTGCCAATGG
TTCGTCAAAGAAATTCTACAAAACCATAAAGACAACTCAAGTATACGCAAGCTCCTTAGGAACCTGGACTTC
TATGTCCTTCCAGTTCTTAACATAGATGGTTATATCTACACTTGGACAACTGATCGTCTTTGGAGGAAATCC
CGTTCACCCCATAATAATGGCACATGTTTTGGGACGGATCTCAATCGAAATTTCAATGCATCTTGGTGTAAG
AGACAAGAAGAGAAACTGAAGAATAACAACAGAGATCTATCAATGGTTCGAATGAAATCCATGTTTGCTATT
GGCTTTTGTTTTACTGCCCTAATGGGAATGTTCAATTCCATATTTGATGGTAGAGTGGTGGCAAAGCTTCCT
TTTACCCCTCTTTCTTACATCCAAGGACTGTCTCATCGAAATCTGCTGGGAGATGACACCACAGACTGTTCC
TTCATTTTCCTGTATATTCTCTGTACTATGTCGATTCGACAGAACATTCAGAAGATTCTCGGCCTTGCCCCT
TCACGAGCCGCCACCAAGCAGGCAGGTGGATTTCTTGGCCCACCACCTCCTTCTGGGAAGTTCTCTTGA

LP273 Full-Length Sequence (310aa):
>ds35878+1_ORF1 (SEQ ID NO: 20) The underlined portion is a predicted signal sequence (Met-1 to Gly-
20). A predicted SP cleavage site is between Gly-20 and Tyr-21 indicated as follows: 1
MKPLLETLYLLGMLVPGGLG^YD 22

MKPLLETLYLLGMLVPGGLGYDRSLAQHRQEIVDKSVSPWSLETYSYNIYHPMGEIYEWMREISEKYKEVVT
QHFLGVTYETHPMYYLKISQPSGNPKKIIWMDCGIHAREWIAPAFCQWFVKEILQNHKDNSSIRKLLRNLDF
YVLPVLNIDGYIYTWTTDRLWRKSRSPHNNGTCFGTDLNRNFNASWCKRQEEKLKNNNRDLSMVRMKSMFAI
GFCFTALMGMFNSIFDGRVVAKLPFTPLSYIQGLSHRNLLGDDTTDCSFIFLYILCTMSIRQNIQKILGLAP
SRAATKQAGGFLGPPPPSGKFS*

LP273 Mature Sequence (290aa):
A predicted mature LP273 is as follows:

YDRSLAQHRQEIVDKSVSPWSLETYSYNIYHPMGEIYEWMREISEKYKEVVTQHFLGVTYETHPMYYLKIS
QPSGNPKKIIWMDCGIHAREWIAPAFCQWFVKEILQNHKDNSSIRKLLRNLDFYVLPVLNIDGYIYTWTTD
RLWRKSRSPHNNGTCFGTDLNRNFNASWCKRQEEKLKNNNRDLSMVRMKSMFAIGFCFTALMGMFNSIFDG
RVVAKLPFTPLSYIQGLSHRNLLGDDTTDCSFIFLYILCTMSIRQNIQKILGLAPSRAATKQAGGFLGPPP
PSGKFS*

Particularly interesting portions or fragments of the full length LP273 polypeptide (SEQ ID NO: 20) include, e.g., a predicted signal sequence from Met-1 to Gly-20; a second predicted signal sequence from Met-1 to Trp-40. Additionally interesting segments of LP273 are transmembrane-like portions Leu-4 to Gly-19; Asn-200 to Asn-228; and His-252 to Gln-278. Additionally interesting segments of LP273 are fragments Leu-10 to Ser-24; Leu-25 to Ser-38; Pro-39 to Ile-63; Gly-77 to Tyr-87; Met-103 to Lys-123; Leu-126 to Leu-138; Arg-140 to Tp-159; Asp-162 to Thr-180; Asn-187 to Met-207; Met-210 to Phe-227; Asn-228 to Arg-253; Leu-256 to Leu-284; Ser-289 to Leu-300; Leu10 to Leu-19; Tyr-21 to Tyr-45; Asn-48 to Lys-68; Glu-69 to Thr-79; Thr-82 to Asn-96; Lys-98 to Gly-106; Ile-107 to Phe-117; Phe-121 to Leu-139; Leu-142 to Ile-156; Tyr-157 to Thr-180; Leu-182 to Arg-209; Ser-212 to Phe-241; Gln-248 to Asp-258; Thr-260 to Lys-282; Thr-1 to Pro-16 Gly-17 to Trp-40; Arg-61 to Gln-73; Ile-90 to Lys-99; Trp-112 to Ile-125; Leu-126 to Arg-136; Leu-138 to Ile-152; Arg-166 to Asn-185; Trp-190 to Val-208; Ser-212 to Asn-228; Gln-248 to Cys-263; and Pro-288 to Pro-334, whose discovery was based on an analysis of hydrophobicity, hydropathicity, and hydrophilicity plots. Additional interesting sections of LP273 are the portions of the polypeptide from Thr-7 to Tyr-21; Asp-22 to Asp-34; Tyr-47 to Lys-66; Thr-79 to Gln-92; Gly-95 to Ala-109; Arg-110 to Lys-123; Val-146 to Asp-162; Pro-171 to Phe-186; Asp-204 to Gly-217; Phe-218 to Val-236; Thr-260 to Leu-286; Asp-22 to Glu-43; Met-60 to Gly-76; Pro-84 to Ser-94; Gly-95 to Asp-104; Lys-123 to Leu-138; Trp-165 to Leu-182; Phe-186 to Leu-205; Gly-249 to Asp-262; Ile-284 to Gln-295; Asp-22 to Val-37; Tyr-57 to Val-70; Gln-127 to Leu-139 and Phe-186 to Ser-206. These fragments were discovered based on analysis of antigenicity plots. Additionally interesting portions of LP273 are segments that were discovered based on a PROSITE analysis to be zinc-binding-like regions or zinc carboxypeptidase-like motifs: Lys-99 to Phe-121 (KIIWMDCGIHAREWIAPAFCQWF); Tyr-50 to Ile-90; Lys-99 to Trp-112; His-129 to Arg-169; Asn-173 to Asn-187; Leu-76 to Leu-88; Lys-99 to Ile-113; Gly-179 to Asn-187; and Tyr-50 to Gln194. Generally, proteins possessing such motifs appear to be structurally and functionally related members of the following family of zinc carboxypeptidases: Carboxypeptidase A1 (EC 3.4.17.1), is a pancreatic digestive enzyme that can removes all C-terminal amino acids with the exception of Arg, Lys and Pro; Carboxypeptidase A2 (EC 3.4.17.15), is a pancreatic digestive enzyme with a specificity similar to that of carboxypeptidase A1, but with a preference for bulkier C-terminal residues; Carboxypeptidase B (EC 3.4.17.2), is also a pancreatic digestive enzyme, but it preferentially removes C-terminal Arg and Lys; Carboxypeptidase N (EC 3.4.17.3) (also known as arginine carboxypeptidase), is a plasma enzyme that protects the body from potent vasoactive and inflammatory peptides containing C-terminal Arg or Lys (such as kinins or anaphylatoxins) which are released into the circulation; Carboxypeptidase H (EC 3.4.17.10) (also known as enkephalin convertase or carboxypeptidase E), is an enzyme located in secretory granules of pancreatic islets, adrenal gland, pituitary and brain (typically, this enzyme removes residual C- terminal Arg or Lys remaining after initial endoprotease cleavage during prohormone processing); Carboxypeptidase M (EC 3.4.17.12), is a membrane bound Arg and Lys specific enzyme that normally acts on peptide hormones at local tissue sites where it could control their activity before or after interaction with specific plasma membrane receptors; Mast cell carboxypeptidase (EC 3.4.17.1), is an enzyme with a specificity to carboxypeptidase A, but found in the secretory granules of mast cells; *Streptomyces griseus* carboxypeptidase (Cpase SG) (EC 3.4.17), which combines the specificities of mammalian carboxypeptidases A and B (Narahashi Y. J., 1990 Biochem. 107:879-886); *Thermoactinomyces vulgaris* carboxypeptidase T (EC 3.4.17.18) (CPT), which also combines the specificities of carboxypeptidases A and B (Teplyakov, et al., 1992 Eur. J. Biochem. 208:281-288); AEBP1, a transcriptional repressor active in preadipocytes, which seems to regulate transcription by cleavage of other transcriptional proteins (He, et al., 1995 Nature 378:92-96); and Yeast hypothetical protein YHR132c. Note that these proteins belong to families M14A/M14B in the classification of peptidases (Rawlings & Barrett 1995 Meth. Enzymol. 248:183-228). Typically, all of the enzymes in this family of proteins bind an atom of zinc. Three conserved residues are implicated in the binding of the zinc atom: two histidines and a glutamic acid. Two signature motifs have been discovered that will typically contain these three zinc-ligands: one consensus pattern is (PK)-x-(LIVMFY)-x-(LIVMFY)-x(4)-H-(STAG)-x-E-x-(LIVM)- (STAG)-x(6)- (LIVMFYTA) where H and E are the zinc ligands. Typically, practically all sequences exhibiting this pattern belong to a member of the family described above. A second consensus pattern is the following: H- (STAG)-x(3)- (LIVME)-x(2)- (LIVMFYW)-P- (FYW) where H is a zinc ligand. Typically, practically all sequences exhibiting this pattern belong to a member of the family described above. Generally, if a protein includes both signature motifs, the probability of it being a eukaryotic zinc carboxypeptidase approaches 100%. Further, particularly interesting LP273 secondary structures (e.g., such as a helix, a strand, or a coil) are the following LP273 helical structures: Leu-4 to Leu-8; Arg-23 to Arg-26; Ile-56 to Val-70; Pro-115 to Gln-127; Ser-134 to Asn-141; Trp-190 to Lys-199; Leu-205 to Met-207; Met-210 to Lys-211; Gln-278 to Leu-284; and Arg-290 to Lys-294. Particularly interesting coil structures are Pro-16 to Gly-201 Kts-35 to Trp-45; Trp-80 to Pro-84; Ser-91 to Lys-98; Cys-105 to His-108; His-129 to Asp-131; Leu-142 to Phe-144; Asn-151 to Tyr-155; Thr-160 to Thr-161; Ser-168 to Phe-186; Ile-216 to Gly-217; Phe-231 to Gly-233; Pro-240 to Pro-243; Gly-249 to Asp-262; Gly-285 to Pro-288; and Ala-296 to Ser-310. Particularly interesting strands are Tyr-87 to Ile-90; Lys-99 to Met-103; Tyr-145 to Leu-147; Ile-156 to Thr-158; and Val-235 to Ala-237. Further encompassed by the invention are contiguous amino acid residue combinations of any of the predicted secondary structures described above. For example, one helix-coil-strand-coil-strand motif of LP273 combines the Ser-134 to Asn-141 helix, with the Leu-142 to Phe-144 coil; with the Tyr-145 to Leu-147 strand; Asn-151 to Tyr-155 coil, and the Ile-156 to Thr-158 strand to form an interesting fragment of contiguous amino acid residues from Ser-134 to Tyr-155. Other combinations of contiguous amino acids are contemplated as can be easily determined.

### FEATURES OF LP NO: 11 (LP277)

LP277 (CPAh2; human carboxypeptidase 2) is an alternatively spliced version of LP190 (CPAh; human carboxypeptidase). LP190 was previously disclosed in provisional application USSN 60/241,813, which was filed 19-OCT-2000. LP277 differs from LP190 due to an insertion of an additional exon that is missing from LP190. The translation of the additional exon results in the production of a truncated protein because of an early stop codon. The N-terminal portion of LP277 (from about Met-1 to about Lys-178) shows sequence identity with a similar portion of LP190. However, due to the insertion of the additional exon (absent from LP190), the C-terminal portion of LP277 is a distinct fragment of about 10 amino acid residues (from about Gly-179 to about Val-188). Analysis of an alignment comparing LP277 with human carboxypeptidase A1 protein (GenBank Accession No. X67318) and LP190, suggests that LP277 is characterized as having a signal peptide portion about 33 amino acid residues in length (from about Met-1 to about Gly-33), an activation peptide portion about 74 amino acid residues in length (from about Gln-34 to about Ile-107), and a pseudo-proteinase domain portion about 81 amino acid residues in length (from about Lys-108 to about Val-188). The pseudo-proteinase domain fragment of LP277 results from the insertion of the extra exon since its translation produces a truncated protein. In comparison with LP190, LP277 is missing specific amino acid residues that are essential to carboxypeptidase functioning. LP190 contains residues Arg-198, Asn-271, Arg-272, Glu-300, Tyr-325, and Try-375, which are believed to be necessary for substrate binding during carboxypeptidase activity. In contrast, truncation of LP277 aborts these residues. Consequently, lacking an intact proteinase domain, LP277 appears to be an inactive carboxypeptidase, however, LP277 may function as a modulator of endogenous carboxypeptidase activity (as described herein) either by competing for substrate binding with other functioning carboxypeptidases or by competing for conversion to an activated state. Testing a protein for carboxypeptidase activity is routine and would not require undue experimentation given the teachings supplied herein (e.g., as to the LP277 sequence) and given teachings in the art for methods of determining whether a suspected protein has carboxypeptidase activity (see, e.g., Skidgel, R. A., "Assays for Arginine/Lysine Carboxypeptidase" 1991 Methods in Neurosciences (6)373-385; Thomas, et al., "An Improved Spectrophotometic Assay for Human Plasma Carboxypeptidase N" 1980 Analytical Biochemistry (108) 348-353; and Thomas, et al., "Comparative Studies on Human Carboxypeptidase B and N" 1979 Archives of Biochemistry and Biophysics (197) 487-492). Consequently, compositions comprising LP277 polypeptides (fragments thereof), polynucleotides (fragments thereof), and/or antibodies, activators and inhibitors of LP277 will be useful comprising a composition for the diagnosis, prognosis, and/or treatment of conditions, syndromes, disorders, or pathological states involving carboxypeptidase activity. Particularly, in conditions, syndromes, disorders, or pathological states involving the expression of LP277 (such as, e.g., in the cardiovascular system, the immune system, and ovarian tumors). Additionally, sequence encoding LP277 polypeptide has been localized to chromosome 7q31. Moreover, the following diseases, conditions, syndromes, disorders, or pathological states have also been mapped to this region of the human chromosome: autism (Warburton, et al., 2000 Am J Med. Genet 96(2):228-234); speech and language disorder (Lai, et al., 2000 Am J Hum Genet 67(2):357-68); cancer (Zenklusen, et al., 2000 Oncogene 19(13):1729-1730) such as, for example, prostate cancer (Latil, et al., 1995 Clin Cancer Res. (11):1385-1389) and breast cancer (Devilee, et al., 1997 Genes Chromosomes Cancer 18(3):193-199); fluctuating sensori-neural hearing loss (Abe, et al., 1999 Am J Med. Genet 82(4):322-328); and congenital chloride diarrhea (Hoglund, et al., 1996 Genome Res. 1996 Mar;6(3):202-210). Accordingly, an isolated and/or recombinant DNA molecule of LP277 (or fragment thereof) meets the statutory utility requirement of 35 U.S.C. 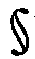 101 as it can be used to hybridize near one or more of the above stated diseases and thus serve as a marker for a such a disease gene. Additionally, compositions comprising LP277 polypeptides or polynucleotides (or fragments thereof), LP277 agonists or antagonists, and/or binding compositions (e.g., LP277 antibodies) will also be useful for diagnosis, prognosis, treatment, amelioration, and/or intervention of a disease, condition, or state associated with such an above referenced disease. It has been discovered that LP277 sequences (SEQ ID NO: 21) are expressed primarily in the following number of LIFESEQ GOLD™ database tissue and cDNA libraries: Cardiovascular System 1/68; Exocrine System 1/64; Genitalia, Female 1/106; Genitalia, Male 1/114; Hemic and Immune System 2/159; and Skin 1/15. LP277 was cloned from testis tissue, accordingly, compositions comprising LP277: polypeptides (or fragments thereof), polynucleotides (or fragments thereof), and/or antibodies are also useful for the treatment of defects in or wounds to tissues including, for example, but not limited to the reproductive organs.
Table 10: Primate, e.g., human, LP277 polynucleotide sequence (SEQ ID NO: 21) and corresponding polypeptide (SEQ ID NO: 22). The ORF for LP277 is 76-642 bp (with the start (ATG) and stop codons (TAG) identified in bold lettering in case numbering is misidentified one skilled in the art could determine the open reading frame without undue experimentation).

LP277 DNA sequence (1770 bp)(ORF = 76-642):
ATGCAGGGCACCCCTGGAGGCGGACGCGCCCTGGCCATCCCCCGTGGACAGGCGGACACTCCTGGTCTTC
AGCTTTATCCTGGCAGCAGCTTTGGGCCAAATGAATTTCACAGGGGACCAGGTTCTTCGAGTCCTGGCCAAA
GATGAGAAGCAGCTTTCACTTCTCGGGGATCTGGAGGGCCTGAAACCCCAGAAGGTGGACTTCTGGCGTGGC
CCAGCCAGGCCCAGCCTCCCTGTGGATATGAGAGTTCCTTTCTCTGAACTGAAAGACATCAAAGCTTATCTG
GAGTCTCATGGACTTGCTTACAGCATCATGATAAAGGACATCCAGGTGCTGCTGGATGAGGAAAGACAGGCC
ATGGCGAAATCCCGCCGGCTGGAGCGCAGCACCAACAGCTTCAGTTACTCATCATACCACACCCTGGAGGAG
ATATATAGCTGGATTGACAACTTTGTAATGGAGCATTCCGATATTGTCTCAAAAATTCAGATTGGCAACAGC
TTTGAAAACCAGTCCATTCTTGTCCTGAAGGGGCCATGGTCTTCTCTCTCTAATGTTGTCTGA

LP277 Full-Length Sequence (188aa):
>ds36416+1_ORF1 (SEQ ID NO: 22)  The underlined portion is a predicted
signal sequence (Met-1 to Gly-33).  A predicted SP cleavage site is
between Gly-33 and Gln-34 indicated as follows: 1
MQGTPGGGTRPGPSPVDRRTLLVFSFILAAALG^QM 35

MQGTPGGGTRPGPSPVDRRTLLVFSFILAAALGQMNFTGDQVLRVLAKDEKQLSLLGDLEGLKPQKVDFWRG
PARPSLPVDMRVPFSELKDIKAYLESHGLAYSIMIKDIQVLLDEERQAMAKSRRLERSTNSFSYSSYHTLEE
IYSWIDNFVMEHSDIVSKIQIGNSFENQSILVLKGPWSSLSNVV*
[?]

LP277 Mature Sequence (155aa):
LP277 DNA contains an additional exon when compared with LP190.A
predicted mature LP277 is as follows:

QMNFTGDQVLRVLAKDEKQLSLLGDLEGLKPQKVDFWRGPARPSLPVDMRVPFSELKDIKAYLESHGLAYS
IMIKDIQVLLDEERQAMAKSRRLERSTNSFSYSSYHTLEEIYSWIDNFVMEHSDIVSKIQIGNSFENQSIL
VLKGPWSSLSNVV*

Particularly interesting portions or fragments of the full length LP277 polypeptide (SEQ ID NO: 22) include, e.g., a predicted signal sequence from Met-1 to Gly-33. Additionally interesting segments of LP277 are fragments Gly-7 to Asp-17; Thr-20 to Gln-34; Leu-62 to Ser-77; Gly-100 to Gln-111; Glu-117 to Asn-132; Ser-133 to Ser-147; Phe-26 to Asp-40; Arg-44 to Gly-57; Leu-59 to Gly-72; Pro-76 to Phe-86; Glu-88 to Gly-100; Tyr-136 to Ile-149; Asn-151 to Ile-163; Gly-7 to Arg-19; Thr-20 to Lys-32; Gly-33 to Val-42; Asp-58 to Phe-69; Arg-71 to Arg-83; Phe-86 to Leu-101; Leu-114 to Arg-125; Arg-126 to His-140; Val-153 to Ile-163; and Gln-164 to Ser-173, whose discovery was based on an analysis of hydrophobicity, hydropathicity, and hydrophilicity plots. Additional interesting sections of LP277 are the portions of the polypeptide from Gly-7 to Arg-18; Ile-27 to Gln-41; Val-42 to Ser-54; Pro-73 to Glu-88; Leu-89 to His-99; Gly-100 to Leu-113; Glu-143 to Glu-155; Ser-157 to Asn-171; Glu-172 to Ser-182; Thr-9 to Arg-18; Asp-40 to Lys-48; Lys-51 to Gly-61; Lys-63 to Pro-76; Leu-78 to Glu-88; Glu-88 to Ser-98; Glu-116 to Glu-128; Leu-127 to His-140; His-156 to Asn-167; and Asp-115 to Ser-130. These fragments were discovered based on analysis of antigenicity plots. An additionally interesting portion of LP273 is a segment that was discovered based on a sequence analysis (Pfam) to be a zinc carboxypeptidase-like motif: Tyr-139 to Lys-178 (YHTLEEIYSWIDNFVMEHSDIVSKIQIGNSFENQSILVLK). Generally, proteins possessing such a motif appear to be structurally and functionally related to members of the zinc carboxypeptidase family (as described above). Further, particularly interesting LP277 secondary structures (e.g., such as a helix, a strand, or a coil) are the following LP277 helical structures: Leu-22 to Ala-30; Gln-41 to Ala-47; Glu-50 to Leu-55; Leu-89 to Leu-96; Met-106 to Glu-128; and Leu-142 to Phe-152. Particularly interesting coil structures are Met-1 to Arg-18; Gln-34 to Gly-39; Gly-61 to Gln-65; Trp-70 to Pro-85; His-99 to Leu-101; Ser-130 to Tyr-139; His-156 to Asp-158 Gly-166 to Asn-171; and Gly-179 to Val-188. Particularly interesting strands are Ser-161 to Gln-164. Further encompassed by the invention are contiguous amino acid residue combinations of any of the predicted secondary structures described above. For example, one helix-coil-helix motif of LP277 combines the Met-106 to Glu-128 helix, the Ser-130 to Tyr-139 coil; and the Leu-142 to Phe-152 helix to form an interesting fragment of contiguous amino acid residues from Met-106 to Phe-152. Other combinations of contiguous amino acids are contemplated as can be easily determined.

### FEATURES OF LP NO: 12 (LP287)

LP287 is an alternatively spliced version of LP281 (described above). LP287 exhibits similarity at the amino acid level to members of the latent TGF-beta-binding family of proteins (LTBPs) (see, e.g., Saharinen, et al. 1998 J Biol. Chem. 273(29): 18459-18469 and Oklu & Hesketh 2000 Biochem J 352 Pt 3:601-610), delta and notch type proteins (see, e.g., WO200006726-A2, which was filed 12-JUL-1999 from 99WO-US15710) and members of the fibulin protein family (fibulins are secreted glycoproteins found in association with extracellular matrix structures such as connective tissue fibers, basement membranes and blood clot)s. These associations are attributed to the ability of fibulins to interact with other extracellular matrix proteins such as fibronectin, laminins, nidogen, perlecan, fibrillin and fibrinogen suggesting that fibulins play a role in the formation and/or stabilization of extracellular matrix structures as well effecting cellular behavior (e.g., motility, metastasis, etc.). One similarity between LTBPs, Delta and Notch related proteins, and fibulin is the presence in these types of molecules of EGF-like domains. For example, Ca2+ binding epidermal growth factor-like repeats contained in predicted protein structures are characteristic of the extracellular matrix proteins, fibrillin and fibulin. EGF-like domains have been shown to be important for protein-protein interactions, as exemplified by the association of Notch and its ligands Delta and Serrate via EGF domains (Rebay, et al. 1991 Cell 67, 687-699). Loss or malfunction of EGF-like domains has been shown to play a role in disease conditions (see below). Similar domains in LTBPs are believed to play a role as structural matrix proteins (see below). One common theme is the role of such extracellular like molecules in the regulation of vascular growth and maturation during development and in lesions of injured vessels. For example, in regenerating skin, fibulin-2 is a late component of the cutaneous microfibrillar apparatus with an earlier existence in a fibrillar matrix mediated by fibronectin suggesting that fibulins interact with both fibronectin fibrils and fibrillin microfibrils (see, e.g., Ragunath, et al. 1999 J Invest Dermatol 112(1):97-101).

LTBPs are involved in the assembly, secretion, and targeting of TGFs to sites at which TGFs are stored and/or activated, thus LTBPs play critical roles in controlling and directing the activity ofTGFs. LTBPs have been shown to bind and regulate TGF beta cytokines. Recent evidence suggests that the differential expression of LTBP isoforms occurs during the development of coronary heart disease, and that modulation of LTBP function ―and hence of TGF-beta activity― is associated with a variety of disease states (e.g., such as conditions of cell proliferation, for example, certain cancers) (see, e.g., Oklu & Hesketh 2000 Biochem J 352 Pt 3:601-610). LTBP levels are also altered in other pathological conditions, including solar elastosis, solar keratosis, and pseudoxanthoma elasticum, pancreatitis, rheumatoid arthritis, glomerulosclerosis, hypertension, muscular dystrophy, carcinoid heart disease, and tuberculous pleurisy (see, e.g., Oklu & Hesketh, supra and the references cited therein). Evidence also suggests that abnormal levels of TGF beta 1 in serum and in artial vessel walls are associated with the development of atherosclerotic type conditions (Grainger, et al. 1995 Nat. Med. (N.Y.) 1, 74-79). TGF beta 1 is postulated to be a protective cytokine that inhibits the development of vascular lesions and their progresive deterioration (see, e.g., Metcalfe and Grainger 1995 Biochem. Soc. Trans. 23, 403-406). Furthemore, the protective properties of drugs such as tamoxifen on the cardiovascular system of mice and monkeys and in breast cancer clinical trials may be derived in part from their capacity to increase the concentration of active TGF beta 1 in vessel walls (see, e.g., Williams, et al. 1997 Arterioscler. Thromb. Vasc. Biol. 17, 403-408; Grainger, et al. 1995 Nat. Med. (N.Y.) 1, 1067-1073; Reckless, et al. 1997 Circulation 95, 1542-1548; McDonald and Stewart 1991 Br. Med. J. 303, 435-437). Thus, control of TGF beta 1 activity, via LTPB-like molecules, is likely to be important in regulating vascular health. Accordingly, due to the possession of similar structural features (e.g., EGF-like or cysteine type domains) by LP281 or LP287, one skilled in the art could easily determine the usefulness of LTPB-like molecules (or fragments thereof) on the properties of drugs such as tamoxifen (e.g., by testing, for example, through competitive binding assays, the effect on the release of TGFs with tamoxifen and/or in combination with or without various concentrations of LP281 or LP287). Thus, the effect of tamoxifen like drugs could be tested using LP281 or LP287. LTBP molecules may also exert effects independently of those associated with TGF; for example, LTBPs may function as structural matrix proteins. This is exemplified by the close identity between LTBPs and members of the fibrillin family of extracellular matrix proteins. Mutations in fibrillin have been linked directly to Marfan's syndrome (MFS), a heritable disorder of connective tissue. MFS lies at one end of a phenotypic continuum, with people in the general population who have one or another of the features of MFS at the other end, and those with a variety of other conditions in between. Mutations in FBN1, the gene that encodes fibrillin-1, are responsible for MFS and (in a few patients) other disorders in the continuum. In addition to skeletal, ocular, and cardiovascular features, patients with MFS have involvement of the skin, integument, lungs, and muscle tissue. Over the past 30 years, evolution of aggressive medical and surgical management of the cardiovascular problems (especially mitral valve prolapse, aortic dilatation, and aortic dissection) has resulted in considerable improvement in life expectancy for patients with these conditions. The similarity of LTBPs with fibrillin at the amino acid level suggests that the anomalous expression of LTBPs (and LTBP-like proteins) may be associated with similar disease conditions. A unifying feature among fibrillin, LTBPs, LP281, LP287, and Delta related protein are similar domains and structural-like motifs (e.g., such as cysteine and EGF-like domains; see herein for descriptions of their locations in LP281 and LP287), whose loss or absence (e.g., through point mutations, frame shifts, exon deletions, etc.) has been shown to lead to disease conditions. For example, cysteine deletions in fibrillin are associated with Marfan's syndrome (Ades, et al. 1996 J. Med. Genet. 33, 665-671; Dietz, et al. 1993 Science 259, 680―683; and Liu, et al. 1997 Nat. Genet. 16, 328―329) while severe forms of the disease also result from mutations within EGF-like domains or from the skipping of exons that encode EGF-like domains (Liu, et al. 1996 Hum. Mol. Genet. 5, 1581-1587). These findings suggest variant forms of LP281 and LP287 lacking EGF-like domains or cysteines may also be involved in the development of similar disease conditions of extracellular matrices.

Sequence encoding LP287 polypeptide has been localized to chromosome 2. It has been discovered that LP287 sequences (SEQ ID NO: 23) are expressed primarily in the following number of LIFESEQ GOLD™ database tissue and cDNA libraries: Connective Tissue 1/47; Embryonic structures 1/21; Genitalia, Female 3/106; Genitalia, Male 3/114; Hemic and Immune System 5/159; Musculoskeletal System 3/47; Nervous System 3/198; and Skin 1 /15. LP281 is expressed in hemic and immune system (e.g., specifically, in spleen, lymph nodes, and thymus) and musculoskeletal system (e.g., specifically, in synovium, and cartilage). Consequently, based on these expression patterns, compositions comprising LP281 polypeptides (or fragments thereof), polynucleotides (or fragments thereof), and/or LP281 antibodies (or LP281 binding compositions) are also useful for the treatment of defects in or wounds to tissues and organs including, but not limited to, cartilage, bone, spleen, lymph nodes, thymus, connective tissue; and joints (e.g., arthritis).
Table 11: Primate, e.g., human, LP287 polynucleotide sequence (SEQ ID NO: 23) and corresponding polypeptide (SEQ ID NO: 24). The ORF for LP287 is 13-852 bp (with the start (ATG) and stop codons (TAG) identified in bold lettering in case numbering is misidentified one skilled in the art could determine the open reading frame without undue experimentation).

LP287 DNA sequence (1210bp)(ORF = 13-852):
LP287 ORF = 13-852 (start (atg) and stop (tga) codons are bold).

ATGGTGCCCAGCTCTCCGCGCGCTCTTCCTTCTGCTCCTGATCCTCGCCTGCCCCGAGCCGCGGGCTTCC
CAGAACTGTCTCAGCAAACAGCAGCTCCTCTCGGCCATCCGCCAGCTGCAGCAGCTGCTGAAGGGCCAGGAG
ACACGCTTCGCCGAGGGCATCCGCCACATGAAGAGCCGGCTGGCCGCGCTGCAGAACTCTGTGGGCAGGGTG
GGCCCAGATGCCCTTCCAGTTTCCTGCCCGGCTCTGAACACCCCCGCAGACGGCAGAAAGTTTGGAAGCAAG
TACTTAGTGGATCACGAAGTCCATTTTACCTGCAACCCTGGGTTCCGGCTGGTCGGGCCCAGCAGCGTGGTG
TGTCTTCCCAATGGCACCTGGACAGGGGAGCAGCCCCACTGTAGAGGTATCAGTGAATGCTCCAGCCAGCCT
TGTCAAAATGGTGGTACATGTGTAGAAGGAGTCAACCAGTACAGATGCATTTGTCCTCCAGGAAGGACTGGG
AACCGCTGTCAGCATCAGGCCCAGACTGCCGCCCCCGAGGGCAGCGTGGCCGGCGACTCCGCCTTCAGCCGC
GCGCCGCGCTGTGCGCAGGTGGAGCGGGCTCAGCACTGCAGCTGCGAGGCCGGATTCCACCTGAGCGGCGCC
GCCGGCGACAGCGTCTGCCAGGATGTGGATGAATGTGTGGGCCTGCAGCCGGTGTGCCCCCAGGGGACCACA

TGCATCAACACCGGTGGAAGCTTCCAGTGTGTCAGCCCTGAGTGCCCCGAGGGCAGCGGCAATGTGAGCTAC
GTGAAGACGTCTCCATTGTATTTTGGAGAGCCAGTCTTCCAGCTCTGA

LP287 Full-Length Sequence (279aa):
>ds37942Fibulin1h.con+1_ORF1 (SEQ ID NO: 24)
The underlined portion is a predicted signal sequence (Met-1 to Ala-
23). A predicted SP cleavage site is between Ala-23 and Ser-24
indicated as follows: 1 MVPSSPRALFLLLLILACPEPRA^SQ 25

MVPSSPRALFLLLLILACPEPRASQNCLSKQQLLSAIRQLQQLLKGQETRFAEGIRHMKSRLAALQNSVGRV
GPDALPVSCPALNTPADGRKFGSKYLVDHEVHFTCNPGFRLVGPSSVVCLPNGTWTGEQPHCRGISECSSQP
CQNGGTCVEGVNQYRCICPPGRTGNRCQHQAQTAAPEGSVAGDSAFSRAPRCAQVERAQHCSCEAGFHLSGA
AGDSVCQDVDECVGLQPVCPQGTTCINTGGSFQCVSPECPEGSGNVSYVKTSPLYFGEPVFQL*

LP287 Mature Sequence (256aa):
A predicted mature LP287 is as follows:

SQNCLSKQQLLSAIRQLQQLLKGQETRFAEGIRHMKSRLAALQNSVGRVGPDALPVSCPALNTPADGRKFG
SKYLVDHEVHFTCNPGFRLVGPSSVVCLPNGTWTGEQPHCRGISECSSQPCQNGGTCVEGVNQYRCICPPG
RTGNRCQHQAQTAAPEGSVAGDSAFSRAPRCAQVERAQHCSCEAGFHLSGAAGDSVCQDVDECVGLQPVCP
QGTTCINTGGSFQCVSPECPEGSGNVSYVKTSPLYFGEPVFQL*

Particularly interesting portions or fragments of the full length LP287 polypeptide (SEQ ID NO: 24) include, e.g., a predicted signal sequence from Met-1 to Ala-23. Additionally interesting segments of LP287 are fragments Lys-11 to Ser-24; Gln-25 to Leu-43; Leu-44 to Arg-61; Lys-59 to Ser-68; Gly-70 to Val-79; Cys-81 to Thr-106; Asn-108 to Cys-121; Gly-125 to Cys-145; Arg-159 to Ser-183; Phe-190 to Cys-205 Gly-246 to Ser-268; Leu-11 to Cys-27; Glu-47 to Glu-66; Ser-68 to Gly-90; Thr-106 to Asn-124; Pro-163 to Ser-183; Ala-193 to Phe-211; Asp-224 to Glu-246; Gly-260 to Leu-270; Ala-8 to Cys-18; Por-19 to Glu-32; Leu-43 to Leu-62; Ala-64 to Pro-78; Pro-82 to Leu-98; Asn-124 to Ile-137; Ser-138 to Cys-160; Gly-165 to Phe-190; Cys-196 to Phe-211; Ser-214 to Leu-231; Pro-233 to Cys-250; and Ser-252 to Pro-269, whose discovery was based on an analysis of hydrophobicity, hydropathicity, and hydrophilicity plots. Additional interesting sections of LP287 are the portions of the polypeptide from Ile-15 to Ser-24; Asn-26 to Gln-42; Glu-48 to His-57; Met-58 to Asn-67; Ser-68 to Val-79; Lys-92 to His-104; Asn-108 to Ser-118; Val-119 to Gly-136; Cys-140 to Val-155; Gly-154 to Cys-171; Ser-220 to Pro-233; Pro-236 to Pro-253; Pro-253 to Val-265; Pro-21 to Ser-35; Gln-41 to Arg-56; Lys-59 to Gly-70; Gly-70 to Cys-81; Ser-80 to Arg-91; Asn-85 to Tyr-97; Glu-130 to Cys-151; Arg-135 to Val-152; Thr-167 to Ala-185; Arg-192 to His-204; Arg-217 to Glu-227; Val-225 to Gln-232; Gly-238 to Ser-252; and Gln-249 to Val-262. These fragments were discovered based on analysis of antigenicity plots. A further interesting portion of LP287 (based on PROSITE analysis) is the segment Ala-8 to Cys-18 (ALFLLLLILAC), which has been discovered to be a a lipoprotein lipid attachment-like site (as previously described herein). Further interesting portions of LP287 are the segments: Cys-160 to Cys-171 (CICPPGRTGNRC), Cys-140 to Cys-171 (CSSQPCQNGGTCVEGVNQYRCICPPGRTGNRC), and Cys-228 to Pro-269 (CVGLQPVCPQGTTCINTGGSFQCVSPECPEGSGNVSYVKTSP) which have been discovered to be EGF-like domains. Additionally interesting segments of LP287 are the segments: Asp-224 to Cys-250 (DVDECVGLQPVCPQGTTCINTGGSFQC), Cys-134 to Cys-145 (CRGISECSSQPC), and Cys-151 to Arg-166 (CVEGVNQYRCICPPGR) which are all identified as a calcium-binding EGF-like domains. As in non-calcium binding EGF-like domains, there are six conserved cysteines and the structure of both types of domains is very similar since calcium-binding induces only strictly local structural changes (see, e.g., Selander-Sunnerhagen, et al. 1992 J. Biol. Chem. 267:19642-19649). Deletions in LTPBs of calcium binding sites and EGF-like domains have been suggested to be associated with, respectively, increased LTPB degradation and anomalous targeting of TGF beta; both of which may cause disease conditions (Oklu and Hesketh, 2000 Biochem. J. 352, 601-610). Loss of similar domains in LP287 may have similar effects (see also, the discussion above for LP281 domains). Further interesting segments of LP287 are the segments: Gly-136 to Asn-147 (GISECSSQPCQN), Gly-148 to Val-155 (GGTCVEGV), and Asn-156 to Arg-166 (NQYRCICPPGR), which are all identified as Type II EGF-like signatures (aspartic acid or asparagine residues in these Type II EGF-like signatures of a number of proteins typically undergo post-translational hydroxylation to form either erythro-beta-hydroxyaspartic acid or erythro-beta-hydroxyasparagine; see, e.g., Stenflo, et al. 1988 J. Biol. Chem. 263:21-24). An additionally interesting portion of LP287 is the segment: Cys-81 to Cys-134 (CPALNTPADGRKFGSKYLVDHEVHFTCNPGFRLVGPSSVVCLPNGTWTGEQPHC ), which has been discovered to be a sushi-like domain (see, e.g., Ichinose, et al. 1990 J Biol. Chem. 265:13411-13414; Kato & Enjyoji 1991 Biochemistry 30:11687-11694). Further, particularly interesting LP 277 secondary structures (e.g., such as a helix, a strand, or a coil) are the following LP287 helix structures: Arg-7 to Leur-14; Cys-27 to Leu-34; Thr-49 to Gln-66; and Arg-195 to Arg-201. Particularly interesting coil structures are Met-1 to Ser-4; Ala-17 to Ser-24; Gly-70 to Ser-95; Cys-109 to Phe-111; Gly-115 to Ser-117; Pro-123 to Gly-149; Val-155 to Asn-156; Cys-162 to Arg-170; Ala-178 to Ser-188; Ser-206 to Gly-210; Ser-214 to Val-221; and Asp-226 to Cys-229. Particularly interesting strands are Tyr-97 to Leu-98; Val-103 to Phe-105; Val-119 to Cys-121; Arg-159 to Cys-160; Thr-240 to Ile-242; Glu-249 to Cys-250; and Ser-263 to Val-265. A particularly interesting coiled coil exists from Ser-29 to Arg-56. Further encompassed by the invention are contiguous amino acid residue combinations of any of the predicted secondary structures described above. For example, one coil-strand-strand-coil-coil-strand-coil motif of LP287 combines the Gly-70 to Ser-95 coil; with the Tyr-97 to Leu-98 strand; the Val-103 to Phe-105 strand, the Cys-109 to Phe-111 coil, with the Gly-115 to Ser-117 coil, the Val-119 to Cys-121 strand, and the Pro-123 to Gly-149 coil to form an interesting fragment of contiguous amino acid residues from Gly-70 to Gly-149. Other combinations of contiguous amino acids are contemplated as can be easily determined.

### FEATURES OF LP NO: 13 (LP209)

LP209 is a novel composition that exhibits sequence similarity to members of the OX2 receptor/ligand protein family. LP209 may exist in both a soluble (a 264 amino acid extracellular portion is indicated) and cell bound form (a putative transmembrane is indicated and hydrophobic fragments exist near both the N- and C-terminal regions suggesting that LP209 exists in a membrane bound receptor-like configuration). Other examples of receptors that also have naturally soluble forms exist in the literature (see, e.g., TNFR-1). OX2 is membrane glycoprotein containing two immunoglobulin superfamily (IgSF) domains that interacts with other cell surface proteins. OX2 has single transmembrane domain with a short cytoplasmic tail. OX2 receptor/ligand interactions play a regulatory role in immune/inflammatory processes apparently through interactions of the IgSF-like domains on the OX2 ligand and OX2 receptor. OX2 protein is expressed in recirculating B-cells, activated T-cells, dedritic cells, vascular endothelium, degenerating corpora lutea and both central and peripheral neurons. Additionally, OX2 is an important regulator of cells of the macrophage lineage. In mice lacking OX2, macrophage-type cells (including brain microglia) are more numerous and they exhibit an activated phenotype. After facial nerve transection, damaged OX2-deficient neurons elicit an accelerated microglial response. Moreover, in the CNS, lack of OX2 results in a more rapid onset of experimental autoimmune encephalomyelitis (EAE). While outside the brain, disruption of OX2 ligand-OX2 receptor interaction hastens susceptibility to collagen-induced arthritis (CIA) in mice that are normally resistant to this disease. Overall, these data suggest that OX2 functions in a variety of tissues as an inhibitory regulator for cells of the macrophage lineage. Consequently, prevention of OX2 receptor/ligand interactions exacerbates diseases, conditions, or syndromes involving immune and/or inflammatory mediated activity (especially, in activities mediated by cells of the macrophage lineage, such as, for example, Gauchers disease, Hodgkins disease, rheumatoid arthritis, encephalomyelitis, neural trauma, HIV disease, Krabbe disease, allergic disease, cardiovascular disease (e.g.,atherosclerosis, Coats Disease, Lebers miliary aneurysm), respiratory disease (e.g., asthma), Chrons disease, Kawasaki disease, ect.). LP209 was tested in various assay systems that have been designed to assign functions to novel proteins using a variety of cell-based techniques. Briefly, in assays testing cell proliferation (similar to those described herein), various cell types are sub-maximally stimulated using fetal bovine serum (FBS), and the effect of an LP (such as here, for example, LP209) on the growth rate of various cell types is tested by comparing the growth of specific cells with (or without) the addition of varing concentrations of the LP. This type of assay allows one skilled in the art to determine whether LP209 has a positive or negative effect on cell growth rate. The results of such testing indicated that LP209 influenced both cell cycle and cell proliferation. LP209 was shown to increase proliferation in both a human prostate cell line (LNCaP) and in a human ovarian cell line (PA1). Additionally, in these assays, LP209 was shown to have an inhibitory effect on the proliferation of human vein endothelial cells (HUVEC) when compared to a control buffer. These data suggest that LP209 could have a negative regulatory role in cell cycle progression. Given the similarity between OX2 and LP209, and the discovery of specific LP209 features (e.g., such as, the presence of hydrophobic fragments near both the N- and C-terminal regions) these data also suggest that LP209 ligand may be expressed on the surface of HUVEC cells (OX2 is a transmembrane receptor protein), as a cell surface receptor. Endothelial cells can play a very active role in a variety of inflammatory processes; their activation & proliferation often induces them to produce cytokines which are mediators of inflammation. Thus, down-regulation of the proliferation of HUVEC cells may indicate that LP209 plays a role in the down-regulation of endothelial cell activation. Down-regulation of endothelial cell activation has multiple implications in regulation of a variety of inflammatory diseases and in the treatment of diseases of cell proliferation (e.g., cancers) via the inhibition of angiogenesis, since endothelial cell proliferation is an important component of angiogenesis. For testing AKT or ERK kinase activity, various cell types are stimulated with an LP (e.g., here either LP209 or a positive control stimulant) or left unstimulated. Cells are then lysed and the lysates run on an SDS-PAGE gel. Subsequently, the proteins are transferred from the gel to nitrocellulose membranes. These membranes are then probed with antibodies specific for phosphorylated AKT or phosphorylated ERK kinases since phosphorylated forms of these kinases are indicative of the active forms of these enzymes. The results of assays testing for the presence of kinase activity (similar to assays described herein) indicate LP209 induces the activity of AKT kinase in a human erythroblast cell line (TF-1). Furthermore, in human astrocytes (U373), LP209 induced ERK kinase activity. Together, these data indicate that LP209 can signal, respectively, via a protein Kinase C pathway and an ERK/MAP kinase pathway.

Applicants used the LP209 sequence to create a soluble LP209 fusion protein (LP209-Fc) by joining 264 amino acids of a predicted extracellular domain of LP209 (Met-1 to Ala-264) to the following human Fc region

(IEPKSCDKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISKAK GQPREPQEYTLPPSREEMTKNQVSLTCLVKGFYPSDIAVEWESNGQPENNYKTTPPVLDSDGSFFL YSKLTVDKSRWQQGNVFSCSVMHEALHNHYTQKSLSLSPGKRI). LP209-Fc differs from

LP209-Fc-FLIS by lacking the flag antibody epitope-Hexahsitidine ("FLIS" tag)(Tyr-501 to His-515). LP209-Fc is useful, for example, as an antagonist or agonist of cells of the macrophage lineage that respond to signaling via an OX2-like pathway. For example, as an antagonist, soluble LP209-Fc would compete with the natural LP209 ligand on LP209 presenting cells to inhibit functioning OX2-like LP209 receptor/ligand complexes from forming, thus preventing inhibitory signals in cells of the macrophage lineage and allowing, for instance, macrophage activation. For instance, virally infected cells (such as, e.g., cells infected with herpes simplex) escape immune surveillance by expressing OX2-like proteins to inhibit activation of immune cells or to prevent macrophage-secreted signaling molecules (e.g., cytokines and chemokines) from orchestrating an immune response. However, LP209-Fc could compete with a virally infected cell presentation of OX2-like molecules thus preventing a virally directed inhibition of immune cells (e.g., like those in the macrophage lineage). Similarly, in situations where cells LP209 presenting cells are lacking or absent (e.g., in autoimmune related conditions), soluble LP209-Fc could act as an agonist to activate inhibitory signaling through an LP209 (OX2-like) receptor/ligand complex and thus dampen an unwanted immune response. It has been discovered that LP209 sequence (SEQ ID NO: 25) are expressed in the following number of LIFESEQ GOLD™ database tissue and cDNA libraries: Cardiovascular System 1 /68; Exocrine Glands 2/64; Genitalia, Female 1/106; Hemic and Immune System 2/159; Musculoskeletal System 1/47; and Respiratory System 1/93. LP209 is expressed in the hemic and immune system (e.g., specifically, in spleen, lymph nodes, and thymus) and musculoskeletal system (e.g., specifically, in synovium, and cartilage). Additionally, a Northern blot (the membrane is the CLONTECH Human 12-lane MTN blot) was performed using full-length LP209 with DIG labeling. The results of the Northern blot demonstrated a strong signal from liver of approximately 1.9kb in size. An additional longer exposure demonstrates other LP209 bands at approximately 2.4kb and 4.2kb, which were seen only in spleen and placenta indicating that alternative spliced mRNA versions of LP209 exist (see below, LP209b). Consequently, based on these expression patterns, compositions comprising LP209 polypeptides (or fragments thereof), polynucleotides (or fragments thereof), and/or LP209 antibodies (or LP209 binding compositions) are also useful for the treatment of defects in or wounds to tissues and organs including, but not limited to, cartilage, bone, spleen, lymph nodes, thymus, connective tissue; and joints (e.g., arthritis).
Table 12: Primate, e.g., human, LP209 polynucleotide sequence (SEQ ID NO: 25) and corresponding polypeptide (SEQ ID NO: 26). The ORF for LP209 is 257-1303 bp (with the start (ATG) and stop codons (TAG) identified in bold typeface in case numbering is misidentified one skilled in the art could determine the open reading frame without undue experimentation).

LP209 Full-Length Sequence (348aa):
>btzz1528428-encoded polypeptide
The underlined portion is a predicted signal sequence (Met-1 to Ala-24).
A predicted SP cleavage site is between Ala-24 and Glu-25 indicated as
follows: 1 MLCPWRTANLGLLLILTIFLVAEA^E 25





*

An LP209 Mature Sequence (324aa):
Using the signal sequence as predicted, a correspondingly predicted
mature LP209 protein is as follows:

EGAAQPNNSLMLQTSKENHALASSSLCMDEKQITQNYSKVLAEVNTSWPVKMATNAVLCCPPIALRNLIIIT
WEIILRGQPSCTKAYRKETNETKETNCTDERITWVSRPDQNSDLQIRPVAITHDGYYRCIMVTPDGNFHRGY
HLQVLVTPEVTLFQNRNRTAVCKAVAGKPAAQISWIPEGDCATKQEYWSNGTVTVKSTCHWEVHNVSTVTCH
VSHLTGNKSLYIELLPVPGAKKSAKLYIPYIILTIIILTIVGFIWLLKVNGCRKYKLNKTESTPVVEEDEMQ
PYASYTEKNNPLYDTTNKVKASQALQSEVDTDLHTL*

An LP209 Cell Membrane Sequence (348aa):
The underlined portion indicates a predicted transmembrane domain
(YIPYIILTIIILTIVGFIWLLKVNGCRKY; Tyr-267 to Tyr-295). The double
underlined portion
(LNKTESTPVVEEDEMQPYASYTEKNNPLYDTTNKVKASQALQSEVDTDLHTL) indicates a
predicted cytoplasmic portion.

MLCPWRTANLGLLLILTIFLVAEAEGAAQPNNSLMLQTSKENHALASSSLCMDEKQITQNYSKVLAEVNTSW
PVKMATNAVLCCPPIALRNLIIIITWEIILRGQPSCTKAYRKETNETKETNCTDERITWVSRPDQNSDLQIRP
VAITHDGYYRCIMVTPDGNFHRGYHLQVLVTPEVTLFQNRNRTAVCKAVAGKPAAQISWIPEGDCATKQEYW
SNGTVTVKSTCHWEVHNVSTVTCHVSHLTGNKSLYIELLPVPGAKKSAKLYIPYIILTIIILTIVGFIWLLK
VNGCRKYKLNKTESTPVVEEDEMQPYASYTEKNNPLYDTTNKVKASQALQSEVDTDLHTL*

An LP209 Extracellular Sequence (264aa):

MLCPWRTANLGLLLILTIFLVAEAEGAAQPNNSLMLQTSKENHALASSSLCMDEKQITQNYSKVLAEVNTSW
PVKMATNAVLCCPPIALRNLIIITWEIILRGQPSCTKAYRKETNETKETNCTDERITWVSRPDQNSDLQIRP

VAITHDGYYRCIMVTPDGNFHRGYHLQVLVTPEVTLFQNRNRTAVCKAVAGKPAAQISWIPEGDCATKQEYW
SNGTVTVKSTCHWEVHNVSTVTCHVSHLTGNKSLYIELLPVPGAKKSA

LP209-Fc-FLIS Construct (515aa):
A Flag-tagged/His-tagged ("FLIS") version of the first 264 amino acids
of a predicted extracellular domain of LP209 (Met-1 to Ala-264)is shown
below. A predicted signal sequence is from Met-1 to Ala-24. Predicted
glycosylation sites are indicated in italics. The human Fc region of
the construct is indicated by underlining (Ile-265 to Ile-499). The
flag antibody epitope-Hexahsitidine ("FLIS" tag) is indicated by double
underlining (Tyr-501 to His-515).

MLCPWRTANLGLLLILTIFLVAEAEGAAQPNNSLMLQTSKENHALASSSLCMDEKQITQNYSKVLAEVNTSW
PVKMATNAVLCCPPIALRNLIIITWEIILRGQPSCTKAYRKETNETKETNCTDERITWVSRPDQNSDLQIRP
VAITHDGYYRCIMVTPDGNFHRGYHLQVLVTPEVTLFQNRNRTAVCKAVAGKPAAQISWIPEGDCATKQEYW
SNGTVTVKSTCHWEVHNVSTVTCHVSHLTGNKSLYIELLPVPGAKKSAIEPKSCDKTHTCPPCPAPELLGGP
SVFLFPPKPKDTLMISRTPEVTCVVVDVSHEDPEVKFNWYVDGVEVHNAKTKPREEQYNSTYRVVSVLTVLH
QDWLNGKEYKCKVSNKALPAPIEKTISKAKGQPREPQEYTLPPSREEMTKNQVSLTCLVKGFYPSDIAVEWE
SNGQPENNYKTTPPVLDSDGSFFLYSKLTVDKSRWQQGNVFSCSVMHEALHNHYTQKSLSLSPGKRIDYKDD
DDKHVHHHHHH*

Particularly interesting portions or fragments of the full length LP209 polypeptide include, e.g., a discovered putative signal-like sequence from Met-1 to Ala-24. Additionally interesting portions of LP209 are nine potential glycosylation sites: Asn-31 to Ser-33; Asn-60 to Ser-62; Asn-69 to Ser-71; Asn-116 to Thr-118; Asn-122 to Thr-124; asn-185 to Thr-187; Asn-218 to Thr-220; Asn-233 to Ser-235; and Asn-247 to Ser-249. Additionally interesting segments of LP209 are predicted transmembrane-like regions: Trp-5 to Ala-27; Arg-90 to Lys-113; and Tyr-267 to Tyr-295. Additionally interesting segments of LP209 are discovered fragments Gly-11 to Gln-29; Pro-30 to Glu-41; Ala-44 to Gln-56; Ile-57 to Thr-70; Ser-71 to Arg-102; Pro-105 to Asn-137; Trp-203 to His-228; Gly-259 to Asn-290; Cys-292 to Ala-315; Ser-316 to Ser-339; Gly-11 to Pro-30; Thr-70 To Pro-105; Ser-106 to Pro-134; Ala-146 to Met-157; Pro-160 To Thr-175; Gly-195 To Glu-214; Trp-216 to Trp229; Glu-2230 to His-243; Leu-244 to Leu-256; Pro-258 to Gly-291; Tyr-295 to Glu-312; Tyr-317 to Gln-338; Asn-9 to Val-21; Glu-23 to Leu-34; Leu-36 to Leu-50; Cys-50 to Leu-65; Leu-82 to Arg-102; Gly-103 to Thr-129; Val-131 to Arg-143; Phe-181 to Gln-200; Pro-05 to Ser-217; Ser-217 to Trp-229; His-230 to Leu-250; Leu-266 To Thr-279; Ile-280 to Val-289; Asn-298 to Pro-313; and Ser-316 to Ala-336, whose discovery was based on an analysis of hydrophobicity, hydropathicity, and hydrophilicity plots. Additional interesting sections of LP209 are the discovered portions of LP209 from Ala-8 to Gly-26; Thr-38 to Leu-50; Asn-69 to Cys-84; Pro-85 to Cys-107; Lys-119 to Ile-142; Alaa-146 to Phe-164; Pro-197 to Ser-217; Cys-227 to Leu-250; Ile-268 to Asn-290; Pro-304 to Glu-319; Leu-324 to Asp-342; Ala-27 to Asp-42; Ala-44 to Ile-57; Thr-58 to Val-64; Asn-69 to Thr-78; Lys-109 to Asp-125; Ser-138 to Ala-146; Thr-124 to Gln-136; Tyr-152 to Phe-163; Gly-162 to Leu-173; Gln-182 to Lys-196; Cys-209 to Thr-222; Lys-224 to Thr-236; Val-237 to Lys-248; Lys-262 to Asn-290; Tyr-295 to Lys-320; Lys-320 to Val-331; and Lys-330 to Gln-338. These fragments were discovered based on analysis of antigenicity plots. Further, particularly interesting LP209 secondary structures (e.g., such as a helix, a strand, or a coil) that have been discovered are the following LP209 helix structures: Thr-58 to Gln-59; and Tyr-111 to Arg-112. Particularly interesting discovered coil structures are Met-1 to Cys-3; Ala-27 to Asn-32; Ser-47 to Cys-51; Asn-69 to Lys-75; Cys-84 to Ile-87; Arg-102 to Ser-106; Glu-117 to Asp-125; Arg-133 to Asp-1339; Thr-148 to Gly-151; Thr-159 to Gly-167; Thr-175 to Glu-177; Gln-182 to Asn-185; Gln-195 to Ala-198; Pro-205 to Thr-211; Ser-217 to Gly-219; Asn-233 to Val-234; Leu-244 to Lys-248; Pro-256 to Ala-264; Val-289 to Arg-293; Asn-298 to Pro-304; Asp-309 to Tyr-314; Thr-318 to Thr-327; and Leu-345 to Leu-348. Particularly interesting discovered strand structures are: Ile-128 to Val-131; Gln-141 to Ile-142; Tyr-153 to Val-158; His-169 to Leu-173; Thr-179 to Phe-181; Gln-200 to Trp-203; Val-221 to Lys-224; Thr-236 to Val-241; Leu-250 to Leu-254; Leu-266 to Tyr-267; Trp-285 to leu-287; and Lys-294 to Leu-297. A particularly interesting coiled coil exists from Ser-24 to Glu-53. Further encompassed by the invention are contiguous amino acid residue combinations of any of the predicted secondary structures described above. For example, one strand-coil-strand-coil-strand-coil motif of LP209 combines the Tyr-153 to Val-158 strand; with the Thr-159 to Gly-167 coil, with the His-169 to Leu-173 strand; the Thr-175 to Glu-177 coil, the Thr-179 to Phe-181 strand, and the Gln-182 to Asn-185 coil to form an interesting fragment of contiguous amino acid residues from Tyr-153 to Asn-185. Other combinations of contiguous amino acids are contemplated as can be easily determined.

### FEATURES OF LP NO: 14 (LP209b)

LP209b is a novel variant of LP209 (described above). The LP209b polypeptide is encoded by an alternatively spliced mRNA of LP209. LP209b differs from LP209 by exhibiting a 24 amino acid residue deletion in the N-terminal portion (EAEGAAQPNNSLMLQTSKENHALA; corresponding to the Glu-23 to Ala-46 portion of full-length LP209) and by being truncated at an earlier amino acid residue than LP209 (see, Table 13 below). LP209b comprises primarily the extracellular portion of LP209 without the predicted transmembrane and cytoplasmic portions. Sequence encoding LP209b was found in a clone derived from metastatic breast tumor tissue. Accordingly, LP209b may be a natural secreted variant form of LP209 that has similar functions to those of LP209-Fc (e.g., such as soluble receptor that competes for LP209-like ligand thus blocking the function of the normal ligand-receptor pair).
Table 13: Primate, e.g., human, LP209b polynucleotide sequence (SEQ ID NO: 27) and corresponding polypeptide (SEQ ID NO: 28). The ORF for LP209b is 112-264 bp (with the start (ATG) and stop codons (TAG) identified in bold typeface in case numbering is misidentified one skilled in the art could determine the open reading frame without undue experimentation).

LP209b DNA sequence (265 bp) (ORF = 112-264):
LP209b (start (atg) codon is indicated in bold typeface and underlined).

ATGCTCTGCCCTTGGAGAACTGCTAACCTAGGGCTACTGTTGATTTTGACTATCTTCTTAGTGGCCGCTTCA
AGCAGTTTATGTATGGATGAAAAACAGATTACACAGAACTACTCGAAAGTACTCGCAGAAGTTAACACTTCA
TGGCCTGTAA

LP209b Full-Length Sequence (51aa):

MLCPWRTANLGLLLILTIFLVAASSSLCMDEKQITQNYSKVLAEVNTSWPV

LP209b exhibits sequence identity with Human Secreted Protein encoded by gene 85 SEQ ID NO:208 of W0200006698-A1. Particularly interesting portions or fragments of the full length LP209b polypeptide include discovered fragments Thr-7 to Ser-26; Leu-27 to Glu-44; Leu-10 to Ala-22; and Ala-23 to Lys-40, whose discovery was based on an analysis of hydrophobicity, hydropathicity, and hydrophilicity plots. Additional interesting sections of LP209b are the discovered portions of LP209b from Leu 12 to Val-21; Ser-26 to Asn-37; Glu-44 to Ser-48 and Ser-25 to Lys-40. These fragments were discovered based on analysis of antigenicity plots. Further, particularly interesting LP209b secondary structures (e.g., such as a helix, a strand, or a coil) that have been discovered are the following LP209b helix structures: Arg-7 to Thr-7; Leu-13 to Leu-16; and Glu-31 to Val-41. Particularly interesting discovered coil structures are Met-1 to Cys-3; Ser-24 to Met-29; and Ser-48 to Val-51. Further encompassed by the invention are contiguous amino acid residue combinations of any of the predicted secondary structures described above. For example, one coil-helix motif of LP209b combines the Ser-24 to Met-29 coil, and the Glu-31 to Val-41 helix to form an interesting fragment of contiguous amino acid residues from Ser-24 to Val-41. Other combinations of contiguous amino acids are contemplated as can be easily determined.

### FEATURES OF LP NO: 15 (LP209c)

LP209c is another novel variant of LP209 that also exhibits sequence similarity and/or identity to members of the OX2 receptor/ligand protein family. LP209c is also a splice variant of LP209 and is also characterized as a natural soluble variant of LP209. LP209c differs from LP209 by exhibiting a deletion of approximately 24 amino acids at the N-terminad portion of the sequence and a four amino acid difference just N-terminad to the transmembrane region of LP209. LP209c can be tested as described herein using any of the various assay systems designed to assign functions to novel proteins by employing any of a variety of cell-based techniques. Applicants used the LP209c sequence to create a soluble LP209c fusion protein (LP209c-Fc) by joining LP209c (Met-1 to Ala-264) to the following human Fc region:

(IEPKSCDKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISKAK GQPREPQEYTLPPSREEMTKNQVSLTCLVKGFYPSDIAVEWESNGQPENNYKTTPPVLDSDGSFFL YSKLTVDKSRWQQGNVFSCSVMHEALHNHYTQKSLSLSPGKRI). LP209c-Fc differs from LP209c-Fc-FLIS by lacking the flag antibody epitope-Hexahsitidine ("FLIS" tag)(Tyr-501 to His-515).

It has been discovered that LP209c sequence (SEQ ID NO: 29) is expressed in the following number of LIFESEQ GOLD™ database tissue and cDNA libraries: Cardiovascular System 1/68; Exocrine Glands 2/64; Genitalia, Female 1/106; Hemic and Immune System 2/159; Musculoskeletal System 1/47; and Respiratory System 1/93. LP209c is expressed in the hemic and immune system (e.g., specifically, in spleen, lymph nodes, and thymus) and musculoskeletal system (e.g., specifically, in synovium, and cartilage
Table 14: Primate, e.g., human, LP209c polynucleotide sequence (SEQ ID NO: 29) and corresponding polypeptide (SEQ ID NO: 30). The ORF for LP209c is 1-714 bp (with the start (ATG) and stop codons (TAG) identified in bold typeface in case numbering is misidentified one skilled in the art could determine the open reading frame without undue experimentation).

LP209c DNA sequence (714 bp) (ORF = 1-714):
LP209c (start (atg) and stop (tga) codons are indicated in bold typeface and underlined).

ATGCTCTGCCCTTGGAGAACTGCTAACCTAGGGCTACTGTTGATTTTGACTATCTTCTTAGTGGCCGCTTCA
AGCAGTTTATGTATGGATGAAAAACAGATTACACAGAACTACTCGAAAGTACTCGCAGAAGTTAACACTTCA
TGGCCTGTAAAGATGGCTACAAATGCTGTGCTTTGTTGCCCTCCTATCGCATTAAGAAATTTGATCATAATA
ACATGGGAAATAATCCTGAGAGGCCAGCCTTCCTGCACAAAAGCCTACAGGAAAGAAACAAATGAGACCAAG
GAAACCAACTGTACTGATGAGAGAATAACCTGGGTCTCCAGACCTGATCAGAATTCGGACCTTCAGATTCGT
CCAGTGGCCATCACTCATGACGGGTATTACAGATGCATAATGGTAACACCTGATGGGAATTTCCATCGTGGA
TATCACCTCCAAGTGTTAGTTACACCTGAAGTGACCCTGTTTCAAAACAGGAATAGAACTGCAGTATGCAAG
GCAGTTGCAGGGAAGCCAGCTGCGCAGATCTCCTGGATCCCAGAGGGCGATTGTGCCACTAAGCAAGAATAC
TGGAGCAATGGCACAGTGACTGTTAAGAGTACATGCCACTGGGAGGTCCACAATGTGTCTACCGTGACCTGC
CACGTCTCCCATTTGACTGGCAACAAGAGTCTGTACATAGAGCTACTTCCTGAAAATATAAATTGA

LP209c Full-Length Sequence (237aa):
The underlined portion is a predicted signal sequence (Met-1 to Ala-
22). A predicted SP cleavage site is between Ala-22 and Ala-23
indicated as follows: 1 MLCPWRTANLGLLLILTIFLVA^A 23

MLCPWRTANLGLLLILTIFLVAASSSLCMDEKQITQNYSKVLAEVNTSWPVKMATNAVLCCPPIALRNLIII
TWEIILRGQPSCTKAYRKETNETKETNCTDERITWVSRPDQNSDLQIRPVAITHDGYYRCIMVTPDGNFHRG
YHLQVLVTPEVTLFQNRNRTAVCKAVAGKPAAQISWIPEGDCATKQEYWSNGTVTVKSTCHWEVHNVSTVTC
HVSHLTGNKSLYIELLPENIN

An LP209c Mature Sequence (215aa):
Using the signal sequence as predicted, a correspondingly predicted
mature LP209c protein is as follows:

ASSSLCMDEKQITQNYSKVLAEVNTSWPVKMATNAVLCCPPIALRNLIIITWEIILRGQPSCTKAYRKETN
ETKETNCTDERITWVSRPDQNSDLQIRPVAITHDGYYRCIMVTPDGNFHRGYHLQVLVTPEVTLFQNRNRT
AVCKAVAGKPAAQISWIPEGDCATKQEYWSNGTVTVKSTCHWEVHNVSTVTCHVSHLTGNKSLYIELLPEN
IN*

Particularly interesting portions or fragments of the full length LP209c polypeptide include, e.g., a discovered putative signal-like sequence from about Met-1 to about Ala-24. Additionally interesting portions of LP209c are nine potential glycosylation sites: from about Asn-31 to about Ser-33; from about Asn-60 to about Ser-62; from about Asn-69 to about Ser-71; from about Asn-116 to about Thr-118; from about Asn-122 to about Thr-124; from about Asn-185 to about Thr-187; from about Asn-218 to about Thr-220; from about Asn-233 to about Ser-235; and from about Asn-247 to about Ser-249. Additionally interesting segments of LP209c are predicted transmembrane-like regions: from about Trp-5 to about Ala-27; from about Arg-90 to about Lys-113; and from about Tyr-267 to about Tyr-295. Other interesting segments of LP209c are discovered portions of LP209c from about Asn-9 to about Ala-23 (NLGLLLILTIFLVAA); from about Ser-25 to about Asn-37 (SSLCMDEKQITQN); from about Val-58 to about Asn-68 (VLCCPPIALRN); from about Leu-69 to about Arg-79 (LIIITWEIILR); from about Gly-80 to about Asn-93 (GQPSCTKAYRKETN); from about Glu-94 to about Arg-104 (ETKETNCTDER); from about Tyr-145 to about Phe-158 (YHLQVLVTPEVTLF); from about Gln-159 to about Ala-176 (QNRNRTAVCKAVAGKPAA); from about Glu-183 to about Tyr-192 (EGDCATKQEY); from about Trp-193 to about His-205 (EGDCATKQEY); from about Trp-206 to about Asn-224 (WEVHNVSTVTCHVSHLTGN); from about Gly-11 to about Cys-28 (GLLLILTIFLVAASSSLC); from about Met-29 to about Tyr-38 (MDEKQITQNY); from about Ser-39 to about Trp-49 (SKVLAEVNTSW); from about Val-51 to about Leu-66 (VKMATNAVLCCPPIAL); from about Arg-67 to about Arg-79 (RNLIIITWEIILR); from about Gly-80 to about Lys-90 (GQPSCTKAYRK); from about Glu-91 to about Arg-104 (ETNETKETNCTDER); from about Ile-105 to about Asp-116 (ITWVSRPDQNSD); from about Leu-117 to about Met-134 (LQIRPVAITHDGYYRCIM); from about Val-135 to about Leu-147 (VTPDGNFHRGYHL); from about Thr-156 to about Cys-167 (TLFQNRNRTAVC); from about Lys-168 to about Ile-178 (KAVAGKPAAQI); from about Trp-180 to about Lys-189 (WIPEGDCATK); from about Gln-190 to about Thr-199 (QEYWSNGTVT); from about Val-198 to about Asn-21 0 (VTVKSTCHWEVHN); from about Gly-11 to about Ala-23 (GLLLILTIFLVAA); from about Ser-24 to about Gln-33 (SSSLCMDEKQ); from about Ile-34 to about Glu-44 (SSSLCMDEKQ); from about Val-45 to about Ala-57 (VNTSWPVKMATNA); from about Leu-59 to about Asn-68 (LCCPPIALRN); from about Leu-69 to about Leu-78 (LIIITWEIIL); from about Arg-79 to about Arg-89 (RGQPSCTKAYR); from about Lys-90 to about Arg-104 (KETNETKETNCTDER); from about Ile-105 to about Pro-121 (KETNETKETNCTDER); from about Ala-123 to about Cys-132 (AITHDGYYRC); from about Ile-133 to about His-146 (IMVTPDGNFHRGYH); from about Glu-154 to about Val-166 (EVTLFQNRNRTAV); from about Cys-167 to about Gln-177 (CKAVAGKPAAQ); from about Ile-178 to about Tyr-192 (ISWIPEGDCATKQEY); from about Trp-193 to about Trp-206 (WSNGTVTVKSTCHW); from about Glu-207 to about Cys-216 (EVHNVSTVTC); and from about His-217 to about Leu-227 (HVSHLTGNKSL); whose discoveries were based on an analysis of hydrophobicity, hydropathicity, and hydrophilicity plots. Additional interesting sections of LP209c are the discovered portions of LP209c from about Thr-7 to about Ser-25 (TANLGLLLILTIFLVAASS); from about Thr-47 to about Pro-62 (TSWPVKMATNAVLCCP); from about Pro-63 to about Thr-73 (PIALRNLIIIT); from about Trp-74 to about Ser-83 (WEIILRGQPS); from about Cys-84 to about Asn-93 (CTKAYRKETN); from about Glu-94 to about Glu-103 (ETKETNCTDE); from about Arg-104 to about Leu-117 (RITWVSRPDQNSDL); from about Gln-118 to about Tyr-130 (QIRPVAITHDGYY); from about Arg-131 to about Asn-140 (RCIMVTPDGN); from about Phe-141 to about Thr-152 (FHRGYHLQVLVT); from about Pro-153 to about Asn-162 (PEVTLFQNRN); from about Arg-163 to about Pro-174 (RTAVCKAVAGKP); from about Ala-175 to about Ala-187 (AAQISWIPEGDCA); from about Thr-188 to about Ser-202 (TKQEYWSNGTVTVKS); and from about Val-211 to about Gly-223 (VSTVTCHVSHLTG). These fragments were discovered based on analysis of antigenicity plots. Further, particularly interesting LP209c segments are LP secondary structures (e.g., such as a helix, a strand, or a coil). Particularly interesting LP209c coil structures are the following: from about Met-1 to about Cys-3; from about Ser-24 to about Met-29; from about Asn-46 to about Trp-49; from about Cys-61 to about Ile-64; from about Arg-79 to about Ser-83; from about Glu-94 to about Asp-102; from about Arg-110 to about Asp-116; from about His-126 to about Gly-128; from about Thr-136 to about Gly-144; from about Thr-152 to about Glu-154; from about Asn-160 to about Asn-162; from about Gly-172 to about Ala-175; from about Pro-182 to about Thr-188; from about Ser-194 to about Gly-196; from about Val-211 to about Val-211; from about Leu-221 to about Lys-225; and from about Leu-232 to about Asn-237. Particularly interesting strand structures are from about Val-58 to about Leu-59; from about Ile-105 to about Val-108; from about Gln-118 to about Ile-119; from about Tyr-130 to about Val-135; from about His-146 to about Leu-150; from about Thr-156 to about Phe-158; from about Gln-177 to about Trp-180; from about Val-198 to about Lys-201; from about Thr-213 to about Val-218; and from about Ser-226 to about Leu-231. Further encompassed by the invention are contiguous amino acid residue combinations of any of the predicted secondary structures described above. For example one coil-strand-coil-strand-coil motif of LP209c combines the Val-211 to about Val-211 coil; the Thr-213 to Val-218 strand; the Leu-221 to Lys-225 coil; the Ser-226 to Leu-231 strand; and the Leu-232 to Asn-237 coil to form an interesting fragment of contiguous amino acid residues from about Val-211 to about Asn-237. Other combinations of contiguous amino acids are contemplated as can be easily determined.

LP209d is another novel variant of LP209. LP209d is encoded by an alternatively spliced mRNA of LP209. LP209d differs from LP209 by exhibiting a 24 amino acid residue deletion in the N-terminal portion (EAEGAAQPNNSLMLQTSKENHALA; corresponding to the Glu-23 to Ala-46 portion of full-length LP209). LP209d differs from LP209c by being truncated at an earlier amino acid residue than LP209c (see, Table 15 below). LP209d comprises primarily a truncated extracellular portion of LP209c. Sequence encoding LP209d was also found in a clone derived from metastatic breast tumor tissue. Accordingly, LP209d may be another natural secreted variant form of LP209c that has similar functions to those of LP209c-Fc (e.g., such as soluble receptor that competes for LP209c-like ligand thus blocking the function of the normal ligand-receptor pair).
Table 15: Primate, e.g., human, LP209d polynucleotide sequence (SEQ ID NO: 31) and corresponding polypeptide (SEQ ID NO: 32). The ORF for LP209d is 112-264 bp (with the start (ATG) identified in bold typeface. If numbering is misidentified one skilled in the art could determine the open reading frame without undue experimentation).

LP209d DNA sequence (265 bp) (ORF = 112-264):
LP209d (start (atg) codon is indicated in bold typeface and underlined).

GGATCCTGTACTGAGAAGTTGACCAGAGAGGGTCTCACCATGCGCACAGTTCCTTCTGTACCTGTGTGGAG
GAAAAGTACTGAGTGAAGGGCAGAAAAAGAGAAAACAGAAATGCTCTGCCCTTGGAGAACTGCTAACCTAG
GGCTACTGTTGATTTTGACTATCTTCTTAGTGGCCGCTTCAAGCAGTTTATGTATGGATGAAAAACAGATT
ACACAGAACTACTCGAAAGTACTCGCAGAAGTTAACACTTCATGGCCTGTAA

LP209d Full-Length Sequence (51aa):
A predicted SP cleavage site is between Ala-22 and Ala-23 indicated as
follows: 1 MLCPWRTANLGLLLILTIFLVA^A 23

MLCPWRTANLGLLLILTIFLVAASSSLCMDEKQITQNYSKVLAEVNTSWPV

An LP209d Mature Sequence (29aa):
Using the signal sequence as predicted, a correspondingly predicted
mature LP209d is as follows:

ASSSLCMDEKQITQNYSKVLAEVNTSWPV

### FEATURES OF LP NO: 17 (LP293)

LP293 is a novel secreted polypeptide. LP293 has a signal peptide-like portion at its N-terminal. LP293 exhibits sequence similarity at the amino acid level to embryonic serine protease-1 (Xenopus) (Yamada, et al., 2000 Gene 252(1-2): 209-216) and LP293 contains the active sites of serine proteases (trypsin family) and trypsin-like domains. LP293 is expressed primarily in IL-5 activated eosinophils, and eosinophils exhibiting hyper-eosinophilia, and in asthma patients. Eosinophils are a type of polymorphonuclear leukocyte containing eosin-staining granules. Eosinophils are known to destroy parasitic organisms and play a major role in allergic reactions. They also secrete chemical mediators that can cause broncho-constriction in asthma (Sampson, et al., 2000 Clin Exp Allergy 2000 Jun;30 Suppl 1:22-7; Walsh, et al, 1999 Crit Rev Clin Lab Sci 1999 Oct;36(5):453-96). Eosinophils make up one to three percent of the total white blood cell count. Accordingly, compositions comprising LP293 polypeptides, polynucleotides, its agonists/antagonists, and/or antibodies are also useful for the treatment of parasite infection, allergy, and asthma. Recently, it has been shown that a serine protease CD26/dipeptidyl-peptidase IV (CD26/DPP IV) plays an important role in immune function (Sozzani, et al.2000 Pharm Acta Helv 74(2-3):305-312). CD26/DPP IV functions by removing NH2-terminal dipeptides from several chemokines and thus, profoundly affects their biological activity. Chemokines are a superfamily of proteins that play a central role in immune and inflammatory reactions and in viral infections. Chemokine receptors can function as entry/fusion co-receptors for human immunodeficiency virus (HIV)-1 infection, and regulation of receptor expression by cytokines may be relevant for viral infection. Consequently, post-translational processing of chemokines can profoundly affect their interaction with receptors. For instance, Kaposi's sarcoma (KS)-associated herpes virus 8 encodes for three chemokine-like proteins that show homology with MIP cluster of CC chemokines. These viral chemokines possess a partial agonist activity for certain chemokine receptors and may function as receptor antagonists. This biological activity could represent a strategy developed by the KS-associated herpes virus 8 to subvert immunity impairing the generation of an effective anti-viral immune response. In a similar manner, LP293 may function to modulate immune activity by postranslation modification of the known and useful chemokine proteins. Furthermore, CD26/DPP IV has been shown to play a role in T-cell proliferation and chemotaxis and of fibroblast activation in liver disease (e.g., human cirrhosis) (McCaughan, et al. 2000 Immunol Rev 174:172-191). Consequently, LP293 may play a similar role by activating immune cells in such conditions. Moreover, growing evidence suggests that, through its interactions with cytokines and degradative enzymes, the extracellular matrix (ECM) microenvironment has a specialized role in providing intrinsic signals for coordinating actions of cells of the immune system (e.g., leukocytes). Recent advances also reveal that enzymatic modifications (such as through serine proteases) to ECM moieties and cytokines induce distinctive cellular responses, and are likely to be part of a mechanism that regulates the perpetuation or arrest of inflammation. LP293 may be important in such a role by its ability to enzymatically modify the ECM microenvironment during the inflammatory response.

It has been discovered that LP293 sequence (SEQ ID NO: 33) are expressed in the following number of LIFESEQ GOLD™ database tissue and cDNA libraries: Genitalia, Female 1/106; Germ Cells 1/5; Hemic and Immune System 5/159; Musculoskeletal System 1/47; and Nervous System 1/198. Consequently, based on the expression pattern of LP293, its homology to proteins with known functions, and literature suggesting the role of such proteins in human conditions, diseases, syndromes, etc., it is likely that compositions comprising LP293 polypeptides (or fragments thereof), polynucleotides (or fragments thereof), and/or LP293 antibodies (or LP293 binding compositions), and related reagents are also useful for the diagnosis, prognosis, treatment, amelioration, and/or intervention of a disease, condition, or state including, but not limited to, e.g., cell proliferative, autoimmune/inflammatory, cardiovascular, neurological, and developmental disorders.
Table 16: Primate, e.g., human, LP293 polynucleotide sequence (SEQ ID NO: 33) and corresponding polypeptide (SEQ ID NO: 34). The ORF for LP293 is 185-751 bp (with the start (ATG) and stop codons (TAG) identified in bold typeface in case numbering is misidentified one skilled in the art could determine the open reading frame without undue experimentation).

LP293 DNA sequence (1653 bp) (ORF = 185-751):
LP293 >ds38134 (start (atg) and stop (tga) codons are indicated in bold
typeface and underlined). A polyadenylation signal is indicated in
italic typeface.

ATGAGAGGGGTTTCCTGTCTCCAGGTCCTGCTCCTTCTGGTGCTGGGAGCTGCTGGGACTCAGGGAAGGAA
GTCTGCAGCCTGCGGGCAGCCCCGCATGTCCAGTCGGATCGTTGGGGGCCGGGATGGCCGGGACGGAGAGT
GGCCGTGGCAGGCGAGCATCCAGCATCGTGGGGCACACGTGTGCGGGGGGTCGCTCATCGCCCCCCAGTGG
GTGCTGACAGCGGCGCACTGCTTCCCCAGTGCCCCTCCCAGAGTGGCGACCGCTACAAGGAGTAAGGGTGC
CGCTGCTGGACTCGCGCACCTGCGACGGCCTCTACCACGTGGGCGCGGACGTGCCCAGGCTGAGCGCATTG
TGCTGCCTGGGAGTCTGTGTGCCGGCTACCCCCAGGGCCACAAGGACGCCTGCCAGGTGTGCACCCAGCCT
CCCCAGCCTCCGGAGTCCCCTCCCTGTGCCCAGCACCCTCCCTCCCTGAACTCCAGGACCCAGGACATCCC
AACTCAGGCTCAGGATCCTGGCCTCCAACCTAGAGGCACCACGCCAGGGGTCTGGAACCCTGAGAACTGA

LP293 Full-Length Sequence (188aa):

(SEQ ID NO: 34) The underlined portion is a predicted signal sequence
(Met-1 to Gly-22). A predicted SP cleavage site is between Gly-22 and
Arg-23 indicated as follows: 1 MRGVSCLQVLLLLVLGAAGTQG^RK 24

MRGVSCLQVLLLLVLGAAGTQGRKSAACGQPRMSSRIVGGRDGRDGEWPWQASIQHRGAHVCGGSLIAPQWV
LTAAHCFPSAPPRVATATRSKGAAAGLAHLRRPLPRGRGRAQAERIVLPGSLCAGYPQGHKDACQVCTQPPQ
PPESPPCAQHPPSLNSRTQDIPTQAQDPGLQPRGTTPGVWNPEN*

An LP293 Mature Sequence (166aa):
A predicted mature LP293 sequence is as follows:

RKSAACGQPRMSSRIVGGRDGRDGEWPWQASIQHRGAHVCGGSLIAPQWVLTAAHCFPSAPPRVATATRSKG
AAAGLAHLRRPLPRGRGRAQAERIVLPGSLCAGYPQGHKDACQVCTQPPQPPESPPCAQHPPSLNSRTQDIP
TQAQDPGLQPRGTTPGVWNPEN*

Particularly interesting portions or fragments of the full length LP293 polypeptide include, e.g., a discovered putative signal peptide-like sequence from Met-1 to Gly-22. Additionally interesting portions of LP293 are two trypsin-like domains: Ile-37 to Arg-85 Arg-117 to Gln-137; and a serine protease, trypsin family-like active site: Leu-73 to Cys-78. Suggesting that LP293 may perform a peptidase like activity based on this homology. Trypsin-like protein domains are recognized in all proteins in families S1, S2A, S2B, S2C, and S5 in the classification of peptidases (Rawlings & Barrett 1994 Meth Enzymol 244:19-61; Sprang, et al., 1987 Science 237:905-909). Active sites in serine protease-like proteins of the trypsin family, typically involve the catalytic activity of serine proteases of the trypsin family, which are provided by a charge relay system involving an aspartic acid residue hydrogen-bonded to a histidine, which itself is hydrogen-bonded to a serine. The sequences near the active site serine and histidine residues are well conserved in this family of proteases (Brenner 1988 Nature 334:528-530). Given LP293's sequence similarity to known serine proteases and the discovery of trypsin-like domains and a serine protease, trypsin family-like active site it is likely that LP293 possesses protease activity. Based on the teachings supplied herein, one skilled in the art would be able to easily determine enzymatic like activity for LP293 using common assay techniques, for example, such as those described in the journal BioTechniques (September, 1994), titled "A New Protease Activity Assay Using Fluorescence Polarization." Such techniques are well known in the literature and kits performing such assays are also commercially available (see, e.g., the Beacon® Protease Activity Detection Kit from the Pan Vera Corporation of Madison, Wisconsin). An additionally interesting segment of LP293 is a discovered transmembrane-like region from Met-1 to Ala-26. Additionally interesting segments of LP293 are discovered fragments Leu-11 to Gln-21; Lys-24 to Arg-36; Ile-37 to Arg-57; Cys-63 to Ala-82; Ala-97 to Ala-115; Gly-131 to Pro-145; Pro-149 to Glu-163; Asp-164 to Gly-178; Lys-24 to Gly-43; Gly-46 to Gln-55; Leu-66 to Ala-82; Lys-93 to Gly-111; Gln-114 to Gln-130; Gln-141 to Pro-150; Ser-157 to Asp-171; Leu-7 to Gly-16; Ala-18 to Arg-36; Val-38 to Pro-49; Ala-52 to Ser-65; Ala-76 to Val-86; Ala-87 to Gly-98; Leu-106 to Arg-117; Ser-123 to Val-138; Thr-140 to Ser-157; Leu-158 to Gly-173; and Leu-174 to Val-183, whose discovery was based on an analysis of hydrophobicity, hydropathicity, and hydrophilicity plots. Additional interesting sections of LP293 are the discovered portions of LP293 from Leu-7 to Ala-18; Lys-24 to Met-33; Cys-28 to Gly-40; Val-38 to Glu-47; Arg-44 to Ile-54; Cys-62 to Trp-71; Val-72 to Ala-82; Arg-85 to Ala-95; Gly-94 to Leu-106; Ala-113 to Gly-127; Tyr-128 to Gln-137; Cys-139 to Pro-149; Ser-160 to Asp-171; Gly-173 to Val-183; Gln-21 to Pro-31; Met-33 to Asp42; Asp-45 to Ala-52; His-56 to Gly-64; Ser-65 to Ala-76; Ala-82 to Gly-94; Thr-88 to Gly-98; Gly-98 to Arg-108; Leu-106 to Gln-114; Arg-108 to Leu-120; Gly-131 to Gln-144; Ala-152 to Pro-166; and Asp-164 to Pro-176. These fragments were discovered based on analysis of antigenicity plots. Further, particularly interesting LP293 secondary structures (e.g., such as a helix, a strand, or a coil) that have been discovered are the following LP293 helix structures: Cys-6 to Gln-8; and Gly-98 to Leu-102. Particularly interesting discovered coil structures are Met-1 to Arg-2; Ala-18 to Thr-20; Ala-26 to Met-33; Gly-39 to Pro-49; Arg-57 to Gln-70; Ala-75 to Pro-84; Ser-92 to Gly-94; Arg-104 to Arg-112; Leu-120 to Ser-123; Gly-127 to Ala-135; and Gln-141 to Asn-188. Particularly interesting discovered strand structures are Arg-36 to Ile-37; Val-72 to Thr-74; and Gln-137 to Val-138. Further encompassed by the invention are contiguous amino acid residue combinations of any of the predicted secondary structures described above. For example, one coil-strand-coil motif of LP293 combines the Gly-127 to Ala-135 coil, with the Gln-137 to Val-138 strand, and the Gln-141 to Asn-188 coil to form an interesting fragment of contiguous amino acid residues from Gly-127 to Asn-188. Other combinations of contiguous amino acids are contemplated as can be easily determined.

### FEATURES OF LP NO: 18 (LP294)

LP294 is a novel polypeptide (SEQ ID NO: 35) that is a splice variant of LP293. LP294 exhibits sequence similarity at the amino acid level to human serine proteinases as described above (e.g., such as the serine proteinase designated as prostasin (Yu, et al., 1995 J Biol Chem 270(22):13483-89). LP294 contains the active sites of serine proteinases (trypsin family), trypsin-like domains, and apple-like domains. LP294 has a signal peptide-like portion at its N-terminal. LP294 is expressed primarily in IL-5 activated eosinophils, and eosinophils exhibiting hyper-eosinophilia, and in asthma patients. Eosinophils are a type of polymorphonuclear leukocyte containing eosin-staining granules. Eosinophils are known to destroy parasitic organisms and play a major role in allergic reactions. They also secrete chemical mediators that can cause broncho-constriction in asthma (Sampson, et al., 2000 Clin Exp Allergy 30 Suppl 1:22-7; Walsh, et al, 1999 Crit Rev Clin Lab Sci 36(5):453-96). Eosinophils make up one to three percent of the total white blood cell count. Accordingly, compositions comprising LP294 polypeptides, polynucleotides, its agonists/antagonists, and/or antibodies are also useful for the treatment of parasite infection, allergy, and asthma.

Recently, it has been shown that a serine protease CD26/dipeptidyl-peptidase IV (CD26/DPP IV) plays an important role in immune function (Sozzani, et al.2000 Pharm Acta Helv 74(2-3):305-312). Furthermore, CD26/DPP IV has been shown to play a role in T-cell proliferation and chemotaxis and of fibroblast activation in liver disease (e.g., human cirrhosis)(McCaughan, et al. 2000 Immunol Rev 174:172-191). Consequently, LP294 may play a similar role by activating immune cells in such conditions.

Furthermore, since it has been shown that serine proteases facilitate several steps in cancer progression, it is useful to identify serine proteases that are most suitable for drug targeting by using indicators of actual enzyme activity in a biological sample not simply levels of messenger RNA or an immunoassay of the suspect protein. Accordingly, an automated microtiter plate assay can be used to allow detection of a suspected protease (such as, e.g., LP294) in tissue samples of patients with a proliferative disease condition (for example, see, e.g., the proteomic screen for proteases in colorectal carcinomas developed by McKerrow, et al., 2000 Mol Med. (5): 450-460, which is incorporated by reference herein for these teachings). Such an analysis can identify proteases whose activities may be essential for tumor progression and are not completely balanced by endogenous inhibitors. Such proteases are logical targets for efforts to produce low molecular weight protease inhibitors as a potential chemotherapy. Employing such an assay on LP294 to test its serine protease-like activity in a biological sample would allow a determination of its role in diseases of cell proliferation, such as, e.g., colon cancer.

LP294's homology to proteins involved in blood coagulation (e.g., plasma kallikrein, coagulation factor XI, and plasminogen) and the possession of apple domains, which have been shown to be involved in binding other members of the coagulation cascade (such as, e.g., kininogen, and factor XIIa) suggest that LP294 may also be participate in the blood coagulation system. Furthermore, at least two additional pieces of evidence suggest that LP294 may also participate in inflammatory processes. One is based on the observation that after injury there is typically a simultaneous activation of the innate immune response and the coagulation system. This simultaneous activation has been discovered to be a phylogenetically-conserved, ancient and adaptive response that can be traced back to the early stages of eukaryotic evolution and which persists today so that the same proinflammatory stimuli that activate elements of the human clotting cascade also activate phagocytic effector cells (neutrophils, monocytes, and macrophages). Thus, a complex and highly integrated linkage between systemic inflammation and coagulation is maintained in all vertebrates (see, e.g., Opal S. M. 2000 Crit Care Med. (9 Suppl): S77-80). The second piece of evidence supporting this view is the expression data for LP294, which is primarily in IL-5 activated eosinophils, and eosinophils exhibiting hyper-eosinophilia, and in asthma patients. Thus, supporting the linkage between the immune system and the coagulation system.

It has been discovered that LP294 sequence (SEQ ID NO: 35) are expressed in the following number of LIFESEQ GOLD™ database tissue and cDNA libraries: Genitalia, Female 1/106; Germ Cells 1/5; Hemic and Immune System 5/159; Musculoskeletal System 1/47; and Nervous System 1/198.

Consequently, based on the expression pattern of LP294, its homology to proteins with known functions, and literature suggesting the role of such proteins in human conditions, diseases, syndromes, etc., it is likely that compositions comprising LP294 polypeptides (or fragments thereof), polynucleotides (or fragments thereof), and/or LP294 antibodies (or LP294 binding compositions), and related reagents are also useful for the diagnosis, prognosis, treatment, amelioration, and/or intervention of a disease, condition, or state including, but not limited to, e.g., cell proliferative, autoimmune/inflammatory, coagulative, cardiovascular, neurological, and developmental disorders.
Table 17: Primate, e.g., human, LP294 polynucleotide sequence (SEQ ID NO: 35) and corresponding polypeptide (SEQ ID NO: 36). The ORF for LP294 is 52-894 bp (with the start (ATG) and stop codons (TAG) identified in bold typeface and underlined in case numbering is misidentified one skilled in the art could determine the open reading frame without undue experimentation). 
LP294 DNA sequence (1653 bp) (ORF = 52-894):
LP294 >ds38140 (start (atg) and stop (tga) codons are indicated in bold typeface and underlined). A
polyadenylation signal is indicated in italic typeface.

ATGAGAGGGGTTTCCTGTCTCCAGGTCCTGCTCCTTCTGGTGCTGGGAGCTGCTGGGACTCAGGGAAGGAAG
TCTGCAGCCTGCGGGCAGCCCCGCATGTCCAGTCGGATCGTTGGGGGCCGGGATGGCCGGGACGGAGAGTGG
CCGTGGCAGGCGAGCATCCAGCATCGTGGGGCACACGTGTGCGGGGGGTCGCTCATCGCCCCCCAGTGGGTG
CTGACAGCGGCGCACTGCTTCCCCAGGAGGGCACTGCCAGCTGAGTACCGCGTGCGCCTGGGGGCGCTGCGT
CTGGGCTCCACCTCGCCCCGCACGCTCTCGGTGCCCGTGCGACGGGTGCTGCTGCCCCCGGACTACTCCGAG
GACGGGGCCCGCGGCGACCTGGCACTGCTGCAGCTGCGTCGCCCGGTGCCCCTGAGCGCTCGCGTCCAACCC
GTCTGCCTGCCCGTGCCCGGCGCCCGCCCGCCGCCCGGCACACCATGCCGGGTCACCGGCTGGGGCAGCCTC
CGCCCAGGAGTGCCCCTCCCAGAGTGGCGACCGCTACAAGGAGTAAGGGTGCCGCTGCTGGACTCGCGCACC
TGCGACGGCCTCTACCACGTGGGCGCGGACGTGCCCCAGGCTGAGCGCATTGTGCTGCCTGGGAGTCTGTGT
GCCGGCTACCCCCAGGGCCACAAGGACGCCTGCCAGGGTGATTCTGGGGGACCTCTGACCTGCCTGCAGTCT
GGGAGCTGGGTCCTGGTGGGCGTGGTGAGCTGGGGCAAGGGTTGTGCCCTGCCCAATCGTCCAGGGGTCTAC
ACCAGTGTGGCCACATATAGCCCCTGGATTCAGGCTCGCGTCAGCTTCTAA
 
LP294 Full-Length Sequence (280aa):
>ds38140+2_ORF1(SEQ ID NO: 36) The underlined portion is a predicted signal sequence (Met-1 to Gly-
22). A predicted SP cleavage site is between Gly-22 and Arg-23 indicated as follows: 1
MRGVSCLQVLLLLVLGAAGTQG^RK 24
 
MRGVSCLQVLLLLVLGAAGTQGRKSAACGQPRMSSRIVGGRDGRDGEWPWQASIQHRGAHVCGGSLIAPQW
VLTAAHCFPRRALPAEYRVRLGALRLGSTSPRTLSVPVRRVLLPPDYSEDGARGDLALLQLRRPVPLSARV
QPVCLPVPGARPPPGTPCRVTGWGSLRPGVPLPEWRPLQGVRVPLLDSRTCDGLYHVGADVPQAERIVLPG
SLCAGYPQGHKDACQGDSGGPLTCLQSGSWVLVGVVSWGKGCALPNRPGVYTSVATYSPWIQARVSF*
 
An LP294 Mature Sequence (258aa):
A predicted mature LP294 sequence is as follows:

RKSAACGQPRMSSRIVGGRDGRDGEWPWQASIQHRGAHVCGGSLIAPQWVLTAAHCFPRRALPAEYRVRLGA
LRLGSTSPRTLSVPVRRVLLPPDYSEDGARGDLALLQLRRPVPLSARVQPVCLPVPGARPPPGTPCRVTGWG
SLRPGVPLPEWRPLQGVRVPLLDSRTCDGLYHVGADVPQAERIVLPGSLCAGYPQGHKDACQGDSGGPLTCL
QSGSWVLVGVVSWGKGCALPNRPGVYTSVATYSPWIQARVSF*
 LP293 and LP294 are alternatively spliced mRNAs from the same genomic DNA. LP294 shows sequence identity with various human serine proteases such as, e.g., the mature forms of alpha-tryptase (Vanderslice, et al. 1990 Proc. Natl. Acad. Sci. U. S. A. 87, 3811-3815) or the catalytic chains of acrosin (Adham, et al. 1990 Hum. Genet. 84, 125-128), plasma kallikrein (Chung, et al. 1986 Biochemistry 25:2410-2417), coagulation factor XI (Fujikawa, et al. 1986 Biochemistry 25:2417-2424), serine protease hepsin (Leytus, et al. 1988 Biochemistry 27:1067-1074), and plasminogen (Forsgren, et al. 1987 FEBS Lett. 213:254-260). A catalytic triad of histidine (H), aspartic acid (D), and serine (S), which have been shown to be essential for enzymatic activity (see, e.g., Yu, et al. 1995 J. Biol. Chem. 270 (22): 13483-89), are found in LP294 (the catalytic His-Asp-Ser triad in LP294 is formed by His-77 (LTAA**H**CFPR); Asp-126 (GARG**D**LALL) ; and Ser-231 (CQGD**S**GGPL)). Furthermore, alignment with other serine proteinases, demonstrates that LP294 contains an aspartic acid residue (D) at amino acid residue position Asp-225 (QGHK**D**ACQ), indicating that LP294 has trypsin-like activity (also, LP294 exhibits a trypsin-like domain and serine protease, trypsin family-like active sites further suggest that it has trypsin-like activities). The conserved Cys at LP294 residue Cys-146 (QPVCLPVP) is conserved in all of the serine proteinases which LP294 has been aligned with. This cysteine residue has been discovered to be involved in the formation of an interchain disulfide bond with the noncatalytic chain in plasma kallikrein, coagulation factor XI, and acrosin (see, e.g., McMullen, et al. 1991a Biochemistry 30, 2050-2056;McMullen, et al. 1991b Biochemistry 30, 2056-2060; and Topfer-Petersen, et al. 1990 FEBS Lett. 275, 139-142). Therefore, it is likely that this Cys residue in LP294 plays a similar role either by allowing binding with another molecule or by permitting LP294 hetero- or homodimer formation. Additionally, a conserved Asp residue (QGHKDACQG) is present in LP294 at position Asp-225, which is located six residues before the active Ser site (Ser-231). Similarly located Asp residues in other serine proteinases have been shown to be placed at the bottom of a substrate-binding pocket in trypsin that is involved in an interaction with an Arg or Lys residue on a corresponding substrate (see, e.g., Ruhlmann et al., 1973 J. Mol. Biol. 77, 417-436; and Yu, et al. 1995 J. Biol. Chem. 270 (22): 13483-89). Moreover, two Glycine residues (Gly-252 (VVSW**G**KGCA) and Gly-262 (PNRP**G**VYTS) are conserved in LP294 and other serine proteinases. The counterparts of these two Gly residues in trypsin and prostatin have been shown to be present at the entrance of the substrate-binding pocket and permit entry of large amino acid side chains. Thus, these features in LP294's primary structure additionally reinforce its trypsin-like functionality. Particularly interesting portions or fragments of the full length LP294 polypeptide include, e.g., a discovered putative signal peptide-like sequence from Met-1 to Gly-22. Additionally interesting portions of LP294 are: a trypsin-like domain from Ile-37 to Ile-274:); and serine protease, trypsin family-like active sites: Leu-73 to Cys-78 (LTAAHC) and Asp-225 to Thr-236 (DACQGDSGGPLT). Trypsin-like protein domains are recognized in all proteins in families having the S1, S2A, S2B, S2C, and S5 classification of peptidases (see, e.g., Rawlings & Barrett, 1994 Meth Enzymol 244:19-61; and Sprang, et al., 1987 Science 237:905-909). Active sites in serine protease-like proteins of the trypsin family, typically involve the catalytic activity of serine proteases of the trypsin family, which are provided by a charge relay system involving an aspartic acid residue hydrogen-bonded to a histidine, which itself is hydrogen-bonded to a serine. Sequences near the active site serine and histidine residues are well conserved in this family of proteases (see, e.g., Brenner 1988 Nature 334:528-530). Analysis of LP294 demonstrates that it possesses a characteristic catalytic triad of histidine (H), aspartic acid (D), and serine (S) residues, which have been shown to be essential for enzymatic activity in other serine proteinases (see, e.g., Yu, et al. 1995 J. Biol. Chem. 270 (22): 13483-89). The residues of the triad in LP294 are (His-77 (LTAAHCFPR); Asp-126 (GARG**D**LALL); and Ser-231 (CQGD**S**GGPL)). Analysis of this alignment also demonstrates that LP294 contains a conserved aspartic acid residue (D) at amino acid residue position Asp-225 (QGHK**D**ACQ). Similar placement of an Asp residue in other serine proteinases is interpreted as indicating trypsin-like activity (LP294 also possesses, as indicated above, both a trypsin-like domain and serine protease trypsin-family-like active sites, which further suggest that it possesses trypsin-like activity). A conserved Cys residue in LP294 (Cys-146; VQPVCLPVP), is also conserved in all of the serine proteinases of the alignment. This cysteine residue has been shown to be involved in the formation of an interchain disulfide bond with the noncatalytic chain in plasma kallikrein, coagulation factor XI, and acrosin (see, e.g., McMullen, et al. 1991a Biochemistry 30, 2050-2056; McMullen, et al. 1991b Biochemistry 30, 2056-2060; and Topfer-Petersen, et al. 1990 FEBS Lett. 275, 139-142). Therefore, it is likely that Cys-146 of LP294 plays a similar role (e.g., either by participating in binding with another molecule or by permitting LP294 hetero- or homodimer formation). Additionally, at LP294 position Asp-225 ―six residues before the active Ser site (Ser-231)― is an important conserved Asp residue (QGHKDACQG). Similarly placed Asp residues in other serine proteinases have been shown to be located at the bottom of the substrate-binding pocket of trypsin and to interact with an Arg or Lys residue on a corresponding substrate (see, e.g., Ruhlmann et al., 1973 J. Mol. Biol. 77, 417-436; and Yu, et al. 1995 J. Biol. Chem. 270 (22): 13483-89). Moreover, two LP294 Glycine residues (Gly-252 (WSW**G**KGCA) and Gly-262 (PNRP**G**VYTS); are also conserved in other serine proteinases. The counterparts of these two Gly residues in trypsin and prostatin have been shown to be present at the entrance of the substrate-binding pocket and to permit entry of large amino acid side chains. Consequently, analyzing the data as a whole, LP294's primary structure reinforce the view that it possesses enzymatic-like functionality.

Given its sequence homology to serine proteinases, its possession of a trypsin-like domain, its possession of serine protease, trypsin-family-like active sites, and the conservation of primary features with other serine proteinases, it is likely that LP294 possesses similar catalytic properties. Based on the teachings supplied herein, one skilled in the art would be able to easily determine enzymatic like activity for LP294 using common assay techniques. Testing a protein for trypsin activity is routine in the art and would not require undue experimentation given the teachings supplied herein (e.g., as to the LP294 sequence) and given teachings in the art for methods of determining whether a suspected protein has trypsin activity. For instance, LP294 could easily be tested for trypsin-like activities, using synthetic substrates (see, e.g., Yu et al. 1994 J. Biol. Chem. 269, 18843-18848 and the teachings supplied therein, which are hereby incorporated by reference for these teachings). For example to test LP294 for arginine amidolytic activities one could use the substrate D-Pro-Phe-Arg-MCA and D-Phe-Phe-Arg-MCA. To test for lysine amidolytic activitiy one would use, for example, a substrate such as succinyl-Ala-Phe-Lys-MCA and t-butyloxycarbonyl-Val-Leu-Lys-MCA. To test for enzymatic activity on chymotrypsin substrates one would use, for example, a substrate such as succinyl-Ala-Ala-Pro-Phe-MCA, Ala-Ala-Phe-AMC, or Suc-Leu-Leu-Val-Tyr-AMC. Trypsin-like activity could be assayed, for example with Boc-Leu-Ser-Thr-Arg-AMC. Other methods for testing are known in the art and would be easily available. For example, such as those described in the journal BioTechniques (September, 1994), entitled "A New Protease Activity Assay Using Fluorescence Polarization." Such techniques are well known in the literature and kits performing such assays are commercially available (see, e.g., the Beacon® Protease Activity Detection Kit from the Pan Vera Corporation, Madison, Wisconsin). Additionally interesting portions of LP294 are: the chymotrypsin-like domain signatures of serine proteases: Gly-63 to Cys-78 (GGSLIAPQWVLTAAHC); Gly-122 to Val-136 (GARGDLALLQLRRPV); and Lys-224 to Thr-236 (KDACQGDSGGPLT); and the apple-domain-like signatures of serine proteases: Gly-64 to Arg-96

(GSLIAPQWVLTAAHCFPRRALPAEYRVRLGALR), Leu-97 to Pro-135
(LGSTSPRTLSVPVRRVLLPPDYSEDGARGDLALLQLRRP), Val-136 to Pro-170

Apple domains are found in the two related plasma serine proteases that are activated by factor XIIA― plasma kallikrein (EC 3.4.21.34) and coagulation factor XI (EC 3.4.21.27). Both of these serine proteases share the same domain topology― an N-terminal region containing four tandem repeats of about 90 amino acids, and a C-terminal catalytic domain. This 90 amino-acid repeated domain contains six conserved cysteines that form three disulfide bonds (linking the first and sixth, second and fifth, and third and fourth cysteines) (see, e.g., McMullen, et al. 1991 Biochemistry 30:2050-2056; and McMullen, et al. 1991 Biochemistry 30:2050-2056). Since this domain can be drawn in the shape of an apple it has been designated the "apple domain." Apart from exhibiting these cysteine residues, there also exist a number of other conserved amino acid positions that can be identified in an apple domain. Accordingly, Applicants have developed a consensus pattern that they have used to identify apple domains in novel polypeptide sequences. This consensus sequence is: C-x(3)-[LIVMFY]-x(5)-[LIVMFY]-x(3)-[DENQ]-[LIVMFY]-x(10)- C-x(3)-C-T-x(4)-C-x-[LIVMFY]-F-x-[FY]-x(13,14)-C-x- [LIVMFY]-[RK]-x-[ST]-x(14,15)-S-G-x-[ST]-[LIVMFY]-x(2)-C -. Currently, all existing sequences that have been identified as belonging to the class of proteins possessing apple domains also possess this consensus pattern. Accordingly, since domains in LP294 were discovered using this consensus pattern, it is likely that LP294 is also a member of the grouping of proteins possessing apple domains.

Moreover, based on homology searches and structural prediction methods, it has also recently been shown that (1) the N-terminal N domains of members of the plasminogen/hepatocyte growth factor family, (2) the apple domains of the plasma prekallikrein/coagulation factor XI family, and (3) domains of various nematode proteins belong to a superfamily module, designated the PAN module (see, e.g., Tordai, et al. 1999 FEBS Lett. 461 (1-2): 63-67). Patterns of conserved residues in PAN correspond to secondary structural elements of the known three-dimensional structure of hepatocyte growth factor N domain and therefore, a similar fold is predicted for members of the PAN superfamily module. Based on functional information of apple- and N-domains, PAN modules have been shown to exhibit diverse biological functions by mediating protein-protein or protein-carbohydrate interactions. Consequently, it is expected that LP294 will also have such a predicted fold structure and likewise mediate protein-protein or protein-carbohydrate interactions like other members of the PAN module.

Additionally interesting segments of LP294 are discovered fragments Leu-11 to Gly-22; Lys-24 to Arg-36; Ile-37 to Arg-57; Cys-62 to His-77; Pro-108 to Asp-121; Pro-156 to Trp-177; Pro-220 to Pro-234; Thr-236 to Asn-259; Leu-11 to Arg-23; Lys-24 to Gly-43; Cys-62 to Ala-83; Pro-102 to Pro-116; Leu-132 to Gln-143; Gln-143 to Cys-160; Val-162 to Arg-184; Gly-200 to Leu-215; Pro-234 to Trp-251; Ser-250 to Gly-262; Leu-7 to Gly-16; Ala-17 to Arg-36; Ile-37 to Pro-49; Ala-52 to Ser-65; Ala-76 to Val-90; Leu-97 to Pro-108; Pro-116 to Asp-126; Pro-150 to Val-172; Asp-189 to Arg-208; Ser-214 to Pro-234; and Ser-250 to Ser-271, whose discovery was based on an analysis of hydrophobicity, hydropathicity, and hydrophilicity plots. Additional interesting sections of LP294 are the discovered portions of LP294 from Cys-28 to Ile-54; Cys-62 to Trp-71; Val-72 to Arg-82; Leu-84 to Ala-94; Ala-123 to Ser-139; Val-145 to Thr-158; Gly-157 to Val-172; Val-172 to Gly-182; Pro-204 to Gly-218; Tyr-219 to Gln-228; Gly-229 to Ser-240; Gly-241 to Lys-253; Gly-254 to Ala-268; Tyr-264 to Gln-275; Thr-20 to Pro-31; Met-33 to Gly-43; Gly-43 to Ala-52; Gln-21 to Ala-52; His-56 to Gly-63; Leu-92 to Ser-106; Val-112 to Leu-127; Pro-150 to Trp-165; Pro-154 to Gly-164; Val-185 to Thr-192; Cys-193 to Pro-204; Tyr-219 to Leu-235; and Gly-252 to Tyr-270. These fragments were discovered based on analysis of antigenicity plots. Further, particularly interesting LP294 secondary structures (e.g., such as a helix, a strand, or a coil) that have been discovered are the following LP294 helix structures: Leu-127 to Gln-131. Particularly interesting discovered coil structures are Met-1 to Arg-2; Ala-18 to THr-20; Cys-28 to Met-33; Gly-40 to Pro-49; Arg-56 to Gly-57; Gly-63 to Gln-70; Ala-75 to Arg-81; Leu-97 to Arg-103; Leu-114 to Gly-125; Arg-133 to Pro-144; Leu-147 to Pro-159; Gly-166 to Leu-180; Asp-189 to Gly-195; Gly-200 to Val-203; Leu-211 to Ser-214; Gly-218 to Lys-224; Gly-229 to Gly-233; Gln-239 to Ser-242; Trp-251 to Gly-262; Thr-269 to Ser-271; and Ser-279 to Phe-280. Particularly interesting discovered strand structures are Arg-36 to Ile-37; Arg-110 to Leu-113; Tyr-197 to His-198; Leu-235 to Leu-238; and Trp-243 to Val-249. Further encompassed by the invention are contiguous amino acid residue combinations of any of the predicted secondary structures described above. For example, one strand-coil-helix-coil motif of LP294 combines the Arg-110 to Leu-113 strand, with the Leu-114 to Gly-125 coil, with the Leu-127 to Gln-131 helix, and the Arg-133 to Pro-144 coil to form an interesting fragment of contiguous amino acid residues from Arg-110 to Pro-144. Other combinations of contiguous amino acids are contemplated as can be easily determined.

### FEATURES OF LP NO: 19 (LP295)

LP295 is a novel human polypeptide (SEQ ID NO: 38) that exhibits amino acid sequence identity to various serpin proteins. The serpins are a superfamily of proteins, typically 350―400 amino acids in length, with a diverse set of functions including, but not limited to, inhibition of serine proteases in the vertebrate blood coagulation cascade (see, e.g., Huber and Carrell 1989 Biochemistry 28: 8951-8966; and Marshall 1993 Philos. Trans. R. Soc. Lond. B Biol. Sci. B342: 101-119). Serpins are of clinical interest because mutations in these compositions cause a number of disease conditions―for example, blood clotting disorders, emphysema, cirrhosis, and dementia―many of which are consequences of polymerization (see, e.g., Carrell & Lomas 1997 Lancet 350: 134-138). Serpins have a conserved set of secondary structural elements whose nomenclature (Schechter and Berger 1967 Biochem. Biophys. Res. Commun. 27: 157-162) has developed based on a naming system given to the canonical structure of native alpha-1-antitrypsin (Elliott et al. 1996 Nat. Struct. Biol. 3: 676-681). Typically, serpins contain three beta-sheets and nine alpha-helices. The portion of a serpin designated as the reactive center loop (RCL), is crucial for the function of inhibitory serpins by undergoing large structural changes that alter the folding topology of the molecule. In alpha-1-antitrypsin, the RCL comprises residues P17-P4' and it contains a scissile bond (between residues P1 and P1'), which is cleaved by its cognate target protease. Using the LP295 sequence taught herein and its identity with known serpins, Applicants have discovered similar secondary structures in LP295.

Five conformational states―native, cleaved, latent, gamma, and polymeric― characteristically appear in serpin crystal structures. They differ primarily in the structure of the RCL region (see Whisstock et al. 1998 Trends Biochem. Sci. 23: 63-67). In the native state, the RCL is exposed and, for inhibitory serpins, accessible for interaction with its cognate protease. Upon cleavage of the scissile bond, the reactive center loop forms an additional strand inserted into the A beta sheet, with concomitant conformational changes elsewhere in the molecule (Stein and Chothia, 1991 J. Mol. Biol. 221: 615-621; Whisstock, et al. 2000a J. Mol. Biol. 296: 685-699). Cleavage is typically associated with an increase in stability (resistance to denaturing conditions). The native to cleaved conformational change is designated as the "stressed to relaxed" (S→R) transition (Carrell and Owen 1985 Nature 317: 730-732). A substrate of the native conformation is seen in the X-ray crystal structure of antithrombin, in which the RCL is partially inserted into the A beta-sheet (Carrell, et al. 1994 Structure 2: 257-270; Schreuder, et al. 1994 Nat. Struct. Biol. 1: 48-54; Whisstock, et al. 2000b J. Mol. Biol. 301: 1287-1305).

The latent state is an uncleaved state in which the RCL is inserted into the A beta-sheet, as in the cleaved form; this is an alternative R state. The latent state was first seen in the crystal structure of Plasminogen Activator Inhibitor-1 (PAI-1; Mottonen, et al. 1992 Nature 355: 270-273). The transition in PAI-1 from the native, active form to the latent, non-inhibitory conformation provides a fine level of functional control, limiting the active lifetime of PAI-1 to a few hours (Levin and San-tell 1987 Blood 70: 1090-1098). The latent state also occurs in the crystal structure of antithrombin (Carrell, et al. 1994 Structure 2: 257-270; Skinner et al. 1997 J. Mol. Biol. 266: 601-609), and there is evidence for its existence in alpha-1-antitrypsin (Lomas, et al. 1995 J. Biol. Chem. 268: 516-521) and alpha-1- antichymotrypsin (Gooptu, et al. 2000 Proc. Natl. Acad. Sci. 97: 67-72). Two additional conformational states have recently been structurally characterized in serpins. One, in gamma-antichymotrypsin (which contains the mutation Leu55→Pro), presents an intermediate conformation between the native and latent state (Gooptu et al. 2000 Proc. Natl. Acad. Sci. 97: 67-72). The second, discovered by X-ray crystal structure of cleaved alpha-1-antitrypsin polymers, confirms the loop―sheet mechanism of polymerization (Lomas et al. 1992 Nature 357: 605-607; Huntington et al. 1999 J. Mol. Biol. 293: 961-964; Dunstone, et al. 2000 Protein Sci. 9: 429-443). The S→R transition is integral to the function of inhibitory serpins. The mechanism of inhibition involves the formation of a stable complex between the protease and the cleaved form of the inhibitor, analogous to an enzyme-product complex. Some non-inhibitory serpins, such as CBG, use the S→R transition to control ligand release: the native state of CBG has higher affinity for cortisol than does the cleaved form (Pemberton, et al. 1988 Nature 336: 257-258). Note the difference between this mechanism and that of hemoglobin: once cleaved, CBG releases its ligand, and it cannot be re-used whereas hemoglobin has to develop a complex allosteric mechanism to achieve reversible release of ligands. Some other serpins (e.g., ovalbumin) do not undergo an S→R transition under normal physiological conditions (Wright et al. 1990 J. Mol. Biol. 213: 513-528). The serpins also share certain mechanistic features with non-serpin inhibitor families. Both serpin and non-serpin inhibitors inhibit proteases by forming stable equimolar complexes in which a substrate-like interaction is made between an exposed inhibitor binding loop and an enzyme active site (see, e.g., Potempa, et al. 1994 J. Biol. Chem. 269 15957-15960; Olson, et al. 1994 Semin. Thromb. Hemostasis 20, 373-409; Laskowski, et al. 1980 Annu. Rev. Biochem 49, 593-626; and Bode & Huber. 1992 Eur. J. Biochem. 204, 433-451). However, serpins differ from non-serpin inhibitors in requiring a large inhibitor conformational change to trap proteases in such complexes (see, e.g., Engh, et al. 1990 Protein Eng. 3, 469-477; Schulze, et al. 1990 Eur. J. Biochem. 194, 51-56; Skriver, et al. 1991 J. Biol. Chem. 266, 9216-9221; Carrell, et al. 1991 Nature 353, 576-578; Björk, et al. 1992 J. Biol. Chem. 267, 1976-1982; and Björk, et al. 1993 Biochemistry 32, 6501-6505). In this conformational change, the inhibitor-binding loop is thought to collapse from its exposed position on the protein surface and become inserted into the center of a major beta-sheet comprising the core of the protein. By contrast, in the non-serpin inhibitors, the binding loop is rigidly fixed in an optimal substrate binding, "canonical" conformation capable of tight interaction with the protease with minimal conformational adjustments (Laskowski, et al. 1980 Annu. Rev. Biochem. 49, 593-626; and Bode & Huber. 1992 Eur. J. Biochem. 204, 433-451).

Several regions are important in controlling and modulating serpin conformational changes: (1) The hinge, the P15-P9 portion of the RCL (Hopkins et al. 1993 Biochemistry 32: 7650-7657). The hinge provides mobility essential for the conformational change of the RCL in the S→R transition; (2) The breach, located at the top of the A beta-sheet, the point of initial insertion of the RCL into the A beta-sheet (Whisstock et al. 2000a J. Mol. Biol. 296: 685-699); (3) The shutter, near the center of A beta-sheet (Stein and Carrell 1995 Nat. Struct. Biol. 2: 96-113). The breach and shutter are two important regions that facilitate sheet opening and accept the conserved hinge of the RCL as it inserts (Whisstock et al. 2000a J. Mol. Biol. 296: 685-699); (4) The gate, including strands s3C and s4C, primarily characterized by studies of the transition of active PAI-1 to latency (Mottonen et al. 1992 Nature 355: 270-273; Stein and Carrell 1995 Nat. Struct. Biol. 2: 96-113). To insert fully into the A beta-sheet without cleavage, the RCL has to pass around the helix-turn linking strands s3C and s4C. Characteristically, inhibitory serpins can be recognized by a consensus pattern in their sequences in the hinge region (see, e.g., Gettins, et al. 1996 Serpins: Structure, Function and Biology, RG. Landes, Austin, Texas; Hopkins, et al. 1993 Biochemistry 32:7650-57; and Irving, et al. Genome Research 10:1845-64). One consensus sequence has the following structure: P17=E, P16=E/K/R, P15=G, P14=T/S, P13=E, P12-P9=(A/G/S), in which P15 is usually glycine, P14 is threonine or serine, and positions P12-P9 are occupied by residues with short side chains, such as, e.g., alanine, glycine, or serine. The hinge residues are believed to permit efficient and rapid insertion of the RCL into the A-beta sheet during S→R transition. The corresponding regions of non-inhibitory serpins deviate from the consensus. Mutations of hinge-region residues often convert inhibitory serpins into substrates. In one sequence comparison using the P17-P8-like portion of LP295 (ETGTEATAAT) to compare with such a consensus sequence the results suggest that this region of LP295 exhibits the characteristics of an inhibitory serpin hinge-like region and support an additional assertion that LP295 is a serpin-like protein that has inhibitory-type serpin capability. One consequence of the conformational lability of serpin higher order structure is the possibility of it leading to undesirable conformation formations (e.g., aberrant polymerizations), for example, such as that resulting from the insertion of the RCL of one molecule into the A beta-sheet of another (see, e.g., Mast et al. 1991 Biochemistry 30: 1723-1730; Lomas et al. 1992 Nature 357: 605-607; Huntington et al. 1999 J. Mol. Biol. 293: 449-455; and Dunstone et al. 2000 Protein Sci. 9: 429-443). Mutants, including many in the shutter region of serpins, have been identified that enhance the propensity for serpin polymerization, leading to dysfunction and disease (for a review, see Stein and Carrell 1995 Nat. Struct. Biol. 2: 96-113). Encompassed herein are LP295, and LP295 variants (such as, e.g., amino acid residue variants of the shutter region of LP295), associated with such or similar polymerizations resulting in conditions, disorders, syndromes, or disease states such as, e.g., dementia, Alzheimer's disease, neurofibrillary plaque formation, amyloid-fibril formation, inclusion-body myositis, inclusion-body myopathy syndromes, intrafiber congophilias, neuromuscular junctional disease, hepatic malignant fibrous histocytoma, hepatic inclusion formations, pulmonary inclusion formations, uremic cachexia, muscle cachexia, hepatic damage that may progress to cirrhosis and hepatocellular carcinoma.(see, e.g., Lomas, D. A., 1996 QJM 89(11):807-12). Additionally included herein are methods to represent conformational disease state related to LP295 or an LP295 variant.

Analysis of the LP295 amino acid sequence suggests that it also is an extracellular serpin. The extracellular serpins have been further categorized (based on a phylogenetic analysis of all the currently described extant serpins; see, e.g., Irving et al, 2000 Genome Research 10:1845-1860) into eight separate clades, the largest of which ―clade 'a' in the Irving et al., nomenclature― contains 77 antitrypsin-like extracellular inhibitory serpins. Further analysis of LP295 suggests that is a newly discovered member of this antitrypsin-like grouping. Moreover, within the antitrypsin clade of extracellular inhibitory serpins, LP295 exhibits particularly close amino acid sequence identity to the following serpins: Contrapsin-Like Protease Inhibitor 3 Precursor (CPI-23), Serine Protease Inhibitor 1 (SPI-1); Contrapsin-Like Protease Inhibitor 1 Precursor (CPI-21), Kallikrein-Binding Protein (KBP), Growth Hormone-Regulated Protease Inhibitor (GHR-P63), Serine Protease Inhibitor 2 (SPI-2), Serine Protease Inhibitor 2.3 (SPI-2.3), Thyroid Hormone-Regulated Protein; Serine protease inhibitor (SPI-2.4) rat fragment; Serine protease inhibitor (SPI-2.4); EB22-23; and SPI-2. Interestingly, LP295 contains an arginine residue (Arg-379) at the presumptive P1-like position of the reactive-site peptide bond-like region in LP295, which, in rodent serpins (also know as contrapsins), is a characteristic indicator of trypsin inhibition (see, e.g., Potempa, et al. 1995 Biochem J Feb 15;306 (Pt 1):191-7). Consequently, it is likely that LP295 is a novel trypsin inhibitor. Testing the inhibitory spectrum of LP295 would not require undue experimentation given the teachings in the art regarding assays for determining the inhibitory activity of suspected serpins (such as, e.g., against chymotrypsin, pancreatic elastase, neutrophil elastase, thrombin, plasmin, plasma kallikrein, pancreatic kallikrein, clotting factor Xa, or papain). Such methods are well known in the art and could be adapted here to test LP295 (see, e.g., Yamamoto & Sinohara 1993 J Biol Chem 268(24):17750-53).

Although the chromosomal location of LP295 has currently not been established, it is likely that the genetic locus of LP295 will be found within or near a serpin gene cluster recently described on human chromosome 14. Recently, the first detailed map of a serpin gene cluster in humans was mapped to the 14q32.1 locus (see, e.g., Rollini & Fournier 1997 Genomics 46(3): 409-415). The cluster of known human serpin genes that map to the 14q32.1 locus include genes that are phylogenetically related to LP295. Identified serpin genes at this locus encode: alpha-1-antitrypsin (alpha 1AT, gene symbol PI), corticosteroid-binding globulin (CBG), alpha 1-antichymotrypsin (AACT), protein C inhibitor (PCI), kallistatin (KAL, gene symbol PI4), the polymorphic protease inhibitor (PI) gene, PIL; and the PI-like pseudogene. Given the phylogenetic relationship of LP295 to these genes, it is likely that LP295 also maps to this region of human chromosome 14. Furthermore, recent data indicate an involvement of the serpin genes at this locus in the pathogenesis of Wegener's granulomatosis, a multisystem disease involving necrotizing vasculitis characterized by upper and lower respiratory tracts involvement, progressive glomerulonephritis, and systemic symptoms attributable to small vessel vasculitis (Borgmann, et al., 2001 Clin Immunol (2): 244-248). Consequently, given the similarity of LP295 to members of this gene cluster, LP295 may also be both involved in, and/or a marker for this condition.
**Table 18:** Primate, e.g., human, LP295 polynucleotide sequence (SEQ ID NO: 37) and corresponding polypeptide (SEQ ID NO: 38). The ORF for LP295 is 147-1388 bp (with the start (ATG) and stop codons (TAG) identified in bold typeface and underlined in case numbering is misidentified one skilled in the art could determine the open reading frame without undue experimentation).

LP295 DNA Sequence (1725 bp) (ORF = 147-1388):
LP295 >ds38141 (start (atg) and stop (tga) codons are indicated in bold typeface and underlined). A polyadenylation signal is indicated in italic typeface.
ATGGCCTTCATTGCAGCTTTGGGGCTCTTGATGGCAGGGATCTGCCCTGCTGTCCTCTGTGATGGCACACT
GGGAAGGGACACTCTATCCCATGAAGACCACGGCAAGGGGAGACAACTGCACAGTCTCACATTGGCTTCCA
GCAACACTGACTTCGCATTGAGCCTCTACAAGAAGCTGGCTTTGAGGAATCCAGATAAAAATGTTGTCTTC
TCCCCTCTTAGCATCTCAGCTGCCTTGACCATTTTGTCTCTGGGAGCAAAAGACAGCACCATGGAAGAGAT
TCTAGAAGGTCTCAAGTTCAATCTCACAGAGATCACTGAGGAAGAAATCCACCAGGGCTTTGGGCACCTCC
TACAGAGGCTCAGCCAGCCAGAGGACCAGGTAGAGATCAATACAGGTAGTGCCCTGTTTATTGACAAAGAG
CAGCCGATACTGTCAGAATTCCAGGAGAAGACAAGGGCTCTGTACCAGGCTGAGGCCTTCGTAGCTGACTT
CAAGCAGCCCAATGAGGCCAAAAAGCTCATCAATGACTATGTGAGCAATCAGACCCAGGGGAAGATCGCAG
AACTGTTCTCAGACCTGGAGGAGAGGACATCCATGGTGCTGGTGAACTATCTCCTATTTAAAGGCAAATGG
AAGGCCCCCTTTAACCCCAATGACACAATTGAGTCTGAGTTCTACTTGGATGAGAAGAGGTCTGTGAAGGT
GCCCATGATGAAAATTAAGGAAGTAACCACACCCTATGTCCGGGATGAGGAACTCTCCTGCTCTGTGTTGG
AGCTGAAGTACACAGGAAATGCCAGCGCCCTGTTTATCCTCCCTGACCAAGGCAAGATGCAGCAGGTGGAA
TCCAGCTTGCAACCAGAGACCCTGAAGAAGTGGAAGGACTCTTTGATCCCTAGGATTATAAATGATCTCCG
CATGCCCAAGTTCTCCATCTCCACAGACTACAGTCTAAAAGAGGTCCTTCCAGAGCTGGGCATTAAGAAAG
TCTTCTCCCAGCAAGCTGATCTGAGTAGGATCACAGGGACCAAGGATCTGTATGTCTCTCAAGTGGTCCAC
AAGGCTGTGCTGGATGTGGATGAGACAGGCACAGAAGCAACTGCTGCTACAGGGGTCGCTACAGTCATACG
TAGACAACCTCGAACTCTGAATTTCAACCGGCCATTCATGGTGGTTATCACTGACATGGATAGTCAGTCTA
TCCTCTTTGTTGCCAAAATCACTAACCCCAAGTAA

LP295 Full-Length Sequence (413aa):
>ds38141+3_ORF1(SEQ ID NO: 38)  The underlined portion is a predicted
signal sequence (Met-1 to Ala-17).  A predicted SP cleavage site is
between Ala-17 and Val-18 indicated as follows: 1
MAFIAALGLLMAGICPA^VLCDG 22.  An alternative predicted cleavage site
based on a different signal peptide analysis is between Cys-20 and
Asp-21 indicated as follows: 1 MAFIAALGLLMAGICPAVLC^DG 22.  Both
versions are encompassed herein.
MAFIAALGLLMAGICPAVLCDGTLGRDTLSHEDHGKGRQLHSLTLASSNTDFALSLYKKLALRNPDKNVVF
SPLSISAALTILSLGAKDSTMEEILEGLKFNLTEITEEEIHQGFGHLLQRLSQPEDQVEINTGSALFIDKE
QPILSEFQEKTRALYQAEAFVADFKQPNEAKKLINDYVSNQTQGKIAELFSDLEERTSMVLVNYLLFKGKW
KAPFNPNDTIESEFYLDEKRSVKVPMMKIKEVTTPYVRDEELSCSVLELKYTGNASALFILPDQGKMQQVE
SSLQPETLKKWKDSLIPRIINDLRMPKFSISTDYSLKEVLPELGIKKVFSQQADLSRITGTKDLYVSQVVH
KAVLDVDETGTEATAATGVATVIRRQPRTLNFNRPFMVVITDMDSQSILFVAKITNPK*

An LP295 Mature Sequence (396aa):
A predicted mature LP295 sequence is as follows:

VLCDGTLGRDTLSHEDHGKGRQLHSLTLASSNTDFALSLYKKLALRNPDKNVVFSPLSISAALTILSLGAKD
STMEEILEGLKFNLTEITEEEIHQGFGHLLQRLSQPEDQVEINTGSALFIDKEQPILSEFQEKTRALYQAEA
FVADFKQPNEAKKLINDYVSNQTQGKIAELFSDLEERTSMVLVNYLLFKGKWKAPFNPNDTIESEFYLDEKR
SVKVPMMKIKEVTTPYVRDEELSCSVLELKYTGNASALFILPDQGKMQQVESSLQPETLKKWKDSLIPRIIN
DLRMPKFSISTDYSLKEVLPELGIKKVFSQQADLSRITGTKDLYVSQVVHKAVLDVDETGTEATAATGVATV
IRRQPRTLNFNRPFMVVITDMDSQSILFVAKITNPK*

Three interesting portions of LP295 can define a minimally predicted reactive center-like loop (RCL) region, with an N-terminad side portion having residues Glu-363 to Thr-372 (ETGTEATAAT)and a C-terminad side portion having residues Arg-379 to Arg-380 (RR) which is a pipeline reactive center-like portion of LP295. Between these two areas lies a region from about Thr-372 to Ile-378 (TGVATVI). These three portions of LP295 (ETGTEATAAT), (TGVATVI), and (RR), from about Arg-379 to about Ile-378 exhibit characteristic elements of a reactive center-like loop region which has been shown to be a distinguishing feature of serpins (see, e.g., Carrell & Evans 1992 Curr. Opin. Struct. Biol. 2:438-446). Typically, the reactive center (including the P-P') functions as an ideal substrate, with a central residue that matches the proteolytic specificity of its cognate target protease. For example, in some serpins an arginine is characteristic for a thrombin-like protease (such as, e.g., Arg-379 of LP295), or an alanine (Ala) or methionine (Met) is characteristic for an elastase. One of ordinary skill can use this information to determine specific substrate specificity without undue experimentation. In comparing serpins it is useful to designate the reactive center residue of the serpin as P1 with resides N-terminad to it designated as P2, P3, etc. and C-terminad to it designated as P'1, P'2 etc. Using this notation, the reactive center of a serpin is described as lying on a peptide loop that extends at its N-terminad most portion from about P17 (which is located at the end of strand 5 of the A sheet of the serpin) to about P'4at its C-terminad most portion (which is located at the commencement of the strand 1 of the C sheet of the serpin) (see, e.g., R.W. Carrell pp. 1009-1013, especially Fig. S9b, in the section titled "Serpins" in The Encyclopedia of Molecular Biology, ed. Sir J. Kendrew 1994 Blackwell Science Ltd.); however, others have extended these boundaries (e.g., P19- P'9).

Employing such a notational convention here, a reactive center-like loop region of LP295 extends N terminad from the P1 reactive center arginine residue of Arg-379 and C terminad from the P1' arginine residue of Arg-380. Accordingly (using the notation of Scheter & Berger 1967 Biochem Biophys Res Commun 27:157-162), the P17 → P'4 peptide loop of LP295 starts at LP295 Leu-359 (which is the N-terminad (P17) residue) and continues to LP295 Arg-384 (which is the C-terminad (P'4) residue). This portion of LP295 defines a 25 contiguous amino acid region as follows:
(LDVDETGTEATAATGVATVI←(P1)RR(P'1)→QPR) (where the leftward pointing arrow (←) indicates increasing P values as one moves to the left (N-terminad) from the P1 Arg-379 and the rightward pointing arrow (→)indicates increasing P' values as one moves to the right (C-terminad)from the P'1 Arg-380). Given the importance of RCL for serpin functionality, Applicants embodiments include muteins of, in and around this region, particularly LP295 variants as described herein created by mutagenesis around the P1- P'1 region since it is known as conveying to a unique cleavage site target to its cognate binding partner/s. (see, e.g., Biochem Biophys Acta 2000 Jun 15;1479(1-2):237-46 comparing the inhibitory activity of recombinant human kallistatin and two mutants, Phe388Arg (P1) and Phe387Gly (P2), toward human tissue kallikrein. Similar mutagenesis scheme could be carried out here to design; for example, more potent inhibitory activity of LP295. Additionally, since serpin inhibitors behave as suicide substrates that are cleaved early in the interaction with their target enzyme, for example, testing the specificity of LP295 for various suspected substrates (including testing, e.g., the kinetic parameters of their hydrolysis) could be done using internally (and/or intramolecularly) quenched fluorogenic peptides having specific structures containing particular amino acid pairs designed, e.g., based on the described reactive-center loop sequence of LP295 (e.g., from P17 to about P'13). These tests of LP295 can be adapted from methods known in the art, such as, e.g., those taught by, e.g., Pimenta, et al., *Biochem J* 1999 Apr 15;339 (Pt 2):473-9 and Bourgeois, et al., 1997 *J Biol Chem* Nov 21;272(47):29590-5, whose teaching in this regard are incorporated herein by reference.)

The N-terminad side of the loop-like region defined by residues Glu-363 to Thr-372 (ETGTEATAAT) of LP295 is conserved.

The LP295 ETGTEATAAT region exhibits a high degree of sequence identity to the hinge region of serpins (defined as the P15-P9 or P17-P8 portion of the RCL (depending on various authors, see, e.g., Hopkins, et al. 1993 Biochemistry 32:7650-57; Hopkins & Whisstock, 1994 Science 265:1893-1894; and Schick, et al. 1998 Biochemistry 37: 5258-5266). The hinge region appears to be mechanistically critical for the reactive loop of the serpin to undergo a dramatic conformational change upon cleavage (this native to cleaved change of the serpin is called the "stressed to relaxed" (S→R) transition; Carrel & Owen 1985 Nature 317: 730-32. The hinge provides the mobility essential for the conformational change of the RCL in the S→R transition) and for the formation of a characteristically stable 1:1 complex of an inhibitory serpin and its cognate target protease (even under reducing conditions). Characteristically, inhibitory serpins can be recognized by a consensus pattern in their sequences in the hinge region (see, e.g., Gettins, et al. 1996 Serpins: Structure, Function and Biology, RG. Landes, Austin, Texas; Hopkins, et al. 1993 Biochemistry 32:7650-57; and Irving, et al. Genome Research 10:1845-64). One consensus sequence has the following structure: P17=E, P16=E/K/R, P15=G, P14=T/S, P13=E, P12-P9=(A/G/S), in which P15 is usually glycine, P14 is threonine or serine, and positions P12-P9 are occupied by residues with short side chains, such as, e.g., alanine, glycine, or serine. The hinge residues are believed to permit efficient and rapid insertion of the RCL into the A-beta sheet during S→R transition. Comparison of the P17-P8 portion of LP295 (ETGTEATAAT) to such a consensus sequence suggests that this region of LP295 exhibits characteristics of an inhibitory serpin hinge further supporting an assertion that LP295 is an inhibitory-type serpin protein.

Typically, inhibitory serpins are cleaved by their target cognate protease within a reactive center loop region of about 20 amino acids that is located near the C terminus.

The amino acid located N terminad to the scissile bond (the P1 position) appears to be important for determining the specificity of a serpin for its cognate protease. Possession of such a hinge-like region by LP295 suggests that it also has the capacity to inhibit protease activity, like other inhibitory serpins. Classical inhibitory-type serpins are able to form SDS-stable complexes with their target proteases, even in the presence of reducing agents (see, e.g., Potempa et al. 1994 J. Biol Chem. 269: 15957-60; O' Malley et al. 1997 J. Biol Chem. 272: 5354-59; and Schick et al. 1997 J Biol Chem. 272: 1849-55). Accordingly, it would not require undue experimentation to test the ability of LP295 to form complexes with known proteases (and therefore, inhibit their function). For, example, one of ordinary skill in the art could easily use the teachings herein to express the coding region of LP295 by in vitro transcription/translation to incubate the resulting LP295 product with known proteases (e.g., trypsin, CT, HNE, PR3, thrombin, plasmin catG to test serine proteases; or catB, and catL to test cysteine proteases, or catD to test aspartic proteases) to determine if formation of an SDS-stable complex could be demonstrated. Thus, these features in LP295's primary structure additionally reinforce its serpin-like character.

Characteristic elements of a serpin secondary structure map to a portion of LP295 as follows: an A-helix, a B-helix, etc., are indicated respectively, as hA, hB, etc., while a strand 1 of the A beta-sheet, a strand 2 of the A beta-sheet, etc., are indicated respectively, as s1A, s2A, etc. Typically, members of the serpin superfamily are characterized by a dominant A beta-sheet and a mobile reactive loop region that acts as a pseudosubstrate for the serpin cognate proteinase (see, e.g., Wei, et al., 1994 Nat. Struct. Biol. 1, 251-258; and Baumann, et al., 1991 J. Mol. Biol. 218, 595-606).

To avoid confusion, such particular serpin secondary structures (e.g., hA, hB, etc., or s1A, s2A, etc.), which have been mapped onto the LP295 amino acid sequence, are also given as follows: (TLASSNTDFALSLYKKLALRN) = hA = Thr-44 to Asn-64 of LP295; (VVF) = s6B = Val-69 to Phe-71 of LP295; (PLSISAALTILSLG) = hB =Pro-73 to Gly-86 of LP295; (DSTMEEILEGL) = hC = Asp-89 to Leu-99 of LP295; (EEEIHQGFGHLLQRLS) = hD = Glu-108 to Ser-123 of LP295; (QVEINTGSALFI) = s2A = Gln-128 to Ile-139 of LP295; (SEFQEKTRALYQ) = hE = Ser-147 to Gln-158 of LP295; AFVA = s1A = Ala-161 to Ala-164 of LP295; (PNEAKKLINDYVSNQT) = hF = Pro-169 to Thr-184 of LP295; (MVLVNYLLFK) = s3A = Met-201 to Lys-210 of LP295; (IESEFYL) = s4C = Ile-223 to Leu-229 of LP295; (SVKVPMMKIKEVTT) = s3C = Ser-234 to Thr-247 of LP295; (YVR) = s1B = Tyr-249 to Arg-251 of LP295; (CSVLELKY) = s2B = Cys-257 to Tyr-264 of LP295; (ASALFILP) = s3B = Ala-268 to Pro-275 of LP295; (QQVESS) = hG = Gln-281 to Ser-286 of LP295; (PETLKKWK) = hH = Pro-289 to Lys-296 of LP295; (PRIINDLRMP) = s2C = Pro-301 to Pro-310 of LP295; (FSISTDYS) = s6A = Phe-312 to Ser-319 of LP295; (KEVLPEL) = hI = Lys-321 to Leu-327 of LP295; (VVHKAVLDV) = s5A = Val-353 to Val-361 of LP295; (PFMVVITD) = s4B = Pro-390 to Asp-397 of LP295; and (ILFVAKI) = s5B = Ile-403 to Ile-409 of LP295.

A reactive center loop-like region, which is a distinguishing feature of serpins (see, e.g., Carrell & Evans 1992 Curr. Opin. Struct. Biol. 2:438-446), can be found in LP295 and labeled "P" and "P"' (employing the canonical serpin nomenclature for indicating particular amino acid residues of an RCL region of serpins). To avoid confusion, the canonical serpin nomenclature for an RCL-like region of serpins, from about P19 to about P'10, (as it has been mapped onto the LP2295 amino acid sequence) is as follows:

The "P"' region of LP295 is Val-361 = P19; Asp-362 = P18; Glu-363 = P17; Thr-364 = P16; Gly-365 = P15; Thr-366 = P14; Glu-367 = P13; Ala-368 = P12; Thr-369 = P11; Ala-370 = P10; Ala-371 = P9; Thr-372 = P8; Gly-373 = P7; Val-374 = P6; Ala-375 = P5; Thr-376 = P4; Val-377 = P3; Ile-378 = P2, and Arg-379 = P1.

The "P"' region of LP295 is Arg-380 = P'1; Gln-381 = P'2; Pro-382 = P'3; Arg-383 = P'4; Thr-384 = P'5; Leu-385 = P'6; Asn-386 = P'7; Phe-387 = P"8; Asn-388 = P'9, and Arg-389 = P'10.

A putative P1- P'1 reactive center-like region of LP295 is defined by residues Arg-379 to Arg-380 (RR). An interesting portion of the LP295 RCL-like region at the N-terminad side of the reactive loop region is defined by residues Glu-363 to Thr-372 (ETGTEATAAT).

Particularly interesting portions or fragments of a full length LP295 polypeptide include, e.g., a discovered putative signal peptide-like sequence from Met-1 to Ala-17 (MAFIAALGLLMAGICPA). An alternative predicted cleavage site based on a different signal peptide analysis is between Cys-20 and Asp-21 (MAFIAALGLI,MAGICPAVLC).

Additionally interesting portions of LP295 are a discovered serpin-like domain from Ser-42 to Pro-412; a discovered serpin-like signature motif: Leu-385 to Ile-395 (LNFNRPFMVVI); and the discovered serpin protein-like signatures: Asn-68 to Thr-91 (NVVFSPLSISAALTILSLGAKDST); Ala-172 to Phe-192 (AKKLINDYVSNQTQGKIAELF); Thr-199 to Met-240 (TSMVLVNYLLFKGKWKAPFNPNDTIESEFYLDEKRSVKVPMM); Leu-307 to Phe-333 (LRMPKFSISTDYSLKEVLPELGIKKUF); and Asn-388 to Pro-412 (NRPFMWITDMDSQSILFVAKITNP).

Applicants have developed a signature consensus sequence pattern ([LIVMFY]-x-[LIVMFYAC]-[DNQ]-[RKHQS]-[PST]-F-[LIVMFY]-[LIVMFYC]-x-[LIVMFAH]) to identify putative members of the serpin family of proteins. This consensus sequence has been designed around the well-conserved Pro-Phe (P-F) sequence ([PST]-F) that is found ten to fifteen residues C-terminad of the reactive bond (note: in position 6 of the pattern indicated by the brackets enclosing PST; typically, the Pro residue (P) is found in most serpin family proteins). Currently, all known amino acid sequences that have previously been shown to belong to the serpin family of proteins (except for seven) are detected by this consensus sequence. Further, interesting portions of LP295 are the discovered leuserpin 2-like domain signatures: from Thr-104 to Val-129 (TEITEEEIHQGFGHLLQRLSQPEDQV); Glu-231 to Arg-251 (EKRSVKVPMMKIKEVTTPYVR); and Cys-257 to Glu-282 (CSVLELKYTGNASALFILPDQGKMQQ). Leuserpin 2 is a 5-element fingerprint that provides a signature for the heparin cofactor II, a glycoprotein that is involved in the blood coagulation system. Heparin cofactor II (HCII) exists in human plasma and inhibits thrombin rapidly in the presence of dermatan sulphate or heparin (see, e.g., Zhang, et al. 1994 Biochemistry 33: 3632-3642). In the presence of these glycosaminoglycans, HCII becomes the predominant thrombin inhibitor in place of antithrombin III (AT). It also inhibits chymotrypsin, but in a glycosaminoglycan-independent manner. Not only does HCII function as a thrombin inhibitor, but limited proteolysis near its N-terminus yields biologically active peptide(s) that might participate in inflammation and in wound healing and tissue repair processes (see, e.g., Church, et al. 1991 J. Biol. Chem. 266: 704-709). The sequence of HCII contains a signal peptide of 19 amino acids and a mature protein of 480 amino acids (Blinder, et al. 1988 Biochemistry 27: 752-759). The N-terminal portion of HCII contains two acidic repeats (EDDDYLD and EDDDYID) that may bind to anion-binding exosite I of thrombin to facilitate covalent complex formation (Van Deerlin & Tollefsen, 1991 J. Biol. Chem. 266: 20223-20231). The sequence of HCII shares similarity with anti-thrombin III and other members of the alpha 1-antitrypsin superfamily (Blinder, et al. 1988 Biochemistry 27: 752-759).

Analysis of the primary amino acid structure of LP295 demonstrates that LP295 possesses typical serpin protein characteristics, including its homology to known serpins and serpin-like motifs. Consequently, analyzing the data as a whole, LP295's primary structure reinforces the view that it possesses enzymatic-like functionality.

Given LP295's sequence homology to known serine proteases inhibitors, its possession of a serpin-like domain, a serpin-like signature motif, a leuserpin 2-like domain, serpin protein-like signatures, and other primary features found conserved in other serine protease inhibitors (such as, e.g., a conserved hinge-like region (ETGTEATAAT) N-terminad to a serpin-like reactive site), it is likely that LP295 also possesses catalytic properties similar to other serpins (e.g., the ability to form an inhibitory complex with its cognate serine protease target).

Based on the teachings supplied herein (e.g., the LP295 sequence) and those known in the art (e.g., assay methods to determine serpin activities), one skilled in the art would be able to test LP295 for serpin-like activities without undue experimentation (e.g., using common assay techniques and commercially available reagents).

For instance, it would not require undue experimentation to test the ability of LP295 to form SDS-stable complexes (even in the presence of reducing agents) with known protease targets. This type of testing is valuable, since the formation of such complexes is a classical indicator of an inhibitory type serpin. Specifically, one of ordinary skill in the art could easily use the teachings herein to produce LP295 (e.g., by expressing the coding region of LP295 *using in vitro* transcription/translation) to incubate it with known proteases (such as, e.g., trypsin, chymotrypsin (CT), human neutrophil elastase (HNE), protease 3 (PR3), thrombin, plasmin, cathepsinG (catG) to test serine proteases; cathepsins B or L (catB) (catL) to test cysteine proteases, or cathepsin D (catD) to test aspartic proteases; all of which can be commercially obtained, e.g., Athens Research & Technology, Inc., Athens GA or SIGMA) to test for the formation of SDS-stable complexes.

One non-limiting example of a method of testing LP295 for its capacity to form a stable complex with a target protease is conducted as follows: a PBS reaction buffer (0.01 M phospate buffer, 27 mM KCl, 137 mM NaCl, pH 7.4) is used with catG, plasmin, thrombin, and HNE. A cathpesin reaction buffer (50mM sodium acetate (pH5.5), 4mM dithiothreitol, 1mM EDTA) is used with catL, catB, and catD. A buffer of 0.2M Tris-HCI (pH7.8), 20mM CaC12 is used with CT or trypsin. For PR3, a buffer of 200mM NaCl, 50mM Tris-HCl (pH6.7) is used. To generate an LP295 for example, the complete coding region of LP295 is amplified by PCR using a high fidelity Taq system (Boehringer Mannheim, Germany) and cloned via TA-cloning into pGEM (Promega, Mannheim, Germany). The sequence of the resulting LP295 coding region in the resulting recombinant plasmid is confirmed using standard techniques (e.g., sequence analysis). LP295 is expressed *by in vitro* transcription in the presence of [³⁵S]methionine (Amersham) for 90 minutes at 30°C using a coupled reticulocyte lysate system (Promega) and phage T7 RNA polymerase (Promega). The resulting LP295 translation mixture is diluted fivefold with 50mM Tris-HCl (pH7.4). *The in vitro* translated [³⁵S]LP29 is then size separated by consecutive ultrafiltration using a Microcon 100 and a Microcon 30 (Millipore, Eschborn, Germany). The amount of [³⁵S]LP295 in the retentate is calculated over the incorporated [³⁵S]methionine. At a constant concentration, 542 pM of [³⁵S]LP295 is incubated in an appropriate buffer (as described above) with a chosen protease at the following concentrations: 5.0pM, 125pM, and 500pM for the following times: 15 minutes at 25°C and 15 minutes at 37°C. The resulting proteins are mixed with 2X loading buffer (125 mM Tris-HCl (pH6.8), 20% (v/v) glycerol, 4% (w/v) SDS, 5% (v/v) beta-mercaptoethanol, 0.01% (w/v) bromophenol blue), heated to 100°C for four minutes and separated by SDS-PAGE (10% ProSieve 50 gel (FMC, USA, ME)) using a Tris/Tricine electrode buffer system. Any [³⁵S]LP295 is detected by autoradiography using Hyperfilm (Amersham) (see, e.g., Abts, et al. 1999 J. Mol., Biol. 293: 29-39 for additional details and other references listed therein describing similar techniques, all of which are incorporated herein by reference for these teachings.). An LP295 recombinant protein, could easily be tested for its functionality, such as, e.g., inhibitory specificity, resistance to oxidative and proteolytic inactivation, capability of forming serpin-enzyme complexes and/or determining inhibitory specificity with proteases such as, e.g., trypsin, chymotrypsin, and elastase (e.g., neutrophil elastase). One could easily adapt methods known in the art of serpin biology (see, e.g., Huang, et al. 1995 J Neurochem 64(4):1721-27; or Potempa et al., 1995 Biochem J; 306 (Pt 1):191-7).

For instance, testing a protein for trypsin activity is routine in the art and would not require undue experimentation given the teachings supplied herein (e.g., as to the LP295 sequence) and given teachings in the art for methods of determining whether a suspected protein has trypsin-like activity. For instance, LP295 could easily be tested for trypsin-like activities, using synthetic substrates (see, e.g., Yu, et al. 1994 J. Biol. Chem. 269, 18843-18848 and the teachings supplied therein, which are hereby incorporated by reference for these teachings). For example to test LP295 for arginine amidolytic activities one could use the substrate D-Pro-Phe-Arg-MCA and D-Phe-Phe-Arg-MCA. To test for lysine amidolytic activity one would use, for example, a substrate such as succinyl-Ala-Phe-Lys-MCA and t-butyloxycarbonyl-Val-Leu-Lys-MCA. To test for enzymatic activity on chymotrypsin substrates one would use, for example, a substrate such as succinyl-Ala-Ala-Pro-Phe-MCA, Ala-Ala-Phe-AMC, or Suc-Leu-Leu-Val-Tyr-AMC. Trypsin-like activity could be assayed, for example with Boc-Leu-Ser-Thr-Arg-AMC. Other methods for testing are known in the art are easily available. For example, such as those methods described in the journal BioTechniques (September, 1994), entitled "A New Protease Activity Assay Using Fluorescence Polarization." Such techniques are well known in the literature and kits performing such assays are commercially available (see, e.g., the Beacon™ Protease Activity Detection Kit from the PanVera Corporation, Madison, Wisconsin).

Additionally interesting segments of LP295 are discovered fragments Arg-26 to Asp-33; Arg-38 to Tyr-57; Lys-58 to Pro-73; Pro-73 to Ala-87; Ser-90 to Leu-99; Phe-101 to Gly-114; Leu-122 to Phe-138; Lys-141 to Ala-155; Tyr-157 to Asn-170; Asn-170 to Gln-183; Gly-186 to Ser-200; Val-202 to Ser-225; Phe-227 to Thr-247; Tyr-264 to Lys-279; Met-280 to Trp-295; Asp-297 to Glu-322; Glu-130 to Glu-148; Asn-177 to Ala-189; Arg-198 to Phe-209; Phe-209 to Thr-222; Thr-222 to Arg-233; Val-245 to Cys-257; Ser-258 to Ile-273; Asp-276 to Pro-301; Arg-302 to Lys-330; Ser-334 to His-355; Thr-366 to Val-377; Ile-378 to Val-393; Ala-6 to Val-18; Cis-20 to His-41; Leu-45 to Phe-52; Leu-62 to Val-69; Gly-86 to Glu-94; Thr-104 to Gly-114; Leu-122 to Ser-135; Ser-147 to Ala-161; Ala-164 to Leu-175; Asn-177 to Ala-189; Met-201 to Lys-210; Lys-212 to Glu-226; Phe-227 to Val-237; Ile-242 to Ser-258; Asp-276 to Ser-298; Ser-313 to Glu-322; Lys-331 to Tyr-349; Leu-359 to Thr-376; and Ile-378 to Asn-388, whose discovery was based on an analysis of hydrophobicity, hydropathicity, and hydrophilicity plots. Additional interesting sections of LP295 are the discovered portions of LP295 from Ala-6 to Gly-25; Thr-28 to Ala-46; Ala-53 to Asp-66; Asn-68 to Thr-81; Gly-86 to Lys-100; Phe-101 to Phe-115; Pro-125 to Phe-138; Phe-138 to Gln-150; Glu-151 to Ala-164; Gln-168 to Asp-178; Tyr-179 to Ser-193; Leu-195 to Tyr-206; Asn-218 to Tyr-228; Val-235 to Thr-247; Ala-268 to Leu-287; Glu-288 to Ser-298; Leu-299 to Ser-315; Leu-348 to Val-361; Leu-385 to Lys-408; Leu-19 to Ala-46; Leu-24 to Lys-36; Arg-26 to Lys-36; Gly-37 to Leu-45; Asn-68 to Leu-74; Lys-88 to Gly-99; Ser-123 to Ala-136; Leu-146 to Glu-160; Asn-170 to Gln-183; Thr-184 to Asn-194; Pro-216 to Asp-230; Val-235 to Ile-242; Glu-244 to Ser-256; Ser-256 to Lys-263; Met-280 to Leu-292; Phe-312 to Leu-324; Gln-335 to Lys-346; and Asp-338 to Val-377. These LP295 fragments were discovered based on analysis of antigenicity plots. Further, particularly interesting LP295 structures (e.g., such as a helix, a strand, or a coil) that have been discovered are the following LP295 helix structures: Ile-4 to Leu-9; Phe-52 to Ala-61; Thr-91 to Gly-98; Thr-107 to Phe-115; His-117 to Arg-121; Ile-145 to Ala-164; Glu-171 to Asp-178; Ile-188 to Glu-196; Asn-205 to Leu-208; Gln-281 to Ser-285; Glu-290 to Lys-296; Pro-301 to Ile-304; Lys-330 to Ser-334; and Gln-352 to Lys-356. Particularly interesting discovered coil structures are Ile-14 to Pro-16; Asp-21 to Asp-27; Glu-37 to Arg-38; Ala-46 to Thr-50; Arg-63 to Asn-68; Leu-85 to Lys-88; Lys-100 to Thr-104; Ser-123 to Asp-127; Asn-132 to Ser-135; Asp-140 to Glu-142; Lys-167 to Gln-168; Lys-212 to Thr-222; Thr-246 to Leu-255; Tyr-264 to Ala-268; Pro-275 to Gly-278; Arg-308 to Asp-317; Thr-343 to Asp-347; Val-361 to Thr-366; Gln-381 to Pro-390; Met-398 to Ser-400; and Ile-409 to Lys-413. Particularly interesting discovered strand structures are Ala-17 to Leu-19; Val-129 to Ile-131; Ala-270 to Ile-273; and Met-392 to Ile-395. Further encompassed by the invention are contiguous amino acid residue combinations of any of the predicted secondary structures described above. For example, one helix-coil-coil-strand-coil motif of LP295 combines the Gln-352 to Lys-356 helix, with the Val-361 to Thr-366 and Gln-381 to Pro-390 coils, and the Met-392 to Ile-395 strand to form an interesting fragment of contiguous amino acid residues from Gln-352 to Ile-395. Other combinations of contiguous amino acids are contemplated as can be easily determined.

Table 19 summarizes information corresponding to each "LP No." of the invention as described herein. The column labeled, "Total NT Seq." refers to the total number of nucleotides in a polynucleotide sequence identified by an "LP No." The nucleotide position of SEQ ID NO: X of the putative start codon (methionine) is identified as "5' NT of Start Codon." Similarly, the nucleotide position of SEQ ID NO: X of a predicted signal sequence of an LP protein or polypeptide is identified as "5' NT of First AA of Signal Pep."

The corresponding translated amino acid sequence of a particular NT SEQ ID NO:X, typically beginning with the methionine, is identified as "AA SEQ ID NO: Y," although other reading frames can also be easily translated using techniques known in molecular biology. A polypeptide produced using an alternative open reading frame/s is also specifically encompassed by the present invention. The first and last amino acid position of a SEQ ID NO: Y of the predicted signal peptide is identified as "First AA of Signal Pep" and "Last AA of Signal Pep." The predicted first amino acid position of SEQ ID NO: Y of the secreted portion is identified as "Predicted First AA of Secreted Portion." Finally, the amino acid position of SEQ ID NO: Y of the last amino acid in the open reading frame is identified as "Last AA of ORF."

An LP polypeptide or fragment thereof, identified from SEQ ID NO: Y may be used, e.g., as an immunogen to generate an antibody that specifically and/or selectively binds a protein comprising an LP polypeptide sequence (or fragment thereof) of the invention and/or to a mature LP polypeptide or secreted LP protein, e.g., encoded by a polynucleotide sequence described herein. An LP polypeptide of the invention can be prepared in any manner suitable to those known in the art. Such a polypeptide includes, e.g., naturally occurring polypeptides that are isolated, recombinantly produced polypeptides, synthetically produced polypeptides, or polypeptides produced by any combination of these methods. Means for preparing such polypeptides are well understood in the art. An LP polypeptide (or fragment thereof) may be in the form of, a mature polypeptide, a secreted protein (including the mature form), or it may be a fragment thereof, or it may be a part of a larger polypeptide or protein, such as, e.g., a fusion protein.

It is often advantageous to include with an LP polypeptide (or fragment thereof), e.g., additional amino acid sequence that contains, e.g., secretory or leader sequences, prosequences, sequences that aid in purification, such as, e.g., multiple histidine residues, or an additional sequence for stability during recombinant production. Such variants are also encompassed herein. An LP polypeptide (or fragment thereof) is preferably provided in an isolated or recombinant form, or it may be preferably substantially purified. A recombinantly produced version of an LP polypeptide of the invention, including a secreted polypeptide, can be substantially purified using techniques described herein or otherwise known in the art, such as, e.g., the single-step purification method (Smith and Johnson (1988) Gene 67(1) :31-40). An LP polypeptide (or fragment thereof) can also be purified from natural, synthetic or recombinant sources using techniques described herein or otherwise known in the art, such as, e.g., using an antibody of the invention raised against a secreted protein. The present invention provides an isolated or recombinant LP polynucleotide comprising, or alternatively consisting of, a nucleic acid molecule having a mature polynucleotide sequence of SEQ ID NO: X wherein said polynucleotide sequence or said cDNA encodes at least 12 contiguous amino acids of a mature polypeptide of SEQ ID NO: Y.

### II. Definitions

### LP polynucleotide

As used herein, the term **"LP polynucleotide"** refers to a molecule comprising a nucleic acid sequence contained in a Table herein or in a sequence of SEQ ID NO:X. For example, the polynucleotide can contain the nucleotide sequence of the full length cDNA sequence, including the 5' and 3' untranslated sequences, the coding region, with or without the signal sequence, the secreted protein coding region, as well as fragments, epitopes, domains, and variants of the nucleic acid sequence. An **"LP polynucleotide"** also encompasses, e.g., those polynucleotides that stably hybridize, under stringent hybridization conditions to an LP sequence of a table herein, or to a sequence contained in SEQ ID NO:X. In specific embodiments, an LP polynucleotide sequence is at least 15, at least 30, at least 50, at least 100, at least 125, at least 500, or at least 1000 contiguous nucleotides but are less than or equal to 300 kb, 200 kb, 100 kb, 50 kb, 15 kb, 10 kb, 7.5 kb, 5 kb, 2.5 kb, 2.0 kb, or 1 kb, in length.

An LP polynucleotide sequence can be composed of any polyribonucleotide or polydeoxribonucleotide, which may be unmodified RNA or DNA or modified RNA or DNA. For example, polynucleotides can be composed of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single-and double-stranded regions. In addition, the polynucleotide can be composed of triple-stranded regions comprising RNA or DNA or both RNA and DNA. A polynucleotide may also contain one or more modified bases or DNA or RNA backbones modified for stability or for other reasons. **"Modified"** bases can include, e.g., for example, tritylated bases and unusual bases such as inosine. A variety of modifications can be made to DNA and RNA; thus, the term "polynucleotide" embraces chemically, enzymatically, or metabolically modified forms. **"Altered"** nucleic acid sequences encoding LP include those sequences with deletions, insertions, or substitutions of different nucleotides, resulting in a polypeptide the same as LP or a polypeptide with at least one functional characteristic of LP. Included within this definition are polymorphisms which may or may not be readily detectable using a particular oligonucleotide probe of the polynucleotide encoding LP, and improper or unexpected hybridization to allelic variants, with a locus other than the normal chromosomal locus for the polynucleotide sequence encoding LP.

**"Substantial similarity"** in a nucleic acid sequence comparison context means either that the segments, or their complementary strands, when compared, are identical when optimally aligned, with appropriate nucleotide insertions or deletions, in at least about 50% of the nucleotides, generally at least 56%, more generally at least 59%, ordinarily at least 62%, more ordinarily at least 65%, often at least 68%, more often at least 71%, typically at least 74%, more typically at least 77%, usually at least 80%, more usually at least about 85%, preferably at least about 90%, more preferably at least about 95 to 98% or more, and in particular embodiments, as high at about 99% or more of the nucleotides. Alternatively, substantial similarity exists when the segments will hybridize under selective hybridization conditions, to a strand, or its complement, typically using a sequence derived from SEQ ID X. Typically, selective hybridization will occur when there is at least about 55% similarity over a stretch of at least about 30 nucleotides, preferably at least about 65% over a stretch of at least about 25 nucleotides, more preferably at least about 75%, and most preferably at least about 90% over about 20 nucleotides. See Kanehisa (1984) Nuc. Acids Res. 12:203-213. The length of similarity comparison, as described, may be over longer stretches, and in certain embodiments will be over a stretch of at least about 17 nucleotides, usually at least about 20 nucleotides, more usually at least about 24 nucleotides, typically at least about 28 nucleotides, more typically at least about 40 nucleotides, preferably at least about 50 nucleotides, and more preferably at least about 75 to 100 or more nucleotides, e.g., 150, 200, etc.

For sequence comparison, typically one sequence acts as a reference sequence, to which test sequences are compared. When using a sequence comparison algorithm, test and reference sequences are input into a computer, subsequent coordinates are designated, if necessary, and sequence algorithm program parameters are designated. The sequence comparison algorithm then calculates the percent sequence identity for the test sequence(s) relative to the reference sequence, based on the designated program parameters. Optical alignment of sequences for comparison can be conducted, e.g., by the local homology algorithm of Smith and Waterman (1981) Adv. Appl. Math. 2:482, by the homology alignment algorithm of Needlman and Wunsch (1970) J. Mol. Biol. 48:443, by the search for similarity method of Pearson and Lipman (1988) Proc. Nat'l Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by visual inspection (see generally Ausubel et al., supra). One example of a useful algorithm is PILEUP. Another example of algorithm that is suitable for determining percent sequence identity and sequence similarity is the BLAST algorithm, which is described Altschul, et al. (1990) J. Mol. Biol. 215:403-410. A further indication that two nucleic acid sequences of polypeptides are substantially identical is that the polypeptide encoded by the first nucleic acid is immunologically cross reactive with the polypeptide encoded by the second nucleic acid. Another indication that two nucleic acid sequences are substantially identical is that the two molecules hybridize to each other under stringent conditions.

**"Homologous"** polynucleotide sequences, when compared, exhibit significant similarity (e.g., sequence identity at the nucleotide level). Generally, standards for determining homology between nucleic acid molecules (or polynucleotide sequences) use art known techniques which examine, e.g., the extent of structural similarity or sequence identity between polynucleotide sequences; and/or that determine a phylogenetic relationship (e.g., whether compared sequences are orthologs or paralogs); and/or that are based on the ability of sequences to form a hybridization complex. Hybridization conditions are described in detail herein.

**"Hybridization"** refers to the process by which a polynucleotide strand anneals with a complementary strand through base pairing under defined hybridization conditions. Specific hybridization is an indication that two nucleic acid sequences share a high degree of similarity and/or identity. Specific hybridization complexes form under permissive annealing conditions and remain hybridized after "washing." Washing is particularly important in determining the stringency of the hybridization process, typically, with more stringent conditions allowing less non-specific binding (e.g., binding between polynucleotide sequences that demonstrate less sequence identity or similarity). Permissive conditions for annealing of nucleic acid sequences are routinely determinable by one of ordinary skill in the art and may be consistent among hybridization experiments, whereas wash conditions may be varied among experiments to achieve a desired stringency, and therefore, a particular hybridization specificity.

**"Stringent conditions,"** when referring to homology or substantial similarity and/or identity in the hybridization context, will be stringent combined conditions of salt, temperature, organic solvents, and other parameters, typically those controlled in hybridization reactions. Stringent temperature conditions will usually include temperatures in excess of about 30°C, more usually in excess of about 37°C, typically in excess of about 40°C, characteristically in excess of about 42°C, routinely in excess of about 45°C, usually in excess of about 47°C, preferably in excess of about 50°C, more typically in excess of about 55°C, characteristically in excess of about 60°C, preferably in excess of about 65°C, and more preferably in excess of about 70°C In this context, the term "about" includes, e.g., a particularly recited temperature (e.g., 50°C), and/or a temperature that is greater or lesser than that of the stated temperature by, e.g., one, two, three, four, or five degrees Celsius (e.g., 49°C or 51°C). Stringent salt conditions will ordinarily be less than about 500 mM, usually less than about 450 mM, even more usually less than about 400 mM, more usually less than about 350 mM, even more usually less than about 300 mM, typically less than about 250 mM, even more typically less than about 200 mM, preferably less than about 100 mM, and more preferably less than about 80 mM, even down to less than about 20 mM. In this context, the term "about" includes, e.g., a particularly recited molarity (e.g., 400 mM), and/or a molarity that is greater or lesser than that of the stated molarity by, e.g., three, five, seven, nine, eleven or fifteen millimolar (e.g., 389 mM or 415 mM). It is to be remembered that the combination of parameters is more important than the measure of any single parameter (see, e.g., Wetmur and Davidson (1968) J. Mol. Biol. 31:349-370).

A nucleic acid probe that binds to a target nucleic acid under stringent conditions to form a stable hybridization complex is said to be specific for said target nucleic acid. Preferably, hybridization under stringent conditions should give a signal of at least 2-fold over background, more preferably a signal of at least 3 to 5-fold over background or more. Typically, a hybridization probe is more than 11 nucleotides in length and is sufficiently identical (or complementary) to the sequence of the target nucleic acid (over the region determined by the sequence of the probe) to bind the target under stringent hybridization conditions to form a detectable stable hybridization complex. The term "hybridization complex" refers to a complex formed between two nucleic acid molecules by virtue of the formation of hydrogen bonds between complementary bases. A hybridization complex may be formed in solution (e.g., C₀t or R₀t analysis) or formed between one nucleic acid sequence present in solution and another nucleic acid sequence immobilized on a solid support (such as, e.g., without limitation, paper, plastic, a membrane, a filter, a chip, a pin, glass, or any other appropriate substrate to which cells or their nucleic acids can be complexed with either covalently or non-covalently).

An equation for calculating Tₘ and conditions for nucleic acid hybridization are well known (see, e.g., Sambrook, et al. (1990) Molecular Cloning: A Laboratory Manual (cur. ed.) Vol. 1-3, Cold Spring Harbor Laboratory, Cold Spring Harbor Press, NY, which is incorporated herein by reference and hereinafter referred to as "Sambrook, et al."). A non-limiting example of a high stringency condition of the invention comprises including a wash condition of 68°C in the presence of about 0.2 x SSC and about 0.1% SDS, for 1 hour. Alternatively, temperatures of about 67°C, 63°C, 61°C, 59°C, 57°C, 53°C, 51°C, 49°C, 47°C, 43°C, or 41°C may be used. SSC concentration may be varied from about 0.1 to 2X SSC, with SDS being present at about 0.1%. Typically, blocking reagents are used to block non-specific hybridization. Such blocking reagents include, for instance, sheared, and denatured salmon sperm DNA at about 100-200 ug/ml. Organic solvent, such as, e.g., formamide at a concentration of about 35-50% v/v, may also be used under particular circumstances, such as for a RNA:DNA hybridization. Useful variations on these wash conditions will be readily apparent to those of ordinary skill in the art. Hybridization, particularly under high stringency conditions, may be suggestive of evolutionary similarity between the nucleotides. Such similarity is indicative of a similar functional and/or biological role for the nucleotide sequence and its correspondingly encoded polypeptide sequence.

Another non-limiting example of a stringent hybridization condition comprises, e.g., an overnight incubation at 42°C in a solution comprising 50% formamide, 5x SSC (750 mM NaCl, 75 mM trisodium citrate), 50 mM sodium phosphate (pH 7.6), 5x Denhardt's solution, 10% dextran sulfate, and 20 pg/ml denatured, sheared salmon sperm DNA, followed by washing the filters in 0. 1x SSC at about 65°C. Also contemplated are nucleic acid molecules that hybridize to an LP polynucleotide sequence at lower stringency hybridization conditions. Changes in the stringency of hybridization and signal detection can be accomplished through the manipulation of formamide concentration (lower percentages of formamide result in lowered stringency); salt conditions, or temperature. For example, an alternate stringency condition can comprise, e.g., an overnight incubation at 37°C in a solution comprising 6X SSPE (20X SSPE = 3M NaCl; 0.2M NaH,PO, 0.02M EDTA, pH 7.4), 0.5% SDS, 30% formamide, 100ml salmon sperm blocking DNA; followed by washes at 50°C with 1X SSPE, 0.1% SDS. In addition, to achieve another alternate stringency condition, washes are performed following stringent hybridization at higher salt concentrations (e.g. 5X SSC). Note that variations in the above conditions may be accomplished through the inclusion and/or substitution of alternate blocking reagents used to suppress background in hybridization experiments. Typical blocking reagents include, e.g., Denhardt's reagent, BLOTTO, heparin, denatured salmon sperm DNA, and commercially available proprietary formulations. The inclusion of specific blocking reagents may require modification of a hybridization conditions described herein. A polynucleotide that hybridizes only to polyA+ sequences (such as any 3' terminal polyA+ tract of a cDNA of the invention), or to a complementary stretch of T (or U) residues, is not included, e.g., in the definition of an "LP polynucleotide" since such a polynucleotide would hybridize to any nucleic acid molecule containing a poly (A) stretch or the complement thereof (i.e., practically any double-stranded cDNA clone generated using oligo dT as a primer).

Still another non-limiting example of a stringent hybridization condition is one that employs, e.g.: low ionic strength and high temperature for washing (e.g., 15mM sodium chloride/1.5 mM sodium citrate/0.1% sodium dodecyl sulfate at 50°C); a denaturing agent (during hybridization) such as formamide (e.g., 50% (v/v) formamide with 0.1% bovine serum albumin/0.1% ficoll/0.1% polyvinylpyrrolidone/50 mM sodium phosphate buffer at pH 6.5 with 750 mM sodium chloride/75 mM sodium citrate at 42°C); or 50% formamide, 5X SSC (750 mM sodium chloride, 75 mM sodium citrate), 50 mM sodium phosphate (pH 6.8), 0.1% sodium pyrophosphate, 5X Denhardt's solution, sonicated salmon sperm DNA (50 µg/mL), 0.1% SDS, and 10% dextran sulfate at 42°C with washes at 42°C in 0.2X SSC (30 mM sodium chloride/3 mM sodium citrate) and 50% formamide at 55°C, followed by a high-stringency wash consisting of 0.1X SSC containing EDTA at 55°C.

An **"isolated"** nucleic acid is a nucleic acid molecule or a polynucleotide sequence (e.g., an RNA, DNA, cDNA, genomic DNA, or a mixed polymer) which is substantially separated from other biologic components that naturally accompany a native sequence (e.g., proteins and flanking genomic sequences from the originating species). In a preferable embodiment, the isolated LP sequence is free of association with components that can interfere with diagnostic or therapeutic uses for the sequence including, e.g., enzymes, hormones, and other proteinaceous or non-proteinaceous agents. The term embraces a polynucleotide sequence removed from its naturally occurring environment. For example, an isolated polynucleotide sequence could comprise part of a vector or a composition of matter, or could be contained within a cell, and still be "isolated" because the vector, composition of matter, or cell is not the original environment of the polynucleotide sequence. Moreover, the term encompasses recombinant or cloned DNA isolates, chemically synthesized analogs, or analogs biologically synthesized using heterologous systems. Furthermore, the term includes both double-stranded and single-stranded embodiments. If single-stranded, the polynucleotide sequence may be either the "sense" or the "antisense" strand. A substantially pure molecule includes isolated forms of the molecule.

An isolated nucleic acid molecule will usually contain homogeneous nucleic acid molecules, but, in some embodiments, it will contain nucleic acid molecules having minor sequence heterogeneity. Typically, this heterogeneity is found at the polymer ends or portions of the LP sequence that are not critical to a desired biological function or activity.

The term **"isolated"** does not refer to genomic or cDNA libraries, whole cell total or mRNA preparations, genomic DNA preparations (including those separated by electrophoresis and transferred onto blots), sheared whole cell genomic DNA preparations, or other compositions where the art demonstrates no distinguishing features of a LP polynucleotide sequence of the present invention.

A **"recombinant"** nucleic acid or polynucleotide sequence is defined either by its method of production or its structure. In reference to its method of production, e.g., a product made by a process, the process is use of any genetic engineering technique, e.g., products made by transforming cells with any non-naturally occurring vector are encompassed, as are nucleic acids comprising sequence derived using any synthetic oligonucleotide process. A similar concept is intended for a recombinant LP polypeptide. Specifically included are synthetic nucleic acid molecules which, due to the redundancy of the genetic code, encode polypeptides similar to fragments of these antigens, and fusions of sequences from various different species variants.

### LP protein

As used herein, an **"LP protein"** shall encompass, when used in a protein context, a protein or polypeptide having an amino acid sequence shown in SEQ ID NO: Y or a significant fragment of such a protein or polypeptide, preferably a natural embodiment. The term **"protein"** or **"polypeptide"** is meant any chain of contiguous amino acid residues, regardless of length or postranslation modification (e.g., glycosylation, or phosphorylation).

Further, an LP protein or an LP polypeptide encompass polypeptide sequences that are pre- or pro-proteins. Moreover, the present invention encompasses a mature LP protein, including a polypeptide or protein that is capable of being directed to the endoplasmic reticulum (ER), a secretory vesicle, a cellular compartment, or an extracellular space typically, e.g., as a result of a signal sequence, however, a protein released into an extracellular space without necessarily having a signal sequence is also encompassed. Generally, the polypeptide undergoes processing, e.g., cleavage of a signal sequence, modification, folding, etc., resulting in a mature form (see, e.g., Alberts, et al. (1994) Molecular Biology of The Cell, Garland Publishing, New York, NY, pp. 557-560, 582-592.).

The invention also embraces polypeptides that exhibit similar structure to an LP polypeptide (e.g., one that interacts with an LP protein specific binding composition). These binding compositions, e.g., antibodies, typically bind an LP protein with high affinity, e.g., at least about 100 nM; usually, better than about 30 nM; preferably, better than about 10 nM; and more preferably, at better than about 3 nM.

### Modifications

An LP polypeptide can be composed of amino acids joined to each other by peptide bonds or modified peptide bonds, i.e., peptide isosteres, and may contain amino acids other than the 20 gene-encoded amino acids. The polypeptides may be modified by either natural processes, such as post-translational processing, or by chemical modification techniques that are well known in the art. Such modifications are well described in basic texts and in more detailed monographs, as well as in a voluminous research literature.

Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched, for example, as a result of ubiquitination, and they may be cyclic, with or without branching. Cyclic, branched, and branched cyclic polypeptides may result from posttranslation natural processes or may be made by synthetic methods. Modifications include, e.g., acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination (see, e.g., Creighton (1993) 2nd ed. Proteins-Structure and Molecular Properties, W. H. Freeman and Company, New York; Johnson (1983) ed. Posttranslational Covalent Modification of Proteins, Academic Press, New York, pp. 1-12; Seifter et al. (1990) Meth Enzymol 182:626-646; Rattan et al. (1992) Ann NY Acad Sci 663:48XX).

The encoded protein may also be **"altered,"** and may contain deletions, insertions, or substitutions of amino acid residues that produce a silent change and result in a functionally equivalent LP. Deliberate amino acid substitutions may be made based on similarity in polarity, charge, solubility, hydrophobicity, hydrophilicity, and/or the amphipathic nature of the residues, as long as the biological or immunological activity of the LP is retained. For example, negatively charged amino acids may include aspartic acid and glutamic acid, and positively charged amino acids may include lysine and arginine. Amino acids with uncharged polar side chains having similar hydrophilicity values may include: asparagine and glutamine; and serine and threonine. Amino acids with uncharged side chains having similar hydrophilicity values may include: leucine, isoleucine, and valine; glycine and alanine; and phenylalanine and tyrosine.

**"Substantially pure"** refers to LP nucleic acid or LP protein or polypeptide that are removed from their natural environment and are isolated and/or separated from other contaminating proteins, nucleic acids, and other biologicals. Purity may be assayed by standard methods, and will ordinarily be at least about 50% pure, more ordinarily at least about 60% pure, generally at least about 70% pure, more generally at least about 80% pure, often at least about 85% pure, more often at least about 90% pure, preferably at least about 95% pure, more preferably at least about 98% pure, and in most preferred embodiments, at least 99% pure. Similar concepts apply, e.g., to LP antibodies or nucleic acids of the invention. For example, it may be desirable to purify an LP polypeptide from recombinant cell proteins or polypeptides. Various art known methods of protein purification may be employed (see, e.g., Deutscher, (1990) Methods in Enzymology 182: 83-9 and Scopes, (1982) Protein Purification: Principles and Practice, Springer-Verlag, NY.)

**"Solubility"** of an LP protein or polypeptide is reflected by sedimentation measured in Svedberg units, which are a measure of the sedimentation velocity of a molecule under particular conditions (see, Freifelder (1982) Physical Biochemistry (2d ed.) W.H. Freeman & Co., San Francisco, CA; and Cantor and Schimmel (1980) Biophysical Chemistry parts 1-3, W.H. Freeman & Co., San Francisco, CA). A soluble particle or polypeptide will typically be less than about 30S, more typically less than about 15S, usually less than about 10S, more usually less than about 6S, and, in particular embodiments, preferably less than about 4S, and more preferably less than about 3S. Solubility of a polypeptide or fragment depends upon the environment and the polypeptide. Many parameters affect polypeptide solubility, including temperature, electrolyte environment, size and molecular characteristics of the polypeptide, and nature of the solvent. Typically, the temperature at which the polypeptide is used ranges from about 4° C to about 65° C. Usually the temperature at use is greater than about 18° C and more usually greater than about 22° C. For diagnostic purposes, the temperature will usually be about room temperature or warmer, but less than the denaturation temperature of components in the assay. For therapeutic purposes, the temperature will usually be body temperature, typically about 37° C for humans, though under certain situations the temperature may be raised or lowered in situ *or in vitro.*

The size and structure of the polypeptide should generally be in a substantially stable state, and usually not in a denatured state. The polypeptide may be associated with other polypeptides in a quaternary structure, e.g., to confer solubility, or associated with lipids or detergents in a manner which approximates natural lipid bilayer interactions.

The solvent will usually be a biologically compatible buffer, of a type used for preservation of biological activities, and will usually approximate a physiological solvent. Usually the solvent will have a neutral pH, typically between about 5 and 10, and preferably about 7.5. On some occasions, a detergent will be added, typically a mild non-denaturing one, e.g., CHS (cholesteryl hemisuccinate) or CHAPS (3-[3-cholamidopropyl)-dimethylammonio]-1-propane sulfonate), or a low enough concentration as to avoid significant disruption of structural or physiological properties of the protein.

### Signal Sequence

The present invention encompasses **"mature"** forms of a polypeptide comprising a polypeptide sequence listed in a Table herein, or a polypeptide sequence of SEQ ID NO: Y. Methods for predicting whether a protein has a signal sequence, as well as the cleavage point for that sequence, are known in the art (see, e.g., McGeoch, 1985 Virus Res. 3:271-286 and Henrik Nielsen et al. (1997) Protein Engineering 10:1-6). Employing such known art methods a signal sequence for an LP polypeptide was made. However, cleavage sites may vary and cannot be predicted with absolute certainty. Accordingly, the present invention provides secreted LP polypeptides having a sequence listed in a Table herein, or a polypeptide sequence of SEQ ID NO: Y, in which a particular N-terminus variant polypeptide sequence can begin within five, four, three, two, or one amino acid residues (e.g., +5, +4, +3, +2, +1, or -5, -4, -3, -2, -1) from a particular cleavage point designated as such herein. Similarly, it is also recognized that in some cases, cleavage of a signal sequence of a secreted protein is not uniform, resulting in more than one secreted species for a given protein (e.g., a cleavage variant). Such cleavage variant LP polypeptides, and the polynucleotides encoding them, are also encompassed by the present invention.

Moreover, the signal sequence identified by the above analysis may not necessarily predict a naturally occurring signal sequence. For example, a naturally occurring signal sequence may be further upstream from a predicted signal sequence. However, it is likely that a predicted signal sequence will be capable of directing the secreted protein to the ER Nevertheless, the present invention encompasses a mature LP polypeptide or protein produced by expression of a polynucleotide sequence listed in a Table herein or an LP polynucleotide sequence of SEQ ID NO: X. These LP polypeptides (and fragments thereof), and the polynucleotides encoding them, are also encompassed by the present invention.

### LP Variants

The present invention encompasses variants of an LP polynucleotide sequence disclosed in a table herein or SEQ ID NO: X and/or the complementary strand thereto. The present invention also encompasses variants of a polypeptide sequence disclosed in a table herein or SEQ ID NO: Y. The term **"variant"** refers to a polynucleotide or polypeptide differing from an LP polynucleotide sequence or an LP polypeptide of the present invention, but retaining essential properties thereof. Generally, variants are closely similar overall in structural and/or sequence identity, and, in many regions, identical to an LP polynucleotide or polypeptide of the present invention. For example, the present invention encompasses nucleic acid molecules that comprise, or alternatively consist of, a polynucleotide sequence that is at least: 80%, 85%, 90%, 95%, 96%, 97%, 98%, or 99% identical to, e.g., a polynucleotide coding sequence of SEQ ID NO: X (or a strand complementary thereto); a nucleotide sequence encoding a polypeptide of SEQ ID NO: Y; and/or polynucleotide fragments of any of these nucleic acid molecules (e.g., a fragment as defined herein). Polynucleotides, that stably hybridize to a polynucleotide fragment (as defined herein) under stringent hybridization conditions or lower stringency conditions, are also encompassed by the invention, as are polypeptides (or fragments thereof) encoded by these polynucleotides.

The present invention is also directed to polypeptides that comprise, or alternatively consist of, an amino acid sequence that is at least: 80%, 85%, 90%, 95%, 96%, 97%, 98%, 99% identical to, e.g., a polypeptide sequence of SEQ ID NO: Y (or fragments thereof); a polypeptide sequence encoded by a cDNA contained in a deposited clone, and/or a polypeptide fragment of any of these polypeptides (e.g., those fragments as defined herein). A polynucleotide sequence having at least some "percentage identity," (e.g., 95%) to another polynucleotide sequence, means that the sequence being compared (e.g., the test sequence) may vary from another sequence (e.g. the referent sequence) by a certain number of nucleotide differences (e.g., a test sequence with 95% sequence identity to a reference sequence can have up to five point mutations per each 100 contiguous nucleotides of the referent sequence). In other words, for a test sequence to exhibit at least 95% identity to a referent sequence, up to 5% of the nucleotides in the referent may differ, e.g., be deleted or substituted with another nucleotide, or a number of nucleotides (up to 5% of the total number of nucleotides in the reference sequence) may be inserted into the reference sequence. The test sequence may be: an entire polynucleotide sequence, e.g., as shown in a Table herein, the ORF (open reading frame), or any fragment, segment, or portion thereof (as described herein). As a practical matter, determining if a particular nucleic acid molecule or polynucleotide sequence exhibits at least about: 80%, 85%, 90%, 95%, 96%, 97%, 98% or 99% identity to an LP polynucleotide sequence can be accomplished using any art known method.

Variants encompassed by the present invention may contain alterations in the coding regions, non-coding regions, or both. Moreover, variants in which 1-2,1-5, or 5-10 amino acids are substituted, deleted, or added in any combination are also preferred. Of course, in order of ever-increasing preference, it is highly preferable for a peptide or polypeptide to have an amino acid sequence that comprises an amino acid sequence of the present invention, which contains at least: one, but not more than: 10, 9, 8, 7, 6, 5, 4, 3, 2, or 1 amino acid substitutions. In specific embodiments, the number of additions, substitutions, and/or deletions in an polypeptide sequence of the present invention or fragments thereof (e.g., a mature form and/or other fragments described herein), is at least:1, 2, 3, 4, 5, 6, 7, 8, 9, 10, 11, 12, 13, 14, 15, 16, 17, 18, 19, 20, 21, 22, 23, 24, 25, 10-50, or 50-150; wherein conservative amino acid substitutions are more preferable than non-conservative substitutions.

### LP Polynucleotide and LP Polypeptide Fragments

The present invention is also directed to fragments of an LP polynucleotide. An LP polynucleotide **"fragment"** encompasses a short polynucleotide of a nucleic acid molecule, or a portion of a polynucleotide sequence of SEQ ID NO: X or a complementary strand thereto, or a portion of a polynucleotide sequence encoding a polypeptide of SEQ ID NO: Y (or fragment thereof). Polynucleotide fragments of the invention encompass a polynucleotide sequence that is preferably at least about 15 nucleotides, more preferably at least about: 20, 21, 22, 24, 26, or 29 nucleotides, favorably at least about: 30, 32, 34, 36, 38, or 39 nucleotides, and even more preferably, at least about: 40, 42, 44, 46, 48, or 49 nucleotides, desirably at least about: 50, 52, 54, 56, 58, or 59 nucleotides, particularly at least about 75 nucleotides, or at least about 150 nucleotides in length.

A polynucleotide fragment **"at least 20 nucleotides in length,"** e.g., is intended to include, e.g., 20 or more contiguous bases from a nucleotide sequence shown in SEQ ID NO: X or in a Table herein. In this context **"at least about"** includes, e.g., a specifically recited value (e.g., 20nt), and a value that is larger or smaller by one or more nucleotides (e.g., 5, 4, 3, 2, or 1), at either terminus or at both termini. A polynucleotide fragment has use that includes without limit; e.g., diagnostic probes and primers as discussed herein. Larger fragments (e.g., 50, 150, 500, 600, or 2000 nucleotides) are also useful and preferred.

Representative examples of various lengths of polynucleotide fragments encompassed by the invention, include, e.g., fragments comprising, or alternatively consisting of, a polynucleotide sequence of SEQ ID NO:X from about nucleotide number 1-50, 51-100, 101-150, 151-200, 201-250, 251-300, 301-350, 351-400, 401-450, 451-500, 501-550, 551-600, 651-700, 701-750, 751-800, 800-850, 851-900, 901-950, 951-1000, 1001-1050, 1051-1100, 1101-1150, 1151-1200, 1201-1250, 1251-1300, 1301-1350, 1351-1400, 1401-1450, 1451-1500, 1501-1550, 1551-1600, 1601-1650, 1651-1700, 1701-1750, 1751-1800, 1801-1850, 101851-1900, 1901-1950, 1951-2000, or 2001 to the end of SEQ ID NO:X, or a strand complementary thereto. In this context, the term **"about"** includes, e.g., a particularly recited polynucleotide fragment range herein, and/or ranges that have lengths that are larger or smaller by several nucleotides (e.g., 5, 4, 3, 2, or 1nt), at either terminus or at both termini. Preferably, these fragments encode a polypeptide possessing biological activity as defined herein, e.g., immunogenicity, or antigenicity. More preferably, a polynucleotide fragment can be used as a probe or primer as discussed herein. Furthermore, the present invention also encompasses a polynucleotide that stably hybridizes to a polynucleotide fragment described herein under either stringent or lowered stringency hybridization conditions. Additionally incorporated are polypeptides encoded by a polynucleotide fragment or a hybridized polynucleotide stably bound to a polynucleotide fragment of the invention. Additionally encompassed by the invention is a polynucleotide encoding a polypeptide, which is specifically or selectively bound by an antibody directed to/or generated against a mature polypeptide of the invention (or fragment thereof), e.g., a mature polypeptide of SEQ ID NO: Y.

In the present invention, a **"polypeptide fragment or segment"** encompasses an amino acid sequence that is a portion of SEQ ID NO: Y. Protein and/or polypeptide fragments or segments may be **"free-standing,"** or they may comprise part of a larger polypeptide or protein, of which the fragment or segment forms a portion or region, e.g., a single continuous region of SEQ ID NO: Y connected in a fusion protein. Representative examples of lengths of polypeptide fragments or segments encompassed by the invention, include, e.g., fragments comprising, or alternatively consisting of, from about amino acid residue number 1-20,21-40,41-60, 61-80, 81-100,102-120,121-140,141-160,161-170,171-180, 181-190, 191-200, 201-210, etc., to the end of the mature coding region of a polypeptide of the invention (or fragment thereof).

Preferably, a polypeptide segment of the invention can have a length of contiguous amino acids of a polypeptide of the invention (or fragment thereof) that is at least about: 7, 8, 9, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 110, 120, 130, 140, or 150 contiguous amino acids in length. In this context **"about"** includes, e.g., the specifically recited ranges or values described herein, and it also encompasses values that differ from these recited values by several amino acid residues (e.g., plus or minus 5, plus or minus 4, plus or minus 3, plus or minus 2, or; plus or minus 1 amino acid residues), at either or both ends of the fragment. Further, a polynucleotide encoding such a polypeptide fragment is also encompassed by the invention.

Moreover, a polypeptide comprising more than one of the above polypeptide fragments is encompassed by the invention; including a polypeptide comprising at least: one, two, three, four, five, six, seven, eight, nine, ten, or more fragments, wherein the fragments (or combinations thereof) may be of any length described herein (e.g., a fragment of 12 contiguous amino acids and another fragment of 30 contiguous amino acids, etc.). The invention also encompasses proteins or polypeptides comprising a plurality of distinct, e.g., non-overlapping, segments of specified lengths. Typically, the plurality will be at least two, more usually at least three, and preferably four, five, six, seven, eight, nine, ten, or even more. While length minima are stipulated, longer lengths (of various sizes) may be appropriate (e.g., one of length seven, and two of lengths of twelve). Features of one of the different polynucleotide sequences should not be taken to limit those of another of the polynucleotide sequences. Preferred polypeptide fragments include, e.g., the secreted protein as well as the mature form. Further preferred polypeptide fragments include, e.g., the secreted protein or the mature form having a continuous series of deleted residues from the amino or the carboxy terminus, or both. For example, 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acids can be deleted from the amino terminus of either the secreted polypeptide or the mature form. Similarly, 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30, can be deleted from the carboxy terminus of the secreted protein or mature form. Furthermore, any combination of the above amino and carboxy terminus deletions are preferred. Similarly, polynucleotides encoding these polypeptide fragments are also preferred.

Also preferred are polypeptide fragments or segments (and their corresponding polynucleotide fragments) that characterize structural or functional domains, such as, fragments, or combinations thereof, that comprise e.g., alpha-helix, and alpha-helix forming regions, beta-sheet, and beta-sheet-forming regions, turn, and turn-forming regions, coil, and coil-forming regions, hydrophilic regions, hydrophobic regions, alpha amphipathic regions, beta amphipathic regions, flexible regions, loop regions, hairpin domains, beta-alpa-beta motifs, helix bundles, alpha/beta barrels, up and down beta barrels, jelly roll or swiss roll motifs, transmembrane domains, surface-forming regions, substrate binding regions, transmembrane regions, linkers, immunogenic regions, epitopic regions, and high antigenic index regions. Polypeptide fragments of SEQ ID NO: Y falling within conserved domains are specifically encompassed by the present invention. Moreover, polynucleotides encoding these domains are also encompassed. Other preferred polypeptide segments are biologically active fragments. Biologically active fragments are those exhibiting activity similar, but not necessarily identical, to an activity of an LP polypeptide (or fragment thereof). The biological activity of the fragments may include, e.g., an improved desired activity, or a decreased undesirable activity. Polynucleotides encoding these polypeptide fragments are also encompassed by the invention.

Preferably, the polynucleotide fragments of the invention encode a polypeptide that demonstrates a functional activity. The phrase "functional activity" encompasses a polypeptide segment that can accomplish one or more known functional activities associated with a full-length (complete) polypeptide of invention protein. Such functional activities include, e.g., without limitation, biological activity, antigenicity [ability to bind (or compete with a polypeptide of the invention for binding) to an antibody to a polypeptide of the invention], immunogenicity (ability to generate antibody that binds to a polypeptide of the invention), ability to form multimers with a polypeptide of the invention, and the ability to bind to a receptor or ligand of a polypeptide of the invention.

The functional activity of a polypeptide of the invention (including fragments, variants, derivatives, and analogs thereof) can be assayed by various methods. For example, where one is assaying for the ability to bind or compete with a full-length polypeptide of the invention for binding to an antibody of a polypeptide of the invention, various immunoassays known in the art can be used, including, e.g., without limitation, competitive and non-competitive assay systems using techniques such as radioimmunoassays, ELISA (enzyme linked immunosorbent assay), "sandwich" immunoassays, immunoradiometric assays, gel diffusion precipitation reactions, immunodiffusion assays, in situ immunoassays (using colloidal gold, enzyme or radioisotope labels, for example), western blots, precipitation reactions, agglutination assays (e.g., gel agglutination assays, hemagglutination assays, complement fixation assays, immunofluorescence assays, protein A assays, and immunoelectrophoresis assays, etc.)

In another embodiment, antibody binding is accomplished by detecting a label on the primary antibody. In another embodiment, the primary antibody is detected by detecting binding of a secondary antibody or reagent to the primary antibody. In a further embodiment, the secondary antibody is labeled. Many means are known in the art for detecting binding in an immunoassay and are within the scope of the present invention.

In another embodiment, where a ligand for a polypeptide of the invention is identified, or the ability of a polypeptide fragment, variant or derivative of the invention to multimerize is being evaluated, binding can be assayed, e.g., by using reducing and nonreducing gel chromatography, protein affinity chromatography, and affinity blotting (see generally, Phizicky, et al. (1995) Microbial. Rev. 59:94-123). In another embodiment, physiological correlates of binding of a polypeptide of the invention to its substrates (signal transduction) can be assayed with common techniques. In addition, assays described herein (see, e.g., the "Examples" section of the application), or otherwise known in the art, can routinely be applied to measure the ability of a polypeptide of the invention (its fragments, variants derivatives and analogs thereof) to elicit a related biological activity *(either in vitro* or *in vivo).*

### Epitopes and Antibodies

The present invention encompasses a polypeptide comprising, or alternatively consisting of, an epitope of SEQ ID NO: Y or a table herein; or encoded by a polynucleotide that stably hybridizes to form a hybridization complex, under stringent hybridization conditions (or lower stringency hybridization conditions) as defined herein, to a complement of a sequence of SEQ ID NO: X.

The present invention further encompasses a polynucleotide sequence encoding an epitope of a polypeptide sequence of the invention (such as, e.g., a sequence disclosed in SEQ ID NO: X or a Table herein), a polynucleotide sequence of the complementary strand of a polynucleotide sequence encoding an epitope of the invention, and a polynucleotide sequence that stably hybridizes to a complementary strand under stringent hybridization conditions or lower stringency hybridization conditions as defined herein.

The term **"epitope,"** as used herein, refers to a portion of a polypeptide having antigenic or immunogenic activity in an animal, preferably a mammal, and most preferably in a human. In a preferred embodiment, the present invention encompasses a polypeptide comprising an epitope, as well as the polynucleotide encoding this polypeptide.

An **"immunogenic epitope,"** as used herein, is defined as a portion of a protein or a linearized polypeptide (or fragment thereof) that elicits an antibody response in an animal, as determined by any art known method (e.g., by the methods for generating antibodies described herein or otherwise known, see, e.g., Geysen, et al. (1983) Proc. Natl. Acad. Sci. USA 308 1:3998-4002).

An **"antigenic epitope,"** as used herein, is defined as a portion of a protein or polypeptide to which a binding composition, e.g., an antibody or antibody binding fragment, selectively binds or is specifically immunoreactive with as determined by any known art method, e.g., by an immunoassay described herein. Selective binding excludes non-specific binding but does not necessarily exclude cross-reactivity with other antigens. An antigenic determinant may compete with the intact antigen (i.e., the immunogen used to elicit the immune response) for binding to an antibody. Antigenic epitopes need not necessarily be immunogenic.

The phrase **"specifically binds to"** or is **"specifically immunoreactive with",** when referring to a protein or peptide, refers to a binding reaction which is determinative of the presence of a protein or fragment (e.g., an LP protein) in the presence of a heterogeneous population of proteins and/or other biological components. Typically, the interaction is dependent upon the presence of a particular structure, e.g., an antigenic determinant (or epitope) recognized by a binding composition. For example, if an antibody is specific for epitope "A," the presence of a polypeptide comprising the epitope A, or the presence of free unlabeled A, in a reaction containing free labeled A and the antibody will reduce the amount of labeled A that binds to the antibody. Thus, under designated immunoassay conditions, the specified antibodies bind to a particular protein or polypeptide sequence and do not significantly bind other proteins or other polypeptide sequences that are present in the sample. Specific binding to an antibody under such conditions may require an antibody that is selected for its specificity and/or selectivity for a particular protein. For example, antibodies raised to the protein immunogen with an amino acid sequence depicted in SEQ ID NO: Y can be selected to obtain antibodies specifically immunoreactive with LP proteins or LP polypeptides and not with other proteins or polypeptides. These antibodies will also recognize proteins or polypeptide sequences that have an above average degree of similarity or identity to an LP protein or LP polypeptide sequence. Fragments that function as epitopes can be produced by any conventional means such as, e.g., (1985) Houghten, Proc. Natl. Acad. Sci. USA 82:5131-5135, further described in U.S. Patent No. 4,631,211.

In the present invention, an antigenic or immunogenic epitope preferably contains a polypeptide sequence of at least four, at least five, at least six, at least seven, more preferably at least eight, at least nine, at least 10, at least 11, at least 12, at least 13, at least 14, at least 15, at least 20, at least 25, at least 30, at least 40, at least 50, and, favorably, between about 15 to about 30 contiguous amino acids of a mature polypeptide of SEQ ID NO: Y or a Table herein. Preferred polypeptide fragments of contiguous amino acid residues of SEQ ID NO: Y comprising immunogenic or antigenic epitopes are at least 10, 15, 20, 25, 30, 35, 40, 45, 50, 55, 60, 65, 70, 75, 80, 85, 90, 95, or 100 contiguous amino acid residues in length.

Additional non-exclusive preferred antigenic epitopes include, e.g., the antigenic epitopes disclosed herein, as well as portions thereof. Antigenic epitopes are useful, e.g., to generate antibodies, including monoclonal antibodies that specifically bind the epitope. Preferred antigenic epitopes include, e.g., the antigenic epitopes disclosed herein, as well as any plurality thereof, e.g., at least: two, three, four, five or more of these antigenic epitopes in any combination or structural arrangement. Antigenic epitopes can be used as the target molecules in immunoassays (see, e.g., Wilson, et al. (1984) Cell 37:767-778; Sutcliffe, et al. (1983) Science 219:660-666). Similarly, immunogenic epitopes can be used, e.g., to induce antibodies according to any known art method (see, for instance, Sutcliffe, et al. *supra;* Wilson, et al. *supra;* Chow, et al. Proc. Natl. Acad. Sci. USA 82:910-25914; and Bittle, et al. (1985) J. Gen. Virol. 66:2347-2354.

Preferred immunogenic epitopes include, e.g., an immunogenic epitope disclosed herein, as well as a plurality or any combination thereof, e.g., of at least two, three, four, five or more of these immunogenic epitopes including, e.g., repeats of a particular epitope. A polypeptide comprising a plurality of epitopes may be used to elicit an antibody response with a carrier protein, such as, e.g., an albumin, to an animal system (such as, e.g., a rabbit or a mouse), or, if a polypeptide is of sufficient length (e.g., at least about 25 amino acids), the polypeptide may be presented without a carrier. However, immunogenic epitopes comprising as few as 8 to 10 amino acids have also been shown to be sufficient to generate antibodies and to be useful since they are capable of binding to, e.g., linear epitopes in a denatured polypeptide such as in Western blotting.

Polypeptides or proteins bearing an epitope of the present invention may be used to generate antibodies according to known methods including, e.g., without limitation, *in vivo immunization, in vitro* immunization, and phage display methods (see, e.g., Sutcliffe, et al. *supra;* Wilson, et al. *supra,* and Bittle, et al. (1985) J. Gen. Virol. 66:2347-2354.

### "Binding Composition"

The term **"binding composition"** refers to molecules that bind with specificity and/or selectivity to an LP of the invention or fragment thereof (such as, e.g., in an antibody-antigen interaction). However, other compositions (e.g., antibodies, oligonucleotides, proteins (e.g., receptors), peptides, or small molecules) may also specifically and/or selectivity associate (bind) with the LP in contrast to other molecules. Typically, the association will be in a natural physiologically relevant protein-protein interaction (either covalent or non-covalent) and it may include members of a multi-protein complex (including carrier compounds or dimerization partners). The composition may be a polymer or chemical reagent. A functional analog may be a protein with structural modifications or may be a wholly unrelated molecule (such as, e.g., one that has a molecular shape that interacts with the appropriate binding determinants). The proteins may serve as agonists or antagonists of the binding partner, see, e.g., Goodman, et al. (eds.) (1990) Goodman & Gilman's: The Pharmacological Bases of Therapeutics (cur. ed.) Pergamon Press, Tarrytown, N.Y.

The LP may be used to screen for binding compositions that specifically and/or selectively bind an LP of the invention or fragment thereof (e.g., a binding composition can be a molecule, or part of one, that selectively and/or stoichiometrically binds, whether covalently or not, to one or more specific sites of an LP (or fragment thereof) such as, e.g., in an antigen-antibody interaction, a hormone-receptor interaction, a substrate-enzyme interaction, etc.). At least one and up to a plurality of test binding compositions can be screened for specific and/or selective binding with the LP.

In one embodiment, a binding composition thus identified is closely related to a natural ligand of an LP (such as, e.g., a ligand or fragment thereof, a natural substrate, a structural or functional mimetic, or a natural binding partner; see, e.g., Coligan, et al. (1991) Current Protocols in Immunology 1 (2):Chapter 5.)

### "Binding Agent:LP Complex"

The term "binding agent:LP complex," as used herein, refers to a complex of a binding agent and a LP (or fragment thereof) which is formed by specific and/or selective binding of the binding agent to the respective LP (or fragment thereof). Specific and/or selective binding of the binding agent means that the binding agent has a specific and/or selective binding site that recognizes a site on the LP protein (or fragment thereof). For example, antibodies raised against a LP protein (or fragment thereof) that recognize an epitope on the LP protein (or fragment thereof) are capable of forming a binding agent:LP complex by specific and/or selective binding. Typically, the formation of a binding agent:LP complex allows the measurement of LP protein (or fragment thereof) in a mixture of other proteins and/or biologics.

### "Antibody:LP Complex"

The phrase "antibody:LP complex" refers to an embodiment in which the binding agent, e.g., is an antibody. The antibody may be monoclonal, polyclonal, or a binding fragment of an antibody (including, without limit, e.g., Fv, Fab, or F(ab)2 fragments; diabodies; linear antibodies (Zapata, *et al.,* (1995) Protein Engin. 8(10): 1057-62); single-chain antibody molecules; and multispecific antibodies formed from antibody fragments). Preferably, for cross-reactivity purposes, the antibody is a polyclonal antibody.

### Antibodies

Antibodies can be raised to various LP proteins, including individual, polymorphic, allelic, strain, or species variants, and fragments thereof, both in their naturally occurring (full-length) forms and in their recombinant forms. Additionally, antibodies can be raised to LP proteins in either their active forms or in their inactive forms. Anti-idiotypic antibodies may also be used. Antibodies of the invention include, e.g., without limitation, polyclonal, monoclonal, multispecific, human, humanized or chimeric antibodies, single chain antibodies, Fab fragments, F(ab') fragments, fragments produced by a Fab expression library, anti-idiotypic (anti-Id) antibodies (including, e.g., anti-Id antibodies to antibodies of the invention), and an epitope-binding fragment of any of the above.

As used herein, the phrase **"human antibodies"** includes, e.g., without limitation, antibodies having an amino acid sequence of a human immunoglobulin including, e.g., without limitation, an antibody isolated from a human immunoglobulin library or from an animal transgenic for one or more human immunoglobulins and that do not express endogenous immunoglobulins, as described herein or, as taught, e.g., in U.S. Patent No. 5,939,598. An antibody of the present invention may be monospecific, bispecific, trispecific or of greater multispecificity. Multispecific antibodies may be specific for different epitopes of an LP polypeptide (or fragment thereof) or may be specific for both a polypeptide of the present invention as well as for a heterologous epitope, such as a heterologous polypeptide or solid support material (see, e.g., WO 2093/17715; WO 92/08802; WO 91/00360; WO 92/05793; Tutt, et al. (1991) J. Immunol. 147:60-69; U.S. Patent Nos. 4,474,893; 4,714,681; 4,925,648; 5,573,920; or 5,601,819; or Kostelny, et al. (1992) J. Immunol. 148:1547-1553.

Further encompassed by the present invention is an antibody that selectively binds a polypeptide, which is encoded by a polynucleotide that stably hybridizes, under stringent hybridization conditions (as described herein), to an LP polynucleotide sequence. An antibody of the present invention may also be characterized or specified in terms of its binding affinity to a protein or polypeptide (fragment thereof), or epitope of the invention. A preferred binding affinity of a binding composition, e.g., an antibody or antibody binding fragment, includes, e.g., a binding affinity that demonstrates a dissociation constant or Kd of less than about: 5 X 10⁻²M, 10⁻²M, 5 X 10⁻³M, 10⁻³M, 5 X 10⁻⁴M, 10⁻⁴M, 5 X 10⁻⁵M, 10⁻⁵M, 5 X 1O⁻⁶M, 10⁻⁶M, 5 X 10⁻⁷M, 10⁻⁷M, 5 X 10⁻⁸M, 10⁻⁸M, 5 X 10⁻⁹M, 10⁻⁹M, 5 X 10⁻¹⁰M, 10⁻¹⁰M, 5 X 10⁻¹¹M, 10⁻¹¹M, 5 X 10⁻¹²M, 10⁻¹²M, 5 X 10⁻¹³M, 10⁻¹³M, 5 X 10⁻¹⁴M, 10⁻¹⁴M, 5 X 10⁻¹⁵M, or 10 ¹⁵M.

The invention also encompasses antibodies that competitively inhibit binding of a binding composition to an epitope of the invention as determined by any known art method for determining competitive binding, e.g., the immunoassays described herein. In preferred embodiments, the antibody competitively inhibits binding to the epitope by at least 95%, at least 90%, at least 85%, at least 80%, at least 75%, at least 70%, at least 60%, or at least 50%.

Antibodies of the present invention may act as agonists or antagonists of an LP polypeptide (or fragment thereof). Likewise encompassed by the invention, are neutralizing antibodies that bind a ligand and prevent it binding to a receptor. Similarly encompassed are ligand-binding antibodies that inhibit receptor activation without inhibiting receptor binding. Alternatively, ligand-binding antibodies that activate a receptor are also included. Antibodies of the invention may act as receptor agonists, e.g., by potentiating or activating either all or a subset of the biological activities of the ligand-mediated receptor activation, e.g., by inducing dimerization of a receptor. The antibodies may be specified as agonists, antagonists, or inverse agonists for biological activities comprising the specific biological activities of a peptide of the invention disclosed herein. An antibody agonist can be made using known methods art (see, e.g., WO 96/40281; U.S. Patent No. 5811,097; Deng, et al., Blood 92(6):1981-1988 (1998); Chen, et al., Cancer Res. 58(16):3668-3678 (1998); Harrop, et al., J. Immunol. 161(4):1786-1794 (1998); Zhu, et al., Cancer Res. 58( 15):3209-3214 (1998)).

Antibodies of the present invention may be used, e.g., without limitation, to purify, detect, or target a polypeptide (or fragment thereof) of the present invention for, e.g., *in vitro* and/or *in vivo* diagnostic and therapeutic methods. For example, the antibodies have use in immunoassays for qualitatively and/or quantitatively measuring levels of a polypeptide (or fragment thereof) of the present invention in a biological sample (see, e.g., Harlow, et al., Antibodies: A Laboratory Manual, Cold Spring Harbor Laboratory Press, cur. ed.; incorporated by reference).

The term **"monoclonal antibody**" as used herein is not limited to antibodies produced through hybridoma technology. The term "monoclonal antibody" refers to an antibody that is derived from a single clone, including any eukaryotic, prokaryotic, or phage clone, and not the method by which it is produced. Methods for producing and screening for specific antibodies using hybridoma technology are routine and known in the art. For an overview of the technology for producing human antibodies, see, e.g., Lonberg and Huszar, Int. Rev. Immunol. 13:65-93 (1995). In addition, commercial companies such as, e.g., Abgenix, Inc. (Freemont, CA) and Genpharm (San Jose, CA) can be hired to produce human antibodies.

Completely human antibodies that recognize a selected epitope can be generated by "guided selection" (see, e.g., Jespers, et al. (1988) BioTechnology 12:899-903). Further, antibodies of the invention can, in turn, be used to generate anti-idiotype antibodies that "mimic" a polypeptide (or fragment thereof) of the invention using known techniques (see, e.g., Greenspan & Bona, FASEB J. 7(5):437-444; (1989) and Nissinoff, J. (1991) Immunol. 147(8):2429-2438). The present invention encompasses antibodies recombinantly fused or chemically conjugated (including both covalent and non-covalent conjugations) to a polypeptide (or portion thereof, preferably comprising at least: 10, 20, 30, 40, 50, 60, 70, 80, 90, or 100 contiguous amino acids of a polypeptide of SED ID NO:X) of the present invention to generate fusion proteins. The fusion does not necessarily need to be direct, but may occur through linker sequences.

The antibodies may be specific for antigens other than a polypeptide of the invention (or portion thereof, preferably at least: 10, 20, 30, 40, 50, 60, 70, 80, 90, or 100 contiguous amino acids) of the present invention. For example, antibodies may be used to target an LP polypeptide (or fragment thereof) to particular cell types, *either in vitro or in vivo,* by fusing or conjugating a polypeptide (or fragment thereof) of the present invention to an antibody specific for a particular cell surface receptor. Antibodies fused or conjugated to a polypeptide of the invention may also be used in *in vitro* irnmunoassays and in purification methods using known art methods (see e.g., Harbor, et al., *supra,* and WO 9312 1232; EP 439,095; Naramura et al. (1994) Immunol. Lett. 39:91-99).

The present invention further includes compositions comprising a polypeptide of the invention (or fragment thereof) fused or conjugated to an antibody domain other than a variable region. For example, a polypeptide of the invention (or fragment thereof) may be fused or conjugated to an antibody Fc region, or portion thereof. The antibody portion that is fused to a polypeptide of the invention (or fragment thereof) may comprise a constant region, a hinge region, a CH1 domain, a CH2 domain, and/or a CH3 domain or any combination of whole domains or portions thereof. A polypeptide of the invention (or fragment thereof) may also be fused or conjugated to an antibody portion described herein to form multimers. For example, Fc portions fused to a polypeptide of the invention (or fragment thereof) can form dimers through disulfide bonding between the Fc portions. Higher multimeric forms can be made by fusing the polypeptides to portions of IgA and IgM. Methods for fusing or conjugating a polypeptide of the invention (or fragment thereof) to an antibody portion are known (see, e.g., U.S. Patent Nos. 5,336,603; 5,622,929; 5,359,046; 5,349,053; 5,447,851; 5,112,946; EP 307,434; EP 367,166; WO 96/04388).

In many cases, the Fc part of a fusion protein is beneficial in therapy and diagnosis, and thus can result in, e.g., improved pharmacokinetic properties (see, e.g., EP A232, 2G2). Alternatively, deleting the Fc part after the fusion protein has been expressed, detected, and purified, can be favored. Moreover, an antibody of the present invention (or fragment thereof) can be fused to marker sequences, such as a peptide to facilitate purification. Techniques for conjugating a therapeutic moiety to an antibody are known, see, e.g., Amon, et al., "Monoclonal Antibodies For Immunotargeting Of Drugs In Cancer Therapy", in Monoclonal Antibodies And Cancer Therapy, Reisfeld, et al. (eds.), pp. 243-56 (Alan R. Liss, Inc.1985); Hellstrom, et al., "Antibodies For Drug Delivery", in Controlled Drug Delivery (2nd Ed.), Robinson, et al. (eds.), pp. 623-53 (Marcel Dekker, Inc. 1987). Alternatively, an antibody can be conjugated to a second antibody to form an antibody heteroconjugate as described U.S. Patent No. 4,676,980.

An antibody (or fragment thereof) of the invention may be utilized for immunophenotyping of cell lines and biological samples. The translation product of an LP polynucleotide sequence (or fragment thereof) may be useful as a cell specific marker, or more specifically, as a cellular marker (which is differentially expressed at various stages of differentiation and/or maturation of particular cell types). A particular protein can be measured by a variety of immunoassay methods see, e.g., Stites and Terr (eds.) (1991) Basic and Clinical Immunology (7th ed.); Price and Newman (eds.) (1991) Principles and Practice of Immunoassays Stockton Press, NY; and Ngo (ed.) (1988) Non-isotopic Immunoassays Plenum Press, NY.; Stites and Terr (eds.) Basic and Clinical Immunology (7th ed.) *supra;* Maggio (ed.) Enzyme Immunoassay, supra; and Harlow and Lane Antibodies, A Laboratory Manual, *supra.* The ability of the antibody of interest to immunoprecipitate a particular antigen can be assessed by, e.g., Western blot analysis. One of skill in the art would be knowledgeable as to the parameters are modifiable to increase binding of an antibody to an antigen and to decrease background (e.g., by pre-clearing the cell lysate with sepharose beads). Further discussion of immunoprecipitation protocols can be found in, e.g., Ausubel et al, eds., 1994, Current Protocols in Molecular Biology, Vol. 1, John Wiley & Sons, Inc., New York.

### Therapeutic Uses

The present invention further encompasses antibody-based therapies that involve administering LP antibody to an animal, preferably a mammal, most preferably a primate (e.g., a human), to modulate, treat, inhibit, effect, or ameliorate one or more of the disclosed diseases, disorders, or conditions. An antibody of the invention can be used to modulate, treat, inhibit, ameliorate, or prevent diseases, disorders, or conditions associated with aberrant expression and/or activity of a polypeptide (or fragment thereof) of the invention, including, e.g., without limitation, any one or more of the diseases, disorders, syndromes or conditions described herein. The treatment, amelioration, and/or prevention of diseases, disorders, or conditions associated with aberrant expression and/or activity of a polypeptide of the invention includes, e.g., without limitation, ameliorating symptoms associated with those diseases, disorders or conditions. Antibodies of the invention may be provided in pharmaceutically acceptable compositions as known in the art or as described herein.

### Making LP proteins; Mimetics

DNAs which encode a LP protein or fragments thereof can be obtained by chemical synthesis, screening cDNA libraries, or by screening genomic libraries prepared from a wide variety of cell lines or tissue samples. Methods for doing so, or making expression vectors are either art known or are described herein.

These DNAs can be expressed in a wide variety of host cells for the synthesis of a full-length protein or fragments which can in turn, e.g., be used to generate polyclonal or monoclonal antibodies; for binding studies; for construction and expression of modified molecules; and for structure/function studies. Each LP protein or its fragments can be expressed in host cells that are transformed or transfected with appropriate expression vectors. By **"transformed"** is meant a cell into which (or into an ancestor of which) a DNA molecule has been introduced, by means of recombinant techniques, which encodes an LP polypeptide or fragment thereof.

Expression vectors are typically self replicating DNA or RNA constructs containing the desired antigen gene or its fragments, usually operably linked to appropriate genetic control elements that are recognized in a suitable host cell. The specific type of control elements necessary to effect expression depends on the host cell used. Generally, genetic control elements include a prokaryotic promoter system or a eukaryotic promoter expression control system, and typically include a transcriptional promoter, an optional operator to control the onset of transcription, transcription enhancers to elevate the level of mRNA expression, a sequence that encodes a suitable ribosome binding site, and sequences that terminate transcription and translation. All of the associated elements both necessary and sufficient for the production of LP polypeptide are in operable linkage with the nucleic acid encoding the LP polypeptide (or fragment thereof). Usually, expression vectors also contain an origin of replication that allows the vector to replicate independently of the host cell.

An expression vector will preferably include, e.g., at least one selectable marker. Such markers include, e.g., without limit, dihydrofolate reductase, G418, or neomycin resistance for eukaryotic cell culture and tetracycline, kanamycin or ampicillin resistance genes for culturing in E. *coli* and other bacteria.

The vectors of this invention contain DNAs which encode an LP protein, or a fragment thereof, typically encoding, e.g., a biologically active polypeptide, or protein. The DNA can be under the control of a viral promoter and can encode a selection marker. This invention further contemplates use of expression vectors capable of expressing eukaryotic cDNA coding for a LP (or fragment) in a prokaryotic or eukaryotic host, where the vector is compatible with the host and where the eukaryotic cDNA coding for the protein is inserted into the vector such that growth of the host containing the vector expresses the cDNA in question. Usually, expression vectors are designed for stable replication in their host cells or for amplification to greatly increase the total number of copies of the desirable gene per cell. It is not always necessary to require that an expression vector replicate in a host cell, e.g., it is possible to effect transient expression of the protein or its fragments in various hosts using vectors that do not contain a replication origin that is recognized by the host cell. It is also possible to use vectors that cause integration of an LP protein gene or its fragments into the host DNA by recombination, or to integrate a promoter that controls expression of an endogenous gene.

Vectors, as used herein, encompass plasmids, viruses, bacteriophage, integratable DNA fragments, and other vehicles that enable the integration of DNA fragments into the genome of the host. Expression vectors are specialized vectors that contain genetic control elements that effect expression of operably linked genes. Plasmids are the most commonly used form of vector, but many other forms of vectors that perform an equivalent function are also suitable for use (see, e.g., Pouwels, et al. (1985 and Supplements) Cloning Vectors: A Laboratory Manual Elsevier, N.Y.; and Rodriquez, et al. (eds.) (1988) Vectors: A Survey of Molecular Cloning Vectors and Their Uses Buttersworth, Boston, MA).

Suitable host cells include prokaryotes, lower eukaryotes, and higher eukaryotes. Prokaryotes include both gram negative and gram positive organisms, e.g., E. *coli* and *B. subtilis.* Lower eukaryotes include yeasts, e.g., *S. cerevisiae* and *Pichia,* and species of the genus *Dictyostelium.* Higher eukaryotes include established tissue culture cell lines from animal cells, both of non-mammalian origin, e.g., insect cells, and birds, and of mammalian origin, e.g., human, primates, and rodents.

Prokaryotic host-vector systems include a variety of vectors for many different species. As used herein, E. *coli* and its vectors will be used generically to include equivalent vectors used in other prokaryotes. A representative vector for amplifying DNA is pBR322 or its derivatives. Vectors that can be used to express these proteins or protein fragments include, but are not limited to, such vectors as those containing the lac promoter (pUC-series); trp promoter (pBR322-trp); Ipp promoter (the pIN-series); lambda-pP or pR promoters (pOTS); or hybrid promoters such as ptac (pDR540). See Brosius, et al. (1988) "Expression Vectors Employing Lambda-, trp-, lac-, and Ipp-derived Promoters," in Rodriguez and Denhardt (eds.) Vectors: A Survey of Molecular Cloning Vectors and Their Uses 10:205-236 Buttersworth, Boston, MA. Other representative bacterial vectors include, e.g., without limit, pQE70, pQE60, and pQE-9, (available from QIAGEN, Inc.); pBluescript vectors, Phagescript vectors, pNH8A, pNH16a, pNH18A, pNH46A, (available from Stratagene Cloning Systems, Inc.); and ptrc99a, pKK223-3, pKK233-3, pDR540, pRIT5 (available from Pharmacia Biotech, Inc).

Higher eukaryotic tissue culture cells are typically the preferred host cells for expression of the functionally active LP protein. Non-limiting representative examples of suitable expression vectors include pCDNA1; pCD (Okayama, et al. (1985) Mol. Cell Biol. 5:1136-1142); pMC1neo Poly-A, (Thomas, et al. (1987) Cell 51:503-512); and a baculovirus vector such as pAC 373 or pAC 610. Additional eukaryotic vectors include, e.g., without limit, pWLNE0, pSV2CAT, pOG44, pXT1 and pSG (available from Stratagene); and pSVK3, pBPV, pMSG and pSVL (available from Pharmacia Biotech, Inc.).

A polypeptide (or fragment thereof) of the present invention, and preferably, a mature and/or secreted form, can also be recovered from natural sources, including, e.g., without limit, bodily fluids, tissues, and cells, (whether directly isolated or cultured); products of chemical synthetic procedures; and products produced by recombinant techniques from a prokaryotic or eukaryotic host (including, e.g., bacterial, yeast, higher plant, insect, and mammalian cells).

It is likely that LP proteins need not be glycosylated to elicit biological responses. However, it will occasionally be desirable to express an LP protein or LP polypeptide in a system that provides a specific or defined glycosylation pattern. In this case, the usual pattern will be that provided naturally by the expression system. However, the pattern will be modifiable by exposing the polypeptide, e.g., in unglycosylated form, to appropriate glycosylating proteins introduced into a heterologous expression system. For example, the LP protein gene may be co-transformed with one or more genes encoding mammalian or other glycosylating enzymes. It is further understood that over glycosylation may be detrimental to LP protein biological activity, and that one of skill may perform routine testing to optimize the degree of glycosylation which confers optimal biological activity.

In addition, an LP polypeptide (or fragments thereof) may also include, e.g., an initial modified methionine residue (in some cases because of host-mediated processes). Typically, the N-terminal methionine encoded by the translation initiation codon removed with high efficiency from any protein after translation in all eukaryotic cells. While the N-terminal methionine on most proteins is also efficiently removed in most prokaryotes, for some proteins depending on the nature of the amino acid to which the N-terminal methionine is covalently linked, the removal process is inefficient. In one embodiment, the yeast *Pichia pastoris* is used to express a polypeptide of the present invention(or fragment thereof) in an eukaryotic system (see, e.g., Ellis, et al., Mol. Cell. Biol. 5:1111-21 (1985); Koutz, et al., Yeast 5: 167-77 (1989); Tschopp, et al., Nucl. Acids Res. 15:3859-76 (1987)). Thus, a heterologous coding sequence, such as, e.g., an LP polynucleotide sequence, (or fragment thereof) under the transcriptional regulation of all or part of the *AOX1* regulatory sequence is expressed at exceptionally high levels in *Pichia* yeast grown in the presence of methanol.

In one example, the plasmid vector pPIC9K is used to express polynucleotide sequence encoding a polypeptide of the invention, (or fragment thereof) as set forth herein, in a *Pichea* yeast system essentially as described in *"Pichia* Protocols: Methods in Molecular Biology," D.R Higgins and J. Cregg, eds. The Humana Press, Totowa, NJ, 1998. This expression vector allows expression and secretion of a protein of the invention by virtue of the strong AOX1 promoter linked to the *Pichia pastoris* alkaline phosphatase (PHO) secretory signal peptide located upstream of a multiple cloning site. Many other yeast vectors could be used in place of pPIC9K, such as, e.g., pYES2, pYD1, pTEFI/Zeo, pYES2/GS, pPICZ, pGAPZ, pGAPZalpha, pPIC9, pPIC3.5, PHIL-D2, PHIL-S1, pPIC3.5K, and, PA08, as a skilled in the artisan would appreciate, as long as the proposed expression construct provides appropriately located and operably linked signals for transcription, translation, secretion (if desired), and the like, (including an in-frame stop codon as required).

Furthermore, heterologously expressed proteins or polypeptides can also be expressed in plant cells. For plant cells viral expression vectors (e.g., cauliflower mosaic virus and tobacco mosaic virus) and plasmid expression vectors (e.g., T1 plasmid) are suitable. Such cells are available from a wide range of sources (e.g., the American Tissue Type Culture Collection, Rockland, MD; also, see for example, Ausubel, et al. (cur. ed. and Supplements; expression vehicles may be chosen from those provided e.g., in Pouwels, et al. (Cur. ed..) Cloning Vectors, A Laboratory Manual).

A LP protein, or a fragment thereof, may be engineered to be phosphatidyl inositol (PI) linked to a cell membrane, but can be removed from membranes by treatment with a phosphatidyl inositol cleaving enzyme, e.g., phosphatidyl inositol phospholipase-C. This releases the antigen in a biologically active form, and allows purification by standard procedures of protein chemistry (see, e.g., Low (1989) Biochem. Biophys. Acta 988:427-454; Tse, et al. (1985) Science 230:1003-1008; and Brunner, et al. (1991) J. Cell Biol. 114:1275-1283).

Now that LP proteins have been characterized, fragments or derivatives thereof can be prepared by conventional processes for synthesizing peptides. These include processes such as are described in Stewart and Young (1984) Solid Phase Peptide Synthesis Pierce Chemical Co., Rockford, IL; Bodanszky and Bodanszky (1984) The Practice of Peptide Synthesis Springer-Verlag, New York, NY; and Bodanszky (1984) The Principles of Peptide Synthesis Springer-Verlag, New York, NY. The prepared protein and fragments thereof can be isolated and purified from the reaction mixture by means of peptide separation, for example, by extraction, precipitation, electrophoresis and various forms of chromatography, and the like. An LP protein of this invention can be obtained in varying degrees of purity depending upon its desired use. Purification can be accomplished by use of known protein purification techniques or by the use of the antibodies or binding partners herein described (e.g., in immunoabsorbant affinity chromatography).

### Recombinant Proteins

An LP polypeptide, or fragment thereof, can be used to generate a fusion protein. For example, when fused to a second polypeptide, an LP polypeptide, or fragment thereof, can be used as an antigenic tag or an immunogen.

Antibodies raised against an LP polypeptide (or fragment thereof) can be used to indirectly detect a second protein by binding thereto. In one embodiment, if an LP protein has amino acid sequence portion that targets a cellular location (e.g., based on trafficking signals), that portion of the polypeptide can be used by fusing it to another protein (or fragment) to target a protein. Examples of domains that can be fused to an LP polypeptide (or fragment thereof) include, e.g., not only heterologous signal sequences, but also other heterologous functional regions. A fusion does not necessarily need to be direct, but may occur, e.g., through linker sequences. Moreover, fusion proteins may also be engineered to improve characteristics of an LP polypeptide.

For instance, a region of additional amino acids, particularly charged amino acids, may be added to the N-terminus of the polypeptide to improve stability and persistence during purification from a host cell or during subsequent handling and storage. In addition, peptide moieties can be added to the polypeptide to facilitate purification. Such regions may be removed before final preparation of the polypeptide. Additions of peptide moieties to facilitate handling are familiar and routine art techniques. Moreover, an LP polypeptide (including any fragment thereof, and specifically an epitope) can be combined with parts of the constant domain of an immunoglobulin e.g., (IgA, IgE, IgG, IgM) portions thereof (CH 1, CH2, CH3), and any combination thereof including both entire domains and portions thereof), resulting in a chimeric polypeptide. Such fusion proteins can facilitate purification and often are useful to increase *the in vivo* half-life of the protein (Fountoulakis, et al. (1995) J. Biochem.15 270:3958-3964). Enhanced delivery of an antigen across an epithelial barrier to the immune system has been demonstrated for antigens (e.g., insulin) conjugated to an FcRn binding partner such as IgG or Fc fragments (see, e.g., WO 96/22024 and WO 99/104813). IgG fusion proteins that have a disulfide-linked dimeric structure due to the IgG portion disulfide bonds have also been found more efficient in binding and neutralizing other molecules than monomeric polypeptides or fragments thereof alone (Fountoulakis, et al. (1995) J. Biochem. 270:3958-3964).

Additionally, a fusion protein can comprise various portions of the constant region of an immunoglobulin molecule together with a human protein (or part thereof) EP-A-O 464 533 (Canadian counterpart 2045869). In many cases, the Fc part in a fusion protein is beneficial in therapy and diagnosis, and thus, can result in, e.g., improved pharmacokinetic properties (EP-A 0232 262.). Alternatively, deleting the Fc part after the fusion protein has been expressed, detected, and purified, may be desired. For example, the Fc portion may hinder therapy and/or diagnosis if the fusion protein is used as an immunogen for immunizations. In drug discovery for example, human proteins, such as hIL-5, have been fused with Fc portions for the purpose of high-throughput screening assays to identify hIL-5 antagonists (Bennett, et al. (1995) 1. Molecular Recognition 8:52-58; and Johanson, et al. (1995) J. Biol. Chem. 270:9459-9471).

Furthermore, new constructs may be made by combining similar functional domains from other proteins. For example, protein-binding or other segments may be "swapped" between different new fusion polypeptides or fragments (see, e.g., Cunningham, et al. (1989) Science 243:1330-1336; and O'Dowd, et al. (1988) J. Biol. Chem. 263:15985-15992).

Moreover, an LP polypeptide (or fragment thereof) can be fused to a marker sequence, such as a peptide, to facilitate purification. In preferred embodiments, the marker amino acid sequence is a hexa-histidine peptide, such as, e.g., the tag provided in a pQE vector (QIAGEN, Inc., Chatsworth, CA, 91311), which provides for convenient purification of the fusion protein (Gentz, et al. (1989) Proc. Natl. Acad. Sci. USA 86:821-824). Another useful peptide-purification tag is the "HA" tag, which corresponds to an epitope derived from an influenza hemagglutinin protein (Wilson, et al. (1984) Cell 37:767). Nucleic acid molecules containing LP polynucleotide sequences encoding an LP epitope can also be recombined with a gene of interest as an epitope tag (e.g., the "HA" or flag tag) to aid in detection and purification of the expressed polypeptide. For example, one system purifies non-denatured fusion proteins expressed in human cell lines (Janknecht, et al. (1991) Proc. Natl. Acad. Sci. USA 88:8972-897). In this system, a gene of interest is subcloned into a vaccinia recombination plasmid such that the open reading frame of the sequence of interest is translationally fused to an amino-terminal tag consisting of six histidine residues. The tag serves as a matrix-binding domain for the fusion protein. Extracts from cells infected with the recombinant vaccinia virus are loaded onto Ni2+ nitriloacetic acid-agarose column and histidine-tagged proteins can be selectively eluted with imidazole-containing buffers.

Additionally, LP fusion constructions may be generated through the techniques of gene-shuffling, motif-shuffling, exon shuffling, and/or codon shuffling (collectively referred to as **"DNA shuffling").** DNA shuffling may be employed to modulate an activity of an LP polypeptide. Such methods can be used to generate LP polypeptides (or fragments thereof) with altered activity, as well as agonists and antagonists of an LP polypeptide (see, e.g., U.S. Patent Nos. 5,605,793; 5,811,238; 5,830,721; 5,834,252; and 5,837,458, and Patten, et al. (1997) Cur. Opinion Biotechnol. 8:724-33 30; Harayama, (1998) Trends Biotechnol. 16(2):76-82; Hansson, et al. (1999) J. Mol. Biol. 287:265-76; and Lorenzo and Blasco, (1998) Biotechniques 24(2): 308-13; each of which is incorporated by reference for these DNA shuffling teachings).

### VIII. Functional Variants

**"Derivatives"** of LP protein antigens include amino acid sequence mutants, glycosylation variants, and covalent or aggregate conjugates with other chemical moieties. Covalent derivatives can be prepared by linkage of functionalities to groups which are found in LP protein amino acid side chains or at the N- or C- termini, by any art known means. These derivatives can include, without limitation, aliphatic esters or amides of the carboxyl terminus, or of residues containing carboxyl side chains, O-acyl derivatives of hydroxyl group-containing residues, and N-acyl derivatives of the amino terminal amino acid or amino-group containing residues, e.g., lysine or arginine. Acyl groups are selected from the group of alkyl-moieties including C3 to C18 normal alkyl, thereby forming alkanoyl aroyl species. Covalent attachment to carrier proteins may be important when immunogenic moieties are haptens.

Also provided by the invention is a chemically modified derivative of a polypeptide of the invention (or fragment thereof) that may provide additional advantages such as increased solubility, increased stability increased circulating time, or decreased immunogenicity or antigenicity (see U.S. Patent no: 4,179,337). A chemical moieties for derivatization may be selected from water soluble polymers such as, e.g., polyethyleneglycol, ethylene glycol, propylene glycol, copolymers, carboxymethylcellulose, dextran, polyvinyl alcohol, etc. A polypeptide of the invention, (or fragment thereof) may be modified at random or at predetermined positions within the molecule and may include, e.g., one, two, three, or more attached chemical moieties. The polymer may be of any molecular weight, and may be branched or unbranched. For polyethylene glycol, a preferred molecular weight is between about 1 kDa and about 100 kDa (the term "about" means that in polyethylene glycol preparations, some molecules will weigh more and some will weigh less, than the stated molecular weight).

Other sizes may be used, depending on the desired effect (e.g., the [period of sustained release, the effects, if any, on biological activity, ease in handling, the degree or lack of antigenicity, and other known effects of polyethylene glycol on a protein, polypeptide or an analog). Polyethylene glycol molecules (or other chemical moieties) should be attached with consideration of the effect on functional, immunogenic, and/or antigenic domains of a polypeptide (or fragment thereof). Attachment methods include; e.g., without limit, (coupling PEG to G-CSF); EP 0 401 384, pegylating GM-CSF using tresyl chloride (Malik, et al. (1992) Exp. Hematol. 20:1028-1035). For example, polyethylene glycol may be covalently bound through amino acid residues via a reactive group, such as, e.g., a free amino or carboxyl group. Reactive groups are those to which an activated polyethylene glycol molecule may be bound. Amino acid residues having a free amino group may include, e.g., lysine residues, and N-terminal amino acid residue. Amino acid residues having a free carboxyl group may include, e.g., aspartic acid residues, glutamic acid residues, and C-terminal amino acid residues. Sulfhydryl groups may also be used to attach to a polyethylene glycol molecule. For human, a preferred attachment is at an amino group, such as, e.g., an attachment at the N-terminus or a lysine group.

One may specifically desire a protein, or a polypeptide (or fragment thereof) that is chemically modified at the N-terminus. Using polyethylene glycol as an illustration of the present composition, one may select from a variety of polyethylene glycol molecules (by molecular weight, branching, etc.), the proportion of polyethylene glycol molecules to a protein (polypeptide) molecule in the reaction mix, the type of pegylation reaction to be performed, and the method of obtaining the selected N-terminally pegylated, e.g., polypeptide. The method of obtaining an N-terminally pegylated preparation (by, e.g., separating this moiety from other monopegylated moieties if necessary) may be by purification of the N-terminally pegylated material from a population of pegylated protein molecules. Selective protein chemical modification at the N-terminus may be accomplished by reductive alkylation, which exploits differential reactivity of different types of primary amino groups (lysine versus the N-terminal) available for derivatization in a particular protein. Under appropriate reaction conditions, substantially selective derivatization of a protein or polypeptide (or fragment thereof) at the N-terminus with a carbonyl-group-containing-polymer is achieved.

This invention also encompasses the use of derivatives of an LP protein other than variations in amino acid sequence or glycosylation. Such derivatives may involve covalent or aggregative association with chemical moieties. Generally, these derivatives fall into the three classes: (1) salts, (2) side chain and terminal residue covalent modifications, and (3) adsorption complexes (e.g., with cell membranes). Such covalent or aggregative derivatives are useful as immunogens, as reagents in immunoassays, or in purification methods such as for affinity purification of proteins or other binding proteins. For example, a LP protein antigen can be immobilized by covalent bonding to a solid support such as cyanogen bromide-activated SEPHAROSE, by methods which are well known in the art, or adsorbed onto polyolefin surfaces, with or without glutaraldehyde cross-linking, for use in an assay or purification of anti-LP protein antibodies or its respective binding partner. An LP protein can also be labeled for use in diagnostic assays with a detectable group (such as, e.g., radioiodinated by the chloramine T procedure; covalently bound to rare earth chelates; or conjugated to another fluorescent moiety). Purification of an LP protein may be effected by immobilized antibodies or a binding partner.

A polypeptide of the invention (or fragment thereof) may be as a monomer or a multimer (e.g., a dimer, a trimer, a tetramer, or a higher multimer). Accordingly, the present invention encompasses monomers and multimers of a polypeptide of the invention, (or fragment thereof) including, e.g., their preparation, and compositions (preferably, therapeutic compositions) containing them. In specific embodiments, the polypeptides and/or fragments of the invention are monomers, dimers, trimers, tetramers or higher multimers. In additional embodiments, a multimer of the invention is at least a dimer, at least a trimer, or at least a tetramer. Multimers encompassed by the invention may be homomers or heteromers. As used herein, the term "homomer," refers to a multimer containing only a specific polypeptide (or fragment thereof) corresponding to an amino acid sequence of SEQ ID NO:Y or in a talbe herein (including fragments, variants, splice variants, and fusion proteins, corresponding to these polypeptides as described herein). A homomer may contain a polypeptide having identical or different amino acid sequences. In a specific embodiment, a homomer of the invention is a multimer containing only polypeptides (or fragments thereof) having identical amino acid sequences. In another specific embodiment, a homomer of the invention is a multimer containing polypeptides having different amino acid sequences.

In specific embodiments, a multimer of the invention is a homodimer (e.g., containing polypeptides having identical and/or different amino acid sequences) or a homotrimer (e.g., containing polypeptides having identical and/or different amino acid sequences). In additional embodiments, the homomeric multimer of the invention is at least a homodimer, at least a homotrimer, or at least a homotetramer. As used herein, the term "heteromeric," refers to a multimer containing one or more heterologous polypeptides. In a specific embodiment, a multimer of the invention is a heterodimer, a heterotrimer, or a heterotetramer. In additional embodiments, the heteromeric multimer of the invention is at least a heterodimer, at least a heterotrimer, or at least a heterotetramer. Multimers of the invention may be the result of hydrophobic, hydrophilic, ionic and/or covalent associations and/or may be indirectly linked, by e.g., liposome formation. Thus, in one embodiment, a multimer of the invention, such as, e.g., homodimers or homotrimers, are formed when polypeptides of the invention (or fragments thereof) contact one another in solution.

In another embodiment, a heteromultimer of the invention, such as, e.g., a heterotrimer or a heterotetramer, is formed when, e.g., a polypeptide of the invention contacts an antibody (generated against a polypeptide; or fragment thereof of the invention (including antibodies to the heterologous polypeptide sequence in a fusion protein of the invention)) in solution. In other embodiments, a multimer of the invention is formed by covalent association with and/or between a polypeptide and a binding partner such as mentioned herein (or fragment thereof). Such covalent associations may involve one or more amino acid residues contained in a polypeptide sequence (e.g., as recited in a sequence listing herein, or contained in a polypeptide encoded by a deposited clone specified herein). In one instance, a covalent association is a cross-link, e.g., between cysteine residues. In another instance, the covalent associations are the consequence of chemical or recombinant manipulation. Alternatively, such covalent associations may involve one or more amino acid residues contained in a heterologous polypeptide sequence such as, e.g., a fusion protein of the invention. In one example, covalent associations form with a heterologous sequence contained in a fusion protein of the invention (see, e.g., US Patent No. 5,478,925). In a specific example, a covalent association is between a heterologous sequence contained in an Fc fusion protein of the invention (as described herein). In another specific example, a covalent association of a fusion protein of the invention is with a heterologous polypeptide sequence such as, e.g., oseteoprotegerin (see, e.g., WO 98149305, incorporated by reference for these teachings).

In another embodiment, two or more polypeptides of the invention (or fragment thereof) are joined through peptide linkers. Examples include, e.g., peptide linkers described in U.S. Pat. No. 5,073,627 (incorporated by reference for these teachings). A protein comprising multiple polypeptides of the invention that are separated by peptide linkers may be produced using conventional recombinant DNA technology.

Recombinant fusion proteins comprising a polypeptide of the invention (or fragment thereof) fused to a polypeptide sequence that dimerizes or trimerizes in solution can be expressed in a suitable host cell. The resulting soluble multimeric fusion protein can be recovered from a supernatant using any art known technique or method described herein. Trimeric polypeptides of the invention may offer an advantage of enhanced biological activity (as defined herein). Preferred leucine zipper moieties and isoleucine moieties are those that preferentially form trimers. An example is a leucine zipper derived from lung surfactant protein D (SPD), as described in Hoppe, et al. FEBS Letters 344: 19 1,15(1994) and in U.S. patent application Ser. No. 08/446,922, (each hereby incorporated by reference for these teachings). Other peptides derived from naturally occurring trimeric proteins may be employed when preparing a trimeric polypeptide of the invention.

In another example, polypeptides or proteins of the invention are associated by interactions with a Flag polypeptide sequence (e.g., contained in a fusion protein of the invention having a Flag sequence). In a further embodiment, a protein or a polypeptide of the invention is associated by an interaction with a heterologous polypeptide sequence (contained in a Flag fusion protein of the invention) and an anti-Flag antibody.

A multimer of the invention may be generated using chemical art known techniques. For example, polypeptides (or fragments thereof) desired to be contained in a multimer of the invention may be chemically cross-linked using a linker molecule e.g., linker molecules and linker molecule length optimization techniques are known in the art; see, e.g., US Patent No. 5,478,925, which is incorporated by reference for such teachings. Additionally, a multimer of the invention may be generated using techniques known in the art to form one or more inter-molecule cross-links between the cysteine residues (see, e.g., US Patent No. 5,478,925, incorporated by reference for these teachings). Further, a polypeptide of the invention modified by the addition of cysteine or biotin to the C or N-terminus of a polypeptide can be generated by art known methods (see, e.g., US Patent No. 5,478,925, incorporated by reference for these teachings).

Additionally, a multimer of the invention can be generated by art known methods (see, e.g., US Patent No. 5,478,925, incorporated by reference for these teachings). Alternatively, a multimer of the invention can be generated using other commonly known genetic engineering techniques. In one embodiment, a polypeptide contained in a multimer of the invention is produced recombinantly with fusion protein technology described herein or otherwise known in the art (see, e.g., US Patent No. 5,478,925, incorporated by reference for these teachings). In a specific embodiment, a polynucleotide encoding a homodimer of the invention can be generated by ligating a polynucleotide sequence encoding a polypeptide (or fragment thereof) of the invention to another sequence encoding a linker polypeptide and then subsequently, further to a synthetic polynucleotide encoding the translated product of the polypeptide in the reverse orientation from the original C-terminus to the N-terminus (lacking the leader sequence) (see, e.g., US Patent No. 5,478,925, incorporated by reference for these teachings).

In another embodiment, recombinant techniques described herein or otherwise known in the art can be applied to generate a recombinant polypeptide of the invention (or fragment thereof) that contains a transmembrane domain (or hyrophobic or signal peptide) and that can be incorporated by membrane reconstitution techniques into a liposome (see, e.g., US Patent No. 5,478,925, incorporated by reference for these teachings).

### X. Uses

The present invention provides reagents that will find use in diagnostic and/or therapeutic applications as described herein, e.g., in the description of kits for diagnosis.

An LP polynucleotide sequence (or fragment thereof) can be used in numerous ways, e.g., such as a reagent. The following descriptions (using known art techniques) are non-limiting examples of ways to use an LP polynucleotide sequence (or fragment thereof). For example, an LP polynucleotide sequence (or fragment thereof) is useful for chromosome identification. There exists an ongoing need to identify new chromosome markers, since few chromosome-marking reagents, based on actual sequence data (repeat polymorphisms), are presently available. Each polynucleotide of the present invention can therefore, be used as a chromosome marker.

In another embodiment, the invention encompasses a kit, e.g., for analyzing a sample for the presence of a polynucleotide associated with a proliferative disease, syndrome, disorder, or condition. In a general embodiment, the kit includes, e.g., at least an LP polynucleotide sequence (or fragment thereof) probe containing a polynucleotide sequence that hybridizes with an LP polynucleotide sequence(or fragment thereof) and directions, e.g., such as for disposal. In another specific embodiment, a kit includes, e.g., two polynucleotide probes defining an internal region of an LP polynucleotide sequence, where each probe has one strand containing a 31 mer-end internal to a region the polynucleotide.

In a further embodiment, a probe may be useful as a primer for amplification using a polymerase chain reaction (PCR). Where a diagnosis of a disease, syndrome, disorder or condition has already been made according to conventional methods, the present invention is useful as a prognostic indicator, for a subject exhibiting an enhanced or diminished expression of an LP polynucleotide sequence (or fragment thereof) by comparison to a subject expressing the polynucleotide of the present invention (or fragment thereof) at a level nearer a standard level.

The phrase, **"measuring level of a composition of the present invention"** is intended to mean herein measuring or estimating (either qualitatively and/or quantitatively) a level of, e.g., a polypeptide (or fragment thereof), or a polynucleotide (or fragment thereof) including, e.g., mRNA, DNA, or cDNA, in a first sample (e.g., preferably a biological sample) either directly (e.g., by determining or estimating an absolute protein or mRNA level) or relatively (e.g., by comparing to a polypeptide or mRNA level in a second sample). In one embodiment, the level in the first sample is measured or estimated from an individual having, or suspected of having, a disease, syndrome, disorder or condition and comparing that level to a second level, wherein the second level is obtained from an individual not having and/or not being suspected of having a disease, syndrome, disorder or condition. Alternatively, the second level is determined by averaging levels from a population of individuals not having or suspected of having a disease, syndrome, disorder, or condition.

As is appreciated in the art, once a standard level is determined, it can be used repeatedly as a standard for comparison. A **"biological sample"** is intended to mean herein any sample comprising biological material obtained from, using, or employing, e.g., an organism, body fluid, exudate, lavage product, waste product, cell (or part thereof), cell line, organ, biopsy, tissue culture, or other source originating from, or associated with, a living cell, tissue, organ, or organism, which contains, e.g., a polypeptide (or fragment thereof), a protein (or fragment thereof), a mRNA (or fragment thereof), or polynucleotide sequence (or fragment thereof) of the present invention, including, e.g., without limitation, a sample such as from, e.g., hair, skin, blood, saliva, semen, vomit, synovial fluid, amniotic fluid, breast milk, lymph, pulmonary sputum, urine, fecal matter, a lavage product, etc.

As indicated, a biological sample can include, e.g., without limitation, body fluids (e.g., such as semen, lymph, sera, plasma, urine, synovial fluid and spinal fluid) that contain a polypeptide (or fragment thereof), mRNA (or fragment thereof), a protein (or fragment thereof), or polynucleotide (or fragment thereof) of the present invention, by product, or, waste product; and/or other tissue source found to express a polypeptide (or fragment thereof), mRNA (or fragment thereof), or nucleic acid (or fragment thereof), by product, or, waste product; of the present invention. Methods for obtaining biological samples, e.g., tissue biopsies, body fluids, cells, or waste products from mammals are known in the art. Where the biological sample is to include, e.g., mRNA, a tissue biopsy is a preferred source.

The present invention further encompasses an LP polynucleotide sequence (or fragment thereof) that is chemically synthesized, or reproduced as a peptide nucleic acid (PNA) using art known methods. The use of a PNA is preferred if a polynucleotide (or a fragment thereof) is incorporated, e.g., onto a solid support, or genechip. For the purposes of the present invention, a peptide nucleic acid (PNA) is a polyamide type of polynucleotide analog in which, generally, e.g., the monomeric units for adenine, guanine, thymine and cytosine are available commercially (see, e.g., Perceptive Biosystems). Certain components of a polynucleotide, such as DNA, like phosphorus, phosphorus oxides, or deoxyribose derivatives, are not present in a PNA. Generally, PNAs bind specifically and tightly to complementary DNA strands and are not degraded by nucleases (Nielsen, et al. (1993) Nature 365: 666). In fact, a PNA binds more strongly to DNA than DNA binds to itself, probably, as there is no electrostatic repulsion between PNA/DNA; furthermore, the PNA polyamide backbone is more flexible than DNA. Because of this, PNA/DNA duplexes can bind under a wider range of stringency conditions than DNA/DNA duplexes thus, making it easier to perform multiplex hybridizations. Moreover, smaller probes can be used with PNA than with DNA due to the strong binding.

In addition, it is more likely that single base mismatches can be determined using a PNA/DNA hybridization since, e.g., a single mismatch in a PNA/DNA 15-mer lowers the melting point (Tₘ) by 8°-20°C, versus lowering the melting point 4°-16°C for the DNA/DNA 15-mer duplex. In addition, the absence of charge groups in a PNA molecule means that hybridizations can be done at low ionic strengths and the absence of charge groups with the DNA reduces possible interference by salt.

An LP polypeptide (or fragment thereof), can be used in numerous ways. The following descriptions are non-limiting, exemplars that use art known techniques.

A polypeptide (or fragment thereof) can be used to assay a protein level, e.g., of a secreted protein, in a sample, e.g., such as a bodily fluid by using antibody-based techniques. For example, protein expression in a tissue can be studied by an immunohistological method (see, e.g., Jalkanen, et al. (1985) J. Cell Biol. 101:976-985; Jalkanen, et al. (1987) J. Cell Biol. 105:3087-303096). Another useful antibody-based method for detecting protein or polypeptide expression includes, e.g., an immunoassay like an enzyme linked immunosorbent assay or a radioimmunoassay (RIA). In addition to assaying, e.g., the level of a secreted protein in a sample, a protein can also be detected *by in vivo* imaging. Thus, the invention provides a means for detecting, marking, locating or diagnosing a disease, syndrome, syndrome, disorder, and/or condition comprising assaying the expression of a polynucleotide (or fragment thereof), or a polypeptide (or fragment thereof), of the present invention that is in a sample, e.g., cells or body fluid of an individual by comparing one level of expression with another level of expression, e.g., a standard level of expression to indicate, e.g., a disease, syndrome, disorder, and/or condition, (or predilection to the same), or to make a prognosis or prediction.

Furthermore, an LP polypeptide (or fragment thereof)can be used to treat, prevent, modulate, ameliorate, and/or diagnose a disease, syndrome, condition, and/or a disorder. For example, a subject can be administered a polypeptide (or fragment thereof) of the invention to replace absent or decreased levels of a polynucleotide or polypeptide (e.g., insulin); to supplement absent or decreased levels of a different polynucleotide or polypeptide (e.g., hemoglobin S for hemoglobin B; SOD to catalyze DNA repair proteins); to inhibit the activity of a polynucleotide or polypeptide (e.g., an oncogene or tumor suppressor); to activate a polynucleotide or polypeptide (e.g., by binding to a receptor), to reduce activity of a membrane bound receptor by competing with the receptor for free ligand (e.g., soluble TNF receptors can be used to reduce inflammation), or to bring about a desired response (e.g., blood vessel growth inhibition, enhancement of an immune response to proliferating cells or to an infectious agent).

Similarly, an antibody directed to a polypeptide (or fragment thereof) of the present invention can also be used to treat, prevent, modulate, ameliorate, and/or diagnose a condition, syndrome, state, disease or disorder. For example, administration of an antibody directed to an LP polypeptide (or fragment thereof)can bind and reduce the level of the targeted polypeptide. Similarly, administration of an antibody can activate an LP polypeptide (or fragment thereof), such as by binding to the polypeptide that is bound to a membrane (e.g., a receptor).

### Diagnosis and Imaging Using an LP Antibody

Antibodies of the invention can be used to assay polypeptide levels in a sample, eg., using classical immunohistological methods known to those of skill in the art (see e.g., Jalkanen, et al., J. Cell. Biol. 101:976-985 (1985); Jalkanen, et al., J. Cell. Biol. 105:3087-3096 (1987)). Other antibody-based methods typically useful for detecting polypeptide expression include, e.g., immunoassays, such as the enzyme linked immunosorbent assay (ELISA) and the radioimmunoassay (RIA).

Sequences encoding an LP polypeptide (or fragment thereof) are used for the diagnosis of disorders associated with LP (such as, e.g., LP misexpression, LP overexpression, LP underexpression, etc.). Examples of such disorders include, without limit, a cell proliferative disorder such as actinic keratosis, arteriosclerosis, atherosclerosis, bursitis, cirrhosis, hepatitis, mixed connective tissue disease (MCTD), myelofibrosis, paroxysmal nocturnal hemoglobinuria, polycythemia vera, psoriasis, primary thrombocythemia, and cancers including adenocarcinoma, leukemia, lymphoma, melanoma, myeloma, Hamartoma, sarcoma, teratocarcinoma, and, in particular, a cancer of the adrenal gland, bladder, bone, bone marrow, brain, breast, cervix, gall bladder, ganglia, gastrointestinal tract, heart, kidney, liver, lung, muscle, ovary, pancreas, parathyroid, penis, prostate, salivary glands, skin, spleen, testis, thymus, thyroid, and uterus; an autoimmune/inflammatory disorder such as acquired immunodeficiency syndrome (AIDS), Addison's disease, adult respiratory distress syndrome, allergies, ankylosing spondylitis, amyloidosis, anemia, asthma, atherosclerosis, autoimmune hemolytic anemia, autoimmune thyroiditis, autoimmune polyendocrinopathy-candidiasis-ectodermal dystrophy (APECED), bronchitis, cholecystitis, contact dermatitis, Crohn's disease, atopic dermatitis, dermatomyositis, diabetes mellitus, emphysema, episodic lymphopenia with lymphocytotoxins, erythroblastosis fetalis, erythema nodosum, atrophic gastritis, glomerulonephritis, Goodpasture's syndrome, gout, Graves' disease, Hashimoto's thyroiditis, hypereosinophilia, irritable bowel syndrome, multiple sclerosis, myasthenia gravis, myocardial or pericardial inflammation, osteoarthritis, osteoporosis, pancreatitis, polymyositis, psoriasis, Reiter's syndrome, rheumatoid arthritis, scleroderma, Sjögren's syndrome, systemic anaphylaxis, systemic lupus erythematosus, systemic sclerosis, thrombocytopenic purpura, ulcerative colitis, uveitis, Werner syndrome, complications of cancer, hemodialysis, and extracorporeal circulation, viral, bacterial, fungal, parasitic, protozoal, and helminthic infections, and trauma; a cardiovascular disorder such as congestive heart failure, ischemic heart disease, angina pectoris, myocardial infarction, hypertensive heart disease, degenerative valvular heart disease, calcific aortic valve stenosis, congenitally bicuspid aortic valve, mitral annular calcification, mitral valve prolapse, rheumatic fever and rheumatic heart disease, infective endocarditis, nonbacterial thrombotic endocarditis, endocarditis of systemic lupus erythematosus, carcinoid heart disease, cardiomyopathy, myocarditis, pericarditis, neoplastic heart disease, congenital heart disease, complications of cardiac transplantation, arteriovenous fistula, atherosclerosis, hypertension, vasculitis, Raynaud's disease, aneurysms, arterial dissections, varicose veins, thrombophlebitis and phlebothrombosis, vascular tumors, and complications of thrombolysis, balloon angioplasty, vascular replacement, and coronary artery bypass graft surgery; a neurological disorder such as epilepsy, ischemic cerebrovascular disease, stroke, cerebral neoplasms, Alzheimer's disease, Pick's disease, Huntington's disease, dementia, Parkinson's disease and other extrapyramidal disorders, Amyotrophic lateral sclerosis and other motor neuron disorders, progressive neural muscular atrophy, retinitis pigmentosa, hereditary ataxias, multiple sclerosis and other demyelinating diseases, bacterial and viral meningitis, brain abscess, subdural edema, epidural abscess, suppurative intracranial thrombophlebitis, myelitis and radiculitis, viral central nervous system disease, prion diseases including kuru, Creutzfeldt-Jakob disease, and Gerstmann-Straussler-Scheinker syndrome, fatal familial insomnia, nutritional and metabolic diseases of the nervous system, neurofibromatosis, tuberous sclerosis, cerebelloretinal hemangioblastomatosis, encephalotrigeminal syndrome, mental retardation and other developmental disorders of the central nervous system including Down syndrome, cerebral palsy, neuroskeletal disorders, autonomic nervous system disorders, cranial nerve disorders, spinal cord diseases, muscular dystrophy and other neuromuscular disorders, peripheral nervous system disorders, dermatomyositis and polymyositis, inherited, metabolic, endocrine, and toxic myopathies, myasthenia gravis, periodic paralysis, mental disorders including mood, anxiety, and schizophrenic disorders, seasonal affective disorder (SAD), akathesia, amnesia, catatonia, diabetic neuropathy, tardive dyskinesia, dystonias, paranoid psychoses, post-therapeutic neuralgia, Tourette's disorder, progressive supranuclear palsy, corticobasal degeneration, and familial frontotemporal dementia; and a developmental disorder such as renal tubular acidosis, anemia, Cushing's syndrome, achondroplastic dwarfism, Duchenne and Becker muscular dystrophy, epilepsy, gonadal dysgenesis, WAGR syndrome (Wilms' tumor, aniridia, genitourinary abnormalities, and mental retardation), Smith-Magenis syndrome, myelodysplastic syndrome, hereditary mucoepithelial dysplasia, hereditary keratodermas, hereditary neuropathies such as Charcot-Marie-Tooth disease and neurofibromatosis, hypothyroidism, hydrocephalus, seizure disorders such as Syndenham's chorea and cerebral palsy, spina bifida, anencephali, craniorachischisis, congenital glaucoma, cataract, and sensorineural hearing loss. Sequences encoding an LP polypeptide (or fragment thereof) are used in Southern or northern analysis; dot blot or other membrane-based technologies; PCR technologies; in dipstick, pin, and multiformat ELISA-like assays; and in microarrays utilizing fluids or tissues from a subject; to detect an altered LP polypeptide (or fragment thereof) expression. Such qualitative or quantitative methods are well known in the art.

### Therapeutic Uses

This invention also provides reagents with significant therapeutic value. An LP protein or polypeptide (naturally occurring or recombinant), fragments thereof, and antibodies thereto, along with compounds identified as having binding affinity to an LP, are useful in the treatment of conditions associated with abnormal physiology or development, including abnormal proliferation, e.g., cancerous conditions, or degenerative conditions. Abnormal proliferation, regeneration, degeneration, and atrophy may be modulated by appropriate therapeutic treatment using a composition(s) provided herein. For example, a disease or disorder associated with abnormal expression or abnormal signaling by an LP protein is a target for an agonist or antagonist of the protein.

Other abnormal developmental conditions are known in cell types shown to possess LP mRNA by northern blot analysis (see, e.g., Berkow (ed.) The Merck Manual of Diagnosis and Therapy, Merck & Co., Rahway, N.J.; Thorn et al. Harrison's Principles of Internal Medicine, McGraw-Hill, N.Y.; and Rich (ed.) Clinical Immunology; Principles and Practice, Mosby, St. Louis (cur. ed.); and below). Developmental or functional abnormalities, (e.g., of the neuronal, immune, or hematopoetic system) cause significant medical abnormalities and conditions which may be susceptible to prevention or treatment using compositions provided herein.

Recombinant LP or LP antibodies can be purified and administered to a subject for treatment. These reagents can be combined for use with additional active or inert ingredients, e.g., in conventional pharmaceutically acceptable carriers or diluents, e.g., immunogenic adjuvants, along with physiologically innocuous stabilizers and excipients. These combinations can be sterile filtered and placed into dosage forms as by lyophilization in dosage vials or storage in stabilized aqueous preparations. This invention also contemplates use of antibodies or binding fragments thereof, including forms which are not complement binding.

Another therapeutic approach included within the invention involves direct administration of reagents, formulations, or compositions by any conventional administration techniques (such as, e.g., without limit, local injection, inhalation, or systemic administration) to a subject. The reagents, formulations, or compositions included within the bounds and metes of the invention may also be targeted to a cell by any of the methods described herein (e.g., polynucleotide delivery techniques). The actual dosage of reagent, formulation, or composition that modulates a disease, disorder, condition, syndrome, etc., depends on many factors, including the size and health of an organism, however one of one of ordinary skill in the art can use the following teachings describing methods and techniques for determining clinical dosages (see, e.g., Spilker (1984) Guide to Clinical Studies and Developing Protocols, Raven Press Books, Ltd., New York, pp. 7-13, 54-60; Spilker (1991) Guide to Clinical Trials, Raven Press, Ltd., New York, pp. 93-101; Craig and Stitzel (eds. 1986) Modern Pharmacology, 2d ed., Little, Brown and Co., Boston, pp. 127-33; Speight (ed. 1987) Avery's Drug Treatment: Principles and Practice of Clinical Pharmacology and Therapeutics, 3d ed., Williams and Wilkins, Baltimore, pp. 50-56; Tallarida, et al. (1988) Principles in General Pharmacology, Springer-Verlag, New York, pp. 18-20; and U.S. Pat. Nos. 4,657,760; 5,206,344; and 5,225,212.). Generally, in the range of about between 0.5 fg/ml and 500 µg/ml inclusive final concentration are administered per day to a human adult in any pharmaceutically acceptable carrier. Furthermore, animal experiments provide reliable guidance for the determination of effective does for human therapy. Interspecies scaling of effective doses can be performed following art known principles (e.g., see, Mordenti and Chappell (1989) "The Use of Interspecies Scaling in Toxicokinetics," in Toxicokinetics and New Drug Development; Yacobi, et al. (eds.) Pergamon Press, NY).

Effective doses can also be extrapolated using dose-response curves derived *from in vitro* or animal-model test systems. For example, for antibodies a dosage is typically 0.1 mg/kg to 100 mg/kg of a recipients body weight. Preferably, a dosage is between 0.1 mg/kg and 20 mg/kg of a recipients body weight, more preferably 1 mg/kg to 10 mg/kg of a recipients body weight. Generally, homo-specific antibodies have a longer half-life than hetero-specific antibodies, (e.g., human antibodies last longer within a human host than antibodies from another species, e.g., such as a mouse, probably, due to the immune response of the host to the foreign composition). Thus, lower dosage of human antibodies and less frequent administration is often possible if the antibodies are administered to a human subject. Furthermore, the dosage and frequency of administration of antibodies of the invention may be reduced by enhancing uptake and tissue penetration (e.g., into the brain) by using modifications such as, e.g., lipidation.

The invention also provides a pharmaceutical pack or kit comprising one or more containers filled with one or more of the ingredients of the compositions of the invention and instructions such as, e.g., for disposal (typically, in a form prescribed by a governmental agency regulating the manufacture, use or sale of pharmaceuticals or biological products).

The quantities of reagents necessary for effective treatment will depend upon many different factors, including means of administration, target site, physiological state of the patient, and other medicaments administered. Thus, treatment dosages should be titrated to optimize safety and efficacy. Typically, dosages *used in vitro* may provide useful guidance in the amounts useful for in situ administration of these reagents. Animal testing of effective doses for treatment of particular disorders will provide further predictive indication of human dosage. Various considerations are described, e.g., in Gilman, et al. (eds.) (1990) Goodman and Gilman's: The Pharmacological Bases of Therapeutics (8th ed.) Pergamon Press; and (1990) Remington's Pharmaceutical Sciences (1 7th ed.) Mack Publishing Co., Easton, PA. Methods for administration are discussed therein and below, e.g., for oral, intravenous, intraperitoneal, or intramuscular administration, transdermal diffusion, and others. Pharmaceutically acceptable carriers will include water, saline, buffers, and other compounds described, e.g., in the Merck Index, Merck & Co., Rahway, NJ. Dosage ranges would ordinarily be expected to be in amounts lower than 1 mM concentrations, typically less than about 10 µM concentrations, usually less than about 100 nM, preferably less than about 10 pM (picomolar), and most preferably less than about 1 fM (femtomolar), with an appropriate carrier. Slow release formulations, or a slow release apparatus will often be utilized for continuous administration.

LP protein, fragments thereof, and antibodies to it or its fragments, antagonists, and agonists, may be administered directly to the host to be treated or, depending on the size of the compounds, it may be desirable to conjugate them to carrier proteins such as ovalbumin or serum albumin prior to their administration. Therapeutic formulations may be administered in any conventional dosage formulation. While it is possible for the active ingredient to be administered alone, it is preferable to present it as a pharmaceutical formulation. Formulations typically comprise at least one active ingredient, as defined above, together with one or more acceptable carriers thereof. Each carrier should be both pharmaceutically and physiologically acceptable in the sense of being compatible with the other ingredients and not injurious to the patient. Formulations include those suitable for oral, rectal, nasal, or parenteral (including subcutaneous, intramuscular, intravenous and intradermal) administration. The formulations may conveniently be presented in unit dosage form and may be prepared by any methods well known in the art of pharmacy. See, e.g., Gilman, et al. (eds.) (1990) Goodman and Gilman's: The Pharmacological Bases of Therapeutics (8th ed.) Pergamon Press; and (1990) Remington's Pharmaceutical Sciences (17th ed.) Mack Publishing Co., Easton, PA; Avis, et al. (eds.) (1993) Pharmaceutical Dosage Forms: Parenteral Medications Dekker, NY; Lieberman, et al. (eds.) (1990) Pharmaceutical Dosage Forms: Tablets Dekker, NY; and Lieberman, et al. (eds.) (1990) Pharmaceutical Dosage Forms: Disperse Systems Dekker, NY. The treatment of this invention may be combined with or used in association with other therapeutic agents.

The present invention also provides a pharmaceutical composition. Such a composition comprises, e.g., a therapeutically effective amount of a composition of the invention in a pharmaceutically acceptable carrier. As used herein, the term **"pharmaceutically acceptable carrier"** means a carrier approved by a federal regulatory agency of the United States of America, or a regulatory/administrative agency of a state government of the United States or a carrier that is listed in the U.S. Pharmacopeia or other pharmacopeia; which is generally recognized by those in the art for use in an animal, e.g., a mammal, and, more particularly, in a primate, e.g., a human primate.

Various delivery systems are known and can be used to administer, e.g., a composition, formulation, antibody polypeptide (or fragment thereof), or polynucleotide (or fragment thereof) of the invention. For example, delivery can use liposomes, microparticles, microcapsules, recombinant cells, receptor-mediated endocytosis (see, e.g., Wu and Wu (1987) J. Biol. Chem. 262:4429-4432), inclusion of a nucleic acid molecule as part of a retroviral or other vector, etc. Methods of administration include, e.g., without limit, intradermal, intramuscular, intraperitoneal, intravenous, subcutaneous, intranasal, epidural, and oral routes.

An LP can be useful in ameliorating, treating, preventing, modulating, and/or diagnosing a disease, disorder, syndrome, or condition of the immune system, by, e.g., activating or inhibiting the proliferation, differentiation, or mobilization (chemotaxis or directed movement) of an immune cell. Typically, immune cells develop through a process called hematopoiesis, producing myeloid (platelets, red blood cells, neutrophils, and macrophages) and lymphoid (B and T lymphocytes) cells from pluripotent stem cells.

The etiology of an immune disease, disorder, syndrome, or condition may be genetic and/or somatic, (e.g., such as some forms of cancer or some autoimmune conditions acquired by e.g., chemotherapy or toxins or an infectious agent, e.g., a virus or prion-like entity. Moreover, an LP can be used to mark or detect a particular immune system disease, syndrome, disorder, state, or condition.

An LP can be useful in ameliorating, treating, preventing, modulating, and/or diagnosing a disease, disorder, syndrome, and/or a condition of a hematopoietic cell. An LP could be used to increase or inhibit the differentiation or proliferation of a hematopoietic cell, including a pluripotent stem cell such an effect can be implemented to treat, prevent, modulate, or ameliorate a disease, disorder, syndrome, and/or a condition associated with a decrease in a specific type of hematopoietic cell. An example of such an immunologic deficiency, disease, disorder, syndrome, and/or condition includes, e.g., without limitation, a blood condition (e.g. agammaglobulinemia, digammaglobulinemia), ataxia telangiectasia, common variable immunodeficiency, Digeorge Syndrome, HIV infection, HTLV-BLV infection, leukocyte adhesion deficiency syndrome, lymphopenia, phagocyte bactericidal dysfunction, severe combined immunodeficiency (SCIDs), Wiskott-Aldrich Disorder, anemia, thrombocytopenia, or hemoglobinuria.

Moreover, an LP can be used to modulate hemostatic or thrombolytic activity. For example, increasing hemostatic or thrombolytic activity can treat or prevent a blood coagulation condition such as e.g., afibrinogenemia, a factor deficiency, a blood platelet disease (e.g. thrombocytopenia), or a wound resulting from e.g., trauma, surgery, etc. Alternatively, a composition of the invention can be used to decrease hemostatic or thrombolytic activity or to inhibit or dissolve a clotting condition. Such compositions can be important in a treatment or prevention of a heart condition, e.g., an attack infarction, stroke, or mycardial scarring.

An LP may also be useful in ameliorating, treating, preventing, modulating and/or diagnosing an autoimmune disease, disorder, syndrome, and/or condition such as results, e.g., from the inappropriate recognition by a cell of the immune system of the self as a foreign material. Such an inappropriate recognition results in an immune response leading to detrimental effect destruction on the host, e.g., on a host cell, tissue, protein, or moiety, e.g., a carbohydrate side chain. Therefore, administration of an LP which inhibits a detrimental immune response, particularly, e.g., a proliferation, differentiation, or chemotaxis of a T-cell, may be effective in detecting, diagnosing, ameliorating, or preventing such an autoimmune disease, disorder, syndrome, and/or condition. Examples of autoimmune conditions that can be affected by the present invention include, e.g., without limit Addison's Disease syndrome hemolytic anemia, anti-phospholipid syndrome, rheumatoid arthritis, dermatitis, allergic encephalomyelitis, glomerulonephritis, Goodpasture's Syndrome, Graves' Disease syndrome, Multiple Sclerosis, Myasthenia Gravis, Neuritis, Ophthalmia, Bullous Pemphigoid, Pemphigus, Polyendocrinopathies, Purpura, Reiter's Disease syndrome, Stiff-Man Syndrome, Autoimmune Thyroiditis, Systemic Lupus Erythematosus, Autoimmune Pulmonary Inflammation, Guillain-BarreSyndrome, insulin dependent diabetes mellitis, and autoimmune inflammatory eye disease.

Similarly, allergic reactions and conditions, such as asthma (e.g., allergic asthma) or other respiratory problems, may also be ameliorated, treated, modulated or prevented, and/or diagnosed by an LP polynucleotide or polypeptide (or fragment thereof), or an agonist or antagonist thereto. Moreover, such inventive compositions can be used to effect, e.g., anaphylaxis, hypersensitivity to an antigenic molecule, or blood group incompatibility.

An LP may also be used to modulate, ameliorate, treat, prevent, and/or diagnose organ rejection or graft-versus-host disease (GVHD). Generally speaking, organ rejection occurs by a host's, immune-cell destruction of a transplanted tissue or cell. A similarly destructive immune response is involved in GVHD, however, in this case, transplanted foreign immune cells destroy host tissues and/or cells. Administration of a composition of the invention, which ameliorates or modulates such a deleterious immune response (e.g., a deleterious proliferation, differentiation, or chemotaxis of a T cell), can be effective in modulating, ameliorating, diagnosing, and/or preventing organ rejection or GVHD.

Similarly, an LP may also be used to detect, treat, modulate, ameliorate, prevent, and/or diagnose an inflammation, e.g., by inhibiting the proliferation and/or differentiation of a cell involved in an inflammatory response, or an inflammatory condition (either chronic or acute), including, e.g., without limitation, chronic prostatitis, granulomatous prostatitis and malacoplakia, an inflammation associated with an infection (such as, e.g., septic shock, sepsis, or systemic inflammatory response syndrome (SIRS)), ischemia-reperfusion injury, endotoxin lethality, arthritis, complement-mediated hyperacute rejection, nephritis, cytokine or chemokine induced lung injury, inflammatory bowel disease syndrome, Crohn's disease syndrome, or a condition resulting from an over production of a cytokine(s) (e.g., TNF or IL-1.)

An LP can be used to modulate, ameliorate, treat, prevent, and/or diagnose a hyperproliferative disease, condition, disorder, or syndrome (such as, e.g., a neoplasm) via direct or indirect interactions. For example, such as by initiating the proliferation of cells that, in turn, modulate a hyperproliferative state; or by increasing an immune response (e.g., by increasing the antigenicity of a protein involved in a hyperproliferative condition); or by causing the proliferation, differentiation, or mobilization of a specific cell type (e.g., a T-cell). A desired effect using a composition of the invention may also be accomplished either by, e.g., enhancing an existing immune response, or by initiating a new immune response. Alternatively, the desired result may be effected either by, e.g., diminishing or blocking an existing immune response, or by preventing the initiation of a new immune response.

Examples of such hyperproliferative states, diseases, disorders, syndromes, and/or conditions include, e.g., without limitation, a neoplasm of the colon, abdomen, bone, breast, digestive system, liver, pancreas, peritoneum, endocrine system (e.g., an adrenal gland, a parathyroid gland, the pituitary, the testicles, the ovary, the thymus, or the thyroid), eye, head, neck, nervous system (central or peripheral), the lymphatic system, pelvis, skin, spleen, thorax, and urogenital system. Similarly, other hyperproliferative conditions, include, e.g., without limit hypergammaglobulinemia, lymphoproliferative conditions, paraproteinemias, purpura, sarcoidosis, Hamartoma, Sezary Syndrome, Waldenstron's Macroglobulinemia, Gaucher's Disease syndrome, histiocytosis, and other hyperproliferative states.

One preferred embodiment utilizes an LP to inhibit aberrant cellular division, through a polynucleotide delivery technique. Thus, the present invention provides a method for treating, preventing, modulating, ameliorating, preventing, inhibiting, and/or diagnosing cell proliferative diseases, disorders, syndromes, and/or conditions described herein by inserting into an abnormally proliferating cell a composition of the present invention, wherein said composition beneficially modulates an excessive condition of cell proliferation, e.g., by inhibiting transcription and/or translation. Another embodiment comprises administering one or more active copies of an LP polynucleotide sequence to an abnormally proliferating cell. For example in one embodiment, an LP polynucleotide sequence is operably linked in a construct comprising a recombinant expression vector that is effective in expressing a polypeptide (or fragment thereof) corresponding to the polynucleotide of interest. In another preferred embodiment, the construct encoding a polypeptide or fragment thereof, is inserted into a targeted cell utilizing a retrovirus or an adenoviral vector (see, e.g., Nabel, et al. (1999) Proc. Natl. Acad. Sci. USA 96: 324-326). In a still preferred embodiment, the viral vector is defective and only transforms or transfects a proliferating cell but does not transform or transfects a non-proliferating cell. Moreover, in a still further preferred embodiment, an LP polynucleotide sequence is inserted into a proliferating cell either alone, (or in combination with, or fused to, another polynucleotide sequence, which can subsequently be modulated via an external stimulus (e.g., a magnetic signal, a specific small molecule, a chemical moiety or a drug administration, etc.) that acts on an upstream promoter to induce expression of the LP polypeptide (or fragment thereof).

As such, a desired effect of the present invention (e.g., selectively increasing, decreasing, or inhibiting expression of an LP polynucleotide sequence) may be accomplished based on using an external stimulus.

An LP sequence may be useful in repressing the expression of a gene or an antigenic composition, e.g., an oncogenic retrovirus. By **"repressing the expression of a gene"** is meant, e.g., the suppression of the transcription of a 'gene', the degradation of a 'gene' transcript (pre-message RNA), the inhibition of splicing of a 'gene', the destruction of mRNA, the prevention of a post-translational modification of a polypeptide, the destruction of a polypeptide, or the inhibition of a normal function of a protein.

Local administration to an abnormally proliferating cell may be achieved by any art known method or technique discussed herein including, e.g., without limit to transfection, electroporation, microinjection of cells, or in vehicles (such as a liposome, lipofectin, or a naked polynucleotide). Encompassed delivery systems include, without limit, retroviral vectors (Gilboa, J. Virology 44:845 (1982); Hocke, Nature 320:275 (1986); Wilson, et al., Proc. Natl. Acad. Sci. U.S.A. 85:3014); vaccinia virus systems (Chakrabarty, et al., Mol. Cell Biol. 5:3403 (1985); Yates, et al., Nature 3 13:8 12 (1985). Preferably a retroviral, or adenoviral delivery system (as known in the art or described herein) is used to specifically deliver a recombinant construct or to transfect a cell that is abnormally proliferating. An LP polynucleotide sequence may be delivered directly to the site of a cell proliferation, e.g., in an internal organ, body cavity, and the like by use of, e.g., an imaging device used to guide the recombinant construct. Alternatively, administration to an appropriate location may be carried out at a time of surgical intervention.

By **"cell proliferative condition"** is meant any human or animal disease, syndrome, disorder, condition, or state, affecting any cell, tissue, any site or any combination of organs, tissues, or body parts, which is characterized by a single or multiple local abnormal proliferation of cells, groups of cells, or tissues, whether benign or malignant. Any amount of LP may be administered as long as it has a desired effect on the treated cell, e.g., a biologically inhibiting effect on an abnormally proliferating cell. Moreover, it is possible to administer more than one LP polynucleotide or polypeptide (or fragment thereof), or an agonist or antagonist thereto, simultaneously to the same site.

By **"biologically inhibiting"** is meant a partial or total inhibition of mitotic activity and/or a decrease in the rate of mitotic activity or metastatic activity of a targeted cell. A biologically inhibitory dose can be determined by assessing the effects of an LP on abnormally proliferating cell division in a cell or tissue culture, tumor growth in an animal or any other art known method. In another embodiment, an LP can be useful to inhibit angiogenesis associated with abnormally proliferative cells or tissues, either alone, or as a protein fusion, or in combination with another LP polynucleotide or polypeptide (or fragment thereof), or an agonist or antagonist, thereto. In a preferred embodiment, a desired anti-angiogenic effect may be achieved indirectly, e.g., through the inhibition of hematopoietic, tumor-specific cells, such as, e.g., tumor-associated macrophages (see e.g., Joseph, et al. (1998) J Natl. Cancer Inst. 90(21): 1648-53). Alternatively, in a desired anti-angiogenic effect may be achieved directly, (e.g., see Witte, et al., (1998) Cancer Metastasis Rev. 17(2): 155-61).

An LP, including a protein fusion, may be useful in inhibiting an abnormally proliferative cell or tissue, via an induction of apoptosis. An LP may act either directly, or indirectly to induce apoptosis in a proliferative cell or tissue, e.g., by activating the death-domain FA receptor, such as, e.g., tumor necrosis factor (TNF) receptor-1, CD95 (F&APO-1), TNF-receptor-related apoptosis-mediated protein (TRAMP) and TNF-related apoptosis-inducing ligand (TRAIL) receptor-1 and -2 (see, e.g., Schulze-Osthoff, et al., Eur J Biochem 254 (3): 439-59 (1998), which is hereby incorporated by reference for teachings on apoptotic cell death). Moreover, in another preferred embodiment, an LP may induce apoptosis via other mechanisms, such as, e.g., through the activation of a pathway that subsequently activates apoptosis, or through stimulating the expression of a protein(s) that activates an apoptotic pathway, either alone or in combination with small molecule drugs or adjuvants, such as apoptonin, galectins, thioredoxins, anti-inflammatory proteins (see e.g., Mutat Res 400 (1-2):447-55 (1998), Med Hypotheses. 50(5): 423-33 (1998), Chem Biol Interact. Apr 24; lll-l12:23-34 (1998), J Mol Med. 76(6): 402-12(1998), Int J Tissue React; 20 (1):3-15 (1998), which are all hereby incorporated by reference for these teachings).

An LP is useful in inhibiting cell metastasis either directly as a result of administering a polynucleotide or polypeptide (or fragment thereof), or an agonist or antagonist thereto, (as described elsewhere herein), or indirectly, such as, e.g., by activating or increasing the expression of a protein known to inhibit metastasis, such as, e.g., an alpha integrin, (see, e.g., Cur. Top Microbial Immunol 1998; 23 1: 125-4 1, which is hereby incorporated by reference for these teachings). Such a desired effect can be achieved either alone using an LP or in combination with e.g., a small molecule drug or an adjuvant.

An LP, or a protein fusion thereto, is useful in enhancing the immunogenicity and/or antigenicity of a proliferating cell or tissue, either directly, (such as would occur if e.g., an LP polypeptide (or fragment thereof) 'vaccinated' the immune system to respond to a proliferative antigen or immunogen), or indirectly, (such as in activating, e.g., the expression a of protein known to enhance an immune response (e.g. a chemokine), to an antigen on an abnormally proliferating cell).

An LP may be used to, modulate, ameliorate, effect, treat, prevent, and/or diagnose a cardiovascular disease, disorder, syndrome, and/or condition. As described herein, including, e.g., without limitation, cardiovascular abnormalities, such as arterio-arterial fistula, arteriovenous fistula, cerebral arteriovenous malformations, congenital heart defects, pulmonary atresia, and Scimitar Syndrome peripheral artery disease, syndrome, such as limb ischemia. Additional cardiovascular disorders encompass, e.g., congenital heart defects which include, e.g., aortic coarctation, car triatriatum, coronary vessel anomalies, crisscross heart, dextrocardia, patent ductus arteriosus, Ebstein's anomaly, Eisenmenger complex, hypoplastic left heart syndrome, levocardia, tetralogy of fallot, transposition of great vessels, double outlet right ventricle, tricuspid atresia, persistent truncus arteriosus, and heart septal defects, such as e.g., aortopulmonary septal defect, endocardial cushion defects, Lutembacher's Syndrome, trilogy of Fallot, and ventricular heart septal defects. Further cardiovascular conditions include, e.g., heart disease syndrome, such as, e.g., arrhythmias, carcinoid heart disease syndrome, high cardiac output, low cardiac output, cardiac tamponade, endocarditis (including bacterial endocarditis), heart aneurysm, cardiac arrest, congestive heart failure, congestive cardiomyopathy, paroxysmal dyspnea, cardiac edema, heart hypertrophy, congestive cardiomyopathy, left ventricular hypertrophy, right ventricular hypertrophy, post-infarction heart rupture, ventricular septal rupture, heart valve disease, myocardial disease, myocardial ischemia, pericardial effusion, pericarditis (including constrictive and tuberculous pericarditis), pneumopericardium, post-pericardiotomy syndrome, pulmonary heart disease syndrome, rheumatic heart disease syndrome, ventricular dysfunction, hyperemia, cardiovascular pregnancy complications, Scimitar Syndrome, cardiovascular syphilis, and cardiovascular tuberculosis. Further cardiovascular disorders include, e.g., arrhythmias including, e.g., sinus arrhythmia, atrial fibrillation, atrial flutter, bradycardia, extra systole, Adams-Stokes Syndrome, bundle-branch block, sinoatrial block, long QT syndrome, parasystole, Lown-Ganong-Levine Syndrome, Mahaim-type preexcitation syndrome, Wolff Parkinson-White syndrome, sick sinus syndrome, and ventricular fibrillation tachycardias. Tachycardias encompassed with the cardiovascular condition described herein include, e.g., paroxysmal tachycardia, supraventricular tachycardia, accelerated idioventricular rhythm, atrioventricular nodal re-entry tachycardia, ectopic atrial tachycardia, ectopic junctional tachycardia, sinoatrial nodal re-entry tachycardia, sinus tachycardia, Torsades de Pointes Syndrome, and ventricular tachycardia. Additional cardiovascular disorders include, e.g., heart valve disease such as, e.g., aortic valve insufficiency, aortic valve stenosis, heart murmurs, aortic valve prolapse, mitral valve prolapse, tricuspid valve prolapse, mitral valve insufficiency, mitral valve stenosis, pulmonary atresia, pulmonary valve insufficiency, pulmonary valve stenosis, tricuspid atresia, tricuspid valve insufficiency, and tricuspid valve stenosis. Myocardial conditions associated with cardiovascular disease include, e.g., myocardial diseases such as, e.g., alcoholic cardiomyopathy, congestive cardiomyopathy, hypertrophic cardiomyopathy, aortic subvalvular stenosis, pulmonary subvalvular stenosis, restrictive cardiomyopathy, Chagas cardiomyopathy, endocardial fibroelastosis, endomyocardial fibrosis, Kearns Syndrome, myocardial reperfusion injury, and myocarditis.

Cardiovascular conditions include, e.g., myocardial ischemias such as, e.g., coronary disease syndrome, such as e.g., angina pectoris, coronary aneurysm, coronary arteriosclerosis, coronary thrombosis, coronary vasispasm, myocardial infarction, and myocardial stunning. Cardiovascular diseases also encompassed herein include, e.g., vascular diseases such as e.g., aneurysms, angiodysplasia, angiomatosis, bacillary angiomatosis, Hippel-Lindau Disease syndrome, Klippel-Trenaunay-Weber Syndrome, Sturge-Weber Syndrome, angioneurotic edema, aortic disease, Takayasu's Arteritis, aortitis, Leriche's Syndrome, arterial occlusive disease, arteritis, enarteritis, polyarteritis nodosa, cerebrovascular disease, diabetic angiopathies, diabetic retinopathy, embolism, thrombosis, erythromeialgia, hemorrhoids, hepatic veno-occlusive disease syndrome, hypertension, hypotension, ischemia, peripheral vascular diseases, phlebitis, pulmonary veno-occlusive disease syndrome, Raynaud's disease syndrome, CREST syndrome, retinal vein occlusion, Scimitar syndrome, superior vena cava syndrome, telangiectasia, ataxia telangiectasia, hereditary hemorrhagic telangiectasia, varicocele, varicose veins, varicose ulcer, vasculitis, and venous insufficiency. Cardiovascular conditions further include, e.g., aneurysms such as, e.g., dissecting aneurysms, false aneurysms, infected aneurysms, ruptured aneurysms, aortic aneurysms, cerebral aneurysms, coronary aneurysms, heart aneurysms, and iliac aneurysms. Arterial occlusive cardiovascular conditions include, e.g., arteriosclerosis, intermittent claudication, carotid stenosis, fibromuscular dysplasias, mesenteric vascular occlusion, Moyamoya disease syndrome, renal artery obstruction, retinal artery occlusion, and thromboangiitis obliterans.

Cerebrovascular cardiovascular conditions include, e.g., carotid artery disease, cerebral amyloid angiopathy, cerebral aneurysm, cerebral anoxia, cerebral arteriosclerosis, cerebral arteriovenous malformation, cerebral artery disease, cerebral embolism and thrombosis, carotid artery thrombosis, sinus thrombosis, Wallenberg's syndrome, cerebral hemorrhage, epidural hematoma, subdural hematoma, subarachnoid hemorrhage, cerebral infarction, cerebral ischemia (including transient cerebral ischemia), subclavian steal syndrome, periventricular leukomalacia, vascular headache, cluster headache, migraine, and vertebrobasilar insufficiency. Embolic cardiovascular conditions include, e.g., air embolisms, amniotic fluid embolisms, cholesterol embolisms, blue toe syndrome, fat embolisms, pulmonary embolisms, and thromboembolisms. Thrombotic cardiovascular conditions include, e.g., coronary thrombosis, hepatic vein thrombosis, retinal vein occlusion, carotid artery thrombosis, sinus thrombosis, Wallenberg's syndrome, and thrombophlebitis. Ischemic conditions include, e.g., cerebral ischemia, ischemic colitis, compartment syndromes, anterior compartment syndrome, myocardial ischemia, reperfusion injuries, and peripheral limb ischemia. Vasculitic conditions include, e.g., aortitis, arteritis, Behcet's Syndrome, Churg-Strauss Syndrome, mucocutaneous lymph node syndrome, thromboangiitis obliterans, hypersensitivity vasculitis, Schoenlein-Henoch purpura, allergic cutaneous vasculitis, and Wegener's granulomatosis. An LP can be beneficial in ameliorating critical limb ischemia and coronary disease. An LP may be administered using any art known method, described herein An LP may administered as part of a therapeutic composition or formulation, as described in detail herein. Methods of delivering an LP are also described in detail herein.

### Anti-Hemopoietic Activity

The naturally occurring balance between endogenous stimulators and inhibitors of angiogenesis is one in which inhibitory influences typically predominate (see, e.g., Rastinejad, et al., Cell 56345-355 (1989)). When neovascularization occurs under normal physiological conditions, such as wound healing, organ regeneration, embryonic development, and female reproductive processes, angiogenesis is stringently regulated, and delimited spatially and temporally. In pathological angiogenesis such as, e.g., during solid tumor formation, these regulatory controls fail and unregulated angiogenesis can become pathologic by sustaining progression of many neoplastic and non-neoplastic diseases. A number of serious diseases are dominated by abnormal neovascularization (including, e.g., solid tumor growth and metastases, arthritis, some types of eye conditions, and psoriasis; see, e.g., reviews by Moses, et al., Biotech. 9630-634 (1991); Folkman, et al., N. Engl. J. Med., 333: 1757-1763 (1995); Auerbach, et al., J. Microvasc. Res. 29:401-4 11 (1985); Folkman, "Advances in Cancer Research", eds. Klein and Weinhouse, Academic Press, New York, pp. 175-203 (1985); Patz, Am. J. Opthalmol. 94:7 15-743 (1982); and Folkman, et al., Science 221:7 19-725 (1983).

In a number of pathological conditions, angiogenesis contributes to a disease-state, e.g., for example, significant data have accumulated suggesting that solid tumor formation is dependent on angiogenesis (see, e.g., Folkman and Klagsbrun, Science 235:442-447 (1987)). In another embodiment of the invention, administration of an LP provides for the treatment, amelioration, modulation, diagnosis, and/or inhibition of a disease, disorder, syndrome, and/or condition associated with neovascularization. Malignant and metastatic conditions that can be effected in a desired fashion using an LP include, e.g., without limitation, a malignancy, solid tumor, and a cancer as described herein or as otherwise known in the art (for a review of such disorders, syndromes, etc. see, e.g., Fishman, et al., Medicine, 2d Ed., J. B. Lippincott Co., Philadelphia (1985)). Thus, the present invention provides a method of ameliorating, modulating, treating, preventing, and/or diagnosing an angiogenesis-related disease and/or disorder, comprising administering to a subject in need thereof a beneficially effective amount of an LP. For example, cancers that may be so affected using a composition of the invention includes, e.g., without limit a solid tumor, including e.g., prostate, lung, breast, ovarian, stomach, pancreas, larynx, esophagus, testes, liver, parotid, biliary tract, colon, rectum, cervix, uterus, endometrium, kidney, bladder, thyroid cancer; primary tumors and metastases; melanomas; glioblastoma; Kaposi's sarcoma; leiomyosarcoma; non-small cell lung cancer; colorectal cancer; advanced malignancies; and blood born tumors such as e.g., leukemia.

Moreover, an LP may be delivered topically, to treat or prevent cancers such as, e.g., skin cancer, head and neck tumors, breast tumors, and Kaposi's sarcoma. Within yet another aspect, an LP may be utilized to treat superficial forms of bladder cancer by, e.g., intravesical administration into the tumor, or near the tumor site; via injection or a catheter. Of course, the appropriate mode of administration will vary according to the cancer to be treated. Other modes of delivery are discussed herein. An LP may also be useful in modulating, ameliorating, treating, preventing, and/or diagnosing another disease, disorder, syndrome, and/or condition, besides a cell proliferative condition (e.g., a cancer) that is assisted by abnormal angiogenic activity. Such close group conditions include, e.g., without limitation, benign tumors, e.g., such as hemangiomas, acoustic neuromas, neurofibromas, trachomas, and pyogenic granulomas; atherosclerotic plaques; ocular angiogenic diseases, e.g., diabetic retinopathy, retinopathy of prematurity, macular degeneration, cornea graft rejection, neovascular glaucoma, retrolental fibroplasia, rubeosis, retinoblastoma, uvietis and Pterygia (abnormal blood vessel growth) of the eye; rheumatoid arthritis; psoriasis; delayed wound healing; endometriosis; vasculogenesis; granulations; hypertrophic scars (keloids); nonunion fractures; scleroderma; trachoma; vascular adhesions; myocardial angiogenesis; coronary collaterals; cerebral collaterals; arteriovenous malformations; ischemic limb angiogenesis; Osler-Webber Syndrome; plaque neovascularization; telangiectasia; hemophiliac joints; angiofibroma; fibromuscular dysplasia; wound granulation; Crohn's disease; and atherosclerosis.

For example, within another aspect of the present invention methods are provided for modulating, ameliorating, treating, preventing, and/or diagnosing hypertrophic scars and keloids, comprising administering an LP to a site of hypertrophic scar or keloid formation. Within one embodiment, the method involves a direct injection into a hypertrophic scar or keloid, to provide a beneficial effect, e.g., by preventing progression of such a lesion. This method is of particular value to a prophylactic treatment of a condition known to result in the development of a hypertrophic scar or a keloid (e.g., burns), and is preferably initiated after the proliferative phase of scar formation has had time to progress (approximately, e.g., 14 days after the initial injury), but before hypertrophic scar or keloid development. As noted above, the present invention also provides methods for ameliorating, treating, preventing, and/or diagnosing neovascular diseases of the eye, including e.g., corneal graft neovascularization, neovascular glaucoma, proliferative diabetic retinopathy, retrolental fibroplasia and macular degeneration. Moreover, ocular diseases, disorders, syndromes, and/or conditions associated with neovascularization that can be modulated ameliorated, treated, prevented, and/or diagnosed with an LP include, e.g., without limit; neovascular glaucoma, diabetic retinopathy, retinoblastoma, retrolental fibroplasia, uveitis, retinopathy of premature macular degeneration, corneal graft neovascularization, as well as other inflammatory eye diseases, ocular tumors, and diseases associated with choroidal or iris neovascularization (see, e.g., reviews by Waltman, et al., (1978) Am. J. Ophthal. 8.51704-710 and Gartner, et al., (1978) Sun. Ophthd. 22:291-3 12). Thus, within one aspect of the present invention methods are provided for treating or preventing neovascular diseases of the eye such as corneal neovascularization (including corneal graft neovascularization), comprising administering to a patient a therapeutically effective amount of an LP composition to the cornea, such that the formation of blood vessels is inhibited or delayed. Briefly, the cornea is a tissue that normally lacks blood vessels. In certain pathological conditions however, capillaries may extend into the cornea from the pericorneal vascular plexus of the limbus. When the cornea becomes vascularized, it also becomes clouded, resulting in a decline in the patient's visual acuity. Visual loss may become complete if the cornea completely opacifies. A wide variety of diseases, disorders, syndromes, and/or conditions can result in corneal neovascularization, including e.g., corneal infections (e.g., trachoma, herpes simplex keratitis, leishmaniasis and onchocerciasis), immunological processes (e.g., graft rejection and Stevens-Johnson's syndrome), alkali burns, trauma, inflammation (of any cause), toxic and nutritional deficiency states, and as a complication of using contact lenses.

Within particularly preferred embodiments, an LP composition may be prepared for topical administration in saline (combined with any of the preservatives and anti-microbial agents commonly used in ocular preparations), and administered in drop form to the eye. The solution or suspension may be prepared in its pure form and administered several times daily. Alternatively, anti-angiogenic compositions, prepared as described herein, may also be administered directly to the cornea. Within preferred embodiments, an anti-angiogenic composition is prepared with a muco-adhesive polymer, which binds to the cornea.

Within further embodiments, an anti-angiogenic factor or anti-angiogenic LP composition may be utilized as an adjunct to conventional steroid therapy. Topical therapy may also be useful prophylactically in corneal lesions that are known to have a high probability of inducing an angiogenic response (such as, e.g., a chemical burn). In these instances, the treatment (likely in combination with steroids) may be instituted immediately to help prevent subsequent complications. Within other embodiments, an LP composition may be injected directly into the corneal stroma using microscopic guidance by an ophthalmologist. The preferred site of injection may vary with the morphology of the individual lesion, but the goal of the administration is to place a composition of the invention at the advancing front of the vasculature (i.e., interspersed between the blood vessels and the normal cornea). In most instances, this would involve perilimbic corneal injection to "protect" the cornea from advancing blood vessels. This method may also be utilized shortly after a corneal insult to prophylactically prevent corneal neovascularization. In such a situation, the composition could be injected into the perilimbic cornea interspersed between the corneal lesion and its undesired potential limbic blood supply. Such methods may also be utilized in a similar fashion to prevent capillary invasion of transplanted corneas. In a sustained-release form, injections might only be required 2-3 times per year. A steroid could also be added to the injection solution to reduce inflammation resulting from the injection itself.

Within another aspect, methods are provided for treating or preventing neovascular glaucoma, comprising administering to a patient a therapeutically effective amount of an LP to the eye, such that the formation of blood vessels is inhibited. In one embodiment, the composition may be administered topically to the eye to treat or prevent early forms of neovascular glaucoma. Within other embodiments, the composition may be implanted by injection into the region of the anterior chamber angle. Within other embodiments, the composition may also be placed in any location such that the composition is continuously released into the aqueous humor. Within another aspect, methods are provided for treating or preventing proliferative diabetic retinopathy, comprising administering to a patient a therapeutically effective amount of an LP to the eyes, such that the formation of blood vessels is inhibited. Within a particularly preferred embodiment, proliferative diabetic retinopathy may be treated by injection into the aqueous or the vitreous humor, to increase the local concentration of a composition of the invention in the retina. Preferably, this treatment should be initiated before the acquisition of severe disease requiring photocoagulation. Within another aspect of the present invention, methods are provided for treating or preventing retrolental fibroplasia, comprising administering to a patient a beneficially effective amount of an LP to the eye, such that the formation of blood vessels is inhibited. The composition may be administered topically, via intravitreous injection and/or via intraocular implants. Additional, diseases, disorders, syndromes, and/or conditions that can be modulated, ameliorated, treated, prevented, and/or diagnosed with an LP include, e.g., without limitation, hemangioma, arthritis, psoriasis, angiofibroma, atherosclerotic plaques, delayed wound healing, granulations, hemophilic joints, hypertrophic scars, nonunion fractures, Osler-Weber syndrome, pyogenic granuloma, scleroderma, trachoma, and vascular adhesions.

Moreover, diseases, disorders, states, syndromes, and/or conditions that can be modulated, ameliorated, treated, prevented, and/or diagnosed with an LP include, e.g., without limitation, solid tumors, blood born tumors such as leukemias, tumor metastasis, Kaposi's sarcoma, benign tumors (e.g., hemangiomas), acoustic neuromas, neurofibromas, trachomas, and pyogenic granulomas, rheumatoid arthritis, psoriasis, ocular angiogenic diseases, e.g., diabetic retinopathy, retinopathy of prematurity, macular degeneration, corneal graft rejection, neovascular glaucoma, retrolental fibroplasia, rubeosis, retinoblastoma, and uvietis, delayed wound healing, endometriosis, vasculogenesis, granulations, hypertrophic scars (keloids), nonunion fractures, scleroderma, trachoma, vascular adhesions, myocardial angiogenesis, coronary collaterals, cerebral collaterals, arteriovenous malformations, ischemic limb angiogenesis, Osler-Webber Syndrome, plaque neovascularization, telangiectasia, hemophiliac joints, angiofibroma fibromuscular dysplasia, wound granulation, Crohn's disease, syndrome, atherosclerosis, birth-control inhibition of vascularization necessary for embryo implantation during the control of menstruation, and diseases that have angiogenesis as a pathologic consequence such as, e.g., cat scratch disease (Rochele minalia quintosa), ulcers *(Helicobacter pylori),* Bartonellosis and bacillary angiomatosis.

In another embodiment as a birth control method, an amount of an LP sufficient to block embryo implantation is administered before or after intercourse and fertilization have occurred, thus providing an effective method of birth control, possibly a "morning after" method. An LP may also be used in controlling menstruation or administered either as a peritoneal lavage fluid or for peritoneal implantation in the treatment of endometriosis.

An LP may be utilized in a wide-variety of surgical procedures. For example, within one aspect of the present invention a compositions (in the form of, e.g., a spray or film) may be utilized to coat or spray an area before removal of a tumor, to isolate normal surrounding tissues from malignant tissue, and/or to prevent the spread of disease to surrounding tissues. Within other aspects, an LP composition (e.g., in the form of a spray) may be delivered via endoscopic procedures to coat tumors, or inhibit angiogenesis in a desired locale. Within yet another aspect, surgical meshes that have been coated with an anti-angiogenic composition of the invention may be utilized in a procedure in which a surgical mesh might be utilized. For example, a surgical mesh laden with an anti-angiogenic composition may be utilized during cancer resection surgery (e.g., abdominal surgery subsequent to colon resection) to provide support to the structure, and to release an amount of the anti-angiogenic factor. Within further aspects of the present invention, methods are provided for treating tumor excision sites, comprising administering an LP to the resection margins of a tumor after excision, such that the local recurrence of cancer and the formation of new blood vessels at the site is inhibited.

Within one embodiment, an anti-angiogenic composition of the invention is administered directly to a tumor excision site (e.g., applied by swabbing, brushing or otherwise coating the resection margins of the tumor with the anti-angiogenic composition). Alternatively, an anti-angiogenic composition may be incorporated into a known surgical paste before administration. Within a particularly preferred embodiment, an anti-angiogenic composition of the invention is applied after hepatic resections for malignancy, and after neurosurgical operations. Within another aspect, administration can be to a resection margin of a wide variety of tumors, including e.g., breast, colon, brain, and hepatic tumors. For example, within one embodiment, anti-angiogenic compositions may be administered to the site of a neurological tumor after excision, such that the formation of new blood vessels at the site is inhibited.

### Diseases at the Cellular Level

Diseases associated with increased cell survival or the inhibition of apoptosis that could be modulated, ameliorated, treated, prevented, and/or diagnosed by an LP include, e.g., cancers (such as, e.g., follicular lymphomas, carcinomas with p53 mutations, and hormone-dependent tumors, including, e.g., but without limit, colon cancer, cardiac tumors, pancreatic cancer, melanoma, retinoblastoma, glioblastoma, lung cancer, intestinal cancer, testicular cancer, stomach cancer, neuroblastoma, myxoma, myoma, lymphoma, endothelioma, osteoblastoma, osteoclastoma, osteosarcoma, chondrosarcoma, adenoma, breast cancer, prostate cancer, Kaposi's sarcoma and ovarian cancer); autoimmune conditions (such as, e.g., multiple sclerosis, Sjogren's syndrome, Hashimoto's thyroiditis, biliary cirrhosis, Behcet's disease syndrome, Crohn's disease syndrome, polymyositis, systemic lupus erythematosus, immune-related glomerulonephritis, and rheumatoid arthritis); viral infections (such as, e.g., herpes viruses, pox viruses, and adenoviruses); inflammation; graft v. host disease syndrome, acute graft rejection, and chronic graft rejection.

In preferred embodiments, an LP is used to inhibit growth, progression, and/or metastases of cancers such as, in particular, those listed herein. Additional diseases, states, syndromes, or conditions associated with increased cell survival that could be modulated, ameliorated, treated, prevented, or diagnosed by an LP include, e.g., without limitation, progression, and/or metastases of malignancies and related disorders such as leukemia including acute leukemias (such as, e.g., acute lymphocytic leukemia, acute myelocytic leukemia, including myeloblastic, promyelocytic, myelomonocytic, monocytic, and erythroleukemia) and chronic leukemias (e.g., chronic myelocytic, chronic granulocytic, leukemia, and chronic lymphocytic leukemia)), polycythemia Vera, lymphomas (e.g., Hodgkin's disease, and non-Hodgkin's disease), multiple myeloma, Waldenstrom's macroglobulinemia, heavy chain disease, syndrome, and solid tumors including, e.g., without limitation, sarcomas and carcinomas (such as, e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma, and retinoblastoma).

Diseases associated with increased apoptosis that could be modulated, ameliorated, treated, prevented, and/or diagnosed by an LP include, e.g., AIDS, conditions (such as, e.g., Alzheimer's disease syndrome, Parkinson's disease syndrome, Amyotrophic lateral sclerosis, Retinitis pigmentosa, Cerebellar degeneration and brain tumor, or prion associated disease); autoimmune conditions (such as, e.g., multiple sclerosis, Sjogren's syndrome, Hashimoto's thyroiditis, biliary cirrhosis, Behcet's disease syndrome, Crohn's disease syndrome, polymyositis, systemic lupus erythematosus, immune-related glomerulonephritis, and rheumatoid arthritis); myelodysplastic syndromes (such as aplastic anemia), graft v. host disease syndrome; ischemic injury (such as that caused by myocardial infarction, stroke and reperfusion injury); liver injury (such as, e.g., hepatitis related liver injury, ischemia reperfusion injury, cholestosis (bile duct injury), and liver cancer); toxin-induced liver disease (such as, e.g., that caused by alcohol), septic shock, cachexia, and anorexia.

### Wound Healing and Epithelial Cell Proliferation

In accordance with yet a further aspect of the invention, there is provided a process for using an LP to stimulate epithelial cell proliferation and basal keratinocytes for the purpose of, e.g., wound healing, to stimulate hair follicle production, and to heal a dermal wound. An LP composition may be clinically useful in stimulating wound healing including e.g., surgical wounds, excisional wounds, deep wounds involving damage of the dermis and epidermis, eye tissue wounds, dental tissue wounds, oral cavity wounds, diabetic ulcers, dermal ulcers, cubitus ulcers, arterial ulcers, venous stasis ulcers, burns resulting from exposure heat or chemicals, abnormal wound healing conditions associated with e.g., uremia, malnutrition, vitamin deficiency and wound healing complications associated with systemic treatment with steroids, radiation therapy, anti-neoplastic drugs, and anti-metabolites. An LP could be used to promote dermal reestablishment after dermal loss.

An LP could be used to increase the adherence of skin grafts to a wound bed and to stimulate re-epithelialization from the wound bed. The following is a non-exhaustive list of grafts that an LP could be used to increase adherence to: a wound bed, autografts, artificial skin, allografts, autodermic grafts, autoepidermic grafts, avascular grafts, Blair-Brown grafts, bone grafts, brephoplastic grafts, cutis grafts, delayed grafts, dermic grafts, epidermic grafts, fascia grafts, full thickness grafts, heterologous grafts, xenografts, homologous grafts, hyperplastic grafts, lamellar grafts, mesh grafts, mucosal grafts, Ollier-Thiersch grafts, omenpal grafts, patch grafts, pedicle grafts, penetrating grafts, split skin grafts, and thick split grafts. An LP can be used to promote skin strength and to improve the appearance of aged skin. It is believed that an LP will also produce changes in hepatocyte proliferation, and epithelial cell proliferation in, for example, the lung, breast, pancreas, stomach, small intestine, and large intestine. Epithelial cell proliferation can be effected in epithelial cells such as, e.g., sebocytes, hair follicles, hepatocytes, type II pneumocytes, mucin-producing goblet cells, and other epithelial cells or their progenitors which are contained within the skin, lung, liver, and gastrointestinal tract.

An LP may: promote proliferation of endothelial cells, keratinocytes, and basal keratinocytes; it could also be used to reduce the side effects of gut toxicity that result from radiation, chemotherapy treatments or viral infections, it may have a cytoprotective effect on the small intestine mucosa; it may also stimulate healing of mucositis (mouth ulcers) that result from chemotherapy and viral infections, it could further be used in full regeneration of skin in full and partial thickness skin defects, including burns, (i.e., re-population of hair follicles, sweat glands; and sebaceous glands), treatment of other skin defects such as psoriasis, it also could be used to treat epidermolysis bullosa, a defect in adherence of the epidermis to the underlying dermis which results in frequent, open and painful blisters by accelerating re-epithelialization of these lesions; it could also be used to treat gastric and doudenal ulcers and help heal by scar formation of the mucosal lining and regeneration of glandular mucosa and duodenal mucosal lining more rapidly. Inflammatory bowel diseases, such as Crohn's disease and ulcerative colitis, are diseases that result in destruction of the mucosal surface of the small or large intestine, respectively. Thus, an LP could be used to promote resurfacing of a mucosal surface to aid more rapid healing and to prevent progression of inflammatory bowel disease resulting in a desired effect, e.g., such as on the production of mucus throughout the gastrointestinal tract and the protection of intestinal mucosa from injurious substances that are ingested or following surgery. An LP could be used to treat a condition associated with the under expression of an LP polynucleotide sequence or an LP polypeptide of the present invention (or fragment thereof), or an agonist or antagonist thereto.

Moreover, an LP could be used to prevent and heal damage to the lungs due to various pathological states, such as, e.g., stimulating proliferation and differentiation to promote repair of alveoli and bronchiolar epithelium. For example, emphysema, inhalation injuries, that (e.g., from smoke inhalation) and burns, which cause necrosis of the bronchiolar epithelium and alveoli could be effectively ameliorated, treated, prevented, and/or diagnosed using a polynucleotide or polypeptide of the invention (or fragment thereof), or an agonist or antagonist thereto. Also, an LP could be used to stimulate the proliferation of and differentiation of type II pneumocytes, to help treat or prevent hyaline membrane diseases, such as e.g., infant respiratory distress syndrome and bronchopulmonary displasia, (in premature infants). An LP could stimulate the proliferation and/or differentiation of a hepatocyte and, thus, could be used to alleviate or treat a liver condition such as e.g., fulminant liver failure (caused, e.g., by cirrhosis), liver damage caused by viral hepatitis and toxic substances (e.g., acetaminophen, carbon tetrachloride, and other known hepatotoxins). In addition, an LP could be used treat or prevent the onset of diabetes mellitus. In patients with newly diagnosed Types I and II diabetes, where some islet cell function remains, an LP could be used to maintain the islet function so as to alleviate, modulate, ameliorate, delay, or prevent permanent manifestation of the disease. In addition, an LP could be used as an auxiliary in islet cell transplantation to improve or promote islet cell function.

### Neurological Diseases

Nervous system diseases, disorders, syndromes, states, and/or conditions that can be modulated, ameliorated, treated, prevented, and/or diagnosed with an LP composition include, e.g., without limitation, nervous system injuries diseases, disorders, states, syndromes, and/or conditions that result in either a disconnection or misconnection of an axon or dendrite; a diminution or degeneration of a cell (or part of a cell) of the nervous system (such as, e.g., without limitation, neurons, astrocytes, microglia, macroglia, oligodendroglia, Schwann cells, and ependymal cells); demyelination or improper mylenation; neural cell dysfunction (such as, e.g., failure of neurotransmitter release or uptake); or interference with mylenization. Nervous system lesions that may be modulated, ameliorated, treated, prevented, and/or diagnosed in a subject using an LP composition of the invention, include, e.g., without limitation, the following lesions of either the central (including spinal cord and brain) or peripheral nervous system: (1) ischemic lesions, in which a lack of oxygen in a portion of the nervous system results in neuronal injury or death, including e.g., cerebral infarction (or ischemia), or spinal cord infarction (or ischemia); (2) traumatic lesions, including, e.g., lesions caused by physical injury or associated with surgery (e.g., lesions that sever a portion of the nervous system), or compression injuries; (3) malignant lesions, in which a portion of the nervous system is comprised by malignant tissue, which is either a nervous system associated malignancy or a malignancy derived from non-nervous-system tissue; (4) infectious lesions, in which a portion of the nervous system is comprised because of infection (e.g., by an abscess or associated with infection by human immunodeficiency virus, herpes zoster, or herpes simplex virus or with Lyme disease, syndrome, tuberculosis, syphilis); (5) degenerative lesions, in which a portion of the nervous system is comprised because of a degenerative process including, without limit, degeneration associated with Parkinson's disease syndrome, Alzheimer's disease syndrome, Huntington's chorea, or Amyotrophic lateral sclerosis (ALS); (6) lesions associated with a nutritional condition, in which a portion of the nervous system is comprised by a nutritional disorder (or a disorder of metabolism including, without limit, vitamin B 12 deficiency, folic acid deficiency, Wernicke disease, syndrome, tobacco-alcohol amblyopia, Marchiafava-Bignami disease (primary degeneration of the corpus callosum), and alcoholic cerebellar degeneration; (7) neurological lesions associated with systemic diseases including, e.g., without limitation, diabetes (diabetic neuropathy, Bell's palsy), systemic lupus erythematosus, carcinoma, or sarcoidosis; (8) lesions caused by toxic substances including e.g., alcohol, lead, or a neurotoxin; and (9) demyelinating lesions in which a portion of the nervous system is comprised by a demyelinating cause (including, e.g., without limitation, multiple sclerosis, human immunodeficiency virus-associated myelopathy, transverse myelopathy or various etiologies, progressive multifocal leukoencephalopathy, and central pontine myelinolysis).

In a preferred embodiment, an LP can be used to protect a neuronal cell from the damaging effects of cerebral hypoxia; cerebral ischemia, cerebral infarction; stroke; or a neural cell injury associated with a heart attack. An LP, which is useful for producing a desired effect in a nervous system condition, may be selected by testing for biological activity in promoting survival and/or differentiation of neural cell. For example, an LP that elicits any of the following effects may be useful according to the invention: (1) increased survival time of neurons in culture; (2) increased or decreased sprouting of a neural in culture *or in vivo;* (3) increased or decreased production of a neuron-associated molecule e.g., such as a neurotransmitter in culture *or in vivo,* e.g., choline acetyltransferase or acetylcholinesterase with respect to a motor neuron; or (4) decreasing a symptom of neuronal *d*ysfunction *in vivo* or in a model system, e.g., such as a mouse model for Parkinsons Syndrome. Such an effect may be measured by any known art method.

In a preferred, non-limiting embodiment any art known method can be used to: measure increased neuronal survival (such as, e.g., described in Arakawa, et al. (1990) J. Neurosci. 10:3507-3515); detect increased or decreased sprouting (such as, e.g., described in Pestronk, et al. (1980) Exp. Neurol. 70:65-82; Brown, et al. (1981) Ann. Rev. Neurosci. 4:17-42); measure increased production of a neuron-associated molecule (e.g., by bioassay, enzymatic assay, antibody binding, Northern blot assay, etc., depending on the molecule to be measured); and measure motor neuron dysfunction (by, e.g., assessing the physical manifestation of motor neuron disorder, e.g., weakness, motor neuron conduction velocity, or functional disability in a model system). In specific embodiments, motor neuron diseases, disorders, syndromes, and/or conditions that may be modulated, ameliorated, treated, prevented, and/or diagnosed using an LP composition include, e.g., without limitation, infarction, infection, exposure to toxin, trauma, surgical damage, degenerative disease or malignancy (that may affect motor neurons as well as other components of the nervous system), as well as conditions that selectively affect neurons such as, e.g., without limitation, Amyotrophic lateral sclerosis progressive spinal muscular atrophy, progressive bulbar palsy, primary lateral sclerosis, infantile and juvenile muscular atrophy, progressive bulbar paralysis of childhood (Fazio-Londe syndrome), poliomyelitis post polio syndrome, and Hereditary Motorsensory Neuropathy (Charcot-Marie-Tooth Disease).

### Infectious Disease

An LP composition can be used to modulate, ameliorate, treat, prevent, and/or diagnose an effect of an infectious agent in a subject or associated with a condition. For example, by increasing an immune response e.g., particularly increasing the proliferation and differentiation a of B and/or a T cell, infectious diseases may be modulated, ameliorated, treated, prevented, and/or diagnosed. The immune response may be increased either by enhancing an existing immune response, or by initiating a new immune response. Alternatively, an LP may also directly inhibit an infectious agent, without necessarily eliciting an immune response. Viruses are a type of an infectious agent that can cause diseases, disorders, syndromes, and/or conditions that may be modulated, ameliorated, treated, prevented, and/or diagnosed using an LP composition of the invention. Examples of such viruses, include, e.g., without limitation, the following DNA and RNA viruses and viral families: *Arbovirus, Adenoviridae, Arenaviridae, Arterivirus, Birnaviridae, Bunyaviridae, Caliciviridae, Circoviridae, Coronaviridae, Dengue, EBV, HIV, Flaviviridae, Hepadnaviridae (Hepatitis), Herpesviridae* (such as, e.g., *Cytomegalovirus, Herpes Simplex, Herpes Zoster), Mononegavirus* (e.g., *Paramyxoviridae, Morbillivirus, Rhabdoviridae), Orthomyxoviridae* (e.g., *Influenza A, Influenza B,* and *parairifluenza), Papilomavirus, Papovaviridae, Parvoviridae, Picornaviridae, Poxviridae* (such as, e.g., *Smallpox* or *Vaccinia), Reoviridae* (e.g., *Rotavirus), Retroviridae* (such as, e.g., *HTLV-I HTLV-II, Lentivirus),*and *Togaviridae* (e.g., R*ubivirus).* Typically, viruses of these families can cause a variety of undesired conditions, including, but not limited to: e.g., arthritis, bronchiollitis, respiratory syncytial virus, encephalitis, eye infections (e.g., conjunctivitis, keratitis), chronic fatigue syndrome, hepatitis (e.g., of type A, B, C, E, Chronic Active, or Delta), Japanese Bencephalitis, Junin, Chikungunya, Rift Valley fever, yellow fever, meningitis, opportunistic infections (e.g., AIDS), pneumonia, Burkitt's Lymphoma, chickenpox, hemorrhagic fever, Measles, Mumps, Parainfluenza, Rabies, a common cold, Polio, leukemia, Rubella, sexually transmitted diseases, skin diseases (e.g., Kaposi's, warts), and viremia. An LP can be used to modulate, ameliorate, treat, prevent, and/or diagnose any of these symptoms or diseases.

In specific embodiments, an LP composition is used to modulate, ameliorate, treat, prevent, and/or diagnose e.g., meningitis, Dengue, EBV, and/or hepatitis (e.g., hepatitis B). In a further specific embodiment, an LP is administered to a subject that is non-responsive to one or more currently established commercially available, hepatitis vaccines. In a further specific embodiment an LP can be used to modulate, ameliorate, treat, prevent, and/or diagnose AIDS or an AIDS-related syndrome or condition. Similarly, bacterial or fungal agents that can cause a disease, disorder, condition, syndrome, or symptom and that can be ameliorated, treated, prevented, and/or diagnosed by an LP composition of the invention include, e.g., but without limitation, the following: Gram-Negative and Gram-positive bacteria and bacterial families and fungi such as: *Actinomycetales* (e.g., *Corynebacterium, Mycobacterium, Norcardia), Cryptococcus neoformans, Aspergillosis, Bacillaceae* (e.g., *Anthrax, Clostridium), Bacteroidaceae, Blastomycosis, Bordetella, Borrelia* (e.g., *Borrelia burgdorferi), Brucellosis, Candidiasis, Campylobacter, Coccidioidomycosis, Cryptococcosis, Dermatocycoses, E. coli* (e.g., *EnterotoxigenicE. coli* and *Enterohemorrhagic E. coli), Enterobacteriaceae (Klebsiella, Salmonella* (e.g., *Salmonella typhi,* and *Salmonella paratyphi), Serratia, Yersinia), Erysipelothrix, Helicobacter, Legionellosis, Leptospirosis, Listeria, Mycoplasmatales, Mycobacterium leprae, Vibrio cholerae, Neisseriaceae* (e.g., *Acinetobacter,Gonorrhea, Menigococcal), Meisseria meningitidis, Pasteurellacea Infections* (e.g., *Actinobacillus, Heamophilus* (e.g., *Heamophilus influenza type B), Pasteurella), Pseudomonas, Rickettsiaceae, Chlamydiaceae, Syphilis, Shigella spp.,Staphylococcal, Meningiococcal, Pneumococcal* and *Streptococcal* (e.g., *Streptococcus pneumoniae* and *Group B Streptococcus).*

These bacterial or fungal families can cause the following diseases, disorders, conditions, syndromes, or symptoms including, e.g., without limitation, bacteremia, endocarditis, eye infections (conjunctivitis, tuberculosis, uveitis), gingivitis, opportunistic infections (e.g., AIDS related infections), paronychia, prosthesis-related infections, Reiter's Disease syndrome, respiratory tract infections, such as Whooping Cough or Empyema, sepsis, Lyme Disease syndrome, Cat-Scratch Disease syndrome, Dysentery, Paratyphoid Fever, food poisoning, Typhoid, pneumonia, Gonorrhea, meningitis (e.g., meningitis types A and B), Chlamydia, Syphilis, Diphtheria, Leprosy, Paratuberculosis, Tuberculosis, Lupus, Botulism, gangrene, tetanus, impetigo, Rheumatic Fever, Scarlet Fever, sexually transmitted diseases, skin diseases (e.g., cellulitis, dermatocycoses), toxemia, urinary tract infections and wound infections. An LP can be used to modulate, ameliorate, treat, prevent, and/or diagnose any of these diseases, disorders, conditions, syndromes, or symptoms.

In specific embodiments, an LP composition can be used to modulate, ameliorate, treat, prevent, and/or diagnose: tetanus, Diptheria, botulism, and/or meningitis type B. Moreover, parasitic agents causing diseases, disorders, conditions, syndromes, or symptoms that can be modulated, ameliorated, treated, prevented, and/or diagnosed by an LP include, e.g., without limitation, a parasitic agent from any of the following groupings: *Amebiasis, Babesiosis, Coccidiosis, Cryptosporidiosis, Dientamoebiasis, Dourine, Ectoparasitic, Giardiasis, Helminthiasis, Leishmaniasis, Theileriasis, Toxoplasmosis, Trypanosomiasis, Trichomona, Sporozoans* (e.g., *Plasmodium virax, Plasmodium falciparium, Plasmodium malariae,* and *Plasmodium ovale).* These parasites can cause a variety of diseases or symptoms, including, e.g., without limitation: Scabies, *Trombiculiasis,* eye infections, intestinal disease (e.g., dysentery, *giardiasis),* liver disease syndrome, lung disease syndrome, opportunistic infections (e.g., AIDS related conditions), malaria, complications of pregnancy, and toxoplasmosis. An LP composition of the invention can be used to modulate, ameliorate, treat, prevent, and/or diagnose any of these diseases, disorders, conditions, syndromes, or symptoms. In specific embodiments, an LP can be used to modulate, ameliorate, treat, prevent, and/or diagnose malaria.

Preferably, treatment or prevention using an LP is accomplished either by administering an effective amount of an LP composition to a subject, or by removing cells from a subject, delivering an LP then returning the resulting engineered cell to the patient (ex *vivo* therapy). Furthermore, an LP sequence can be used as an antigen in a vaccine to raise an immune response against an infectious disease.

### Regeneration

An LP composition of the invention can be used e.g., to differentiate a cell, tissue; or biological structure, de-differentiate a cell, tissue; or biological structure; cause proliferation in cell or a zone (similar to a ZPA in a limb bud), have an effect on chemotaxis, remodel a tissue (e.g., basement membrane, extra cell matrix, connective tissue, muscle, epithelia), or initiate the regeneration of a tissue, organ, or biological structure (see, e.g., Science (1997) 276:59-87). Regeneration using an LP composition of the invention could be used to repair, replace, remodel, or protect tissue damaged by, e.g., congenital defects, trauma (such as, e.g., wounds, burns, incisions, or ulcers); age; disease (such as, e.g., osteoporosis, osteoarthritis, periodontal disease syndrome, or liver failure), surgery, (including, e.g., cosmetic plastic surgery); fibrosis; re-perfusion injury; or cytokine damage. Tissues that can be regenerated include, e.g., without limitation, organs (e.g., pancreas, liver, intestine, kidney, epithelia, endothelium), muscle (smooth, skeletal, or cardiac), vasculature (including vascular and lymphatics), nervous system tissue, cells, or structures; hematopoietic tissue; and skeletal (bone, cartilage, tendon, and ligament) tissue. Preferably, regeneration occurs with little or no scarring. Regeneration also may include, e.g., angiogenesis.

Moreover, an LP composition may increase the regeneration of an aggregation of special cell types, a tissue, or a matrix that typically is difficult to heal. For example, by increasing the rate at which a tendon/ligament heals after damage. Also encompassed is using an LP prophylactically to avoid damage (e.g., in an interstitial space of a joint or on the cartalagenous capsule of a bone). Specific diseases that could be modulated, ameliorated, treated, prevented, and/or diagnosed using an LP composition include, e.g., without limitation, tendinitis, carpal tunnel syndrome, and other tendon or ligament defects. Examples of non-healing wounds include, wounds that would benefit form regeneration treatment, e.g., without limit pressure ulcers, ulcers associated with vascular insufficiency, surgical wounds, and traumatic wounds.

Similarly, nerve and brain tissue also could be regenerated using an LP. Such nervous system conditions that could be modulated, ameliorated, treated, prevented, and/or diagnosed using an LP composition include, e.g., without limitation, central and peripheral nervous system diseases, neuropathies, or mechanical and traumatic conditions (e.g., spinal cord disorders or syndromes, head trauma, cerebrovascular disease syndrome, and stoke). Specifically, diseases associated with peripheral nerve injuries include, e.g., without limitation, peripheral neuropathy (e.g., resulting from chemotherapy or other medical therapies), localized neuropathies, and central nervous system diseases (e.g., Alzheimer's disease syndrome, Parkinson's disease syndrome, Huntington's disease syndrome, Amyotrophic lateral sclerosis, and Shy-Drager syndrome). All could be ameliorated, treated, prevented, and/or diagnosed using an LP.

### Chemotaxis

An LP may have an effect on a chemotaxis activity. Briefly, chemotactic molecules can attract or mobilize (but may also repeal) cells (e.g., monocytes, fibroblasts, neutrophils, T-cells, mast cells, eosinophils, epithelial and/or endothelial cells) or cell processes (e.g., filopodia, psuedopodia, lamellapodia, dendrites, axons, etc.) to a particular site (e.g., such as inflammation, infection, site of hyperproliferation, the floor plate of the developing spinal cord, etc.). In some instances, such mobilized cells can then fight off and/or modulate a particular trauma, abnormality, condition, syndrome, or disease. An LP may have an effect on a chemotactic activity of a cell (such as, e.g., an attractive or repulsive effect).

A chemotactic molecule can be used to modulate, ameliorate, treat, prevent, and/or diagnose inflammation, infection, hyperproliferative diseases, disorders, syndromes, and/or conditions, or an immune system disorder by increasing the number of cells targeted to a particular location in the body. For example, a chemotactic molecule can be used to attract an immune cell to an injured location in a subject. An LP that had an effect on a chemotactant could also attract a fibroblast, which can be used to modulate, ameliorate, and/or treat a wound. It is also contemplated that an LP may inhibit a chemotactic activity to modulate, ameliorate, treat, prevent, and/or diagnose a disease, disorder, syndrome, and/or a condition.

### XI. Kits

This invention also contemplates use of LP proteins, fragments thereof, peptides, and their fusion products in a variety of diagnostic kits and methods for detecting the presence of LP protein or a binding partner. Typically, the kit will have a compartment containing either a defined LP protein peptide or gene segment or a reagent, which recognizes one or the other, e.g., binding partner fragments or antibodies.

A preferred kit for determining the concentration of, e.g., a LP protein in a sample would typically comprise a labeled compound, e.g., binding partner or antibody, having known binding affinity for the LP protein, a source of LP protein (naturally occurring or recombinant), and a means for separating the bound from free labeled compound, for example, a solid phase for immobilizing the LP protein. Compartments containing reagents, and instructions, will normally be provided. Another diagnostic aspect of this invention involves use of oligonucleotide or polynucleotide sequences taken from the sequence of a LP protein. These sequences are used as probes for detecting levels of the LP protein message in samples from natural sources, or patients suspected of having an abnormal condition, e.g., cancer or developmental problem. The preparation of both RNA and DNA nucleotide sequences, the labeling of the sequences, and the preferred size of the sequences has received ample description and discussion in the literature.

In specific embodiments, a kit may include, e.g., a recombinantly produced or chemically synthesized polypeptide antigen. The polypeptide antigen of the kit may also be attached to a solid support. In a more specific embodiment the detecting means of the above-described kit includes, e.g., a solid support to which said polypeptide antigen is attached. Such a kit may also include, e.g., a non-attached reporter-labeled anti-human antibody. In this embodiment, binding of the antibody to the polypeptide antigen is detected by binding of the reporter-labeled antibody.

### Other Preferred Embodiments

Other preferred embodiments of the claimed invention include an isolated or recombinant nucleic acid molecule comprising a polynucleotide sequence that is at least 95% identical to a polynucleotide sequence of at least about: 10,12,14,16,18, 20, 22, 24, 26, 28, 30,32,34,36,38,40,42,44,46,48,50,52,54,56,58,60,62,64,66,68,70,72,74,76,78, 80, 82, 84, 86, 88, 90, 92, 94, 96, 98,100,110,120,130,140, or 150 contiguous nucleotides of a sequence of SEQ ID NO:X wherein X is any integer as defined in a Table herein. Other preferred embodiments of the claimed invention include an isolated or recombinant nucleic acid molecule comprising a polynucleotide sequence that is at least 95% identical to a polynucleotide sequence of at least about: 10, 12, 14, 16, 18, 20, 22, 24, 26, 28, 30, 32, 34, 36, 38,40,42,44,46,48,50,52,54,56,58,60,62,64,66,68,70,72,74,76,78,80,82,84,86, 88, 90, 92, 94, 96, 98,100,110,120,130,140, or 150 contiguous nucleotides of a mature coding portion of SEQ ID NO:X wherein X is any integer as defined in a Table herein. Also preferred is a nucleic acid molecule wherein said sequence of contiguous nucleotides is include, e.g. in the nucleotide sequence of SEQ ID NO:X in the range of positions beginning with the nucleotide at about the position of the 5' nucleotide of the Clone Sequence and ending with the nucleotide at about the position of the 3' nucleotide of the Clone Sequence as defined for SEQ ID NO:X in a Table herein. Also preferred is a nucleic acid molecule wherein said sequence of contiguous nucleotides is included, e.g., in the nucleotide sequence of SEQ ID NO:X in the range of positions beginning with the nucleotide at about the position of the 5' nucleotide of the Start Codon and ending with the nucleotide at about the position of the 3' nucleotide of the Clone Sequence as defined for SEQ ID NO:X in a Table herein. Similarly preferred is a nucleic acid molecule comprising polynucleotide sequence of SEQ ID NO:X in the range of positions beginning with the nucleotide at about the position of the 5' nucleotide of a correspondingly encoded First Amino Acid of a Signal Peptide and ending with the nucleotide at about the position of the 3' nucleotide of a Clone Sequence as defined for SEQ ID NO:X in a Table herein. Also preferred is an isolated or recombinant nucleic acid molecule comprising a polynucleotide sequence that is at least 95% identical to a polynucleotide sequence of at least about: 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 110, 120, 130, 140, or 150 contiguous nucleotides in at least one polynucleotide sequence fragment of SEQ ID NO:X More preferably said polynucleotide sequence that is at least 95% identical to one, exhibits 95% sequence identity to at least: 2, 3, 4, 5, 6, 7, 8, 9, 10, or more polynucleotide fragments 22, 24, 2G, 28, 30, 32, 34, 36, 38, 40, 42, 44, 4G, 48, 50, 52, 54, 56, 58, 60, 62, 64, 66, 68, 70, 72, 74, 76, 78, 80, 82, 84, 86, 88, 90, 92, 94, 96, 98, 100, 110, 120, 130, 140, or 150 contiguous nucleotides in length of the mature coding portion of SEQ ID NO:X., wherein any one such fragment is at least 21 contiguous nucleotides in length. Further preferred is an isolated or recombinant nucleic acid molecule comprising a polynucleotide sequence that is at least 95% identical to a polynucleotide sequence of at least about: 200, 250, 300, 350, 400, 450, or 500 contiguous nucleotides of the mature coding portion of SEQ ID NO:X. Also preferred is an isolated or recombinant nucleic acid molecule comprising a polynucleotide sequence that is at least 95% identical to a sequence of at least about: 10,12,14,16,18, 20, 22, 24,26,28,30,32,34,36, 38,40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 110, 120, 130, 140, or 150 contiguous nucleotides in at least one nucleotide sequence fragment of SEQ ID NO:X, wherein the length of at least one such fragment is about 200, 250, 300, 350, 400, 450, or 500 contiguous nucleotides of SEQ ID NO:X. Another preferred embodiment is an isolated or recombinant nucleic acid molecule comprising a polynucleotide sequence that is at least 95% identical to a sequence of SEQ ID NO:X beginning with the nucleotide at about the position of the 5' Nucleotide of the First Amino Acid of the Signal Peptide and ending with the nucleotide at about the position of the 3' Nucleotide of a Clone Sequence as defined for SEQ ID NO:X in a Table herein. A further preferred embodiment is an isolated or recombinant nucleic acid molecule comprising a polynucleotide sequence, which is at least 95% identical to the complete mature coding portion of SEQ ID NO:X or a species variant thereof. Also preferred is an isolated or recombinant nucleic acid molecule comprising polynucleotide sequence that hybridizes under stringent hybridization conditions to a mature coding portion of a polynucleotide of the invention (or fragment thereof), wherein the nucleic acid molecule that hybridizes does not hybridize under stringent hybridization conditions to a nucleic acid molecule having a nucleotide sequence consisting of only A residues or of only T residues. Thus, the invention provides an assay system or kit for carrying out a diagnostic method. The kit generally includes, e.g., a support with surface-bound recombinant antigens, and a reporter-labeled anti-human antibody for detecting surface-bound anti-antigen antibody.

The broad scope of this invention is best understood with reference to the following examples, which are not intended to limit the invention to specific embodiments.

### EXAMPLES

### General Methods

Many of the standard methods described herein are described or referenced, e.g., in Maniatis, et al. (Cur. ed..) Molecular Cloning, A Laboratory Manual Cold Spring Harbor Laboratory, Cold Spring Harbor Press, NY; Sambrook, et al.; Ausubel, et al., Biology Greene Publishing Associates, Brooklyn, NY; or Ausubel, et al. (1987 and Supplements) Current Protocols in Molecular Biology Wiley/Greene, NY; Innis, et al. (eds.) (1990) PCR Protocols: A Guide to Methods and Applications Academic Press, NY. Methods for protein purification include such methods as ammonium sulfate precipitation, column chromatography, electrophoresis, centrifugation, crystallization, and others. See, e.g., Ausubel, et al. (1987 and periodic supplements); Deutscher (1990) "Guide to Protein Purification," Methods in Enzymology vol. 182, and other volumes in this series; Coligan, et al. (1995 and supplements) Current Protocols in Protein Science John Wiley and Sons, New York, NY; P. Matsudaira (ed.) (1993) A Practical Guide to Protein and Peptide Purification for Microsequencing, Academic Press, San Diego, CA; and manufacturer's literature on use of protein purification products, e.g., Pharmacia, Piscataway, NJ, or Bio-Rad, Richmond, CA. Combination with recombinant techniques allow fusion to appropriate segments (epitope tags), e.g., to a FLAG sequence or an equivalent which can be fused, e.g., via a protease-removable sequence. See, e.g., Hochuli (1989) Chemische Industrie 12:69-70; Hochuli (1990) "Purification of Recombinant Proteins with Metal Chelate Absorbent" in Setlow (ed.) Genetic Engineering, Principle and Methods 12:87-98, Plenum Press, NY; and Crowe, et al. (1992) QIAexpress: The High Level Expression and Protein Purification System QUIAGEN, Inc., Chatsworth, CA. Standard immunological techniques are described, e.g., in Hertzenberg, et al. (eds. 1996) Weir's Hanbook of Experimental Immunology vols. 1-4, Blackwell Science; Coligan (1991) Current Protocols in Immunology Wiley/Greene, NY; and Methods in Enzymology volumes. 70, 73, 74, 84, 92, 93, 108, 116, 121, 132, 150, 162, and 163. Assays for neural cell biological activities are described, e.g., in Wouterlood (ed. 1995) Neuroscience Protocols modules 10, Elsevier; Methods in Neurosciences Academic Press; and Neuromethods Humana Press, Totowa, NJ. Methodology of developmental systems is described, e.g., in Meisami (ed.) Handbook of Human Growth and Developmental Biology CRC Press; and Chrispeels (ed.) Molecular Techniques and Approaches in Developmental Biology Interscience. FACS analyses are described in Melamed, et al. (1990) Flow Cytometry and Sorting Wiley-Liss, Inc., New York, NY; Shapiro (1988) Practical Flow Cytometry Liss, New York, NY; and Robinson, et al. (1993) Handbook of Flow Cytometry Methods Wiley-Liss, New York, NY.

### Example 1: Isolation of LP clones

Standard methods are used to isolate full length genes from a cDNA library made from an appropriate source, e.g., human cells. The appropriate sequence is selected, and hybridization at high stringency conditions is performed to find a full length corresponding gene using standard techniques. The full length, or appropriate fragments, of human genes are used to isolate a corresponding monkey or other primate gene. Preferably, a full length coding sequence is used for hybridization. Similar source materials as indicated above are used to isolate natural genes, including genetic, polymorphic, allelic, or strain variants. Other species variants are also isolated using similar methods. With a positive clone, the coding sequence is inserted into an appropriate expression vector. This may be in a vector specifically selected for a prokaryote, yeast, insect, or higher vertebrate, e.g., mammalian expression system. Standard methods are applied to produce the gene product, preferably as a soluble secreted molecule, but will, in certain instances, also be made as an intracellular protein. Intracellular proteins typically require cell lysis to recover the protein, and insoluble inclusion bodies are a common starting material for further purification. With a clone encoding a vertebrate LP protein, recombinant production means are used, although natural forms may be purified from appropriate sources. The protein product is purified by standard methods of protein purification, in certain cases, e.g., coupled with immunoaffinity methods. Immunoaffinity methods are used either as a purification step, as described above, or as a detection assay to determine the separation properties of the protein.

Preferably, the protein is secreted into the medium, and the soluble product is purified from the medium in a soluble form. Alternatively, as described above, inclusion bodies from prokaryotic expression systems are a useful source of material. Typically, the insoluble protein is solubilized from the inclusion bodies and refolded using standard methods. Purification methods are developed as described herein. The product of the purification method described above is characterized to determine many structural features. Standard physical methods are applied, e.g., amino acid analysis and protein sequencing. The resulting protein is subjected to CD spectroscopy and other spectroscopic methods, e.g., NMR, ESR, mass spectroscopy, etc. The product is characterized to determine its molecular form and size, e.g., using gel chromatography and similar techniques. Understanding of the chromatographic properties will lead to more gentle or efficient purification methods. Prediction of glycosylation sites may be made, e.g., as reported in Hansen, et al. (1995) Biochem. J. 308:801-813. The purified protein is also be used to identify other binding partners of an LP of the invention as described, e.g., in Fields and Song (1989) Nature 340:245-246.

### Example 2: Tissue Distribution of an LP Polynucleotide

Tissue distribution of mRNA expression of a polynucleotide of the present invention (or fragment thereof) is determined using protocols for Northern blot analysis, described (among others) by, e.g., Sambrook, et al. For example, a cDNA probe produced using common techniques is labeled with P³² using the Rediprime™ DNA labeling system (Amersham Life Science), according to manufacturer's instructions. After labeling, the probe is purified using CHROMA SPIN-100™ column (Clontech Laboratories, Inc.), according to manufacturer's protocol number PT1200-1. The purified, labeled probe is then used to examine various human tissues for mRNA expression. Multiple Tissue Northern (MTN) blots containing various human tissues (H) or human immune system tissues (IM) (Clontech) are examined with the labeled probe using Express Hyb™ hybridization solution (Clontech) according to manufacturer's protocol number PTll90-1. After hybridization and washing, blots are mounted and exposed to film (overnight at -70 °C), and the films are subsequently developed according to standard procedures.

### Example 3: Chromosomal Mapping of an LP Polynucleotide

An oligonucleotide primer set is designed according to the sequence at the 5' end of a SEQ ID NO:X identified sequence. This primer preferably spans about 100 nucleotides. This primer set is then used in a polymerase chain reaction under the following set of conditions: 30 seconds, 95 °C; 1 minute, 56 °C; 1 minute, 70 °C. This cycle is repeated 32 times followed by one 5-minute cycle at 70 °C. Human, mouse, and hamster DNA is used as template in addition to a somatic cell hybrid panel containing individual chromosomes or chromosome fragments (Bios, Inc). The reaction is analyzed on either 8% polyacrylamide gels or 3.5 % agarose gels. Chromosome mapping is determined by the presence of an approximately 100bp PCR fragment in a particular somatic cell hybrid.

### Example 4: Production of a Secreted LP Protein for a High-Throughput Screening Assay

The following protocol produces a supernatant containing an LP polypeptide (or fragment thereof) to be tested. This supernatant can then be used in a variety of screening assays (such as, e.g., those taught herein). First, dilute Poly-D-Lysine (644 587 Boehringer-Mannheim) stock solution (1 mg/ml in PBS) 1:20 in PBS (w/o calcium or magnesium 17-5 16F Biowhittaker) to obtain a working stock solution of 50 ug/ml. Add 200 ul of this solution to each well (24-well plates) and incubate (RT for 20 min). Distribute the solution over each well (a 12-channel pipetter may be used with tips on every other channel). Aspirate off the Poly-D-Lysine solution and rinse with 1 ml PBS (Phosphate Buffered Saline). The PBS should remain in the well until just before plating the cells and plates may be coated (up to two weeks in advance) with poly-lysine. Plate 2933: cells (do not carry cells past P+20) at 2 × 10⁵ cells/well in 0.5 ml DMEM (Dulbecco's Modified Eagle Medium) (with 4.5 G/L glucose and L-glutamine5 (12-604F Biowhittaker))/10% heat inactivated FBS (14-503F Biowhittaker)/lx Pinstripe (17-602E Biowhittaker). Let the cells grow overnight.

The next day, mix in a sterile solution basin: 300 ul Lipofectamine (18324-012 Gibco; BRL) and 5ml Optimem I (31985070 Gibco; BRL) per 96-well plate. With a small volume multi-channel pipetter, aliquot approximately 2 ug of an expression vector containing an LP polynucleotide insert of the invention, produced by any art known methods or as taught herein, into an appropriately labeled 96-well round-bottom plate. With a multi-channel pipetter, add 50 µl of the Lipofectamine/Optimem I mixture to each well. Pipette up and down gently to mix. Incubate at RT for 15-45 minutes. After about 20 minutes, use a multichannel pipetter to add 150µl of Optimem I to each well. As a control, transfect one plate of vector DNA lacking an insert with each set of transfections.

Preferably, transfections should be performed by splitting the following tasks between two individuals to reduce the time, and to insure that the cells do not spend too much time in PBS. First, person A aspirates off the media from four 24-well plates of cells, and then person B rinses each well with 0.5-1 ml of PBS. Person A then aspirates off the PBS rinse, and person B (using a 12-channel pipetter with tips on every other channel) adds 200µl of DNA/Lipofectamine/Optimem I complex first to the odd wells, then to the even wells (of each row on the 24-well plates). Incubate at 37 °C for 6 hours. While cells are incubating, prepare appropriate media, either 1 % BSA in DMEM with Ix penstrep, or CHO-5 media (116.6 mg/L of CaCl₂ (anhyd); 0.00130mg/L CuSO₄5H₂O; 0.050 mg/L of Fe(NO₃)₃-9H₂0; 0.417 mg/L of FeSO₄-7H₂O; 311.80 mg/L of KC1; 28.64 mg/L of MgCl₂; 48.84 mg/L of MgSO₄ 6995.50 mg/L of NaCl; 2400.0 mg/L of NaHCO₃; 62.50 mg/L of NaH₂PO₄-H₂O; 71.02 mg/L of Na₂HPO₄; 0.4320 mg/L of ZnSO₄-7H₂O; 0.002 mg/L of Arachidonic Acid; 1.022 mg/L of Cholesterol; 0.070 mg/L of DL-alpha-Tocopherol-Acetate; 0.0520 mg/L of Linoleic Acid; 0.010 mg/L of Linolenic Acid; 0.010 mg/L of Myristic Acid; 0.010 mg/L of Oleic Acid; 0.010 mg/L of Palmitric Acid; 0.010 mg/L of Palmitic Acid; 100 mg/L of Pluronic F-68; 0.010 mg/L of Stearic Acid; 2.20 mg/L of Tween 80; 4551 mg/L of D-Glucose; 130.85 mg/ml of L-Alanine; 147.50 mg/ml of L-Arginine-HCL; 7.50 mg/ml of L-Asparagine-H₂O; 6.65 mg/ml ofL-Aspartic Acid; 29.56 mg/ml of L-Cystine-2HCL-H₂O; 31.29 mg/ml of L-Cystine-2HCL; 7.35 mg/ml of L-Glutamic Acid; 365.0 mg/ml of L-Glutamine; 18.75 mg/ml of Glycine; 52.48 mg/ml of L-Histidine-HCL-H₂O; 106.97 mg/ml of L-Isoleucine; 111.45 mg/ml of L-Leucine; 163.75 mg/ml of L-Lysine HCL; 32.34 mg/ml of L-Methionine; 68.48 mg/ml of L-Phenylalanine; 40.0 mg/ml of L-Proline; 26.25 mg/ml of L-Serine; 101.05 mg/ml of L-Threonine; 19.22 mg/ml of L-Tryptophan; 91.79 mg/ml of L-Tryrosine-2Na-2H₂O; 99.65 mg/ml of L-Valine; 0.0035 mg/L of Biotin; 3.24 mg/L of D-Ca Pantothenate; 11.78 mg/L of Choline Chloride; 4.65 mg/L of Folic Acid; 15.60 mg/L of i-Inositol; 3.02 mg/L of Niacinamide; 3.0 mg/L of Pyridoxal HCL; 0.031 mg/L of Pyridoxine HCL; 0.319 mg/L of Riboflavin; 3.17 mg/L of Thiamine HCL; 0.365 mg/L of Thymidine; and 0.680 mg/L of Vitamin B₁₂; 25 mM of HEPES Buffer; 2.39 mg/L of Na Hypoxanthine; 0.105 mg/L of Lipoic Acid; 0.081 mg/L of Sodium Putrescine-2HCL; 55.0 mg/L of Sodium Pyruvate; 0.0067 mg/L of Sodium Selenite; 20uM of Ethanolamine; 0.122 mg/L of Ferric Citrate; 41.70 mg/L of Methyl-B-Cyclodextrin complexed with Linoleic Acid; 33.33 mg/L of Methyl-B-Cyclodextrin complexed with Oleic Acid; and 10 mg/L of Methyl-B-Cyclodextrin complexed with Retinal) with 2mm glutamine, and 1X penstrep (BSA (81-068-3 Bayer) 100gm dissolved in 1L DMEM for a 10% BSA stock solution). Filter the media and collect 50 ul for endotoxin assay in 15m1 polystyrene conical.

The transfection reaction is terminated, preferably by splitting tasks (as above) at the end of the incubation period. Person A aspirates off the transfection media, while person B adds 1.5 ml appropriate media to each well. Incubate at 37 °C for 45 or 72 hours depending on the media used (1 %BSA for 45 hours or CHO-5 for 72 hours). On day four, using a 300 ul multichannel pipetter, aliquot 600µl in one 1 ml deep well plate and the remaining supernatant into a 2 ml deep well. The supernatants from each well can then be used in an assay taught herein. It is specifically understood that when activity is obtained in an assay described herein using a supernatant, the activity originates either from the polypeptide (or fragment thereof) directly (such as, e.g., from a secreted protein or fragment thereof) or by the polypeptide (or fragment thereof) inducing expression of another protein(s), which is/are then released into the supernatant. Thus, the invention provides a method of identifying a polypeptide (or fragment thereof) in a supernatant characterized by an activity in a particular assay taught herein.

### Example 5: Construction of a GAS Reporter Construct

One signal transduction pathway involved in cellular differentiation and proliferation is a Jaks-STATS pathway. Activated proteins in a Jaks-STATS pathway have been shown to bind to gamma activation site "GAS" elements or interferon-sensitive responsive element ("ISRE"), which are located, e.g., in the promoter region of many genes. Typically, binding, e.g., by a protein, to such an element alters expression of an associated gene. GAS and ISRE elements are recognized by a class of transcription factors called Signal Transducers and Activators of Transcription, or "STATS." The Stat1 and Stat3 members of the STATS family are present in many cell types, (as is Stat2) probably, because the response to IFN-alpha is widespread. Stat4, however, is more restricted to particular cell types though, it has been found in T helper class I cells after their treatment with IL-12. Stat 5 (originally designated mammary growth factor) has been found at higher concentrations in cells besides breast cells, e.g., myeloid cells. Stat 5 is activated in tissue culture cells by many cytokines.

After tyrosine phosphorylation (by kinases known as the Janus Kinase Family or "Jaks"), members of the STATS family typically translocate from the cytoplasm to the nucleus of the cell. Jaks represent a distinct family of soluble tyrosine kinases and include, e.g., Tyk2, Jak1, Jak2, and Jak3. These Jak kinases display significant sequence similarity to each other and, generally, are catalytically inactive in resting cells. However, Jaks are catalytically activated by a wide range of receptors (summarized in the Table below, adapted from Schidler and Darnell (1995) Ann. Rev. Biochem. 64:621-51). One cytokine receptor family, which is capable of activating a Jak, is divided into two groups (Class 1 and 2). Class 1 includes, e.g., receptors for IL-2, IL-3, IL-4, IL-6, IL-7, IL-9, IL-11, IL-12, IL-15, Epo, PRL, GH, G-CSF, GM-CSF, LIF, CNTF, and thrombopoietin; while Class 2 includes, e.g., IFN-a, IFN-g, and IL-10. The Class 1 receptors share a conserved cysteine motif (a set of four conserved cysteines and one tryptophan) and a WSXWS motif (a membrane proximal region encoding Trp-Ser-Xxx-Trp-Ser). Thus, after a ligand binds a receptor, Jaks are typically activated and, in turn, subsequently activate STATS, which translocate and bind to GAS transcriptional elements (located in the nucleus of the cell). This entire process of sequential activation is encompassed in a typical Jaks-STATS signal transduction pathway. Therefore, activation of a Jaks-STATS pathway (reflected by binding of a GAS or 1 SRE element) is used to indicate that an LP polypeptide (or fragment thereof) is involved in the proliferation and/or differentiation of a cell. For instance, growth factors and cytokines are examples of proteins that are known to activate a Jaks-STATS pathway. Consequently, by using a GAS element linked to a reporter molecule, an activator of a Jaks-STATS pathway is identified.

| | | ***JAKS*** | | | ***STATS*** | **GAS(elements) or ISRE** |
|---|---|---|---|---|---|---|
| Ligand | tyk2 | Jak1 | Jak2 | Jak3 | | |
| ***IFN family*** | | | | | | |
| IFN-a/B | + | + | - | - | 1,2,3 | ISRE |
| IFN-G | | + | + | - | 1 | GAS (IRF1>Lys6>IFP) |
| IL-10 | + | ? | ? | - | 1,3 | |

| **gp130 family** | | | | | | |
|---|---|---|---|---|---|---|
| IL-6 (Pleiotrophic) | + | + | + | ? | 1,3 | GAS (IRF1>Lys6>IFP) |
| IL-11 (Pleiotrophic) | ? | + | ? | ? | 1,3 | |
| OnM (Pleiotrophic) | ? | + | + | ? | 1,3 | |
| LIF (Pleiotrophic) | ? | + | + | ? | 1,3 | |
| CNTF (Pleiotrophic) | -/+ | + | + | ? | 1,3 | |
| G-CSF (Pleiotrophic) | ? | + | ? | ? | 1,3 | |
| IL-12 (Pleiotrophic) | + | - | + | + | 1,3 | |

| **g-C family** | | | | | | |
|---|---|---|---|---|---|---|
| IL-2 (lymphocytes) | - | + | - | + | 1,3,5 | GAS |
| IL-4 (lymph/myeloid) | - | + | - | + | 6 | GAS (IRF1=IFP>>Ly6)(IgH) |
| IL-7 (lymphocytes) | - | + | - | + | 5 | GAS |
| IL-9 (lymphocytes) | - | + | - | + | 5 | GAS |
| IL-13 (lymphocyte) | - | + | ? | ? | 6 | GAS |
| IL-15 | ? | + | ? | + | 5 | GAS |

| **gp140 family** | | | | | | |
|---|---|---|---|---|---|---|
| IL-3 (myeloid) | - | - | + | - | 5 | GAS (IRF1>IFP>>Ly6) |
| IL-5 (myeloid) | - | - | + | - | 5 | GAS |
| GM-CSF (myeloid) | - | - | + | - | 5 | GAS |

| ***Growth hormone family*** | | | | | | |
|---|---|---|---|---|---|---|
| GH | ? | - | + | - | 5 | |
| PRL | ? | +/- | + | - | 1,3,5 | |
| EPO | ? | - | + | - | 5 | GAS (B-CAS>IRF1=IFP»Ly6) |

| ***Receptor Tyrosine Kinases*** | | | | | | |
|---|---|---|---|---|---|---|
| EGF | ? | + | + | - | 1,3 | GAS (IRF1) |
| PDGF | ? | + | + | - | 1,3 | |
| CSF-1 | ? | + | + | - | 1,3 | GAS (not IRF1) |

To construct a synthetic GAS containing promoter element, like that described in an assays taught herein, a PCR based strategy is employed to generate a GAS-SV40 promoter sequence. The 5' primer contains four tandem copies of the GAS binding site found in the IRF1 promoter, which has previously been shown to bind STATS after induction by a range of cytokines (see, e.g., Rothman, et al. (1994) Immunity 1:457-468). Although, however, it is possible to use other GAS or ISRE elements. The 5' primer also contains 18bp of sequence complementary to the SV40 early promoter sequence and is flanked with an XhoI site. The sequence of the 5' primer is:

5′:GCGCCTCGAGATTTCCCCGAAATCTAGATTTCCCCGAAATGATTTCCCCGAAATGATTTCCCCGAAATA
TCTGCCATCTCAATTAG:3′ (SEQ ID NO:39)

The downstream primer, which is complementary to the SV40 promoter and is flanked with a Hind III site, is: 5':GCGGCAAGCTTTTTGCAAAGCCTAGGC:3' (SEQ ID NO:40). PCR amplification is performed using the SV40 promoter template present in a B-gal:promoter plasmid (Clontech). The resulting PCR fragment is digested with XhoI/Hind III and subcloned into BLSK2- (Stratagene). Sequencing with forward and reverse primers confirms that the insert contains the following sequence:

5':CTCGAGATTTCCCCGAAATCTAGATTTCCCCGAAATGATTCCCCGAAATGATTTCCCCGAAATATCTGC
CATCTCAATTAGTCAGCAACCATAGTCCCGCCCCTAACTCCGCCCATCCCGCCCCTAACTCCGCCCAGTTCC
GCCCATTCTCCGCCCCATGGCTGACTAATTTTTTTTATTTATGCAGAGGCCGAGGCCGCCTCGGCCTCTGAG
CTATTCCAGAAGTAGTGAGGAGGCTTTTTTGGAGGCCTAGGCTTTTGCAAAAAGCTT:3' (SEQ ID
NO:41)

With this GAS promoter element linked to the SV40 promoter, a GAS:SEAP2 reporter construct is next engineered. Here, the reporter molecule is a secreted alkaline phosphatase (SEAP). Clearly, in this or in any of the other assays described herein, any applicable reporter molecule is used instead of SEAP without undue experimentation. For example, using art known methods, such as, e.g., without limitation, chloramphenicol acetyltransferase (CAT), luciferase, alkaline phosphatase, B-galactosidase, green fluorescent protein (GFP), or any protein (detectable by an antibody or detectable binding partner) could be substituted for SEAP. Once the above sequence is confirmed, the synthetic GAS-SV40 promoter element is subcloned into a pSEAP-Promoter vector (Clontech) using HindIII and XhoI. This, effectively, replaces the SV40 promoter with the amplified GAS:SV40 promoter element to create a GAS-SEAP vector. However, since the resulting GAS-SEAP vector does not contain a neomycin resistance gene it is not a preferred embodiment for use in mammalian expression systems. To generate stable mammalian cell lines that express a GAS-SEAP reporter, the GAS-SEAP cassette is removed (using Sal1 and NotI) from the GAS-SEAP vector and inserted into a backbone vector containing a neomycin resistance gene, such as, e.g., pGFP-1 (Clontech), using these restriction sites in the multiple cloning site, to create a GAS-SEAP/Neo vector. Once the GAS-SEAP/Neo vector is transfected into a mammalian cell, it can also be used as a reporter molecule for GAS binding as taught in an assay as described herein.

Similar constructs is made using the above description and replacing GAS with a different promoter sequence. For example, construction of reporter-molecules containing NFK-B and EGR promoter sequences are applicable. Additionally, however, many other promoters is substituted using a protocols described herein, e.g., SRE, IL-2, NFAT, or Osteocalcin promoters is substituted, alone or in combination with another (e.g., GAS/NF-KB/EGR, GAS/NF-KB, I1-2/NFAT, or NF-KB/GAS). Similarly, other cell lines is used to test reporter construct activity, such as, e.g., without limitation, HELA (epithelial), HUVEC (endothelial), Reh (B-cell), Saos-2 (osteoblast), HUVAC (aortic), or Cardiomyocyte cell lines. Alternatively, testing whether an LP polypeptide (or fragment thereof) is involved in a JAK/STATs signal transduction pathway can be performed (without undue experimentation) by adopting a method as described, e.g., in Ho, et al. (1995) Mol. Cell. Biol. 15:5043-5-53. Furthermore, it may be possible to test the JAK/STATs signal transduction pathway for blockage using an LP composition of the invention. Additionally, standard methods exist for testing whether an LP polypeptide (or fragment thereof) of the invention is involved in a STAT signaling pathway (e.g., such methods are described, e.g., in Starr, et al. (1997) Nature 387:917-921; Endo, et al. (1997) Nature 387:921-924; and Naka, et al. Nature 387:924-929 and can be employed here without undue experimentation).

### Example 6: High-Throughput Screening Assay for T-cell Activity.

The following protocol is used to assess T-cell activity by identifying factors and/or determining whether a supernate (described herein) containing an LP polypeptide (or fragment thereof) modulates the proliferation and/or differentiation of a T-cell. T-cell activity is assessed using a GAS/SEAP/Neo construct. Thus, a factor that increases SEAP activity indicates an ability to activate a Jaks-STATS signal transduction pathway. One type of T-cell used in this assay is, e.g., a Jurkat T-cell (ATCC Accession No. TIB-152), although other cells can also be used such as, e.g., without limitation, Molt-3 cells (ATCC Accession No. CRL-1552) or Molt-4 cells (ATCC Accession No. CRL-1582).

Jurkat T-cells are lymphoblastic CD4+ Th1 helper cells. To generate stable cell lines, approximately 2 million Jurkat cells are transfected with a GAS-SEAP/Neo vector using DMRIE-C (Life Technologies) in a transfection procedure as described below. Transfected cells are seeded to a density of approximately 20,000 cells per well and any resulting transfectant (resistant to 1 mg/ml genticin) is subsequently selected. Resistant colonies are then expanded and tested for their response to increasing concentrations of interferon gamma. The dose response of a selected clone is then established. Typically, the following method yields a number of cells sufficient for 75 wells (each containing approximately 200 ul of cells). The method can be modified easily (e.g., it can either be scaled up or performed in multiples to generate sufficient numbers of cells for multiple 96 well plates). Jurkat cells are maintained in RPM1 + 10% serum with 1 % Pen-Strep. Combine 2.5 mls of OPTI-MEM (LifeTechnologies) with 10 ug of plasmid DNA in a T25 flask. Add 2.5 ml OPTI-MEM containing 50 µl of DMRIE-C and incubate (RT) for 15-45 min. During incubation, determine the cell concentration, spin down the required number of cells (~10⁷ per transfection), and resuspend in OPTI-MEM to a final concentration of 10⁷ cells/ml. Then add 1 ml of 1 × 10⁷ cells in OPTI-MEM to a T25 flask and incubate at 37 °C for 6 hrs. After incubation, add 10 ml of RPMI + 15% serum.

The Jurkat:GAS-SEAP stable reporter lines are maintained in RPMI + 10% serum, 1 mg/ml Genticin, and 1% Pen-Strep. These cells are treated with supernatants containing an LP polypeptide (or fragment thereof) and/or an induced polypeptide of the invention (or fragment thereof) as produced by a protocol taught herein. On the day of treatment with the supernatant, the cells should be washed, and re-suspended in fresh RPM1 + 10% serum to a density of 500,000 cells per ml. The exact number of cells required depends on the number of supernatants being screened. For one 96 well plate, approximately 10 million cells are required (for 10 plates, 100 million cells). Transfer the cells to a triangular reservoir boat, to dispense the cells into a 96 well dish, using a 12 channel pipette to transfer 200 ul of cells into each well (therefore adding 100,000 cells per well). After all the plates have been seeded, 50 ul of the supernatants are transferred directly from the 96 well plate containing the supernatants into each well using a 12 channel pipette. In addition, a dose of exogenous interferon gamma (0.1 ng, 1.0 ng, 10.0 ng) is added to wells H9, H10, and H11 to serve as additional positive controls for the assay. The 96 well dishes containing Jurkat cells treated with supernatants are placed in an incubator for 48 hrs (note: this time is variable between 48-72 hrs). Then, 35 ul samples from each well are transferred to an opaque 96 well plate using a 12-channel pipette. The opaque plates should be covered (using cellophane), and stored at -20 °C until SEAP assays are performed as described herein or known in the art.. Plates containing the remaining treated cells are placed at 4 °C, and can serve as a source of material for repeated assays on a specific well if so desired. As a positive control, 100 Unit/ml interferon gamma is used to activate Jurkat T cells. Typically, a 30-fold induction or greater is observed in positive control wells. As will be apparent to those of ordinary skill in the art, the above protocol may be used in the generation of both transient, as well as, stably transfected cells.

### Example 7: High-Throughout Screening Assay to Identify Myeloid Activity

The following protocol is used to assess myeloid activity by determining whether an LP polypeptide (or fragment thereof) mediates the proliferation, and/or differentiation of a myeloid cell. Myeloid cell activity is assessed using a GAS/SEAP/Neo construct as described herein. Thus, a factor that increases SEAP activity indicates the ability to activate a Jaks-STATS signal transduction pathway. A typical myeloid cell used in such an assay is U937 (a pre-monocyte cell line) although, other myeloid cells can be used, such as, e.g., without limitation, TF-1, HL60, or KG1.

To transiently transfect U937 cells with a GAS/SEAP/Neo construct a DEAE-Dextran method is used (Kharbanda, et al. (1994) Cell Growth & Differentiation, 5: 259-265). First, 2 × 10⁷ U937 cells are harvested and then washed with PBS. Typically, U937 cells are grown in RPMI 1640 medium containing 10% heat-inactivated fetal bovine serum (FBS) supplemented with 100 units/ml penicillin, and 100 mg/ml streptomycin. Next, suspend the cells in 1 ml of 20 mM Tris-HCl (pH 7.4) buffer containing 0.5 mg/ml DEAE-Dextran, 8 ug GAS-SEAP2 plasmid DNA, 140 mM NaCl, 5 mM KCl, 375 uM Na₂HPO₄₋7H20, 1 mM MgCl₂, and 675 uM CaCl₂. Incubate at 37°C for 45 min. Wash the cells with RPMI 1640 medium containing 10% FBS and then resuspend in 10 ml complete medium and incubate at 37 °C for 36 hr. The GAS-SEAP/U937 stable cells are obtained by growing the cells in 400 ug/ml G418. The G418-free medium is used for routine growth but periodically (every one to two months), the cells should be re-grown in 400 ug/ml G418 for several passages. These cells are tested by harvesting 1×10⁸cells (approximately enough for ten 96-well plate assays) and then washing with PBS. Suspend the cells in 200 ml of the above described growth medium to a final density of 5×10⁵ cells/ml. Plate 200 ul cells/well in a 96-well plate (or 1×10⁵ cells/well). Add 50 ul of supernatant as described herein then, incubate at 37 °C for 48 to 72 hr. As a positive control, 100 Unit/ml interferon gamma is used to activate U937 cells. Typically, a 30-fold induction is observed in wells containing the positive controls. Assay a supernatant according to a SEAP protocol taught herein or art-known.

### Example 8: High-Throughput Screening Assay to Identify Neuronal Activity.

When cells undergo differentiation and proliferation, genes are activated through many different signal transduction pathways. One such gene, EGRI (early growth response gene 1), is induced in various tissues and cell types upon activation. The promoter of EGRI is responsible for such induction. The activation of particular cells is assessed using the EGRI promoter linked to a reporter molecule. Specifically, the following protocol is used to assess neuronal activity in a PC12 cell (rat phenochromocytoma cell). PC12 cells show proliferative and/or differentiative responses (e.g., EGRI expression) upon activation by a number of stimulators, such as, e.g., TPA (tetradecanoyl phorbol acetate), NGF (nerve growth factor), and EGF (epidermal growth factor). Thus, PC12 cells (stably transfected with a construct comprising an EGR promoter operably linked to SEAP reporter) are used in an assay to determine activation of a neuronal cell by an LP polypeptide (or fragment thereof). A EGR/SEAP reporter construct is created as follows: the EGR-I promoter sequence (-633 to +1; Sakamoto, et al. (1991) Oncogene 6:867-871) is PCR amplified from human genomic DNA using the following primers:

5′ GCGCTCGAGGGATGACAGCGATAGAACCCCGG -3′ (SEQ ID NO:42)

5′ GCGAAGCTTCGCGACTCCCCGGATCCGCCTC-3′ (SEQ ID NO:43)

Using a GAS:SEAP/Neo vector (described herein), the EGR1 amplified product is inserted into this vector by linearizing the GAS:SEAP/Neo vector (XhoI/HindIII) and removing the GAS/SV40 stuffer. The EGRI amplified product is restricted using these same enzymes (XhoI/HindIII). Then, the EGRI promoter is ligated to the vector. To prepare 96 well-plates for cell culture, add two mls of a coating solution (dilute (1:30) collagen type I (Upstate Biotech Inc. Cat#08-115) in filter sterilized 30% ethanol) per one 10 cm plate or 50 ml per well of the 96-well plate, and then air dry for 2 hr. Routinely grow PC12 cells on pre-coated 10 cm tissue culture dishes using RPMI-1640 medium (Bio Whittaker) containing 10% horse serum (JRH BIOSCIENCES, Cat. # 12449-78P), 5% heat-inactivated fetal bovine serum (FBS) supplemented with 100 units/ml penicillin and 100 ug/ml streptomycin. Every three to four days, perform a one to four split of the cells. Cells are removed from a plate by scraping and re-suspending (typically, by pipetting up and down more than 15 times). To transfect an EGR/SEAP/Neo construct into PC12 cells use the Lipofectamine protocol taught herein. Produce stable EGR-SEAP/PC12 cells by growing transfected cells in 300 ug/ml G418. The G418-free medium is used for routine growth but periodically (every one to two months), the PC12 cells should be re-grown in 300 ug/ml G41830 for several passages.

To assay a PC12 cell for neuronal activity, a 10 cm plate (containing cells that are around 70 to 80% confluent) is screened by removing the old medium and washing the cells once with PBS. Then, starve the cells overnight in low serum medium (RPMI-1640 containing 1% horse serum, and 0.5% FBS with antibiotics). The next morning, remove the medium, and wash the cells with PBS. Scrape off the cells from the plate and suspend them thoroughly in 2 ml low serum medium. Count the cell number, and add more low serum medium to achieve a final cell density of approximately 5×10⁵ cells/ml. Add 200 ul of the cell suspension to each well of 96-well plate (equivalent to 1×10⁵ cells/well). Add 50 ul of supernatant and store at 37°C for 48 to 72 hr. As a positive control, use a growth factor known to activate PC12 cells through EGR, such as, e.g., 50 ng/ul of Neuronal Growth Factor (NGF). Typically, a fifty-fold or greater induction of SEAP is achieved with a positive control. Assay the supernatant according to a SEAP method described herein.

### Example 9: High-Throughput Screening Assay to Identify T-cell Activity

NF-KB (Nuclear Factor kappa B) is a transcription factor activated by a wide variety of agents including, e.g., inflammatory cytokines (such as, e.g., IL-1, TNF, CD30, CD40, lymphotoxin-alpha, and lymphotoxin-beta); LPS, thrombin; and by expression of certain viral gene products. As a transcription factor, NF-KB typically regulates: the expression of genes involved in immune cell activation; the control of apoptosis (NF- KB appears to shield cells from apoptosis); the development of B-cells or T-cells; anti-viral or antimicrobial responses; and multiple stress responses. Under non-stimulating conditions, NF- KB is retained in the cytoplasm with I-KB (Inhibitor KB). However, upon proper stimulation, I-KB is phosphorylated and degraded, leading to NF-KB translocating into the nucleus of the cell, thereby activating transcription of specific target genes, such as, e.g., IL-2, IL-6, GM-CSF, ICAM-I, and Class 1 MHC. Due to NF-KB's role in transcriptional activation and its ability to respond to a range of stimuli, reporter constructs utilizing the NF-KB promoter element are useful in screening a supernatant produced as described herein. Activators or inhibitors of NF-KB are useful in treating diseases, e.g., inhibitors of NF-KB is used to treat diseases, syndromes, conditions, etc., related to the acute or chronic activation of NF-KB, such as, e.g., rheumatoid arthritis. To construct a vector comprising a NF-KB promoter element, a PCR based strategy is employed. The upstream primer should contain four tandem copies of the NF-KB binding site (GGGGACTTTCCC; SEQ ID NO:44),18 bp of sequence that is complementary to the 5' end of the SV40 early promoter sequence, and that is flanked by the XhoI site:

5′:GCGGCCTCGAGGGGACTTTCCCGGGGACTTTCCGGGGATCCGGACTTTCCATCCTGCCATC
TCAATTAG:3′ (SEQ ID NO:45)

The downstream primer is complementary to the 3' end of the SV40 promoter and is flanked by the Hind III site:

5′:GCGGCAAGCTTTTTGCAAAGCCTAGGC:3′ (SEQ ID NO:46).

A PCR amplification is performed using the SV40 promoter template present in a pB-gal promoter plasmid (Clontech). The resulting PCR fragment is digested with XhoI, and Hind III, then subcloned into BLSK2 (Stratagene). Sequencing with the T7, and T3 primers should confirm that the insert contains the following sequence:

5':CTCGAGGGGACTTTCCCGGGGACTTTCCGGGGACTTTCCGGGACTTTCCATCTGCCATCTCAATTAGTC
AGCAACCATAGTCCCGCCCCTAACTCCGCCCATCCCGCCCCTAACTCCGCCCAGTTCCGCCCATTCTCCGCC
CCATGGCTGACTAATTTTTTTTATTTATGCAGAGGCCGAGGCCGCCTCGGCCTCTGAGCTATTCCAGAAGTA
GTGAGGAGGCTTTTTTGGAGGCCTAGGCTTTTGCAAAAAGCTT:3' (SEQ ID NO: 47)

Next, replace the SV40 minimal promoter element present in the pSEAP2-promoter plasmid (Clontech) with the NF-KB/SV40 fragment using XhoI, and HindIII (note, this vector does not contain a neomycin resistance gene, and therefore, is not preferred for use in a mammalian expression system). To generate a stable mammalian cell line, the NF-KB/SV40/SEAP construct is removed from the above NF-KB/SEAP vector using restriction enzymes Sail, and NotI, and then inserted into a vector having neomycin resistance. For example, the NF-KB/SV40/SEAP construct is inserted into pGFP-1 (Clontech), replacing the GFP gene, after restricting pGFP-1 with SalI, and NotI. After a NF-KB/SV40/SEAP/Neo vector is established, then stable Jurkat T-cells are created and maintained as described herein. Similarly, a method for assaying supernatants with these stable Jurkat T-cells is used as previously described herein. As a positive control, exogenous TNF alpha (at, e.g., concentration of 0.1 ng, 1.0ng, and 10 ng) is added to a control well (e.g., wells H9, H10, and H11). Typically, a 5- to 10-fold activation is observed in the control.

### Example 10: Assay for Reporter Activity (e.g., SEAP)

As a reporter molecule for the assays taught herein, SEAP activity is assessed using the Tropix Phospho-light Kit (Cat. BP-400) according to the following general procedure. The Tropix Phospho-light Kit supplies the dilution, assay, and reaction buffers described below. Prime a dispenser with the 2.5x dilution buffer and dispense 15 ul of 2.5x dilution buffer into Optiplates containing 35 ul of a supernatant. Seal the plates with a plastic sealer and incubate at 65 °C for 30 min. Separate the Optiplates to avoid uneven heating. Cool the samples, until they are maintained at RT for 15 minutes. Empty the dispenser and prime with the assay buffer. Add 50 ml assay buffer and incubate (5 min. at RT). Empty the dispenser and prime with the reaction buffer (see the table below). Add 50 ul reaction buffer and incubate (20 min. at RT). Since the intensity of the chemiluminescent signal is time dependent, and it takes about 10 minutes to read five plates on luminometer, treat five plates at each time and start the second set 10 minutes later. Read the relative light unit in the luminometer using the H12 location on the plate as blank, and print the results. An increase in chemiluminescence indicates reporter activity.

### Reaction Buffer Formulation:

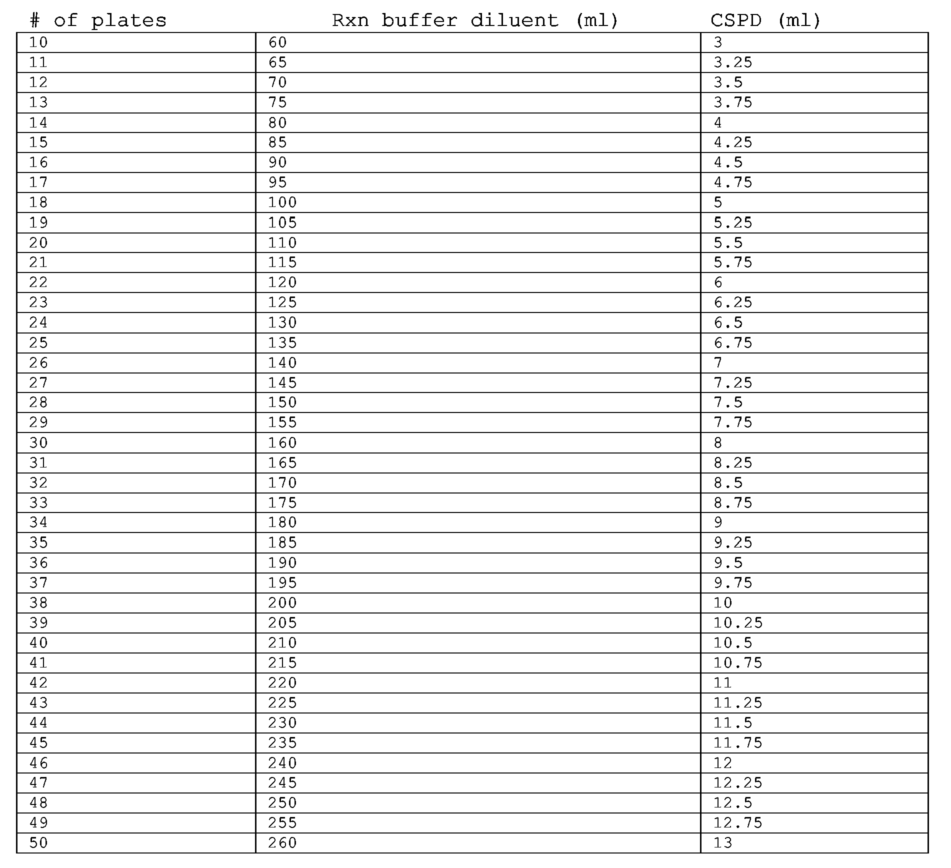

### Example 11: High-Throughput Screening Assay Identifying Changes in Small Molecule Concentration and Membrane Permeability

Binding by a ligand to a receptor can affect: intracellular levels of small molecules (such as, e.g., without limitation, calcium, potassium, and sodium); pH, and a membrane potential of the cell. These alterations are measured in an assay to identify supernatants that bind to a receptor. The following protocol is a non-limiting exemplar for assaying the effects on calcium ions in a cell (such as, e.g., without limitation, Ca⁺⁺ sequestration, removal, uptake, release, etc.) however, this assay can easily be modified to detect other cellular changes (such as, e.g., potassium, sodium, pH, membrane potential) effected by binding of a ligand with a receptor.

The following assay uses Fluorometric Imaging Plate Reader ("FLIPR") to measure changes in fluorescent molecules (Molecular Probes) that bind small molecules, such as, e.g., Ca⁺⁺. Clearly, as would be recognized by the skilled artisan, other fluorescent molecules that can detect a small composition (such as, e.g., a small molecule) can be employed instead of the calcium fluorescent molecule, fluo-4 (Molecular Probes, Inc.; No. F-14202), used here. For adherent cells, seed the cells at 10,000-20,000 cells/well in a Co-starblack 96-well plate with a clear bottom. Incubate the plate in a CO₂ incubator for 20 hours. The adherent cells are washed twice in a Biotek washer with 200 ul of HBSS (Hank's Balanced Salt Solution) leaving 100 ul of buffer after the final wash. A stock solution of 1 mg/ml fluo-4 is made in 10% pluronic acid DMSO. To load the cells with fluo-4, 50 ul of 12 ug/ml fluo-4 is added to each well. The plate is incubated at 37 °C in a CO₂ incubator for 60 min. Wash the plate four times in a Biotek washer with 200 ul of HBSS leaving 100 ul of buffer (as described above). For non-adherent cells, the cells are spun down from culture media. Cells are re-suspended in a 50-ml conical tube to 2-5×10⁶ cells/ml with HBSS. Then, 4 ul of 1 mg/ml fluo-4 solution in 10% pluronic acid DMSO is added to each ml of cell suspension. Subsequently, the tube is placed in a 37 °C water bath for 30-60 min. The cells are washed twice with HBSS, re-suspended to 1×10⁶ cells/ml, and dispensed into a microplate (100 ul/well). The plate is centrifuged at 1000 rpmXg (times gravity) for 5 min. The plate is then washed once in 200 ul Denley Cell Wash followed by an aspiration step to 100 ul final volume. For a non-cell based assay, each well contains a fluorescent molecule, such as, e.g., fluo-4. The supernatant is added to the well, and a change in fluorescence is detected. To measure the fluorescence of intracellular calcium, the FLIPR is set for the following parameters: (1) System gain is 300-800 mW; (2) Exposure time is 0.4 second; (3) Camera F/stop is F/2; (4) Excitation is 488 nm; (5) Emission is 530 nm; and (6) Sample addition is 50 ul. Observance of an increased emission at 530 nm indicates an extracellular signaling event, which has resulted in an increase in the concentration of intracellular Ca⁺⁺.

### Example 12: High-Throughput Screening Assay to Identify Tyrosine Kinase Activity

The Protein Tyrosine Kinases (PTK) represent a diverse group of transmembrane and cytoplasmic kinases. Within the Receptor Protein Tyrosine Kinase (RPTK) group are receptors for a range of mitogenic and metabolic growth factors including, e.g., the PDGF, FGF, EGF, NGF, HGF, and Insulin receptor subfamilies. In addition, a large number of RPTKs have no known corresponding ligand. Ligands for RPTKs include, e.g., mainly secreted small proteins, but also can include membrane-bound proteins, and extracellular matrix proteins.

Activation of an RPTK by a ligand typically involves dimerization of a ligand-mediated receptor resulting in the transphosphorylation of a receptor subunit(s) and subsequent activation of a cytoplasmic tyrosine kinase. Typically, cytoplasmic tyrosine kinases include, e.g., receptor associated tyrosine kinases of the src-family (such as, e.g., src, yes, lck, lyn, and fyn); non-receptor linked tyrosine kinases, and cytosolic protein tyrosine kinases (such as, e.g., Jaks, which mediate, e.g., signal transduction triggered by the cytokine superfamily of receptors such as, e.g., the Interleukins, Interferons, GM-CSF, and Leptin). Because of the wide range of factors that stimulate tyrosine kinase activity, the identification of a novel human secreted protein capable of activating tyrosine kinase signal transduction pathways would be useful. Therefore, the following protocol is designed to identify a novel human secreted protein (or fragments thereof) that activates a tyrosine kinase signal transduction pathway. Seed target cells (e.g., primary keratinocytes) at a density of approximately 25,000 cells per well in a 96 well Loprodyne Silent Screen Plates purchased (Nalge Nunc, Naperville, IL). Sterilize the plates using two 30-minute rinses with 100% ethanol, then rinse with doubly deionized water, and dry overnight. Coat some plates for 2 hr with 100 ml of cell culture grade type I collagen (50 mg/ml), gelatin (2%), polylysine (50 mg/ml) (Sigma Chemicals, St. Louis, MO); 10% Matrigel (Becton Dickinson, Bedford, MA); or calf serum. Then rinse the plates (PBS) and store at 4 °C. Seed 5,000 cells/well in growth medium on a plate and then (after 48 hrs) assay cell growth by estimating the resulting cell number using the Alamar Blue method (Alamar Biosciences, Inc., Sacramento, CA). Use Falcon plate covers (#3071 from Becton Dickinson, Bedford, MA) to cover the Loprodyne Silent Screen Plates. Falcon Microtest III cell culture plates can also be used in some proliferation experiments.

To prepare extracts, seed A431 cells onto nylon membranes of Loprodyne plates (20,000/200ml/well) and culture overnight in complete medium. Quiesce the cells by incubation in serum-free basal medium for 24 hr. Treat the cells with EGF (60 ng/ml) or 50 ul of a supernatant described herein, for 5-20 minutes. After removing the medium, add 100 ml of extraction buffer to each well (20 mM HEPES pH 7.5, 0.15M NaCl, 1% Triton X-100, 0.1 % SDS, 2 mM Na₃VO₄, 2 mM Na₄P₂O₇ and a cocktail of protease inhibitors (Boeheringer Mannheim, Cat No. 1836170; Indianapolis, IN) and shake the plate on a rotating shaker for 5 minutes at 4 °C. Then place the plate in a vacuum transfer manifold and extract filter through the 0.45 mm membrane bottom of each well (using house vacuum). Collect the extracts of a 96-well catch/assay plate in the bottom of the vacuum manifold and immediately place on ice. To clarify an extract by centrifugation, remove the content of a well (after detergent solubilization for 5 min) and centrifuge (15 min at 16,000xG at 4 °C). Test the filtered extracts for levels of tyrosine kinase activity. Although many methods of detecting tyrosine kinase activity are known and can be used without undue experimentation, a non-limiting method is described here for exemplar purposes. Generally, the tyrosine kinase activity of a supernatant is evaluated by determining its ability to phosphorylate a tyrosine residue on a specific substrate (e.g., a biotinylated peptide). An example of a biotinylated peptide useful for this purpose includes, e.g., without limitation, PSK1 (corresponding to amino acid residue numbers 6-20 of the cell division kinase cdc2-p34) and PSK2 (corresponding to amino acid residue numbers 1-17 of gastrin). Both of these biotinylated peptides are substrates for a number of tyrosine kinases and are commercially available (Boehringer Mannheim, Indianapolis, IN).

The tyrosine kinase reaction is set up by adding the following components as follows: First, add 10µl of 5uM biotinylated peptide, then 10 µl ATP/Mg⁺² (5mM ATP/50mM MgCl₂), then 10µl of 5x Assay Buffer (40mM imidazole hydrochloride, pH7.3, 40 mM betaglycerophosphate, 1mM EGTA, 100mM MgCl₂, 5mM MnCl₂, 0.5 mg/ml BSA), then 5µl of Sodium Vanadate (1 mM), and then 5µl of water. Mix the components gently and pre-incubate the reaction mix at 30 °C for 2 min. Initialize the reaction by adding 10µl of the control enzyme or the filtered supernatant. Stop the tyrosine kinase assay reaction by adding 10 ul of 120mm EDTA and place the reactions on ice. Determine tyrosine kinase activity by transferring 50 ul of the reaction mixture to a microtiter plate (MTP) module and incubating at 37 °C for 20 min. This allows the streptavadin coated 96 well plate to associate with the biotinylated peptide. Wash the MTP module four times with 300 ul of PBS per well. Next add 75 ul of anti-phosphotyrosine antibody conjugated to horseradish peroxidase (anti-P-Tyr-POD (0.5µl/ml)) to each well and incubate for one hour at 37 °C. Wash each well as described above. Next, add 100µl of peroxidase substrate solution (Boehringer Mannheim, Indianapolis, IN) and incubate for a minimum of five minutes (up to 30 min) at RT. Measure the absorbance of the sample at 405 nm using an ELISA reader (the level of bound peroxidase activity reflects the level of tyrosine kinase activity and is quantitated using an ELISA reader).

LP-induced tyrosine phosphorylation is determined as follows using any appropriate cell line (such as, e.g., Saos, GH4C1, LNCAP, LLC-PK1, L6, GT1-7, SK-N-MC, U373MG, MCF-7, Ishikawa, PA1, HEP-G2, ECV304, GLUTag, BTC6, HuVEC, TF-1, Balb/C 3T3, HDF, M07E, T1165, THP-1, or Jurkat). On day 1, approximately 2.0 ×10⁴ cells per are plated onto poly-D-lysine-coated wells (96 well plates) containing 100 µL cell propagation media (DMEM:F12 at a 3:1 ratio, 20 mM Hepes at pH 7.5, 5% FBS, and 50 µg/ml Gentamicin) then incubated overnight. On day 2, the propagation media is replaced with 100 µL starvation medium (DMEM:F12 at a 3:1, 20mM Hepes at pH 7.5, 0.5% FBS, and 50 µg/ml Gentamicin) and incubated overnight. On day 3, a 100X stock of pervanadate solution is prepared (100 µL of 100 mM sodium orthovanadate and 3.4 µL of H₂O₂). Cells are stimulated with varying concentrations of an LP of the invention (e.g., 0.1, 0.5, 1.0, 5, and 10 µL of an LP stock solution) and incubated (10 min. at RT). After stimulation, the medium is aspirated and 75 µL lysis buffer (50mM Hepes at pH 7.5, 150 mM NaCl, 10% glycerol, 1% TRITON X-100, 1mM EDTA, 1 mM pervanadate, and BM protease inhibitors) is added to each well (4°C for 15 minutes). Subsequently, 25 µL of 4X loading buffer is added to the cell lysates and the resulting solution is mixed and then heated to 95°C.

Detection of tyrosine phosphorylation is accomplished by Western immunoblotting. Samples of the treated cells (20 µl) are separated using SDS-PAGE 8-16% AA ready gels (Bio-Rad). Separated proteins are subsequently electrotransferred (~1hr at 250 mA) in transfer buffer (25 mM Tris base at pH 8.3, 0.2 M glycine, 20% methanol) to a nitrocellulose membrane that is incubated (1hr at RT) in a blocking buffer (20 mM Tris HCl at pH 7.5, 150 mM NaCl, 0.1% TWEEN-20; 1% BSA). To detect the presence of LP-induced phosphorylated proteins any appropriate commercially available anti-phosphotyrosine antibody is added to a membrane (such as, e.g., a monoclonal antibody that can detect, e.g., Erk-1, Erk-2 kinase, Raf, JNK, p38 MAP, Map kinase kinase (MEK), MEK kinase, Src, Muscle specific kinase (MUSK), IRAK, Tee, and Janus, etc.). The membrane is incubated overnight (4°C with gentle rocking) in a first solution (primary antibody, TBST, and 1% BSA), followed by TBST washing (X3 for 5 min/wash at RT) and incubation (1 hr at RT with gentle rocking) with a second solution (secondary antibody, TBST, and 1% BSA). After the secondary incubation, another series of TBST washes is carried out (X4 for 10 min/wash at RT) and detection of the immuno-identified proteins is visualized by incubating the membranes (10-30 ml of SuperSignal Solution for approximately 1 min at RT). After excess developing solution is removed, the membrane is wrapped (plastic wrap) and exposed to X-ray film (20 sec., 1 min., and 2 min. or longer if needed). LP-induced tyrosine phosphorylation is determined by comparing the number and intensity of immunostained protein bands from treated cells (visual inspection) with the number and intensity of immunostained protein bands from negative control cells (buffer only without LP solution).

### Example 13: High-Throughput Screening Assay To Identify Phosphorylation Activity

An alternative and/or complimentary tyrosine kinase assay, which can also be used detects activation (e.g., phosphorylation) of intracellular signal transduction intermediates. For example, as described herein, such an assay detects tyrosine phosphorylation of an Erk-1 and/or Erk-2 kinase. However, detecting phosphorylation of other molecules, such as, e.g., Raf, JNK, p38 MAP, Map kinase kinase (MEK), MEK kinase, Src, Muscle specific kinase (MUSK), IRAK, Tee, and Janus; as well as any other phosphoserine, phosphotyrosine, or phosphothreonine molecule, can be determined by substituting one of these molecules for an Erk-1 or Erk-2 molecule used as follows. Specifically, assay plates are made by coating the wells of a 96-well ELISA plate with 0.1 ml of protein G (1 ug/ml) for 2 hr at RT. Then, the plates are rinsed with PBS and blocked with 3% BSA/PBS for 1 hr at RT. The protein G plates are subsequently treated for one hour at RT (100 ng/well) using a commercial monoclonal antibody directed against Erk-1 and/or Erk-2 (Santa Cruz Biotechnology). After 3-5 rinses with PBS, the plates are stored at 4 °C until further use. To detect phosphorylation of another molecule (as stated above) modify this step of the method by substituting an appropriate monoclonal antibody, which can detect one of the above-described molecules (such as, e.g., Raf, JNK, p38 MAP, Map kinase kinase (MEK), MEK kinase, Src, Muscle specific kinase (MUSK), IRAK, Tee, Janus, etc.)). Seed A431 cells at 20,000 cells/well in a 96-well Loprodyne filterplate and culture in an appropriate growth medium overnight. Then starve the cells for 48 hr in basal medium (DMEM) and treat for 5-20 minutes with EGF (6.0 ng/well) or with 50 ul of a supernatant described herein. Then, solubilize the cells and filter the cell extract directly into the assay plate. After incubation with the filtered extract for 1 hr at RT, rinse the wells again. As a positive control, use a commercial preparation of MAP kinase (10 ng/well) in place of the extract. Treat the plates (1 hr at RT) with a commercial polyclonal antibody (rabbit; 1 ug/ml) that recognizes a phosphorylated epitope of an Erk-1 and/or an Erk-2 kinase. Biotinylate the antibody using any standard, art-known procedure. Quantitate the amount of bound polyclonal antibody by successive incubations with Europium-streptavidin and Europium fluorescence enhancing reagent in a Wallac DELFIA instrument (using time-resolved fluorescence). Observance of an increased fluorescent signal over background indicates that phosphorylation has occurred.

### Example 14: Method of Detecting Abnormal Levels of an LP Polypeptide in a Sample

An LP polypeptide (or fragment thereof) can be detected in a sample (such as, e.g., a biological sample as described herein). Generally, if an increased or decreased level of the LP polypeptide (compared to a normal level) is detected, then this level of the polypeptide (or fragment thereof) is a useful marker such as, e.g., for a particular cellular phenotype. Methods to detect the level of a polypeptide (or fragment thereof) are numerous, and thus, it is to be understood that one skilled in the art can modify the following exemplar assay to fit a particular need without incurring undue experimentation.

For example, an antibody-sandwich ELISA is used to detect an LP polypeptide (or fragment thereof) in a sample. Wells of a microtiter plate are coated with specific antibodies, at a final concentration of 0.2 to 10 ug/ml. The antibodies (either monoclonal or polyclonal) are produced by any art known method (or as described herein). The wells are treated with an appropriate blocking reagent so that non-specific binding of the LP polypeptide (or fragment thereof) to the well is reduced and/or prevented. The coated wells are then incubated for greater than 2 hours at RT with the sample containing the LP polypeptide (or fragment thereof). Preferably, serial dilutions of the sample containing the suspected polypeptide (or fragment thereof) should be used to validate results. The plates are then washed three times with doubly deionized or distilled water to remove unbound polypeptide. Next, 50 ul of specific antibody-alkaline phosphatase conjugate (at a concentration of 25-400 ng) is added and incubated (2 hours at RT). The plates are again washed three times with doubly deionized or distilled water to remove unbound conjugate. Subsequently, 75 ul of 4-methylumbelliferyl phosphate (MUP) or p-nitrophenylphosphate (NPP) substrate solution is added to each well and incubated (approximately one hour at RT). The reaction is then measured by a microtiter plate reader. A standard curve is prepared, using serial dilutions of a control sample, and the polypeptide concentration is plotted on the X-axis (log scale) with fluorescence or absorbance plotted on the Y-axis (linear scale). The concentration of the polypeptide in the sample can then be interpolated using the standard curve.

### Example 15: Detecting Stimulation or Inhibition of B cell Proliferation and Differentiation

Generation of functional humoral immune responses requires both soluble and cognate signaling between B-lineage cells and their microenvironment a signal may impart a positive stimulus that allows a B-lineage cell to continue its programmed development, or a negative stimulus that instructs the cell to arrest its current developmental pathway. To date, numerous stimulatory and inhibitory signals have been found that influence B cell responsiveness (including, e.g., signals from: IL-2, IL-4, IL-5, IL-6, IL-7, IL-10, IL-4, IL-13, IL-14, and IL-15). Interestingly, a signal by itself can be a weak effector but, in combination with various co-stimulatory proteins, the signal can induce, e.g., activation, proliferation, differentiation, homing, tolerance, and death among B cell populations.
One of the best-studied examples of a B-cell co-stimulatory protein is the class of molecules represented by the TNF-superfamily. Within this family, it has been demonstrated that CD40, CD27, and CD30 along with their respective ligands (CD154, CD70, and CD 153) regulate a variety of immune responses. Assays which allow for the detection and/or observation of the proliferation and/or differentiation of a B-cell population and/or its precursors are useful in determining the effect of a composition of the invention on a B-cell population (e.g., in terms of proliferation and differentiation). Taught herein below are two assays designed to detect the effect of a composition of the invention on the differentiation, proliferation, and/or inhibition of a B-cell population or its precursor.
*In vitro* Assay: An LP polypeptide of the invention (or fragment thereof), is assessed for its ability to induce activation, proliferation, differentiation, inhibition, and/or death in a B-cell and its precursors. The activity of the LP polypeptide on purified human tonsillar B cells (measured qualitatively over the dose range from 0.1 to 10,000 ng/mL) is assessed using a standard B-lymphocyte co-stimulation assay in which purified, tonsillar B cells are cultured in the presence a priming agent (such as, e.g., either formalin-fixed *Staphylococcus aureus* Cowan I (SAC) or immobilized anti-human IgM antibody). A second signal (such as, e.g., IL-2, and IL- 15) synergizes with SAC and IgM crosslinking to elicit B cell proliferation (measured by tritiated-thymidine incorporation). A novel synergizing agent can readily be identified using this assay. The assay involves isolating human tonsillar B cells by magnetic-bead-depletion (MACS) of CD3-positive cells. The resulting cell population is greater than 95% B cells as assessed by expression of CD45R(B220). Various dilutions of each sample are placed into individual wells of a 96-well plate to which are added 10⁵ B-cells suspended in culture medium (RPM1 1640 containing 10% 5FBS, 5 X 10⁻⁵M 2ME, 100U/ml penicillin, 10ug/ml streptomycin, and 10⁻⁵ dilution of SAC) in a total volume of 150µl. Proliferation or inhibition is quantitated by a 20h pulse (1uCi/well) with 3H-thymidine (6.7 Ci/mM) beginning 72h post factor addition. The positive and negative controls are respectively, IL2 and medium.
*In vivo* Assay: BALB/C mice are injected (i.p.) twice daily either with buffer alone or with 10 mg/Kg of an LP polypeptide of the invention (or fragment thereof). Mice receive this treatment for four consecutive days, at which time they are sacrificed and various tissues and serum collected for analyses. Comparison of sections (hemotoxylin and eosin stained) from normals and spleens treated with an LP polypeptide (or fragment thereof) are assessed to identify an effect of the activity of the LP polypeptide (or fragment thereof) on spleen cells (such as, e.g., the diffusion of peri-arterial lymphatic sheaths, and/or significant increases in the nucleated cellularity of the red pulp regions, which may indicate activation of differentiation and proliferation of a B-cell population). Any immunohistochemical technique using any appropriate B cell marker (such as, e.g., anti-CD45R) is used to determine whether a physiological change to a splenic cell (such as, e.g., splenic disorganization) is due to an increased B-cell representation within a loosely defined B-cell zone that infiltrates an established T-cell region. Flow cytometric analyses of spleens from treated mice are used to indicate whether the tested LP polypeptide (or fragment) specifically increases the proportion of ThB+, CD45R dull B cells over control levels. Similarly, an indication of an increased representation of mature B-cells *in vivo* is the detection in a relative increase in serum titers of Ig. Furthermore, determining whether increased B-cell maturation has occurred can also be achieved by comparing serum IgM and IgA levels between LP polypeptide-treated mice and mice treated with buffer only.

### Example 16: T-Cell Proliferation Assay

To assess the effect of an LP polypeptide (or fragment thereof) of the invention on T-cell proliferation (e.g., by measuring CD3-induced proliferation), an assay is performed on PBMCs to measure ³H-thymidine uptake. Ninety-six well plates are coated with 100 µl/well of monoclonal antibody to CD3 (such as, e.g., HIT3a, Pharmingen) or an isotype-matched control mAb (e.g., B33.1) overnight at 4 °C (1 µg/ml in .05M bicarbonate buffer, pH 9.5), then washed X3 (PBS). PBMC are isolated by F/H gradient centrifugation from human peripheral blood and added to quadruplicate wells (5 × 10⁴/well) of mAb coated plates in RPM1 containing 10% FCS and P/S in the presence of varying concentrations of an LP polypeptide (or fragment thereof) (total volume 200 ul). Relevant protein buffer (or medium only) is used as a control. After 48 hr culture at 37 °C, plates are spun for 2 min. at 1000 rpm and 100 µl of supernatant is removed and stored at -20 °C for measurement of IL-2 (or other cytokines) if an effect on proliferation is observed. Wells are supplemented with 100 µl of medium containing 0.5 uCi of ³H-thymidine and cultured at 37 °C for 18-24 hr. Wells are harvested and the amount of incorporation of ³H-thymidine is used as a measure of proliferation. Anti-CD3 by itself is used as a positive control for proliferation. IL-2 (100 U/ml) is also used as a control that enhances proliferation. A control antibody that does not induce proliferation of T cells is used as a negative control for the effect of an LP polypeptide (or fragment thereof).

### Example 17: Effect of an LP polypeptide (or fragment thereof) on the Expression of MHC Class II, Co-stimulatory and Adhesion Molecules and Cell Differentiation of Monocytes and Monocyte-Derived Human Dendritic Cells

Dendritic cells are generated by the expansion of proliferating precursors found in the peripheral blood: adherent PBMC or elutriated monocytic fractions are cultured for 7-10 days with GM-CSF (50 ng/ml) and IL-4 (20 ng/ml). These dendritic cells have the characteristic phenotype of immature cells (e.g., expression of CDI, CD80, CD86, CD40, and MHC class II antigens). Treatment with an activating factor (such as, e.g., TNF-alpha) causes a rapid change in surface phenotype (e.g., an increased expression of MHC class I and II, co-stimulatory and adhesion molecules, down regulation of FQRII, and/or an up regulation of CD83). Typically, these changes correlate with an increased antigen-presenting capacity and/or with a functional maturation of a dendritic cell. A FACS analysis of surface antigens is performed as follows: cells are treated 1-3 days with increasing concentrations of an LP polypeptide (or fragment thereof) or LPS as a positive control, washed with PBS containing 1% BSA and 0.02 mM NaN₃, and then incubated with 1:20 dilution of appropriate FITC- or PE-labeled monoclonal antibodies for 30 minutes at 4 °C. After an additional wash, the labeled cells are analyzed by flow cytometry on a FACScan (Becton Dickinson).

### Effect on the production of cytokines

Cytokines generated by dendritic cells, in particular IL-12, are important in the initiation of T-cell dependent immune responses. IL-12 strongly influences the development of Th-1 helper T-cell immune response, and induces cytotoxic T and NK cell function. An ELISA is used to measure IL-12 release in a dendritic cell that has been exposed to an LP polypeptide of the invention (or fragment thereof) as follows: dendritic cells (10⁶/ml) are treated with increasing concentrations of an LP polypeptide (or fragment thereof) for 24 hours. LPS (100 ng/ml) is added to a cell culture as a positive control. Supernatants from the cell cultures are then collected and analyzed for IL-12 using a commercial ELISA kit (e.g., R & D Systems; Minneapolis, MN). The standard protocol provided with the kit is used to measure IL-12 expression.

### Effect on the expression of MHC Class II, Co-stimulatory, and Adhesion molecules.

Three major families of cell surface antigens is identified on monocytes: adhesion molecules, molecules involved in antigen presentation, and Fc receptor. Modulation of the expression of MHC class II antigens and other co-stimulatory molecules (such as, e.g., B7 and ICAM- 1) may result in changes in the antigen presenting capacity of a monocyte and in an ability to induce T cell activation. Increased expression of Fc receptors may correlate with improved monocyte cytotoxic activity, cytokine release, and phagocytosis. A FACS analysis is used to examine surface antigens as follows: monocytes are treated for 1-5 days with increasing concentrations of an LP polypeptide (or fragment thereof) or LPS (as a positive control), washed with PBS containing 1% BSA and 0.02 mM sodium azide (NaN₃), and then incubated with a 1:20 dilution of appropriate FITC- or PE-labeled monoclonal antibodies for 30 minutes at 4 °C. After an additional wash, the labeled cells are analyzed by flow cytometry on a FACS scanner (Becton Dickinson).

### Monocyte Activation and/or Increased Survival

Assays for molecules that: activate (or, alternatively, inactivate) monocytes; and/or increase monocyte survival (or, alternatively, decrease monocyte survival) are known in the art and may routinely be applied to determine whether a composition of the invention (such as, e.g., a polypeptide or fragment thereof) functions as an inhibitor or activator of a monocyte. Polypeptides (fragments thereof), agonists, or antagonists of the invention is screened using any of the assays described below. For each of these assays, peripheral blood mononuclear cells (PBMC) are purified from single donor leukopacks (American Red Cross, Baltimore, MD) by centrifugation through a Histopaque gradient (Sigma). Monocytes are isolated from PBMC by counterflow centrifugal elutriation.

### Monocyte survival Assay

Human, peripheral-blood monocytes progressively lose viability when cultured in the absence of serum or other stimuli. Their death typically results from internally regulated processes (such as, e.g., apoptosis). Addition to a culture of activating factors, such as, e.g., TNF-alpha dramatically improves PBMC survival and prevents DNA fragmentation. Propidium iodide (PI) staining is used to measure apoptosis as follows: monocytes are cultured for 48 hours in polypropylene tubes in serum-free medium (positive control), in the presence of 100 ng/ml TNF-alpha (negative control), and in the presence of varying concentrations of a composition of the invention (such as, e.g., an LP polypeptide or fragment thereof). Cells are suspended at a concentration of 2 × 10⁶/ml in PBS containing PI at a final concentration of 5 µg/ml, and then incubated at RT for 5 minutes before FACS scan analysis. PI uptake has been demonstrated to correlate with DNA fragmentation in this method.

### Effect on cytokine release

An important function of monocytes/macrophages is their regulatory activity on other cellular populations of the immune system (e.g., through the release of cytokines after appropriate stimulation). An ELISA assay to measure cytokine release is performed as follows: human monocytes are incubated at a density of 5×10⁵ cells/ml with increasing additions of varying concentrations of an LP polypeptide (or fragment thereof) of the invention (controls employ the same conditions without the LP polypeptide). For IL-12 production, the cells are primed overnight with IFN (100 U/ml) in presence of an LP polypeptide (or fragment thereof). LPS (10 ng/ml) is then added. Conditioned media are collected after 24h and kept frozen until use. Measurement of TNF-alpha, IL-1, MCP-1, and IL-8 is then performed using any commercially available ELISA kit (e.g., R & D Systems; Minneapolis, MN) according to a standard protocol provided with the kit.

### Oxidative burst

Purified monocytes are plated in 96-w plate at approximately 1×10⁵ cells/well. Increasing concentrations of a polypeptide of the invention (or fragment thereof) are added to the wells in a total volume of 0.2 ml culture medium (RPM1 1640 + 10% FCS, glutamine and antibiotics). After 3 days incubation, the plates are centrifuged and the medium is removed from the wells. To the macrophage monolayers, 0.2 ml per well of phenol red solution (140 mM NaCl, 10 mM potassium phosphate buffer pH 7.0, 5.5 mM dextrose, 0.56 mM phenol red and 19 U/ml of HRPO) is added, together with a stimulant (200 nM PMA). The plates are incubated at 37°C for 2 hours and the reaction is stopped by adding 20 µl (1N NaOH) per well. The absorbance is read at 610 nm. To calculate the amount of H₂O₂ produced by the macrophages, a standard curve of a H₂O₂ solution of known molarity is performed for each experiment.

### Example 18: Biological Effects of an LP Polypeptide (or fragment thereof) Astrocyte and neuronal cell assays

An LP polypeptide of the invention (or fragment thereof) is tested for its capacity to promote survival, neurite outgrowth, and/or phenotypic differentiation of a cell of the nervous system (such as, e.g., a cortical neuronal cell) and/or for it capacity to induce the proliferation of a cell of the nervous system (such as, e.g., a glial fibrillary acidic protein immunopositive cell like, e.g., an astrocyte). The use of a cortical cell for this assay is based on the prevalent expression of FGF-1 and FGF-2 (basic FGF) in cortical structures and on reported enhancement of cortical neuronal survival after FGF-2 treatment. A thymidine incorporation assay, e.g., is used to assess the effect of the LP on the nervous system cell.

An *in vitro* effect of FGF-2 on cortical or hippocampal neurons shows increased neuronal survival and neurite outgrowth (see, e.g., Walicke, et al. (1986) Proc. Natl. Acad. Sci. USA 83:3012-3016). However, reports from experiments on PC-12 cells suggest that neuronal survival and neurite outgrowth are not necessarily synonymous and that a specific effect may depend not only on which FGF is tested but also on the particular receptor(s) that are expressed on a target cell. Using a primary cortical neuronal culture paradigm, the ability of an LP polypeptide (or fragment thereof) to induce neurite outgrowth and effect neuronal survival compared to FGF-2 is assessed using, e.g., a thymidine incorporation assay.

### Fibroblast and endothelial cell assays.

For proliferation assays, human lung fibroblasts (Clonetics; San Diego, CA) and/or dermal microvascular endothelial cells (Cell Applications; San Diego, CA) are cultured at 5,000 cells/well in a 96-well plate for one day in growth medium. The cells are then incubated for one day in 0.1% BSA basal medium. After replacing the medium with fresh 0.1% BSA medium, the cells are incubated (72 hr) with varying concentrations of an LP polypeptide of the invention (or fragment thereof). Then, Alamar Blue (Alamar Biosciences, Sacramento, CA) is added to each well to a final concentration of 10% and the cells are incubated for 4 hr. Cell viability is measured using a CytoFluorfluorescence reader. For a PGE assay, the human lung fibroblasts are cultured at 5,000 cells/well in a 96-well plate for one day. After a medium change to 0.1% BSA basal medium, the cells are incubated with FGF-2 or an LP polypeptide (or fragment thereof) with (or without) IL-1 alpha for 24 hours. Then supernatants are collected and assayed for PGE, by EIA (Cayman; Ann Arbor, MI). For an IL-6 assay, the human lung fibroblasts are cultured at 5,000 cells/well in a 96-well plate for 24 hrs. After a medium change to 0.1% BSA basal medium, the cells are incubated with FGF-2 or an LP polypeptide (or fragment thereof) with (or without) IL-1 alpha for 24 hours. The supernatants are collected and assayed for IL-6 by ELISA kit (Endogen; Cambridge, MA). Human lung fibroblasts are cultured with FGF-2 or an LP polypeptide (or fragment thereof) for 3 days in basal medium before the addition of Alamar Blue to assess any effect on growth of the fibroblasts. FGF-2 should show a stimulatory effect at about 10-2500 ng/ml, which can then be used to compare any stimulatory effect of an LP polypeptide (or fragment thereof).

### Parkinson Models

The loss of motor function in Parkinson's syndrome is attributed to a deficiency of striatal dopamine due to the degeneration of nigrostriatal dopaminergic projection neurons. A Parkinsonian animal model involves systemic administration of 1-methyl-4 phenyl 1,2,3,6-tetrahydropyridine (MPTP). In the central nervous system, MPTP is taken-up by astrocytes and catabolized to 1-methyl-4-phenyl pyridine (MPP⁺), which is subsequently released. Released MPP⁺ is accumulated in dopaminergic neurons by the high-affinity re-uptake transporter for dopamine. MPP⁺ is then concentrated in mitochondria via an electrochemical gradient where it selectively inhibits nicotinamide adenine disphosphate: ubiquinone oxidoreductionase (complex I) thereby, interfering with electron transport and eventually generating oxygen radicals. In tissue culture, FGF-2 (basic FGF) has trophic activity towards nigral dopaminergic neurons (Ferrari, et al. (1989) Dev. Biol. 133(1):140-147), and administering a striatal gel foam implant containing FGF-2 protects nigral dopaminergic neurons from MPTP toxicity (Otto and Unsicker, (1990) J. Neuroscience 10(6):1912-1921). Based on these reported data for the effect of FGF-2, an LP polypeptide (or fragment thereof) of the invention is evaluated to determine whether it has a similar effect as FGF-2 (such as, e.g., by modulating dopaminergic neuronal survival *(either in vitro or in vivo)* from an effect of MPTP treatment). *An in vitro* dopaminergic neuronal cell culture is prepared by dissecting the midbrain floor plate from gestation day 14 Wistar rat embryos. The tissue is dissociated with trypsin and seeded at a density of 200,000 cells/cm² on polyorthinine-laminin coated glass coverslips. The cells are maintained in Dulbecco's Modified Eagle's medium and F12 medium containing hormonal supplement (N 1). After 8 days *in vitro,* cultures are fixed with paraformaldehyde and processed for immunohistochemical staining of tyrosine hydroxylase (a specific marker for dopaminergic neurons). Dissociated cell cultures are prepared from embryonic rats. The culture medium is changed every third day and the factors are added at that time. Typically, dopaminergic neurons isolated from gestation-day-14 animals are past a point when dopaminergic precursor cells are believed to be proliferating, therefore, an increase in the number of tyrosine hydroxylase immunopositive neurons is interpreted to suggest that a similar increase in the number of surviving dopaminergic neurons would occur if the treatment had occurred *in vitro.* Therefore, if an LP polypeptide (or fragment thereof) prolongs the survival of dopaminergic neurons in an assay as taught herein, it suggests that the polypeptide (or fragment) is used to ameliorate, modulate, treat, or effect a Parkinson's disease, syndrome, condition, or state.

### Example 19: The Effect of an LP Polypeptide on Endothelial Cells

An LP polypeptide (or fragment thereof) is tested for its effect on an endothelial cell (such as, e.g., the effect on the growth of vascular endothelial cells) using the following assay: on day 1, human umbilical vein endothelial cells (HUVEC) are seeded at 2-5 ×10² cells/35 mm dish density in M199 medium containing 4% fetal bovine serum (FBS), 16 units/ml heparin, and 50 units/ml endothelial cell growth supplements (ECGS, Biotechnique, Inc.). On the following day, the medium is replaced with M199 containing 10% FBS, 8 units/ml heparin. An LP polypeptide (or fragment thereof), and positive controls (such as, e.g., VEGF, and basic FGF (bFGF)) are added to the cells at varying concentrations. On days 4, and 6, the medium is replaced. On day 8, cell number is determined with a Coulter Counter. An increase in the number of HUVEC cells indicates that the polypeptide (or fragment thereof) mediates proliferation of vascular endothelial cells.

### Example 20: Stimulatory Effect of an LP Polypeptide on the Proliferation of Vascular Endothelial Cells

An LP polypeptide (or fragment thereof) is tested for its stimulatory effect on an endothelial cell (such as, e.g., a vascular endothelial cell) to evaluate a mitogenic effect. A calorimetric MTS (3-(4,5-dimethylthiazol-2-yl)-5-(3-carboxymethoxyphenyl)-2-(4-sulfophenyl) 2H-tetrazolium) assay with the electron coupling reagent PMS (phenazine methosulfate) is performed (Cell Titer 96 AQ, Promega) based on Leak, et al. (1994) In vitro Cell. Dev. Biol. 30A:512-518 (incorporated herein for its assay teachings). Briefly, cells are seeded in a 96-well plate (5,000 cells/well) in 0.1 mL serum-supplemented medium and allowed to attach overnight. After serum-starvation for 12 hours (in 0.5% FBS conditions), bFGF, VEGF, or an LP polypeptide (or fragment thereof), in 0.5% FBS (either with or without Heparin (8 U/ml), is added to a well of the plate. After 48 hours, 20 mg of MTS/PMS mixture (1:0.05) is added per well and incubated (1 hour at 37°C) before measuring the absorbance (490 nm in an ELISA plate reader). Background absorbance from control wells (some media, no cells) is subtracted, and seven wells are performed in parallel for each condition to test for the presence of mitogenic activity (Leak, et al. *supra*)*.*

### Example 21: Inhibition of PDGF-induced Vascular Smooth Muscle Cell Proliferation

An LP polypeptide (or fragment thereof) is tested for its effect on vascular smooth muscle cell proliferation (e.g., by measuring BrdUrd incorporation) according to an assay of Hayashida, et al. (1996) J. Biol. Chem. 6:271 (36): 21985-21992 (incorporated herein for its assay teachings).

Briefly, subconfluent, quiescent HAoSMC cells grown on 4-chamber slides are transfected with CRP or FITC-labeled AT2-3LP. Then, the cells are pulsed with 10% calf serum and 6mg/ml BrdUrd. After 24 h, immunocytochemistry is performed using BrdUrd Staining Kit (Zymed Laboratories). In brief, after being exposed to denaturing solution, the cells are incubated with biotinylated mouse anti-BrdUrd antibody (4 °C for 2 h) and then incubated with streptavidin-peroxidase and diaminobenzidine. After counterstaining with hematoxylin, cells are mounted for microscopic examination, and BrdUrd-positive cells are counted. A BrdUrd index is calculated as a percentage of the number of BrdUrd-positive cells per number of total cells. Additionally, simultaneous detection of BrdUrd staining (nucleus) and FITC uptake (cytoplasm) is performed for an individual cell by the concomitant use of bright field illumination and dark field, UV fluorescent illumination (see, Hayashida, et al., supra, for details).

### Example 22: Stimulation of Endothelial Migration by an LP

An LP polypeptide (or fragment thereof) is tested for its effect on lymphatic endothelial cell migration. Endothelial cell migration assays are performed using a 48 well micro-chemotaxis chamber (Neuroprobe Inc.; Falk, et al. (1980) J. Immunological Methods : 33:239-247). Polyvinylpyrrolidone-free polycarbonate filters with a pore size of 8 µm (Nucleopore Corp.; Cambridge, MA) are coated with 0.1% gelatin (at least 6 hours at RT) and dried under sterile air. Test substances are diluted to appropriate concentrations in M199 supplemented with 0.25 % bovine serum albumin (BSA), and 10 ul of the final dilution is placed in the lower chamber of a modified Boyden apparatus. Subconfluent, early passage (2-6) HUVEC or BMEC cultures are washed and trypsinized for the minimum time required to achieve cell detachment. After placing the filter between lower and upper chamber, 2.5 × 10⁵cells (suspended in 50 ul M199 containing 1% FBS) are seeded to the upper compartment. The apparatus is then incubated (5 hrs 37°C in a humidified chamber (5% CO₂) to allow cell migration. After the incubation period, the filter is removed and the upper side of the filter (containing non-migrated cells) is scraped to remove cells. Then the filters are fixed with methanol and stained with Giemsa solution (Diff-Quick, Baxter, McGraw Park, IL). Migration is assessed by counting the number of cells occupying three random high-power fields (40x) in each well (measurements in all groups are performed in quadruplicate).

### Example 23: LP Stimulation of Nitric Oxide Production by Endothelial Cells

An LP polypeptide (or fragment thereof) is tested for its effect on nitric oxide production by an endothelial cell according to the following assay.

Nitric oxide released by the vascular endothelium is believed to be a mediator of vascular endothelium relaxation. Nitric oxide is measured in 96-well plates of confluent microvascular endothelial cells after 24 hours starvation and a subsequent 4 hr exposure to various levels of an LP polypeptide (or fragment thereof) or a positive control (such as, e.g., VEGF-1). The presence of nitric oxide in the medium is determined by use of the Griess reagent to measure total nitrite after reduction of nitric oxide-derived nitrate by nitrate reductase. The effect of an LP polypeptide (or fragment thereof) on nitric oxide release is examined on HUVEC cells. Briefly, NO release from a cultured HUVEC monolayer is measured with a NO-specific polarographic electrode connected to a NO meter (Iso-NO, World Precision Instruments Inc.) (1049). Calibration of the electrodes is performed with air-saturated distilled water (ISO) or acidified nitrite (Iso-NO) according to the procedure recommended by the manufacturer. The Iso-NO is prepared by the addition of KNO to a helium-gassed solution of 0.14 M KSO and 0.1 M KI in 0.1 M HSO. The standard calibration curve is obtained by adding graded concentrations of KNO₂ (e.g., 0, 5.0, 10.0, 25, 50, 100, 250, and 500 nmol/L) into the calibration solution containing KI and H₂SO₄. The specificity of the Iso-NO electrode to NO is previously determined by measurement of NO from authentic NO gas (1050). The culture medium is removed and HUVECs are washed twice with Dulbecco's phosphate buffered saline. The cells are then bathed in 5 ml of filtered Krebs-Henseleit solution in 6-well plates, and the cell plates are kept on a slide warmer (Lab Line Instruments Inc.) To maintain the temperature at 37°C, the NO sensor probe is inserted vertically into the wells, keeping the tip of the electrode 2 mm under the surface of the solution, before addition of the different conditions. S-nitroso acetyl penicillamin (SNAP) is used as a positive control. The amount of released NO is expressed as picomoles per 1 × 10⁶ endothelial cells. All values should be established from the means of four to six measurements in each group (number of cell culture wells). See, e.g., Leak, et al. (1995) Biochem. and Biophys. Res. Comm. 217:96-105 (incorporated by reference for teachings on NO assays).

### Example 24: Effect of an LP Polypeptide on Cord Formation/Hematopoiesis

An LP polypeptide (or fragment thereof) is tested in the following assay for its effect on angiogenesis (such as, e.g., endothelial cell differentiation during cord formation such as, e.g., the ability of microvascular endothelial cells to form capillary-like hollow structures when *cultured in vitro).* Microvascular endothelial cells (CADMEC; Cell Applications, Inc.) purchased as proliferating cells (passage 2) are cultured in CADMEC growth medium (Cell Applications, Inc.) and used at passage 5. For *an in vitro* angiogenesis assay, the wells of a 4% cell culture plate are coated (200 ml/well) with attachment factor medium (Cell Applications, Inc.) for 30 min. at 37°C. CADMEC cells are seeded onto the coated wells at 7,500 cells/well and cultured overnight in the growth medium. The growth medium is then replaced with 300 mg chord formation medium (Cell Applications, Inc.) containing either a control buffer or an LP polypeptide (or fragment thereof) (ranging from 0.1 to 100 ng/ml). Commercial VEGF (50 ng/ml; R&D) is used as a positive control. Beta-esteradiol (lng/ml) is used as a negative control. An appropriate buffer (without the polypeptide) is also utilized as a control. Treated cells are then cultured for 48 hr. Any resulting capillary-like chords are quantitated (numbers and lengths) using a video image analyzer (e.g., Boeckeler VIA-170). All assays are done in triplicate.

### Example 25: Effect of an LP Polypeptide on Angiogenesis in a Chick Chorioallantoic Membrane

An LP polypeptide (or fragment thereof) is tested in the following assay for its effect on angiogenesis (such as, e.g., the formation of blood vessels on a chick chorioallantoic membrane (CAM)). The chick chorioallantoic membrane (CAM) is a well-established system to examine angiogenesis. Blood vessel formation on CAM is easily visible and quantifiable.

Fertilized eggs of the White Leghorn chick *(Gallus gallus)* and the Japanese quail *(Cotumix cotumix)* are incubated (37.8°C and 80% humidity). Differentiated CAM of 16-day-old chick and 13-day-old quail embryos is studied as follows: On day 4 of development, a window is made on the shell of a chick egg. The embryos are checked for normal development and the eggs sealed with cellotape. The eggs are further incubated until development day 13 (using standard development stages). Thermanox coverslips (Nunc, Naperville, IL) are cut into disks of about 5 mm in diameter. Sterile and salt-free growth factors and an LP polypeptide (or fragment thereof) (ranging from 0.1 to 100 ng/ml) are dissolved in distilled water and about 3.3 mg/5 ml of the mixture are pipetted on the disks. After air-drying, the inverted disks are applied on a CAM. After 3 days, the specimens are fixed in 3% glutaraldehyde and 2% formaldehyde and rinsed in 0.12 M sodium cacodylate buffer. They are then photographed with a stereo microscope [Wild M8] and embedded for semi- and ultra-thin sectioning using any art known method. Controls are performed with carrier disks alone. The extent of angiogenesis due to a growth factor only, an LP polypeptide only, or a combination of a growth factor and an LP is measured with respect to the degree of angiogenesis found on the untreated controls.

### Example 26: An In Vivo Angiogenesis Assay Using a Matrigel Implant

An LP polypeptide (or fragment thereof) is tested in the following assay for its effect on angiogenesis (such as, e.g., its effect on the ability of an existing capillary network to form new vessels in a capsule of extracellular matrix material (Matrigel) which is implanted in a living rodent). Briefly, varying concentrations of an LP polypeptide (or fragment thereof) are mixed with liquid Matrigel (Becton Dickinson Labware; Kollaborative Biomedical Products) at 4 °C and then injected subcutaneously into a rodent (e.g., a mouse) where it subsequently solidifies into a plug. After 7 days, the plug is removed and examined for the presence of new blood vessels. More specifically, an LP polypeptide (or fragment thereof), preferably a secreted protein, (e.g., such as, 150 ng/ml) is mixed with Matrigel at 4 °C (the Matrigel material is liquid at 4 °C) and then drawn into a cold 3 ml syringe. A female C57BY6 mouse (approximately 8 weeks old) is then injected with approximately 0.5 ml of the mixture at two separate locations (preferably, around the midventral aspect of the abdomen). After 7 days, all injected mice are sacrificed, the Matrigel plugs are removed and cleaned (i.e., all clinging membranes and fibrous tissue is removed). The plugs are then fixed in neutral buffered formaldehyde (10%), embedded in paraffin, sectioned for histological examination, and stained (e.g., Masson's Trichrome). Cross sections from three different regions of each plug are so processed while other elected sections are stained for the presence of vWF. A positive control for this assay is bovine basic FGF (150 ng/ml). Matrigel alone (without an LP polypeptide or FGF) is used as a control to determine basal levels of angiogenesis.

### Example 27: Effect of LP on Ischemia in a Rabbit Lower Limb Model

An LP polypeptide (or fragment thereof) is tested in the following assay for its effect on ischemia using a rabbit hindlimb ischemia model (created by surgical removal of a femoral artery as described by Takeshita, et al. (1995) Am J. Patho 147:1649-16605 and Howell et al., (2000) Nonviral Delivery of the Developmentally Regulated Endothelial Locus-1 (del-1) Gene Increases Collateral Vessel Formation to the Same Extent as hVEGF165 in a Rabbit Hindlimb Ischemia Model, Program No.: 536, Third Annual Meeting of the American Society of Gene Therapy; each of which are incorporated by reference herein for the teachings of this assay).

### Example 28: Effect of an LP Polypeptide on Vasodialation

An LP polypeptide (or fragment thereof) is tested in the following assay for its ability to affect blood pressure in spontaneously hypertensive rats (SHR), such as, e.g., by modulating dilation of the vascular endothelium. In one embodiment, a retrovirally-mediated recombinant construct comprising an LP polypeptide (or fragment thereof) at varying dosages (e.g., 0.5, 1, 10, 30, 100, 300, and 900 mg/kg) is delivered intracardiacally to determine the affect on the development of high blood pressure in a spontaneously hypertensive (SH) rat model of human essential hypertension to determine whether attenuation of high BP is associated with prevention of other pathophysiological changes induced by a hypertensive state. Intracardiac delivery of a polypeptide (or fragment thereof) is administered to 13-14 week old spontaneously hypertensive rats (SHR) according to a method of Martens, et al. (1998) Proc Natl Acad. Sci U S A 95(5):2664-9 (incorporated herein for the teachings of this method). Control SHR and Wister-Kyoto rats (WKY) receive a placebo for the same period. The duration and initiation of treatment, site of administration, among other factors, can influence the reversal of pathophysiological alterations associated with hypertension. At the end of treatment, the effect on arterial systolic blood pressure and the level of perivascular collagen concentration is compared to controls. In addition, the medial cross-sectional area of the aorta is compared to that of untreated SHR. Data on vasuclar lumen changes is expressed as the mean (+/-) of a SEM. Other measurements used to determine treatment outcome are: (1) coronary flow (using the Langendorff-perfused heart model at baseline) after maximum vasodilation in response to adenosine (10(-5) M), after endothelium-dependent vasodilation in response to bradykinin (10(-8) M), and after ecNOS inhibition by nitro-L-arginine methyl ester (L-NAME) (10(-4) M); (2) medial thickening of coronary microvessels and perivascular collagen on histological heart sections; and (3) ecNOS expression by immunohistochemical staining in appropriate vessels using 20-week-old spontaneously hypertensive (SHR) and Wistar-Kyoto control rats (WKY). These measurements are determined by computer-directed color analysis. Statistical analysis are performed with a paired t-test and statistical significance is defined as p<0.05 vs. the response to buffer alone.

### Example 29: Effect of an LP Polypeptide in a Rat Ischemic Skin Flap Model

Current estimates indicate that over 2,000,000 US citizens have chronic wounds each year, and the problem is increasing as the population ages. The cost of caring for chronic wounds reaches into the billions of dollars a year. Clearly, there is a need for better treatment to promote healing of chronic wounds. Ischemia is a major factor contributing to the failure of most chronic wounds to heal. Wound healing involves, e.g., soluble factors that control a series of processes including inflammation, cellular proliferation, and maturation (see, e.g., Robson, M.C. (1997) Wound Repair and Regeneration 5:12-17). Pro-inflammatory cytokines such as tumor necrosis factor (TNF) and Interleukin-1 (IL-1), proteases, protease inhibitors, and growth factors play important roles in normal wound healing. Excessive production of these proteins can impede wound healing (see, e.g., Mast, & Schultz (1996) Wound Repair and Regeneration 4:411-420). Ischemia of wound tissues occurs frequently in subjects having vascular disease (such as, e.g., venous hypertension, arterial insufficiency, or diabetes). Also, extended periods of pressure can cause ischemia in tissue pressure points in persons without nerve function who have lost nerve functions but are otherwise healthy (such as, e.g., quadriplegics or paraplegics). Thus, methods to restore reverse local tissue ischemia would promote healing of many chronic wounds. Delivery of an LP polypeptide (or fragment thereof) to wound cells (e.g., in a recombinant construct encoding the polypeptide or fragment) is used to test a polypeptide of the invention for its ability to treat ischemic, non-healing wounds. In one embodiment an LP polypeptide (or fragment thereof) is used in a rodent single pedicle dorsal skin flap method based on a technique of McFarlane, et al. (1965) Plastic and Reconstructive Surgery 35:177-182 to test angiogenesis.

### Example 30: Effect of an LP Polypeptide in a Peripheral Arterial Disease Model

Angiogenic treatment using an LP polypeptide (or fragment thereof) is a novel therapeutic strategy to obtain restoration of blood flow around an ischemia (e.g., in a case of peripheral arterial disease). To test the ability of an LP polypeptide (or fragment thereof) to modulate such a peripheral arterial disease, the following experimental protocol is used: a) Using a rodent (as in the above described method) one side of the femoral artery is ligated to create ischemic damage to a muscle of the hindlimb (the other non-damaged hindlimb functions as the control); b) an LP polypeptide (or fragment thereof) is delivered to the animal either intravenously and/or intramuscularly (at the damaged limb) at least x3 times per week for 2-3 weeks at a range of dosages (20 mg-500 mg); and c) the ischemic muscle tissue is collected after at 1, 2, and 3 weeks post-ligation for an analysis of expression of an LP polypeptide (or fragment thereof) and histology. Generally, (as above) parameters for evaluation include determining viability and vascularization of tissue surrounding the ischemia, while more specific evaluation parameters may include, e.g., measuring skin blood flow, skin temperature, and factor VIII immunohistochemistry, and/or endothelial alkaline phosphatase reaction. Polypeptide expression during the ischemia, is studied using any art *known in situ* hybridization technique. Biopsy is also performed on the other side of normal muscle of the contralateral hindlimb for analysis as a control.

### Example 31: Effect of an LP Polypeptide in an Ischemic Myocardial Disease Mouse Model

An LP polypeptide (or fragment thereof) is evaluated as a treatment capable of stimulating the development of collateral vessels, and/or restructuring new vessels after coronary artery occlusion. The model is based on Guo, et al. (1999) Proc Natl Acad. Sci U S A. 96:11507-11512 (incorporated herein for these teachings) demonstrating that a robust infarct-sparing effect occurs during the early and the late phases of preconditioning in the mouse and that the quantitative aspects of this effect are consistent with previous experience in other species. The model is useful to elucidate the molecular basis of ischemic preconditioning by making it possible to apply molecular biology techniques to intact animal preparations to dissect the precise role of a specific LP during ischemic events.

### Example 32: Effect of an LP Polypeptide in a Rat Corneal Wound Healing Model

This animal model examines effects of an LP polypeptide (or fragment thereof) for angiogenic or anti-angiogenic activity on the normally avascular cornea. Briefly, the protocol comprises making a 1-1.5 mm long incision from the center of the corneal epithelium of an anesthetized mouse (e.g., a C57BL mouse strain) into the stromal layer then inserting a spatula below the lip of the incision facing the outer corner of the eye to make a pocket (whose base is 1-1.5 mm form the edge of the eye). Next, a pellet comprising an LP polypeptide or fragment thereof, (in a dosage range of about 50 ng-5ug) is positioned within the pocket (being immobilized in a slow release form, e.g., in an inert hydron pellet of approximately 1-2 ml volume). Alternatively, treatment with an LP polypeptide (or fragment thereof) can also be applied topically to the corneal wound in a dosage range of 20 mg-500 mg (daily treatment for five days). Over a 5 to 7 day post-operative period any angiogenic effect (e.g., stimulating the in growth of vessels from the adjacent vascularized corneal limbus) is determined. A photographic record is created by slit lamp photography. The appearance, density and extent of these vessels are evaluated and scored. In some instances, the time course of the progression is followed in anesthetized animals, before sacrifice. Vessels are evaluated for length, density and the radial surface of the limbus from which they emanate (expressed as clock-faced hours). Corneal wound healing is also assessed using any other art known technique.

### Example 33: Effect of an LP Polypeptide in a Diabetic Mouse and Glucocorticoid-Impaired Wound Healing Models

### Diabetic Mouse (db+/db+) as a Model

A genetically-induced diabetic mouse is used to examine the effect of an LP polypeptide (or fragment thereof) on wound healing. Mutant diabetic (db+/db+) mice have a single autosomal recessive mutation on chromosome 4 (db+) are used (Coleman et al. (1982) Proc. Natl. Acad. Sci. USA 72283-293). Typically, homozygous (db-/db-) mice are obese in comparison to their normal heterozygous (db+/db+) littermates. The mutant mice (db+/db+) have unique behavioral characteristics (such as, e.g., polyphagia, polydipsia, and polyuria); characteristic physiology (e.g., elevated blood glucose, increased or normal insulin levels, and suppressed cell-mediated immunity); and specific pathologies (such as, e.g., peripheral neuropathy, myocardial complications, and microvascular lesions, basement membrane thickening, and glomerular filtration abnormalities (see, e.g., Mandel, et al. (1978) J. Immunol. 120: 1375; Debray-Sachs, et al. (1983) Clin. Exp. Immunol. 51 (1):1-7; Leiter, et al. (1985) Am. J. of Pathol. 114:46-55; Norido, et al. (1984) Exp. Neural. 83(2):221-232; Robertson, et al. (1980) Diabetes 29(1):60-67; Giacomelli, et al. (1979) Lab Invest. 40(4):460-473; Coleman, (1982) Diabetes 31 (Suppl):1-6). These homozygous diabetic mice also develop a form of insulin-resistant hyperglycemia that is analogous to human type II diabetes (Mandel, et al. (1978) J. Immunol. 120: 1375-1377). All things considered, healing in the db+/db+ mouse may model the healing observed in humans with diabetes (see, Greenhalgh, et al. (1990) Am. J. of Pathol. 136:1235-1246). Thus, full-thickness, wound-healing using the db+/db+ mouse is a useful well-characterized, clinically relevant, and reproducible model of impaired wound healing in humans. Generally, it is agreed that healing of the diabetic wound is dependent on formation of granulation tissue and re-epithelialization rather than simply by contraction (see, e.g., Gartner, et al. (1992) J. Surg. Res. 52:389; Greenhalgh, *et al* (1990) Am. J. Pathol. 136:1235). Moreover, the diabetic db+/db+ animals have many of the characteristic features observed in Type II diabetes mellitus. Therefore, the genetically-induced db+/db+ diabetic mouse is useful to examine the effect of an LP polypeptide (or fragment thereof) on wound healing according to the following method. Genetically, diabetic female C57BWKsJ mice and their non-diabetic heterozygous littermates are purchased at 6 weeks of age (Jackson Laboratories) and are 8 weeks old at the start of testing. Animals are individually housed and received food and water *ad libitum.* All manipulations are performed using standard aseptic techniques. The wounding protocol is performed generally according to the method of Tsuboi & Rifkin, (1990) Exp. Med. 172:245-251.

### Steroid Impaired Rat Model

The following method is designed to investigate the effect of a topical treatment of varying concentrations of an LP polypeptide (or fragment thereof) on the wound of a healing-impaired rat (methylprednisolone impairment of a full thickness excisional skin wound). The inhibition of wound healing by steroids (such as, e.g., the glucocorticoid methylprednisolone) is well documented *both in vitro and in vivo* (see, e.g., Wahl, (1989) Glucocorticoids and Wound healing. In: Anti-Inflammatory Steroid Action: Basic and Clinical Aspects pp. 280-302; Wahlet, al. (1975) J.Immunol. 115: 476-481; and Werb, et al. (1978) J. Exp. Med. 147:1684-1694). Glucocorticoids (such as methylprednisolone) are believed to retard wound healing by inhibiting angiogenesis, decreasing vascular permeability, fibroblast proliferation, collagen synthesis, and by transiently reducing the level of circulating monocytes. Furthermore, the systemic administration of steroids (such as glucocorticoids) to impair wound healing is a well established method used in rodents, such as, e.g., the rat (see, e.g., Ebert, et al. (1952) An. Intern. Med. 37:701-705; Beck, et al. (1991) Growth Factors. 5: 295-304; Haynes, et al. (1978) J. Clin. Invest. 61: 703-797; Haynes, et al. (1978) J. Clin. Invest. 61: 703-797; and Wahl, (1989), supra); and Pierce, et al. (1989) Proc. Natl. Acad. Sci. USA 86: 2229-2233). Thus, such a model is useful in assessing the effect of an LP polypeptide (or fragment thereof) of the invention on wound healing.

The assays, methods, or examples described herein test the activity of an LP polynucleotide sequence or an LP polypeptide (or fragment thereof). However, an ordinarily skilled artisan could easily modify (without undue experimentation) any exemplar taught herein using a different composition and/or concentration (such as, e.g., an agonist and/or an antagonist of an LP polynucleotide sequence or an LP polypeptide (or fragment thereof) of the invention. It will be clear that the invention may be practiced otherwise than as specifically described in the foregoing description and examples. Numerous modifications and variations of the present invention are possible in light of the above teachings and, therefore, are within the scope of the appended claims. The entire disclosure of each document cited (including patents, patent applications, journal articles, abstracts, laboratory manuals, books, or other disclosures) in the Background of the Invention, Detailed Description, and Examples is hereby incorporated herein by reference for the teachings they were intended to convey. Moreover, the hard copy of the sequence listing submitted herewith and the corresponding computer readable form are both incorporated herein by reference in their entireties, including without reservation, all corresponding drawings, pictures, graphs, diagrams, figures, figure legends, and http sites (including all corresponding information contained therein). The foregoing written specification is considered sufficient to enable a person of ordinary skill in the art to practice the invention. Indeed, various modifications of the invention in addition to those shown and described herein will become apparent from the foregoing description and these modifications also fall within the scope of the appended claims. All references cited herein are incorporated herein by reference to the same extent as if each individual publication or patent application was specifically and individually indicated to be incorporated by reference in its entirety for all purposes. Many modifications and variations of this invention can be made without departing from its spirit and scope, as will be apparent to those skilled in the art. The specific embodiments described herein are offered by way of example only, and the invention is to be limited only by the terms of the appended claims, along with the full scope of equivalents to which such claims are entitled.

SEQUENCE LISTING

SEQ ID NO: 1 is primate LP194 nucleic acid sequence.
SEQ ID NO: 2 is primate LP194 amino acid sequence.
SEQ ID NO: 3 is primate LP263a nucleic acid sequence.
SEQ ID NO: 4 is primate LP263a amino acid sequence.
SEQ ID NO: 5 is primate LP263b nucleic acid sequence.
SEQ ID NO: 6 is primate LP263b amino acid sequence.
SEQ ID NO: 7 is primate LP264 nucleic acid sequence.
SEQ ID NO: 8 is primate LP264 amino acid sequence.
SEQ ID NO: 9 is primate LP265 nucleic acid sequence.
SEQ ID NO: 10 is primate LP265 amino acid sequence.
SEQ ID NO: 11 is primate LP283 nucleic acid sequence.
SEQ ID NO: 12 is primate LP283 amino acid sequence.
SEQ ID NO: 13 is primate LP286 nucleic acid sequence.
SEQ ID NO: 14 is primate LP286 amino acid sequence.
SEQ ID NO: 15 is primate LP284 nucleic acid sequence.
SEQ ID NO: 16 is primate LP284 amino acid sequence.
SEQ ID NO: 17 is primate LP282 nucleic acid sequence.
SEQ ID NO: 18 is primate LP282 amino acid sequence.
SEQ ID NO: 19 is primate LP273 nucleic acid sequence.
SEQ ID NO: 20 is primate LP273 amino acid sequence.
SEQ ID NO: 21 is primate LP277 nucleic acid sequence.
SEQ ID NO: 22 is primate LP277 amino acid sequence.
SEQ ID NO: 23 is primate LP287 nucleic acid sequence.
SEQ ID NO: 24 is primate LP287 amino sequence.
SEQ ID NO: 25 is primate LP209 nucleic acid sequence.
SEQ ID NO: 26 is primate LP209 amino acid sequence.
SEQ ID NO: 27 is primate LP209b nucleic acid sequence.
SEQ ID NO: 28 is primate LP209b amino acid sequence.
SEQ ID NO: 29 is primate LP209c nucleotide sequence.
SEQ ID NO: 30 is primate LP209c amino acid sequence.
SEQ ID NO: 31 is primate LP209d nucleotide sequence.
SEQ ID NO: 32 is primate LP209d amino acid sequence.
SEQ ID NO: 33 is primate LP293 nucleotide sequence.
SEQ ID NO: 34 is primate LP293b amino acid sequence.
SEQ ID NO: 35 is primate LP294 nucleotide sequence.
SEQ ID NO: 36 is primate LP294 amino acid sequence.
SEQ ID NO: 37 is primate LP295 nucleotide sequence.
SEQ ID NO: 38 is primate LP295 amino acid sequence.
SEQ ID NO: 39 is a DNA primer
SEQ ID NO: 40 is a DNA primer
SEQ ID NO: 41 is a DNA primer
SEQ ID NO: 42 is a DNA primer
SEQ ID NO: 43 is a DNA primer
SEQ ID NO: 44 is a DNA primer

## Claims

1. An isolated or recombinant polynucleotide encoding a LP209 polypeptide selected from the group consisting of
a) a polynucleotide encoding a polypeptide according to Table 12;
b) a polypeptide comprising amino acids 25 to 348 of SEQ ID NO: 26;
c) a polynucleotide encoding a mature polypeptide of SEQ ID NO: 26 with less than five amino acid substitutions; and
d) a polynucleotide according to SEQ ID NO: 25 or a polynucleotide which hybridizes thereto at 55°C, less than 500nM salt.

2. A LP209 polypeptide selected from the group consisting of
a) a polypeptide according to Table 12;
b) a polypeptide comprising amino acids 25 to 348 of SEQ ID NO: 26;
c) a mature polypeptide of SEQ ID NO: 26 with less than five amino acid substitutions;

3. A binding compound comprising an antigen binding portion from an antibody which specifically binds to an LP209 protein or polypeptide.

4. The binding compound according to claim 3, wherein said compound is a Fv, Fab, or Fab2 fragment.

5. A polypeptide according to claim 2, for use as a medicament.

6. A binding compound according to either claim 3, or claim 4, for use as a medicament.

7. Use of a binding compound according to either claim 3, or claim 4, in the manufacture of a medicament for the treatment of defects in or wounds to tissues and organs selected from the group consisting of cartilage, bone, spleen, lymph nodes, thymus, connective tissue and joints.

8. Use of a polypeptide according to claim 2, in the manufacture of a medicament for the treatment of defects in or wounds to tissues and organs selected from the group consisting of cartilage, bone, spleen, lymph nodes, thymus, connective tissue and joints.

9. An isolated or recombinant polynucleotide comprising sequence encoding an antigenic polypeptide comprising at least 17 contiguous amino acids from a mature coding portion of SEQ ID NO: Y (LP194; LP263a, LP263b, LP264, LP265, LP283, LP286, LP284, LP282, LP273, LP277, LP287, LP209, LP209b, LP209c, LP209d, LP293, LP294, or LP295).

10. The polynucleotide of Claim 9, encoding:
a) a full length polypeptide of SEQ ID NO: Y or table 1-18;
b) a mature polypeptide of SEQ ID NO: Y or table 1-18;
c) an antigenic fragment at least 12 contiguous amino acid residues in length of SEQ ID NO: Y from an LP of Table 1, 2, 3, 4, 5, 6, 7, 8, 9, 10, 11, 12, 13, 14, 15, 16, 17, or 18;
d) at least two fragments of SEQ ID NO: Y from an LP of Table 1, 2, 3, 4, 5, 6, 7, 8, 9, 10, 11, 12, 13, 14, 15, 16, 17, or 18, wherein said fragment do not overlap;
e) a plurality of fragments of SEQ ID NO: Y from an LP of Table 1, 2, 3, 4, 5, 6, 7, 8, 9, 10, 11, 12, 13, 14, 15, 16, 17, or 18, wherein said fragment do not overlap; or
f) a mature polypeptide of SEQ ID NO:Y with less than five amino acid substitutions.

11. The polynucleotide of Claim 9, which hybridizes at 55° C, less than 500 mM salt, to:
a) the mature coding portion of SEQ ID NO: 1;
b) the mature coding portion of SEQ ID NO: 3;
c) the mature coding portion of SEQ ID NO: 5;
d) the mature coding portion of SEQ ID NO: 7;
e) the mature coding portion of SEQ ID NO: 9;
f) the mature coding portion of SEQ ID NO: 11;
g) the mature coding portion of SEQ ID NO: 13;
h) the mature coding portion of SEQ ID NO: 15.
i) the mature coding portion of SEQ ID NO: 17;
j) the mature coding portion of SEQ ID NO: 19;
k) the mature coding portion of SEQ ID NO: 21;
l) the mature coding portion of SEQ ID NO: 23
m) the mature coding portion of SEQ ID NO: 25;
n) the mature coding portion of SEQ ID NO: 27;
o) the mature coding portion of SEQ ID NO: 29;
p) the mature coding portion of SEQ ID NO: 31;
q) the mature coding portion of SEQ ID NO: 33;
r) the mature coding portion of SEQ ID NO: 35; or
s) the mature coding portion of SEQ ID NO: 37.

12. The polynucleotide of Claim 11, wherein said temperature is at least 65° C, and said salt is less than 300 mM.

13. The polypeptide of Claim 11, comprising at least 30, 32, 34, 36, 38, 39, 40, 42, 44, 46, 48, 49, 50, 52, 54, 56, 58, 59, 75, or at least about 150 contiguous nucleotides to a nucleotide sequence of LP(LP194, LP263a, LP263b, LP264, LP265, LP283, LP286, LP284, LP282, LP273, LP277, LP287, LP209, LP209b, LP209c, LP209d, LP293, LP294, or LP295) of:
a) the mature coding portion of SEQ ID NO: 1;
b) the mature coding portion of SEQ ID NO: 3;
c) the mature coding portion of SEQ ID NO: 5;
d) the mature coding portion of SEQ ID NO: 7;
e) the mature coding portion of SEQ ID NO: 9;
f) the mature coding portion of SEQ ID NO: 11;
g) the mature coding portion of SEQ ID NO: 13;
h) the mature coding portion of SEQ ID NO: 15.
i) the mature coding portion of SEQ ID NO: 17;
j) the mature coding portion of SEQ ID NO: 19;
k) the mature coding portion of SEQ ID NO: 21;
l) the mature coding portion of SEQ ID NO: 23
m) the mature coding portion of SEQ ID NO: 25;
n) the mature coding portion of SEQ ID NO: 27;
o) the mature coding portion of SEQ ID NO: 29;
p) the mature coding portion of SEQ ID NO: 31;
q) the mature coding portion of SEQ ID NO: 33;
r) the mature coding portion of SEQ ID NO: 35 or
s) the mature coding protion of SEQ ID NO: 37.

14. An expression vector comprising a polynucleotide of Claim 9, wherein said temperature is at least 65°C, and said salt is less than 300 mM.

15. The expression vector of Claim 14, which further comprises a plurality of 23 nucleotide segments with identity to the coding portion of SEQ ID NO: X.

16. A host cell containing the expression vector of Claim 14, including a eukaryotic cell.

17. A method of making an antigenic polypeptide comprising expressing a recombinant polynucleotide of Claim 9.

18. A method for detecting a polynucleotide of Claim 9, comprising contacting said polynucleotide with a probe that hybridizes, under stringent conditions, to at least 25 contiguous nucleotides of:
a) the mature coding portion of SEQ ID NO: 1;
b) the mature coding portion of SEQ ID NO: 3;
c) the mature coding portion of SEQ ID NO: 5;
d) the mature coding portion of SEQ ID NO: 7;
e) the mature coding portion of SEQ ID NO: 9;
f) the mature coding portion of SEQ ID NO: 11;
g) the mature coding portion of SEQ ID NO: 13;
h) the mature coding portion of SEQ ID NO: 15.
i) the mature coding portion of SEQ ID NO: 17;
j) the mature coding portion of SEQ ID NO: 19;
k) the mature coding portion of SEQ ID NO: 21;
1) the mature coding portion of SEQ ID NO: 23
m) the mature coding portion of SEQ ID NO: 25;
n) the mature coding portion of SEQ ID NO: 27;
o) the mature coding portion of SEQ ID NO: 29;
p) the mature coding portion of SEQ ID NO: 31;
q) the mature coding portion of SEQ ID NO: 33;
r) the mature coding portion of SEQ ID NO: 35 or
s) the mature coding protion of SEQ ID NO: 37.
to form a duplex, wherein detection of said duplex indicates the presence of said polynucleotide.

19. A kit for the detection of a polynucleotide of Claim 9, comprising a compartment containing a probe that hybridizes, under stringent hybridization conditions, to at least 34 contiguous nucleotides of a polynucleotide of Claim 9 to form a duplex.

20. A binding compound comprising an antibody which specifically binds to at least a 17 contiguous amino acid antigen binding site region of:
a) primate LP194 (SEQ ID NO: 2);
b) primate LP263a (SEQ ID NO: 4);
c) primate LP263b (SEQ ID NO: 6);
d) primate LP264 (SEQ ID NO: 8);
e) primate LP265 (SEQ ID NO: 10);
f) primate LP283 (SEQ ID NO: 12);
g) primate LP286 (SEQ ID NO: 14);
h) primate LP284 (SEQ ID NO: 16);
i) primate LP282 (SEQ ID NO: 18);
j) primate LP273 (SEQ ID NO: 20);
k) primate LP277 (SEQ ID NO: 22);
1) primate LP287 (SEQ ID NO: 24);
m) primate LP209 (SEQ ID NO: 26);
n) primate LP209b (SEQ ID NO: 28);
o) primate LP209c (SEQ ID NO: 30);
p) primate LP209d (SEQ ID NO: 32);
q) primate LP293 (SEQ ID NO: 34);
r) primate LP294 (SEQ ID NO: 36); or
s) primate LP295 (SEQ ID NO: 38).

21. The binding compound of Claim 20, wherein:
a) said antibody binding site is:
i) specifically immunoreactive with a polypeptide of SEQ ID NO: Y;
ii) specifically immunoreactive with a polypeptide of SEQ ID NO: 2;
iii) specifically immunoreactive with a polypeptide of SEQ ID NO: 4;
iv) specifically immunoreactive with a polypeptide of SEQ ID NO: 6;
v) specifically immunoreactive with a polypeptide of SEQ ID NO: 8;
vi) specifically immunoreactive with a polypeptide of SEQ ID NO: 10;
vii) specifically immunoreactive with a polypeptide of SEQ ID NO: 12;
viii) specifically immunoreactive with a polypeptide of SEQ ID NO: 14;
ix) specifically immunoreactive with a polypeptide of SEQ ID NO: 16;
x) specifically immunoreactive with a polypeptide of SEQ ID NO: 18;
xi) specifically immunoreactive with a polypeptide of SEQ ID NO: 20;
xii) specifically immunoreactive with a polypeptide of SEQ ID NO: 22;
xiii) specifically immunoreactive with a polypeptide of SEQ ID NO: 24;
xiv) specifically immunoreactive with a polypeptide of SEQ ID NO: 26;
xv) specifically immunoreactive with a polypeptide of SEQ ID NO: 28;
xvi) specifically immunoreactive with a polypeptide of SEQ ID NO: 30;
xvii) specifically immunoreactive with a polypeptide of SEQ ID NO: 32;
xviii) specifically immunoreactive with a polypeptide of SEQ ID NO: 34;
xix) specifically immunoreactive with a polypeptide of SEQ ID NO: 36;
xx) specifically immunoreactive with a polypeptide of SEQ ID NO: 38;
xxi) raised against a purified or recombinantly produced human LP protein selected from : LP194, LP263a, LP263b, LP264, LP265, LP283, LP286, LP284, LP282, LP273, LP277, LP287, LP209, LP209b, LP209c, LP209d, LP293, LP294, or LP295;
xxii) in a monoclonal antibody, Fab, or F(ab)2; or
b) said binding compound is:
i) an antibody molecule;
ii) a polyclonal antiserum;
iii) detectably labeled;
iv) sterile; or
v) in a buffered composition.

22. A method using the binding compound of Claim 20, comprising contacting said binding compound with a biological sample comprising an antigen, thereby forming an LP binding compound:antigen complex.

23. The method of Claim 22, wherein said biological sample is from a human, and wherein said binding compound is an antibody.

24. A detection kit comprising said binding compound of Claim 21, and:
a) instructional material for the use of said binding compound for said detection; or
b) a compartment providing segregation of said binding compound.

25. A substantially pure or isolated antigenic polypeptide, which binds to said binding composition of Claim 20, and further comprises at least 25 contiguous amino acids from:
a) primate LP194 (SEQ ID NO: 2);
b) primate LP263a (SEQ ID NO: 4);
c) primate LP263b (SEQ ID NO: 6);
d) primate LP264 (SEQ ID NO: 8);
e) primate LP265 (SEQ ID NO: 10);
f) primate LP283 (SEQ ID NO: 12);
g) primate LP286 (SEQ ID NO: 14);
h) primate LP284 (SEQ ID NO: 16);
i) primate LP282 (SEQ ID NO: 18);
j) primate LP273 (SEQ ID NO: 20);
k) primate LP277 (SEQ ID NO: 22);
1) primate LP287 (SEQ ID NO: 24);
m) primate LP209 (SEQ ID NO: 26);
n) primate LP209b (SEQ ID NO: 28);
o) primate LP209c (SEQ ID NO: 30);
p) primate LP209d (SEQ ID NO: 32);
q) primate LP293 (SEQ ID NO: 34);
r) primate LP294 (SEQ ID NO: 36); or
s) primate LP295 (SEQ ID NO: 38);

26. The polypeptide of Claim 25, which:
a) comprises at least a fragment of at least 29 contiguous amino acid residues from a primate LP protein selected from: LP194, LP263a, LP263b, LP264, LP265, LP283, LP286, LP284, LP282, LP273, LP277, LP287, LP209, LP209b, LP209c, LP209d, LP293, LP294, or LP295;
b) is a soluble polypeptide;
c) is detectably labeled;
d) is in a sterile composition;
e) is in a buffered composition;
f) is recombinantly produced, or
g) has a naturally occurring polypeptide sequence.

27. The binding compound of Claim 14, where said compound is an antibody that:
a) is raised against a peptide sequence of a mature polypeptide of Table 1, 2, 3, 4, 5, 6, 7, 8, 9, 10, 11, 12, 13, 14, 15, 16, 17, or 18;
b) is produced in a mammal, or a plant;
c) is immunoselected; or
d) binds to a denatured polypeptide of Table 1, 2, 3, 4, 5, 6, 7, 8, 9, 10, 11, 12, 13, 14, 15, 16, 17, or 18.


SEQUENCE LISTING

<110>  Eli Lilly and Company

<120>  LP MAMMALIAN PROTEINS; RELATED REAGENTS

<130>  X-14462M

<140>  10/470,390
<141>  2003-07-25

<150>  US 60/305,058
<151>  2001-07-13

<150>  US 60/266,359
<151>  2001-02-02

<150>  US 60/270,564
<151>  2001-02-21

<160>  47

<170>  PatentIn version 3.2

<210>  1
<211>  2475
<212>  DNA
<213>  Homo sapiens


<220>
<221>  misc_feature
<222>  (1)..(1)
<223>  LP194

<220>
<221>  CDS
<222>  (168)..(851)

<400>  1
ccggaggagt ccgagaggaa gcggaggcgc gagctggagg cggcggctcc cgtcggcctc      60

cggcaggact gagcgctggg aggccggaag gcgggcgcgc acggcggaga ggcgggcggg     120

aggccggagc atattaatga aaagtgccat aaactgaaaa accaaac atg agg gta      176
                                                    Met Arg Val
                                                    1

gca ggt gct gca aag ttg gtg gta gct gtg gca gtg ttt tta ctg aca      224
Ala Gly Ala Ala Lys Leu Val Val Ala Val Ala Val Phe Leu Leu Thr
    5                   10                  15

ttt tat gtt att tct caa gta ttt gaa ata aaa atg gat gca agt tta      272
Phe Tyr Val Ile Ser Gln Val Phe Glu Ile Lys Met Asp Ala Ser Leu
20                  25                  30                  35

gga aat cta ttt gca aga tca gca ttg gac aca gct gca cgt tct aca      320
Gly Asn Leu Phe Ala Arg Ser Ala Leu Asp Thr Ala Ala Arg Ser Thr
                40                  45                  50

aag cct ccc aga tat aag tgt ggg atc tca aaa gct tgc cct gag aag      368
Lys Pro Pro Arg Tyr Lys Cys Gly Ile Ser Lys Ala Cys Pro Glu Lys
            55                  60                  65

cat ttt gct ttt aaa atg gca agt gga gca gcc aac gtg gtg gga ccc      416
His Phe Ala Phe Lys Met Ala Ser Gly Ala Ala Asn Val Val Gly Pro
        70                  75                  80

aaa atc tgc ctg gaa gat aat gtt tta atg agt ggt gtt aag aat aat      464
Lys Ile Cys Leu Glu Asp Asn Val Leu Met Ser Gly Val Lys Asn Asn
    85                  90                  95

gtt gga aga ggg atc aat gtt gcc ttg gca aat gga aaa aca gga gaa      512
Val Gly Arg Gly Ile Asn Val Ala Leu Ala Asn Gly Lys Thr Gly Glu
100                 105                 110                 115

gta tta gac act aaa tat ttt gac atg tgg gga gga gat gtg gca cca      560

Val Leu Asp Thr Lys Tyr Phe Asp Met Trp Gly Gly Asp Val Ala Pro
                120                 125                 130

ttt att gag ttt ctg aag gcc ata caa gat gga aca ata gtt tta atg      608
Phe Ile Glu Phe Leu Lys Ala Ile Gln Asp Gly Thr Ile Val Leu Met
            135                 140                 145

gga aca tac gat gat gga gca acc aaa ctc aat gat gag gca cgg cgg      656
Gly Thr Tyr Asp Asp Gly Ala Thr Lys Leu Asn Asp Glu Ala Arg Arg
        150                 155                 160

ctc att gct gat ttg ggg agc aca tct att act aat ctt ggt ttt aga      704
Leu Ile Ala Asp Leu Gly Ser Thr Ser Ile Thr Asn Leu Gly Phe Arg
    165                 170                 175

gac aac tgg gtc ttc tgt ggt ggg aag ggc att aag aca aaa agc cct      752
Asp Asn Trp Val Phe Cys Gly Gly Lys Gly Ile Lys Thr Lys Ser Pro
180                 185                 190                 195

ttt gaa cag cac ata aag aac aat aag gat aca aac aaa tat gaa gga      800
Phe Glu Gln His Ile Lys Asn Asn Lys Asp Thr Asn Lys Tyr Glu Gly
                200                 205                 210

tgg cct gaa gtt gta gaa atg gaa gga tgc atc ccc cag aag caa gac      848
Trp Pro Glu Val Val Glu Met Glu Gly Cys Ile Pro Gln Lys Gln Asp
            215                 220                 225

taa tggaaatgtg gagagaattg aagaaagcgc actttcactc ttaatgggag      901

agctataaat ggcagagcta tgtgtaaata ttttaagagc atgcagccat cttggtgtgt      961

gcatgagtat tgtctctttt gatatcagga ttatttattg ctaacgtaaa tagatagcat      1021

tgtaaataat catcacaatg atcaaatcac tgaaccatgt ctccgcacat ttccctaaaa      1081

gtacaatgtt tagactgcta tggtaataca tattttaaat tctaaaagca tacacaatgt      1141

gtaactgaat ggtttgtgaa aaatatattg atatatatac tagttgctat gaaaatatca      1201

tggaataata gggattttag ggtggatact ttattttctt ttatgtttct atatgttgcg      1261

ttgtgatgac attatctttt aaattaaaaa gagatttggc tagttgtgtg tgtaatgtta      1321

ctttacagtc cgactctcct gatgtacctc ttttcatgat ctttttcttt ccttcccaag      1381

aaactgagga atgtttaata tgaaaacata catcggatat gtgaaaagca caacaaaatt      1441

cttaatgtac acagtaaaaa agtaaatata taaatgtaga tggcatttag gaccacagct      1501

tgctggattt gtgttagcta tgggaataac ttgattttgt ataagctatt tagagtgagg      1561

ctggaggtgg cagcttcaca gaactggaga accaggccaa gtcccctccc caacctaatt      1621

aggtcattca ggacagctaa gtcagtatat ttagagcaat actagcatac gtttttctta      1681

attgttatca gcattgacca agtggtttgg aaggaggcat gctttaatat cacaataatt      1741

ttgatttgta aaccaagaaa ttaatcctgt gtttatctaa cttcataata gcaattattg      1801

cccgaagcta tagtggcata tttacaaaag ttcttattac tgggcggact gataacattt      1861

aaaaaataat tgtgtttgac cccaaatgac tttataccca attctacata aaaatataga      1921

agatctatct ttttttgtta ccttcagatg ttcactaaat aactcagttt ttaagcagaa      1981

gttttcaggg cattaaatat atgttgtgta tgaagtatct caaactggaa cataaattta      2041

gtgatcaaac tgccattcac agtgtaaggc agcacttaaa tttcgaacct aaagtttaga      2101

tgcattgtat aaaaaaacct aaaagcagta tctgttattt agctgtaaac caagttggaa      2161

gctattcgga taatttctta aatattgatg aactttggag tactgtttct tccttcaaac      2221

tgaatgtaat taattcatga ataaatgcac cttatatgtt taaacaatct ttgtatactt      2281

ttgggatttt tggtgcttat atgctaaatc acattcagca tgtgtatttt gacatttaaa      2341

atacttccct caattctgta aattaaaaga atagttattt tacagttcca gggattgtga 2401

aataaatgtt gcagtttttt aaaataatga aaataaatac tcttggtttt gctttgtgaa 2461

aaaaaaaaaa aaaa 2475

<210> 2
<211> 227
<212> PRT
<213> Homo sapiens

<400> 2

Met Arg Val Ala Gly Ala Ala Lys Leu Val Val Ala Val Ala Val Phe
1               5                   10                  15

Leu Leu Thr Phe Tyr Val Ile Ser Gln Val Phe Glu Ile Lys Met Asp
            20                  25                  30

Ala Ser Leu Gly Asn Leu Phe Ala Arg Ser Ala Leu Asp Thr Ala Ala
        35                  40                  45

Arg Ser Thr Lys Pro Pro Arg Tyr Lys Cys Gly Ile Ser Lys Ala Cys
    50                  55                  60

Pro Glu Lys His Phe Ala Phe Lys Met Ala Ser Gly Ala Ala Asn Val
65                  70                  75                  80

Val Gly Pro Lys Ile Cys Leu Glu Asp Asn Val Leu Met Ser Gly Val
                85                  90                  95

Lys Asn Asn Val Gly Arg Gly Ile Asn Val Ala Leu Ala Asn Gly Lys
            100                 105                 110

Thr Gly Glu Val Leu Asp Thr Lys Tyr Phe Asp Met Trp Gly Gly Asp
        115                 120                 125

Val Ala Pro Phe Ile Glu Phe Leu Lys Ala Ile Gln Asp Gly Thr Ile
    130                 135                 140

Val Leu Met Gly Thr Tyr Asp Asp Gly Ala Thr Lys Leu Asn Asp Glu
145                 150                 155                 160

Ala Arg Arg Leu Ile Ala Asp Leu Gly Ser Thr Ser Ile Thr Asn Leu
                165                 170                 175

Gly Phe Arg Asp Asn Trp Val Phe Cys Gly Gly Lys Gly Ile Lys Thr
            180                 185                 190

Lys Ser Pro Phe Glu Gln His Ile Lys Asn Asn Lys Asp Thr Asn Lys
        195                 200                 205

Tyr Glu Gly Trp Pro Glu Val Val Glu Met Glu Gly Cys Ile Pro Gln
    210                 215                 220

Lys Gln Asp
225

<210> 3
<211> 763
<212> DNA

<213>  Homo sapiens

<220>
<221>  misc_feature
<222>  (1)..(1)
<223>  LP263a

<220>
<221>  CDS
<222>  (52)..(741)

<220>
<221>  misc_feature
<222>  (502)..(502)
<223>  n = a,g,c, or t

<220>
<221>  misc_feature
<222>  (663)..(663)
<223>  n = a,g,c, or t

<220>
<221>  misc_feature
<222>  (725)..(725)
<223>  n = a,g,c, or t

<400>  3
cagtctcaag tggaagcccc attctacgag gagtggtggt tcctcctggt g atg gct       57
                                                         Met Ala
                                                         1

ctg tcc agc ctg atc gtc atc ctg ctg gtg gtg ttc gcc ctc gtc ctg       105
Leu Ser Ser Leu Ile Val Ile Leu Leu Val Val Phe Ala Leu Val Leu
        5                   10                  15

cac ggg cag aat aag aag tat aag aac tgc agc aca gga aag ggg atc       153
His Gly Gln Asn Lys Lys Tyr Lys Asn Cys Ser Thr Gly Lys Gly Ile
    20                  25                  30

tcc acc atg gag gag tct gtg acc ctg gac aac gga gga ttt gct gcc       201
Ser Thr Met Glu Glu Ser Val Thr Leu Asp Asn Gly Gly Phe Ala Ala
35                  40                  45                  50

ctg gag ctc agc agc cgc cac ctc aat gtc aag agc acc ttc tcc aag       249
Leu Glu Leu Ser Ser Arg His Leu Asn Val Lys Ser Thr Phe Ser Lys
                55                  60                  65

aag aac ggg acc agg tcc cca ccc cgg cct agc ccc ggc ggc ctg cac       297
Lys Asn Gly Thr Arg Ser Pro Pro Arg Pro Ser Pro Gly Gly Leu His
            70                  75                  80

tac tca gac gag gac atc tgc aac aag tac aac ggc gcc gtg ctg acc       345
Tyr Ser Asp Glu Asp Ile Cys Asn Lys Tyr Asn Gly Ala Val Leu Thr
        85                  90                  95

gag agc gtg agc ctc aag gag aag tcg gca gat gca tca gaa tct gag       393
Glu Ser Val Ser Leu Lys Glu Lys Ser Ala Asp Ala Ser Glu Ser Glu
    100                 105                 110

gcc acg gac tct gac tac gag gac gcg ctg ccc aag cac tcc ttc gtg       441
Ala Thr Asp Ser Asp Tyr Glu Asp Ala Leu Pro Lys His Ser Phe Val
115                 120                 125                 130

aac cac tac atg agc gac ccc acc tac tac aac tca tgg aag cgc agg       489
Asn His Tyr Met Ser Asp Pro Thr Tyr Tyr Asn Ser Trp Lys Arg Arg
                135                 140                 145

gcc cag ggc ccg ngc acc tgc gcc gca cag gta cga ggc ggt ggc ggg       537
Ala Gln Gly Pro Xaa Thr Cys Ala Ala Gln Val Arg Gly Gly Gly Gly
            150                 155                 160

ctc cga ggc ggg cgc gca gct gca ccc ggt cat cac cac gca gag cgc       585
Leu Arg Gly Gly Arg Ala Ala Ala Pro Gly His His His Ala Glu Arg
        165                 170                 175

ggg cgg cgt cta cac ccc cgc tgg ccc cgg cgc gcg aac tcc gct cac       633

Gly Arg Arg Leu His Pro Arg Trp Pro Arg Arg Ala Asn Ser Ala His
    180                 185                 190

cgg ctt ctc ctc ctt cgt gtg agc aaa gcn ccg cgc ctc cct cag ggc      681
Arg Leu Leu Leu Leu Arg Val Ser Lys Ala Pro Arg Leu Pro Gln Gly
195                 200                 205                 210

gga acg gag gca act ttc cgg agt cta ttt ttg tta aga caa tnc aac      729
Gly Thr Glu Ala Thr Phe Arg Ser Leu Phe Leu Leu Arg Gln Xaa Asn
                215                 220                 225

tcc aat aac tga gctgaagttt ttgtttaaaa ag                             763
Ser Asn Asn

<210>  4
<211>  229
<212>  PRT
<213>  Homo sapiens

<220>
<221>  misc_feature
<222>  (151)..(151)
<223>  The 'Xaa' at location 151 stands for Ser, Gly, Arg, or Cys.

<220>
<221>  misc_feature
<222>  (225)..(225)
<223>  The 'Xaa' at location 225 stands for Tyr, Cys, Ser, or Phe.

<400>  4

Met Ala Leu Ser Ser Leu Ile Val Ile Leu Leu Val Val Phe Ala Leu
1               5                   10                  15

Val Leu His Gly Gln Asn Lys Lys Tyr Lys Asn Cys Ser Thr Gly Lys
            20                  25                  30

Gly Ile Ser Thr Met Glu Glu Ser Val Thr Leu Asp Asn Gly Gly Phe
        35                  40                  45

Ala Ala Leu Glu Leu Ser Ser Arg His Leu Asn Val Lys Ser Thr Phe
    50                  55                  60

Ser Lys Lys Asn Gly Thr Arg Ser Pro Pro Arg Pro Ser Pro Gly Gly
65                  70                  75                  80

Leu His Tyr Ser Asp Glu Asp Ile Cys Asn Lys Tyr Asn Gly Ala Val
                85                  90                  95

Leu Thr Glu Ser Val Ser Leu Lys Glu Lys Ser Ala Asp Ala Ser Glu
            100                 105                 110

Ser Glu Ala Thr Asp Ser Asp Tyr Glu Asp Ala Leu Pro Lys His Ser
        115                 120                 125

Phe Val Asn His Tyr Met Ser Asp Pro Thr Tyr Tyr Asn Ser Trp Lys
    130                 135                 140

Arg Arg Ala Gln Gly Pro Xaa Thr Cys Ala Ala Gln Val Arg Gly Gly
145                 150                 155                 160

Gly Gly Leu Arg Gly Gly Arg Ala Ala Ala Pro Gly His His His Ala
                165                 170                 175

Glu Arg Gly Arg Arg Leu His Pro Arg Trp Pro Arg Arg Ala Asn Ser
            180                 185                 190

Ala His Arg Leu Leu Leu Leu Arg Val Ser Lys Ala Pro Arg Leu Pro
        195                 200                 205

Gln Gly Gly Thr Glu Ala Thr Phe Arg Ser Leu Phe Leu Leu Arg Gln
    210                 215                 220

Xaa Asn Ser Asn Asn
225

<210>  5
<211>  769
<212>  DNA
<213>  Homo sapiens

<220>
<221>  misc_feature
<222>  (1)..(1)
<223>  LP263b

<220>
<221>  CDS
<222>  (52)..(591)

<220>
<221>  misc_feature
<222>  (269)..(269)
<223>  n=a,c,g, or t

<220>
<221>  misc_feature
<222>  (351)..(351)
<223>  n=a,c,g, or t

<220>
<221>  misc_feature
<222>  (428)..(428)
<223>  n=a,c,g, or t

<400>  5
cagtctcaag tggaagcccc attctacgag gagtggtggt tcctcctggt g atg gct      57
                                                         Met Ala
                                                         1

ctg tcc agc ctg atc gtc atc ctg ctg gtg gtg ttc gcc ctc gtc ctg      105
Leu Ser Ser Leu Ile Val Ile Leu Leu Val Val Phe Ala Leu Val Leu
        5                   10                  15

cac ggg cag aat aag aag tat aag aac tgc agc aca gga aag ggg atc      153
His Gly Gln Asn Lys Lys Tyr Lys Asn Cys Ser Thr Gly Lys Gly Ile
    20                  25                  30

tcc acc atg gag gag tct gtg acc ctg gac aac gga gga ttt gct gcc      201
Ser Thr Met Glu Glu Ser Val Thr Leu Asp Asn Gly Gly Phe Ala Ala
35                  40                  45                  50

ctg gag ctc agc agc cgc cac ctc aat gtc aag agc acc ttc tcc aag      249
Leu Glu Leu Ser Ser Arg His Leu Asn Val Lys Ser Thr Phe Ser Lys
                55                  60                  65

aag aac ggg acc agg tcc cna ccc cgg cct agc ccc ggc ggc ctg cac      297
Lys Asn Gly Thr Arg Ser Xaa Pro Arg Pro Ser Pro Gly Gly Leu His
            70                  75                  80

tac tca gac gag gac atc tgc aac aag tac aac ggc gcc gtg ctg acc      345
Tyr Ser Asp Glu Asp Ile Cys Asn Lys Tyr Asn Gly Ala Val Leu Thr
        85                  90                  95

gag agn gtg agc ctc aag gag aag tcg gca gat gca tca gaa tct gag      393
Glu Xaa Val Ser Leu Lys Glu Lys Ser Ala Asp Ala Ser Glu Ser Glu

100                     105                     110

gcc acg gac tct gac tac gag gac gcg ctg ccc ang cac tcc ttc gtg        441
Ala Thr Asp Ser Asp Tyr Glu Asp Ala Leu Pro Xaa His Ser Phe Val
115                 120                 125                 130

aac cac tac atg agc gac ccc acc tac tac aac tca tgg aag cgc agg        489
Asn His Tyr Met Ser Asp Pro Thr Tyr Tyr Asn Ser Trp Lys Arg Arg
                135                 140                 145

gcc cag ggc cgc gca cct gcg ccg cac agt gta gct atc ctc ctg acg        537
Ala Gln Gly Arg Ala Pro Ala Pro His Ser Val Ala Ile Leu Leu Thr
            150                 155                 160

agc aac ccg tct gcg tac cta agt atg gct ccc cgt ggg tca gcg tcc        585
Ser Asn Pro Ser Ala Tyr Leu Ser Met Ala Pro Arg Gly Ser Ala Ser
        165                 170                 175

tgg tag catggatcca gtctgaaagg tgaggacaac gtggaaactc atgagctgag        641
Trp

cctgcccgct gggacacgtc tccttcccgc gtcaccttct ggtttaggga gccgtcaggt      701

ccctaaacgt tccctacaac tttttctgaa attgtgcaga aaaacagatc tcattaagag      761

aaaaagag                                                               769

<210>  6
<211>  179
<212>  PRT
<213>  Homo sapiens

<220>
<221>  misc_feature
<222>  (73)..(73)
<223>  The 'Xaa' at location 73 stands for Gln, Arg, Pro, or Leu.

<220>
<221>  misc_feature
<222>  (100)..(100)
<223>  The 'Xaa' at location 100 stands for Arg, or Ser.

<220>
<221>  misc_feature
<222>  (126)..(126)
<223>  The 'Xaa' at location 126 stands for Lys, Arg, Thr, or Met.

<400>  6

Met Ala Leu Ser Ser Leu Ile Val Ile Leu Leu Val Val Phe Ala Leu
1               5                   10                  15

Val Leu His Gly Gln Asn Lys Lys Tyr Lys Asn Cys Ser Thr Gly Lys
            20                  25                  30

Gly Ile Ser Thr Met Glu Glu Ser Val Thr Leu Asp Asn Gly Gly Phe
        35                  40                  45

Ala Ala Leu Glu Leu Ser Ser Arg His Leu Asn Val Lys Ser Thr Phe
    50                  55                  60

Ser Lys Lys Asn Gly Thr Arg Ser Xaa Pro Arg Pro Ser Pro Gly Gly
65                  70                  75                  80

Leu His Tyr Ser Asp Glu Asp Ile Cys Asn Lys Tyr Asn Gly Ala Val
                85                  90                  95

Leu Thr Glu Xaa Val Ser Leu Lys Glu Lys Ser Ala Asp Ala Ser Glu
            100                 105                 110

Ser Glu Ala Thr Asp Ser Asp Tyr Glu Asp Ala Leu Pro Xaa His Ser
        115                 120                 125

Phe Val Asn His Tyr Met Ser Asp Pro Thr Tyr Tyr Asn Ser Trp Lys
    130                 135                 140

Arg Arg Ala Gln Gly Arg Ala Pro Ala Pro His Ser Val Ala Ile Leu
145                 150                 155                 160

Leu Thr Ser Asn Pro Ser Ala Tyr Leu Ser Met Ala Pro Arg Gly Ser
                165                 170                 175

Ala Ser Trp

<210>  7
<211>  616
<212>  DNA
<213>  Homo sapiens

<220>
<221>  misc_feature
<222>  (1)..(1)
<223>  LP264

<220>
<221>  CDS
<222>  (20)..(427)

<220>
<221>  misc_feature
<222>  (229)..(229)
<223>  n=a,g,c, or t

<400>  7
cacacatcca ccttaatct gac tca ggc atg ttc ttc aca tta ata ctg tca      52
                     Asp Ser Gly Met Phe Phe Thr Leu Ile Leu Ser
                     1               5                   10

ttt cag cta ctg agt tat ttt ttc tcc tct ttt ttt ttt ttt ttg gca      100
Phe Gln Leu Leu Ser Tyr Phe Phe Ser Ser Phe Phe Phe Phe Leu Ala
            15                  20                  25

gca aag ccg ttg cct ggc ctt gaa gag aaa cca cca ggt tgt ttg gta      148
Ala Lys Pro Leu Pro Gly Leu Glu Glu Lys Pro Pro Gly Cys Leu Val
        30                  35                  40

ctt tca gaa ggg ggt ctt acc tct cac aca act tac ctt cac ccc aca      196
Leu Ser Glu Gly Gly Leu Thr Ser His Thr Thr Tyr Leu His Pro Thr
    45                  50                  55

cct ctc ccc tgt gca gac gtg act gtg cac can ggc aga gct ctc acc      244
Pro Leu Pro Cys Ala Asp Val Thr Val His Xaa Gly Arg Ala Leu Thr
60                  65                  70                  75

aca aca aaa ggg aaa cag gtg ggc ccg gga cgc tct caa aag cag ctc      292
Thr Thr Lys Gly Lys Gln Val Gly Pro Gly Arg Ser Gln Lys Gln Leu
                80                  85                  90

tcc tct ccg aat ctc ttg cga gac aag tgt gtt aaa gcg caa tta gaa      340
Ser Ser Pro Asn Leu Leu Arg Asp Lys Cys Val Lys Ala Gln Leu Glu
            95                  100                 105

ggg caa gaa atc tcc ctg tgg cgc cct gcg gat tat cgc agc tat atc      388
Gly Gln Glu Ile Ser Leu Trp Arg Pro Ala Asp Tyr Arg Ser Tyr Ile
        110                 115                 120

ccg cgg cgc cga gcc ggc gcc aag ggg tgt cgt cgt tag tagagaaacc      437
Pro Arg Arg Arg Ala Gly Ala Lys Gly Cys Arg Arg

125                 130                 135

tctctctctt atcggtgtgt gcaagaacag ggggccgcct cttatctcca gaagaaccga     497

gagtctcata tgtggggaga aagacattct cttcctttgt gtgagatctc gagaagggag     557

acaggcgcct ttgtgttagc acgtgtggtt ctccagaaaa aacgcttgtc ttctgtgcc      616


<210>  8
<211>  135
<212>  PRT
<213>  Homo sapiens

<220>
<221>  misc_feature
<222>  (70)..(70)
<223>  The 'Xaa' at location 70 stands for Gln, or His.

<400>  8

Asp Ser Gly Met Phe Phe Thr Leu Ile Leu Ser Phe Gln Leu Leu Ser
1               5                   10                  15


Tyr Phe Phe Ser Ser Phe Phe Phe Phe Leu Ala Ala Lys Pro Leu Pro
            20                  25                  30


Gly Leu Glu Glu Lys Pro Pro Gly Cys Leu Val Leu Ser Glu Gly Gly
        35                  40                  45


Leu Thr Ser His Thr Thr Tyr Leu His Pro Thr Pro Leu Pro Cys Ala
    50                  55                  60


Asp Val Thr Val His Xaa Gly Arg Ala Leu Thr Thr Thr Lys Gly Lys
65                  70                  75                  80


Gln Val Gly Pro Gly Arg Ser Gln Lys Gln Leu Ser Ser Pro Asn Leu
                85                  90                  95


Leu Arg Asp Lys Cys Val Lys Ala Gln Leu Glu Gly Gln Glu Ile Ser
            100                 105                 110


Leu Trp Arg Pro Ala Asp Tyr Arg Ser Tyr Ile Pro Arg Arg Arg Ala
        115                 120                 125


Gly Ala Lys Gly Cys Arg Arg
    130                 135


<210>  9
<211>  660
<212>  DNA
<213>  Homo sapiens


<220>
<221>  misc_feature
<222>  (1)..(1)
<223>  LP265

<220>
<221>  CDS
<222>  (87)..(395)

<220>
<221>  misc_feature
<222>  (419)..(419)
<223>  n=a,g,c, or t

<400>  9
tgtcgggttt ttcggccatc cttcttgaac ttttgacgcg ttccgatttc tttggtgatt      60

gccttcgctt cacgggtgtg cgcgtg atg ccc tta att gga ata ctt atc gtg      113
                             Met Pro Leu Ile Gly Ile Leu Ile Val
                             1               5

ccc atg tcc aaa cct gcg ggg ctc ttt ttt ctt acc gcg ctt tgc tct      161
Pro Met Ser Lys Pro Ala Gly Leu Phe Phe Leu Thr Ala Leu Cys Ser
10                  15                  20                  25

tgg ccc ctc tct ttg ccc ttg tgc cgt ctg tct gcc ctt cac aac tcg      209
Trp Pro Leu Ser Leu Pro Leu Cys Arg Leu Ser Ala Leu His Asn Ser
                30                  35                  40

ttt tct ttc tgt ctc cgt gac tcg tta gtt ctc ctc tgt gaa ttt tcc      257
Phe Ser Phe Cys Leu Arg Asp Ser Leu Val Leu Leu Cys Glu Phe Ser
            45                  50                  55

ttt gtg gga tta tac gcg gta att tac tcc tgc ctt ctt ctg atg ttg      305
Phe Val Gly Leu Tyr Ala Val Ile Tyr Ser Cys Leu Leu Leu Met Leu
        60                  65                  70

att gcc tgg att agc tct ggt ctt cct gct ctt gct atg ctc gaa tct      353
Ile Ala Trp Ile Ser Ser Gly Leu Pro Ala Leu Ala Met Leu Glu Ser
    75                  80                  85

tgt agc ttt cga gtg atg cat ttg cgt gag ttg cga agt tga              395
Cys Ser Phe Arg Val Met His Leu Arg Glu Leu Arg Ser
90                  95                  100

gtgcgagacg cgagatgcag cganatgttg cagccacaag gtacacagac ctatcgcatc     455

ctatgactga gccgaatgtc cgcgactctc attctacatt gtctacgcct taggacatac     515

gtaacaagag tttattacgc agggcctagt gggaattctg tgacaagtga atgcgtcaaa     575

gctgctagat gtgaatcttg gtaggttaac attccaacgt acgagtgtag tgctacaccg     635

caggcatttg acattttatg tccca                                           660


<210>  10
<211>  102
<212>  PRT
<213>  Homo sapiens

<400>  10

Met Pro Leu Ile Gly Ile Leu Ile Val Pro Met Ser Lys Pro Ala Gly
1               5                   10                  15

Leu Phe Phe Leu Thr Ala Leu Cys Ser Trp Pro Leu Ser Leu Pro Leu
            20                  25                  30

Cys Arg Leu Ser Ala Leu His Asn Ser Phe Ser Phe Cys Leu Arg Asp
        35                  40                  45

Ser Leu Val Leu Leu Cys Glu Phe Ser Phe Val Gly Leu Tyr Ala Val
    50                  55                  60

Ile Tyr Ser Cys Leu Leu Leu Met Leu Ile Ala Trp Ile Ser Ser Gly
65                  70                  75                  80

Leu Pro Ala Leu Ala Met Leu Glu Ser Cys Ser Phe Arg Val Met His
                85                  90                  95

Leu Arg Glu Leu Arg Ser
            100

<210>  11
<211>  3287
<212>  DNA
<213>  Homo sapiens

<220>
<221>  misc_feature
<222>  (1)..(1)
<223>  LP283

<220>
<221>  CDS
<222>  (157)..(2850)

<400>  11
ggcgccgcgg tccgcgcccc gcgactgcag cccccggcct ggccccggcg gggcgccccc      60

tcccctcccc ctcctgcgag ctgggatccg gccggcttcc gccctcccct ggccgcgaga     120

ccggccccgg cggctgggcc gccagtagct ccagcc atg ggc tcg ggg cgc gta      174
                                        Met Gly Ser Gly Arg Val
                                        1               5

ccc ggg ctc tgc ctg ctt gtc ctg ctg gtc cac gcc cgc gcc gcc cag      222
Pro Gly Leu Cys Leu Leu Val Leu Leu Val His Ala Arg Ala Ala Gln
            10                  15                  20

tac agc aaa gcc gcg caa gat gtg gat gag tgt gtg gag ggg act gac      270
Tyr Ser Lys Ala Ala Gln Asp Val Asp Glu Cys Val Glu Gly Thr Asp
        25                  30                  35

aac tgc cac atc gat gct atc tgc cag aac acc ccg agg tca tac aag      318
Asn Cys His Ile Asp Ala Ile Cys Gln Asn Thr Pro Arg Ser Tyr Lys
    40                  45                  50

tgc atc tgc aag tct ggc tac aca ggg gac ggc aaa cac tgc aaa gac      366
Cys Ile Cys Lys Ser Gly Tyr Thr Gly Asp Gly Lys His Cys Lys Asp
55                  60                  65                  70

gtg gat gag tgc gag cga gag gat aat gca ggt tgt gtg cat gac tgt      414
Val Asp Glu Cys Glu Arg Glu Asp Asn Ala Gly Cys Val His Asp Cys
                75                  80                  85

gtc aac atc cct ggc aat tac cgg tgt acc tgc tat gat gga ttc cac      462
Val Asn Ile Pro Gly Asn Tyr Arg Cys Thr Cys Tyr Asp Gly Phe His
            90                  95                  100

ctg gca cat gac gga cac aac tgt ctg gat gtg gac gag tgt gcc gag      510
Leu Ala His Asp Gly His Asn Cys Leu Asp Val Asp Glu Cys Ala Glu
        105                 110                 115

ggc aac ggc ggc tgt cag cag agc tgt gtc aac atg atg ggc agc tat      558
Gly Asn Gly Gly Cys Gln Gln Ser Cys Val Asn Met Met Gly Ser Tyr
    120                 125                 130

gag tgc cac tgc cgg gaa ggc ttc ttc ctc agc gac aac cag cat acc      606
Glu Cys His Cys Arg Glu Gly Phe Phe Leu Ser Asp Asn Gln His Thr
135                 140                 145                 150

tgt atc cag cgg cca gaa gaa gga atg aat tgc atg aac aag aac cac      654
Cys Ile Gln Arg Pro Glu Glu Gly Met Asn Cys Met Asn Lys Asn His
                155                 160                 165

ggc tgt gcc cac att tgc cgg gag aca ccc aag ggg ggt att gcc tgt      702
Gly Cys Ala His Ile Cys Arg Glu Thr Pro Lys Gly Gly Ile Ala Cys
            170                 175                 180

gaa tgc cgt cct ggc ttt gag ctt acc aag aac caa cgg gac tgt aaa      750
Glu Cys Arg Pro Gly Phe Glu Leu Thr Lys Asn Gln Arg Asp Cys Lys
        185                 190                 195

tgt gag ata att ggg atg gca gtg aca tgc aac tat ggt aac ggc ggc      798
Cys Glu Ile Ile Gly Met Ala Val Thr Cys Asn Tyr Gly Asn Gly Gly
    200                 205                 210

tgc cag cac acg tgt gat gac aca gag cag ggt ccc cgg tgc ggc tgc      846

Cys Gln His Thr Cys Asp Asp Thr Glu Gln Gly Pro Arg Cys Gly Cys
215                 220                 225                 230

cat atc aag ttt gtg ctc cat acc gac ggg aag aca tgc atc gag acc      894
His Ile Lys Phe Val Leu His Thr Asp Gly Lys Thr Cys Ile Glu Thr
                235                 240                 245

tgt gct gtc aac aac ggg ggc tgt gac agt aag tgc cat gat gca gcg      942
Cys Ala Val Asn Asn Gly Gly Cys Asp Ser Lys Cys His Asp Ala Ala
            250                 255                 260

act ggt gtc cac tgc acc tgc cct gtg ggc ttc atg ctg cag cca gac      990
Thr Gly Val His Cys Thr Cys Pro Val Gly Phe Met Leu Gln Pro Asp
        265                 270                 275

agg aag acg tgc aaa gat ata gat gag tgc cgc tta aac aac ggg ggc     1038
Arg Lys Thr Cys Lys Asp Ile Asp Glu Cys Arg Leu Asn Asn Gly Gly
    280                 285                 290

tgt gac cat att tgc cgc aac aca gtg ggc agc ttc gaa tgc agt tgc     1086
Cys Asp His Ile Cys Arg Asn Thr Val Gly Ser Phe Glu Cys Ser Cys
295                 300                 305                 310

aag aaa ggc tat aag ctt ctc atc aat gag agg aac tgc cag gat ata     1134
Lys Lys Gly Tyr Lys Leu Leu Ile Asn Glu Arg Asn Cys Gln Asp Ile
                315                 320                 325

gac gag tgt tcc ttt gat cga acc tgt gac cac ata tgt gtc aac aca     1182
Asp Glu Cys Ser Phe Asp Arg Thr Cys Asp His Ile Cys Val Asn Thr
            330                 335                 340

cca gga agc ttc cag tgt ctc tgc cat cgt ggc tac ctg ttg tat ggt     1230
Pro Gly Ser Phe Gln Cys Leu Cys His Arg Gly Tyr Leu Leu Tyr Gly
        345                 350                 355

atc acc cac tgt ggg gat gtg gat gaa tgc agc atc aac cgg gga ggt     1278
Ile Thr His Cys Gly Asp Val Asp Glu Cys Ser Ile Asn Arg Gly Gly
    360                 365                 370

tgc cgc ttt ggc tgc atc aac act cct ggc agc tac cag tgt acc tgc     1326
Cys Arg Phe Gly Cys Ile Asn Thr Pro Gly Ser Tyr Gln Cys Thr Cys
375                 380                 385                 390

cca gca ggc cag ggt cgg ctg cac tgg aat ggc aaa gat tgc aca gag     1374
Pro Ala Gly Gln Gly Arg Leu His Trp Asn Gly Lys Asp Cys Thr Glu
                395                 400                 405

cca ctg aag tgt cag ggc agt cct ggg gcc tcg aaa gcc atg ctc agc     1422
Pro Leu Lys Cys Gln Gly Ser Pro Gly Ala Ser Lys Ala Met Leu Ser
            410                 415                 420

tgc aac cgg tct ggc aag aag gac acc tgt gcc ctg acc tgt ccc tcc     1470
Cys Asn Arg Ser Gly Lys Lys Asp Thr Cys Ala Leu Thr Cys Pro Ser
        425                 430                 435

agg gcc cga ttt ttg cca gag tct gag aat ggc ttc acg gtg agc tgt     1518
Arg Ala Arg Phe Leu Pro Glu Ser Glu Asn Gly Phe Thr Val Ser Cys
    440                 445                 450

ggg acc ccc agc ccc agg gct gct cca gcc cga gct ggc cac aat ggg     1566
Gly Thr Pro Ser Pro Arg Ala Ala Pro Ala Arg Ala Gly His Asn Gly
455                 460                 465                 470

aac agc acc aac tcc aac cac tgc cat gag gct gca gtg ctg tcc att     1614
Asn Ser Thr Asn Ser Asn His Cys His Glu Ala Ala Val Leu Ser Ile
                475                 480                 485

aaa caa cgg gcc tcc ttc aag atc aag gat gcc aaa tgc cgt ttg cac     1662
Lys Gln Arg Ala Ser Phe Lys Ile Lys Asp Ala Lys Cys Arg Leu His
            490                 495                 500

ctg cga aac aaa ggc aaa aca gag gag gct ggc aga atc aca ggg cca     1710
Leu Arg Asn Lys Gly Lys Thr Glu Glu Ala Gly Arg Ile Thr Gly Pro
        505                 510                 515

ggt ggt gcc ccc tgc tct gaa tgc cag gtc acc ttc atc cac ctt aag     1758
Gly Gly Ala Pro Cys Ser Glu Cys Gln Val Thr Phe Ile His Leu Lys

520                 525                 530

tgt gac tcc tct cgg aag ggc aag ggc cga cgg gcc cgg acc cct cca      1806
Cys Asp Ser Ser Arg Lys Gly Lys Gly Arg Arg Ala Arg Thr Pro Pro
535                 540                 545                 550

ggc aaa gag gtc aca agg ctc acc ctg gaa ctg gag gca gag caa ctc      1854
Gly Lys Glu Val Thr Arg Leu Thr Leu Glu Leu Glu Ala Glu Gln Leu
                555                 560                 565

ttt ctc ctc cct gat aca cac ggc cat cca cca cca gcc agc tgt ggg      1902
Phe Leu Leu Pro Asp Thr His Gly His Pro Pro Pro Ala Ser Cys Gly
            570                 575                 580

ctg ccc tgc ctc cga cag cga atg gaa cgg cgg ctg aaa gga tcc ctg      1950
Leu Pro Cys Leu Arg Gln Arg Met Glu Arg Arg Leu Lys Gly Ser Leu
        585                 590                 595

aag atg ctc aga aag tcc atc aac cag gac cgc ttc ctg ctg cgc ctg      1998
Lys Met Leu Arg Lys Ser Ile Asn Gln Asp Arg Phe Leu Leu Arg Leu
    600                 605                 610

gca ggc ctt gat tat gag ctg gcc cac aag ccg ggc ctg gta gcc ggg      2046
Ala Gly Leu Asp Tyr Glu Leu Ala His Lys Pro Gly Leu Val Ala Gly
615                 620                 625                 630

gag cga gca gag ccg atg gag tcc tgt agg ccc ggg cag cac cgt gct      2094
Glu Arg Ala Glu Pro Met Glu Ser Cys Arg Pro Gly Gln His Arg Ala
                635                 640                 645

ggg acc aag tgt gtc cag tgc tcc cca ggg cac tac tac aac acc agc      2142
Gly Thr Lys Cys Val Gln Cys Ser Pro Gly His Tyr Tyr Asn Thr Ser
            650                 655                 660

atc cac cgc tgt att cgc tgt gcc atg ggc tcc tat cag ccc gac ttc      2190
Ile His Arg Cys Ile Arg Cys Ala Met Gly Ser Tyr Gln Pro Asp Phe
        665                 670                 675

cgt cag aac ttc tgc agc cgc tgt cca gga aac aca agc aca gac ttt      2238
Arg Gln Asn Phe Cys Ser Arg Cys Pro Gly Asn Thr Ser Thr Asp Phe
    680                 685                 690

gat ggc tct acc agt gtg gcc caa tgc aag aat cgt cag tgt ggt ggg      2286
Asp Gly Ser Thr Ser Val Ala Gln Cys Lys Asn Arg Gln Cys Gly Gly
695                 700                 705                 710

gag ctg ggt gag ttc act ggc tat att gag tcc ccc aac tac ccg ggc      2334
Glu Leu Gly Glu Phe Thr Gly Tyr Ile Glu Ser Pro Asn Tyr Pro Gly
                715                 720                 725

aac tac cca gct ggt gtg gag tgc atc tgg aac atc aac ccc cca ccc      2382
Asn Tyr Pro Ala Gly Val Glu Cys Ile Trp Asn Ile Asn Pro Pro Pro
            730                 735                 740

aag cgc aag atc ctt atc gtg gta cca gag atc ttc ctg cca tct gag      2430
Lys Arg Lys Ile Leu Ile Val Val Pro Glu Ile Phe Leu Pro Ser Glu
        745                 750                 755

gat gag tgt ggg gac gtc ctc gtc atg aga aag aac tca tcc cca tcc      2478
Asp Glu Cys Gly Asp Val Leu Val Met Arg Lys Asn Ser Ser Pro Ser
    760                 765                 770

tcc att acc act tat gag acc tgc cag acc tac gag cgt ccc att gcc      2526
Ser Ile Thr Thr Tyr Glu Thr Cys Gln Thr Tyr Glu Arg Pro Ile Ala
775                 780                 785                 790

ttc act gcc cgt tcc agg aag ctc tgg atc aac ttc aag aca agc gag      2574
Phe Thr Ala Arg Ser Arg Lys Leu Trp Ile Asn Phe Lys Thr Ser Glu
                795                 800                 805

gcc aac agc gcc cgt ggc ttc cag att ccc tat gtt acc tat gat gag      2622
Ala Asn Ser Ala Arg Gly Phe Gln Ile Pro Tyr Val Thr Tyr Asp Glu
            810                 815                 820

gac tat gag cag ctg gta gaa gac att gtg cga gat ggc cgg ctc tat      2670
Asp Tyr Glu Gln Leu Val Glu Asp Ile Val Arg Asp Gly Arg Leu Tyr
        825                 830                 835

gcc tct gaa aac cac cag gag att tta aag gac aag aag ctc atc aag     2718
Ala Ser Glu Asn His Gln Glu Ile Leu Lys Asp Lys Lys Leu Ile Lys
    840                 845                 850

gcc ttc ttt gag gtg cta gcc cac ccc cag aac tac ttc aag tac aca     2766
Ala Phe Phe Glu Val Leu Ala His Pro Gln Asn Tyr Phe Lys Tyr Thr
855                 860                 865                 870

gag aaa cac aag gag atg ctg cca aaa tcc ttc atc aag ctg ctc cgc     2814
Glu Lys His Lys Glu Met Leu Pro Lys Ser Phe Ile Lys Leu Leu Arg
                875                 880                 885

tcc aaa gtt tcc agc ttc ctg agg ccc tac aaa tag taaccctagg          2860
Ser Lys Val Ser Ser Phe Leu Arg Pro Tyr Lys
            890                 895

ctcagagacc caatttttta agcccccaga ctccttagcc ctcagagccg gcagccccct   2920

accctcagac aaggaactct ctcctctctt tttggaggga aaaaaaaaat atcactacac   2980

aaaccaggca ctctcccttt ctgtctttct agtttccttt ccttgtctct ctctgcctgc   3040

ctctctactg ttcccccttt tctaacacac tacctagaaa agccattcag tactggctct   3100

agtccccgtg agatgtaaag aaacagtaca gccccttcca ctgcccattt taccagctca   3160

cattcccgac cccatcagct tggaagggtg ctagaggccc atcaaggaag tgggtctggt   3220

gggaaacggg gaggggaaag aagggcttct gccattatag ggttgtgcct tgctagtcag   3280

gggccaa                                                             3287

<210>  12
<211>  897
<212>  PRT
<213>  Homo sapiens

<400>  12

Met Gly Ser Gly Arg Val Pro Gly Leu Cys Leu Leu Val Leu Leu Val
1               5                   10                  15

His Ala Arg Ala Ala Gln Tyr Ser Lys Ala Ala Gln Asp Val Asp Glu
            20                  25                  30

Cys Val Glu Gly Thr Asp Asn Cys His Ile Asp Ala Ile Cys Gln Asn
        35                  40                  45

Thr Pro Arg Ser Tyr Lys Cys Ile Cys Lys Ser Gly Tyr Thr Gly Asp
    50                  55                  60

Gly Lys His Cys Lys Asp Val Asp Glu Cys Glu Arg Glu Asp Asn Ala
65                  70                  75                  80

Gly Cys Val His Asp Cys Val Asn Ile Pro Gly Asn Tyr Arg Cys Thr
                85                  90                  95

Cys Tyr Asp Gly Phe His Leu Ala His Asp Gly His Asn Cys Leu Asp
            100                 105                 110

Val Asp Glu Cys Ala Glu Gly Asn Gly Gly Cys Gln Gln Ser Cys Val
        115                 120                 125

Asn Met Met Gly Ser Tyr Glu Cys His Cys Arg Glu Gly Phe Phe Leu
    130                 135                 140

Ser Asp Asn Gln His Thr Cys Ile Gln Arg Pro Glu Glu Gly Met Asn
145                 150                 155                 160

Cys Met Asn Lys Asn His Gly Cys Ala His Ile Cys Arg Glu Thr Pro
                165                 170                 175

Lys Gly Gly Ile Ala Cys Glu Cys Arg Pro Gly Phe Glu Leu Thr Lys
            180                 185                 190

Asn Gln Arg Asp Cys Lys Cys Glu Ile Ile Gly Met Ala Val Thr Cys
        195                 200                 205

Asn Tyr Gly Asn Gly Gly Cys Gln His Thr Cys Asp Asp Thr Glu Gln
    210                 215                 220

Gly Pro Arg Cys Gly Cys His Ile Lys Phe Val Leu His Thr Asp Gly
225                 230                 235                 240

Lys Thr Cys Ile Glu Thr Cys Ala Val Asn Asn Gly Gly Cys Asp Ser
                245                 250                 255

Lys Cys His Asp Ala Ala Thr Gly Val His Cys Thr Cys Pro Val Gly
            260                 265                 270

Phe Met Leu Gln Pro Asp Arg Lys Thr Cys Lys Asp Ile Asp Glu Cys
        275                 280                 285

Arg Leu Asn Asn Gly Gly Cys Asp His Ile Cys Arg Asn Thr Val Gly
    290                 295                 300

Ser Phe Glu Cys Ser Cys Lys Lys Gly Tyr Lys Leu Leu Ile Asn Glu
305                 310                 315                 320

Arg Asn Cys Gln Asp Ile Asp Glu Cys Ser Phe Asp Arg Thr Cys Asp
                325                 330                 335

His Ile Cys Val Asn Thr Pro Gly Ser Phe Gln Cys Leu Cys His Arg
            340                 345                 350

Gly Tyr Leu Leu Tyr Gly Ile Thr His Cys Gly Asp Val Asp Glu Cys
        355                 360                 365

Ser Ile Asn Arg Gly Gly Cys Arg Phe Gly Cys Ile Asn Thr Pro Gly
    370                 375                 380

Ser Tyr Gln Cys Thr Cys Pro Ala Gly Gln Gly Arg Leu His Trp Asn
385                 390                 395                 400

Gly Lys Asp Cys Thr Glu Pro Leu Lys Cys Gln Gly Ser Pro Gly Ala
                405                 410                 415

Ser Lys Ala Met Leu Ser Cys Asn Arg Ser Gly Lys Lys Asp Thr Cys
            420                 425                 430

Ala Leu Thr Cys Pro Ser Arg Ala Arg Phe Leu Pro Glu Ser Glu Asn
        435                 440                 445

Gly Phe Thr Val Ser Cys Gly Thr Pro Ser Pro Arg Ala Ala Pro Ala

450                 455                 460

Arg Ala Gly His Asn Gly Asn Ser Thr Asn Ser Asn His Cys His Glu
465                 470                 475                 480

Ala Ala Val Leu Ser Ile Lys Gln Arg Ala Ser Phe Lys Ile Lys Asp
                485                 490                 495

Ala Lys Cys Arg Leu His Leu Arg Asn Lys Gly Lys Thr Glu Glu Ala
            500                 505                 510

Gly Arg Ile Thr Gly Pro Gly Gly Ala Pro Cys Ser Glu Cys Gln Val
        515                 520                 525

Thr Phe Ile His Leu Lys Cys Asp Ser Ser Arg Lys Gly Lys Gly Arg
    530                 535                 540

Arg Ala Arg Thr Pro Pro Gly Lys Glu Val Thr Arg Leu Thr Leu Glu
545                 550                 555                 560

Leu Glu Ala Glu Gln Leu Phe Leu Leu Pro Asp Thr His Gly His Pro
                565                 570                 575

Pro Pro Ala Ser Cys Gly Leu Pro Cys Leu Arg Gln Arg Met Glu Arg
            580                 585                 590

Arg Leu Lys Gly Ser Leu Lys Met Leu Arg Lys Ser Ile Asn Gln Asp
        595                 600                 605

Arg Phe Leu Leu Arg Leu Ala Gly Leu Asp Tyr Glu Leu Ala His Lys
    610                 615                 620

Pro Gly Leu Val Ala Gly Glu Arg Ala Glu Pro Met Glu Ser Cys Arg
625                 630                 635                 640

Pro Gly Gln His Arg Ala Gly Thr Lys Cys Val Gln Cys Ser Pro Gly
                645                 650                 655

His Tyr Tyr Asn Thr Ser Ile His Arg Cys Ile Arg Cys Ala Met Gly
            660                 665                 670

Ser Tyr Gln Pro Asp Phe Arg Gln Asn Phe Cys Ser Arg Cys Pro Gly
        675                 680                 685

Asn Thr Ser Thr Asp Phe Asp Gly Ser Thr Ser Val Ala Gln Cys Lys
    690                 695                 700

Asn Arg Gln Cys Gly Gly Glu Leu Gly Glu Phe Thr Gly Tyr Ile Glu
705                 710                 715                 720

Ser Pro Asn Tyr Pro Gly Asn Tyr Pro Ala Gly Val Glu Cys Ile Trp
                725                 730                 735

Asn Ile Asn Pro Pro Pro Lys Arg Lys Ile Leu Ile Val Val Pro Glu
            740                 745                 750

Ile Phe Leu Pro Ser Glu Asp Glu Cys Gly Asp Val Leu Val Met Arg
        755                 760                 765

Lys Asn Ser Ser Pro Ser Ser Ile Thr Thr Tyr Glu Thr Cys Gln Thr
    770                 775                 780

Tyr Glu Arg Pro Ile Ala Phe Thr Ala Arg Ser Arg Lys Leu Trp Ile
785                 790                 795                 800

Asn Phe Lys Thr Ser Glu Ala Asn Ser Ala Arg Gly Phe Gln Ile Pro
                805                 810                 815

Tyr Val Thr Tyr Asp Glu Asp Tyr Glu Gln Leu Val Glu Asp Ile Val
            820                 825                 830

Arg Asp Gly Arg Leu Tyr Ala Ser Glu Asn His Gln Glu Ile Leu Lys
        835                 840                 845

Asp Lys Lys Leu Ile Lys Ala Phe Phe Glu Val Leu Ala His Pro Gln
    850                 855                 860

Asn Tyr Phe Lys Tyr Thr Glu Lys His Lys Glu Met Leu Pro Lys Ser
865                 870                 875                 880

Phe Ile Lys Leu Leu Arg Ser Lys Val Ser Ser Phe Leu Arg Pro Tyr
                885                 890                 895

Lys

<210>  13
<211>  2610
<212>  DNA
<213>  Homo sapiens

<220>
<221>  misc_feature
<222>  (1)..(1)
<223>  LP286

<220>
<221>  CDS
<222>  (532)..(2073)

<400>  13
ctgccgggtg tgccgggtgt ccagcgaacc cctttcccaa accttcgggg agaagggagg      60

tgggaggagg caaagaaact acaggcaggg agctggaagg gggggtgggg ggggcaggag     120

acaagaaatc aagacaccag gcagcaggac acacacacac tcacatacac tcacacacat     180

agagaccaac agatagacag ctacctaaag cctgaaagac tgacagcaac acagaaaaaa     240

agaaacaggc agaaagagag acaaagacag aaatagaaac agactaacac acagagtcaa     300

aaatacagag acagaaagac agggagaaag agaaacagaa aattagacac caaagacata     360

cgaacaggga ggaaggccga ctgaaagaaa gacggagaag aggagagaga agccagggcc     420

gagcgtgcca gcaggcggat ggagggcggc ctggtggagg aggagacgta gtggcctggg     480

ctgagctggg tgggccggga gaagcgggtg cctcagagtg ggggtggggg c atg gga      537
                                                         Met Gly
                                                         1

ggg gca ggc att ctg ctg ctg ctg ctg gct ggg gcg ggg gtg gtg gtg       585
Gly Ala Gly Ile Leu Leu Leu Leu Leu Ala Gly Ala Gly Val Val Val
        5                   10                  15

gcc tgg aga ccc cca aag gga aag tgt ccc ctg cgc tgc tcc tgc tct 633
Ala Trp Arg Pro Pro Lys Gly Lys Cys Pro Leu Arg Cys Ser Cys Ser
    20                  25                  30

aaa gac agc gcc ctg tgt gag ggc tcc ccg gac ctg ccc gtc agc ttc 681
Lys Asp Ser Ala Leu Cys Glu Gly Ser Pro Asp Leu Pro Val Ser Phe
35                  40                  45                  50

tct ccg acc ctg ctg tca ctc tca ctc gtc agg acg gga gtc acc cag 729
Ser Pro Thr Leu Leu Ser Leu Ser Leu Val Arg Thr Gly Val Thr Gln
                55                  60                  65

ctg aag gcc ggc agc ttc ctg aga att ccg tct ctg cac ctg ctc ttc 777
Leu Lys Ala Gly Ser Phe Leu Arg Ile Pro Ser Leu His Leu Leu Phe
            70                  75                  80

atc gag gac aat gag att ggc tcc atc tct aag aat gcc ctc aga gga 825
Ile Glu Asp Asn Glu Ile Gly Ser Ile Ser Lys Asn Ala Leu Arg Gly
        85                  90                  95

ctt cgc tcg ctt aca cac cta agc ctg gcc aat aac cat ctg gag acc 873
Leu Arg Ser Leu Thr His Leu Ser Leu Ala Asn Asn His Leu Glu Thr
    100                 105                 110

ctc ccc aga ttc ctg ttc cga ggc ctg gac acc ctt act cac gtg gac 921
Leu Pro Arg Phe Leu Phe Arg Gly Leu Asp Thr Leu Thr His Val Asp
115                 120                 125                 130

ctc cgc ggg aac ccg ttc cag tgt gac tgc cgc gtc ctc tgg ctc ctg 969
Leu Arg Gly Asn Pro Phe Gln Cys Asp Cys Arg Val Leu Trp Leu Leu
                135                 140                 145

cag tgg atg ccc acc gtg aat gcc agc gtg ggg acc ggc gcc tgt gcg 1017
Gln Trp Met Pro Thr Val Asn Ala Ser Val Gly Thr Gly Ala Cys Ala
            150                 155                 160

ggc ccc gcc tcc ctg agc cac atg cag ctc cac cac ctc gac ccc aag 1065
Gly Pro Ala Ser Leu Ser His Met Gln Leu His His Leu Asp Pro Lys
        165                 170                 175

act ttc aag tgc aga gcc ata gag ctg tcc tgg ttc cag acg gtg ggg 1113
Thr Phe Lys Cys Arg Ala Ile Glu Leu Ser Trp Phe Gln Thr Val Gly
    180                 185                 190

gag tcg gca ctg agc gta gag ccc ttc tcc tac caa ggg gag cct cac 1161
Glu Ser Ala Leu Ser Val Glu Pro Phe Ser Tyr Gln Gly Glu Pro His
195                 200                 205                 210

att gtg ctg gca cag ccc ttc gcc ggc cgc tgc ctg att ctc tcc tgg 1209
Ile Val Leu Ala Gln Pro Phe Ala Gly Arg Cys Leu Ile Leu Ser Trp
                215                 220                 225

gac tac agc ctg cag cgc ttc cgg ccc gag gaa gag ctg ccc gcg gcc 1257
Asp Tyr Ser Leu Gln Arg Phe Arg Pro Glu Glu Glu Leu Pro Ala Ala
            230                 235                 240

tcc gtg gtg tcc tgc aag cca ctg gtg ctg ggc ccg agc ctc ttc gtg 1305
Ser Val Val Ser Cys Lys Pro Leu Val Leu Gly Pro Ser Leu Phe Val
        245                 250                 255

ctg gct gcc cgc ctg tgg ggg ggc tca cag ctg tgg gcc cgg ccc agt 1353
Leu Ala Ala Arg Leu Trp Gly Gly Ser Gln Leu Trp Ala Arg Pro Ser
    260                 265                 270

ccc ggc ctg cgc ctg gcc cca acg cag acc ctg gcc ccg cgg cgg ctg 1401
Pro Gly Leu Arg Leu Ala Pro Thr Gln Thr Leu Ala Pro Arg Arg Leu
275                 280                 285                 290

ctg cgg ccc aat gac gcc gag ctc ctg tgg ctg gaa ggg caa ccc tgc 1449
Leu Arg Pro Asn Asp Ala Glu Leu Leu Trp Leu Glu Gly Gln Pro Cys
                295                 300                 305

ttc gtg gtg gcc gat gcc tcc aag gcg ggc agc acc acg ctg ctg tgc 1497
Phe Val Val Ala Asp Ala Ser Lys Ala Gly Ser Thr Thr Leu Leu Cys
            310                 315                 320

cgc gac ggg ccc ggc ttt tac ccg cac cag agc ctg cac gcc tgg cac     1545
Arg Asp Gly Pro Gly Phe Tyr Pro His Gln Ser Leu His Ala Trp His
        325                 330                 335

cgg gac acg gac gct gag gcc ctg gag ctg gac ggc cgg ccc cac ctg     1593
Arg Asp Thr Asp Ala Glu Ala Leu Glu Leu Asp Gly Arg Pro His Leu
    340                 345                 350

ctg ctg gcc tcg gct tcc cag cgg ccc gtg ctc ttc cac tgg acc ggt     1641
Leu Leu Ala Ser Ala Ser Gln Arg Pro Val Leu Phe His Trp Thr Gly
355                 360                 365                 370

ggc cgc ttc gag aga cgc aca gac atc ccc gag gcc gag gat gtc tat     1689
Gly Arg Phe Glu Arg Arg Thr Asp Ile Pro Glu Ala Glu Asp Val Tyr
                375                 380                 385

gcc aca cgc cac ttc cag gct ggt ggg gac gtg ttc ctg tgc ctc aca     1737
Ala Thr Arg His Phe Gln Ala Gly Gly Asp Val Phe Leu Cys Leu Thr
            390                 395                 400

cgc tac att ggg gac tcc atg gtc atg cgc tgg gac ggc tcc atg ttt     1785
Arg Tyr Ile Gly Asp Ser Met Val Met Arg Trp Asp Gly Ser Met Phe
        405                 410                 415

cgt ctg ctg cag caa ctt ccc tcg cgc ggt gcc cac gtc ttc cag cca     1833
Arg Leu Leu Gln Gln Leu Pro Ser Arg Gly Ala His Val Phe Gln Pro
    420                 425                 430

ctg ctc atc gcc agg gac cag ctg gcc atc cta ggc agc gac ttc gcc     1881
Leu Leu Ile Ala Arg Asp Gln Leu Ala Ile Leu Gly Ser Asp Phe Ala
435                 440                 445                 450

ttc agc cag gtc ctc cgc ctt gag cct gac aag ggg ctc ctg gag cca     1929
Phe Ser Gln Val Leu Arg Leu Glu Pro Asp Lys Gly Leu Leu Glu Pro
                455                 460                 465

ctg cag gag ctg ggg cct ccg gcc ctg gtg gcc ccc cgt gcc ttt gcc     1977
Leu Gln Glu Leu Gly Pro Pro Ala Leu Val Ala Pro Arg Ala Phe Ala
            470                 475                 480

cac atc act atg gcc ggc aga cgc ttc ctc ttt gct gct tgc ttt aag     2025
His Ile Thr Met Ala Gly Arg Arg Phe Leu Phe Ala Ala Cys Phe Lys
        485                 490                 495

ggc ccc aca cag atc tac cag cat cac gag atc gac ctc agt gcc tga     2073
Gly Pro Thr Gln Ile Tyr Gln His His Glu Ile Asp Leu Ser Ala
    500                 505                 510

gaccaccaat gggactctgg gcatggctgg ggcccctgga cggccccttg gctggctcct     2133

ggccctactt ggggtgatgg cccgcctgtg agctgctgac cgtgggccac gttcatcagc     2193

cacacgtcta ggccttaagc ccacttctta aaggatctgc acccatgggg ggacatagag     2253

ggtcccagcc tactggacct cccgagctgc ccttcaggtc taaagcaaca tctggacatc     2313

tcggctgggg cagattcccc atcaaaggca acctgggggg tgcaggctgg tgcactgcat     2373

ggagtcgagg tggaggtcac gtgcagagct ccatgaggtc ccggagcccc cctctcaacc     2433

cgctcctacc ccgctggtcc ctctgaaagc accgaggggt tgatggcatt cctttcccat     2493

tctaaactcc acgcagccaa tcgctttcct ggaggagcgc caacctcgaa gacaactcag     2553

cactgggcgg gttcccctct gctgcttgtg cgcatgcgtg cggccgccga ctagtga     2610

<210>  14
<211>  513
<212>  PRT
<213>  Homo sapiens

<400>  14

Met Gly Gly Ala Gly Ile Leu Leu Leu Leu Leu Ala Gly Ala Gly Val
1               5                   10                  15

Val Val Ala Trp Arg Pro Pro Lys Gly Lys Cys Pro Leu Arg Cys Ser
            20                  25                  30

Cys Ser Lys Asp Ser Ala Leu Cys Glu Gly Ser Pro Asp Leu Pro Val
        35                  40                  45

Ser Phe Ser Pro Thr Leu Leu Ser Leu Ser Leu Val Arg Thr Gly Val
    50                  55                  60

Thr Gln Leu Lys Ala Gly Ser Phe Leu Arg Ile Pro Ser Leu His Leu
65                  70                  75                  80

Leu Phe Ile Glu Asp Asn Glu Ile Gly Ser Ile Ser Lys Asn Ala Leu
                85                  90                  95

Arg Gly Leu Arg Ser Leu Thr His Leu Ser Leu Ala Asn Asn His Leu
            100                 105                 110

Glu Thr Leu Pro Arg Phe Leu Phe Arg Gly Leu Asp Thr Leu Thr His
        115                 120                 125

Val Asp Leu Arg Gly Asn Pro Phe Gln Cys Asp Cys Arg Val Leu Trp
    130                 135                 140

Leu Leu Gln Trp Met Pro Thr Val Asn Ala Ser Val Gly Thr Gly Ala
145                 150                 155                 160

Cys Ala Gly Pro Ala Ser Leu Ser His Met Gln Leu His His Leu Asp
                165                 170                 175

Pro Lys Thr Phe Lys Cys Arg Ala Ile Glu Leu Ser Trp Phe Gln Thr
            180                 185                 190

Val Gly Glu Ser Ala Leu Ser Val Glu Pro Phe Ser Tyr Gln Gly Glu
        195                 200                 205

Pro His Ile Val Leu Ala Gln Pro Phe Ala Gly Arg Cys Leu Ile Leu
    210                 215                 220

Ser Trp Asp Tyr Ser Leu Gln Arg Phe Arg Pro Glu Glu Glu Leu Pro
225                 230                 235                 240

Ala Ala Ser Val Val Ser Cys Lys Pro Leu Val Leu Gly Pro Ser Leu
                245                 250                 255

Phe Val Leu Ala Ala Arg Leu Trp Gly Gly Ser Gln Leu Trp Ala Arg
            260                 265                 270

Pro Ser Pro Gly Leu Arg Leu Ala Pro Thr Gln Thr Leu Ala Pro Arg
        275                 280                 285

Arg Leu Leu Arg Pro Asn Asp Ala Glu Leu Leu Trp Leu Glu Gly Gln
    290                 295                 300

Pro Cys Phe Val Val Ala Asp Ala Ser Lys Ala Gly Ser Thr Thr Leu
305                 310                 315                 320

Leu Cys Arg Asp Gly Pro Gly Phe Tyr Pro His Gln Ser Leu His Ala
                325                 330                 335

Trp His Arg Asp Thr Asp Ala Glu Ala Leu Glu Leu Asp Gly Arg Pro
            340                 345                 350

His Leu Leu Leu Ala Ser Ala Ser Gln Arg Pro Val Leu Phe His Trp
        355                 360                 365

Thr Gly Gly Arg Phe Glu Arg Arg Thr Asp Ile Pro Glu Ala Glu Asp
    370                 375                 380

Val Tyr Ala Thr Arg His Phe Gln Ala Gly Gly Asp Val Phe Leu Cys
385                 390                 395                 400

Leu Thr Arg Tyr Ile Gly Asp Ser Met Val Met Arg Trp Asp Gly Ser
                405                 410                 415

Met Phe Arg Leu Leu Gln Gln Leu Pro Ser Arg Gly Ala His Val Phe
            420                 425                 430

Gln Pro Leu Leu Ile Ala Arg Asp Gln Leu Ala Ile Leu Gly Ser Asp
        435                 440                 445

Phe Ala Phe Ser Gln Val Leu Arg Leu Glu Pro Asp Lys Gly Leu Leu
    450                 455                 460

Glu Pro Leu Gln Glu Leu Gly Pro Pro Ala Leu Val Ala Pro Arg Ala
465                 470                 475                 480

Phe Ala His Ile Thr Met Ala Gly Arg Arg Phe Leu Phe Ala Ala Cys
                485                 490                 495

Phe Lys Gly Pro Thr Gln Ile Tyr Gln His His Glu Ile Asp Leu Ser
            500                 505                 510

Ala

<210>  15
<211>  983
<212>  DNA
<213>  Homo sapiens

<220>
<221>  misc_feature
<222>  (1)..(1)
<223>  LP284

<220>
<221>  CDS
<222>  (158)..(943)

<400>  15
ggccgactga aagaaagacg gagaagagga gagagaagcc agggccgagc gtgccagcag      60

gcggatggag ggcggcctgg tggaggagga gacgtagtgg cctgggctga gctgggtggg     120

ccgggagaag cgggtgcctc agagtggggg tgggggc atg gga ggg gca ggc att      175
                                         Met Gly Gly Ala Gly Ile
                                         1               5

ctg ctg ctg ctg ctg gct ggg gcg ggg gtg gtg gtg gcc tgg aga ccc      223

Leu Leu Leu Leu Leu Ala Gly Ala Gly Val Val Val Ala Trp Arg Pro
            10                  15                  20

cca aag gga aag tgt ccc ctg cgc tgc tcc tgc tct aaa gac agc gcc         271
Pro Lys Gly Lys Cys Pro Leu Arg Cys Ser Cys Ser Lys Asp Ser Ala
        25                  30                  35

ctg tgt gag ggc tcc ccg gac ctg ccc gtc agc ttc tct ccg acc ctg         319
Leu Cys Glu Gly Ser Pro Asp Leu Pro Val Ser Phe Ser Pro Thr Leu
    40                  45                  50

ctg tca ctc tca ctc gtc agg acg gga gtc acc cag ctg aag gcc ggc         367
Leu Ser Leu Ser Leu Val Arg Thr Gly Val Thr Gln Leu Lys Ala Gly
55                  60                  65                  70

agc ttc ctg aga att ccg tct ctg cac ctg ctc ctc ttc acc tcc aac         415
Ser Phe Leu Arg Ile Pro Ser Leu His Leu Leu Leu Phe Thr Ser Asn
                75                  80                  85

tcc ttc tcc gtg att gag gac gat gca ttt gcg ggc ctg tcc cac ctg         463
Ser Phe Ser Val Ile Glu Asp Asp Ala Phe Ala Gly Leu Ser His Leu
            90                  95                  100

cag tac ctc ttc atc gag gac aat gag att ggc tcc atc tct aag aat         511
Gln Tyr Leu Phe Ile Glu Asp Asn Glu Ile Gly Ser Ile Ser Lys Asn
        105                 110                 115

gcc ctc aga gga ctt cgc tcg ctt aca cac cta agc ctg gcc aat aac         559
Ala Leu Arg Gly Leu Arg Ser Leu Thr His Leu Ser Leu Ala Asn Asn
    120                 125                 130

cat ctg gag acc ctc ccc aga ttc ctg ttc cga ggc ctg gac acc ctt         607
His Leu Glu Thr Leu Pro Arg Phe Leu Phe Arg Gly Leu Asp Thr Leu
135                 140                 145                 150

act cac gtg gac ctc cgc ggg aac ccg ttc cag tgt gac tgc cgc gtc         655
Thr His Val Asp Leu Arg Gly Asn Pro Phe Gln Cys Asp Cys Arg Val
                155                 160                 165

ctc tgg ctc ctg cag tgg atg ccc acc gtg aat gcc agc gtg ggg acc         703
Leu Trp Leu Leu Gln Trp Met Pro Thr Val Asn Ala Ser Val Gly Thr
            170                 175                 180

ggc gcc tgt gcg ggc ccc gcc tcc ctg agc cac atg cag ctc cac cac         751
Gly Ala Cys Ala Gly Pro Ala Ser Leu Ser His Met Gln Leu His His
        185                 190                 195

ctc gac ccc aag act ttc aag tgc aga gcc ata ggt ggg ggg ctt tcc         799
Leu Asp Pro Lys Thr Phe Lys Cys Arg Ala Ile Gly Gly Gly Leu Ser
    200                 205                 210

cga tgg ggt ggg agg cgg gag atc tgg ggg aaa ggc tgc cag ggc caa         847
Arg Trp Gly Gly Arg Arg Glu Ile Trp Gly Lys Gly Cys Gln Gly Gln
215                 220                 225                 230

gag gct cgt ctc act ccc tgc cct gcc att tcc cgg agt ggg aag acc         895
Glu Ala Arg Leu Thr Pro Cys Pro Ala Ile Ser Arg Ser Gly Lys Thr
                235                 240                 245

ctg agc aag cag cac tgc ctt cct gag ccc cag ttt tct cat ctg taa         943
Leu Ser Lys Gln His Cys Leu Pro Glu Pro Gln Phe Ser His Leu
            250                 255                 260

agtgggggta ataaacagtg atataggagt gcaaaaaaaa                             983

<210>  16
<211>  261
<212>  PRT
<213>  Homo sapiens

<400>  16

Met Gly Gly Ala Gly Ile Leu Leu Leu Leu Leu Ala Gly Ala Gly Val
1               5                   10                  15

Val Val Ala Trp Arg Pro Pro Lys Gly Lys Cys Pro Leu Arg Cys Ser
            20                  25                  30

Cys Ser Lys Asp Ser Ala Leu Cys Glu Gly Ser Pro Asp Leu Pro Val
        35                  40                  45

Ser Phe Ser Pro Thr Leu Leu Ser Leu Ser Leu Val Arg Thr Gly Val
    50                  55                  60

Thr Gln Leu Lys Ala Gly Ser Phe Leu Arg Ile Pro Ser Leu His Leu
65                  70                  75                  80

Leu Leu Phe Thr Ser Asn Ser Phe Ser Val Ile Glu Asp Asp Ala Phe
                85                  90                  95

Ala Gly Leu Ser His Leu Gln Tyr Leu Phe Ile Glu Asp Asn Glu Ile
            100                 105                 110

Gly Ser Ile Ser Lys Asn Ala Leu Arg Gly Leu Arg Ser Leu Thr His
        115                 120                 125

Leu Ser Leu Ala Asn Asn His Leu Glu Thr Leu Pro Arg Phe Leu Phe
    130                 135                 140

Arg Gly Leu Asp Thr Leu Thr His Val Asp Leu Arg Gly Asn Pro Phe
145                 150                 155                 160

Gln Cys Asp Cys Arg Val Leu Trp Leu Leu Gln Trp Met Pro Thr Val
                165                 170                 175

Asn Ala Ser Val Gly Thr Gly Ala Cys Ala Gly Pro Ala Ser Leu Ser
            180                 185                 190

His Met Gln Leu His His Leu Asp Pro Lys Thr Phe Lys Cys Arg Ala
        195                 200                 205

Ile Gly Gly Gly Leu Ser Arg Trp Gly Gly Arg Arg Glu Ile Trp Gly
    210                 215                 220

Lys Gly Cys Gln Gly Gln Glu Ala Arg Leu Thr Pro Cys Pro Ala Ile
225                 230                 235                 240

Ser Arg Ser Gly Lys Thr Leu Ser Lys Gln His Cys Leu Pro Glu Pro
                245                 250                 255

Gln Phe Ser His Leu
            260

<210>  17
<211>  1555
<212>  DNA
<213>  Homo sapiens

<220>
<221>  misc_feature
<222>  (1)..(1)
<223>  LP282

<220>
<221>  CDS

<222>  (27)..(1433)

<400>  17
gctcgctgcc cttggctctc gtcgcc atg gcc tcc gtc gcc cag gag agc gcg       53
                             Met Ala Ser Val Ala Gln Glu Ser Ala
                             1               5

ggc tcg cag cgc cgg cta ccg ccg cgt cac ggg gcg ctg cgc ggg ctg       101
Gly Ser Gln Arg Arg Leu Pro Pro Arg His Gly Ala Leu Arg Gly Leu
10                  15                  20                  25

cta ctg ctc tgc ctg tgg ctg cca agc ggc cgt gcg gcc ttg ccg ccc       149
Leu Leu Leu Cys Leu Trp Leu Pro Ser Gly Arg Ala Ala Leu Pro Pro
                30                  35                  40

gcg gcg ccg ctg tcc gaa ctg cac gcg cag ctg tcg ggc gtg gag cag       197
Ala Ala Pro Leu Ser Glu Leu His Ala Gln Leu Ser Gly Val Glu Gln
            45                  50                  55

ctg ctg gag gag ttc cgc cgg caa ctg cag cag gag cgg cct cag gag       245
Leu Leu Glu Glu Phe Arg Arg Gln Leu Gln Gln Glu Arg Pro Gln Glu
        60                  65                  70

gag ctg gag ctg gag ctg cgt gcg ggc ggc ggc ccc cag gag gac tgc       293
Glu Leu Glu Leu Glu Leu Arg Ala Gly Gly Gly Pro Gln Glu Asp Cys
    75                  80                  85

ccg ggc ccg ggc agc ggc ggc tac agc gca atg cct gac gcc atc atc       341
Pro Gly Pro Gly Ser Gly Gly Tyr Ser Ala Met Pro Asp Ala Ile Ile
90                  95                  100                 105

cgc acc aag gac tcc ctg gcg gcg ggt gcc agc ttc ctg cgg gcg ccg       389
Arg Thr Lys Asp Ser Leu Ala Ala Gly Ala Ser Phe Leu Arg Ala Pro
                110                 115                 120

gcg gcc gtg cgg ggc tgg cgg caa tgc gtg gcg gcc tgc tgc tcc gag       437
Ala Ala Val Arg Gly Trp Arg Gln Cys Val Ala Ala Cys Cys Ser Glu
            125                 130                 135

ccg cgc tgc tcc gtg gcc gtg gtg gag ctg ccc cgg cgc ccc gcg ccc       485
Pro Arg Cys Ser Val Ala Val Val Glu Leu Pro Arg Arg Pro Ala Pro
        140                 145                 150

ccg gca gcc gtg ctc ggc tgc tac ctc ttc aac tgc acg gcg cgc ggc       533
Pro Ala Ala Val Leu Gly Cys Tyr Leu Phe Asn Cys Thr Ala Arg Gly
    155                 160                 165

cgc aac gtc tgc aag ttc gcg ctg cac agc ggc tac agc agc tac agc       581
Arg Asn Val Cys Lys Phe Ala Leu His Ser Gly Tyr Ser Ser Tyr Ser
170                 175                 180                 185

ctc agc cgc gcg ccg gac ggc gcc gcc ctg gcc acc gcg cgc gcc tcg       629
Leu Ser Arg Ala Pro Asp Gly Ala Ala Leu Ala Thr Ala Arg Ala Ser
                190                 195                 200

ccc cgg cag gaa aag gat gcg cct cca ctt agc aag gct ggg cag gat       677
Pro Arg Gln Glu Lys Asp Ala Pro Pro Leu Ser Lys Ala Gly Gln Asp
            205                 210                 215

gtg gtt ctg cat ctg ccc aca gac ggg gtg gtt cta gac ggc cgc gag       725
Val Val Leu His Leu Pro Thr Asp Gly Val Val Leu Asp Gly Arg Glu
        220                 225                 230

agc aca gat gac cac gcc atc gtc cag tat gag tgg gca ctg ctg cag       773
Ser Thr Asp Asp His Ala Ile Val Gln Tyr Glu Trp Ala Leu Leu Gln
    235                 240                 245

ggg gac ccg tca gtg gac atg aag gtg cct caa tca gga acc ctg aag       821
Gly Asp Pro Ser Val Asp Met Lys Val Pro Gln Ser Gly Thr Leu Lys
250                 255                 260                 265

ctg tcc cac cta cag gag gga acc tac acc ttc cag ctg acc gtg acg       869
Leu Ser His Leu Gln Glu Gly Thr Tyr Thr Phe Gln Leu Thr Val Thr
                270                 275                 280

gac act gcc ggg cag aga agc tct gac aac gtg tca gtg aca gtg ctt       917
Asp Thr Ala Gly Gln Arg Ser Ser Asp Asn Val Ser Val Thr Val Leu

285                     290                     295

cgc gca gcc tac tcc aca gga gga tgt ttg cac act tgc tca cgc tac      965
Arg Ala Ala Tyr Ser Thr Gly Gly Cys Leu His Thr Cys Ser Arg Tyr
        300                     305                     310

cac ttc ttc tgt gac gat ggc tgc tgc att gac atc acg ctc gcc tgc      1013
His Phe Phe Cys Asp Asp Gly Cys Cys Ile Asp Ile Thr Leu Ala Cys
    315                     320                     325

gat gga gtg cag cag tgt cct gat ggg tct gat gaa gac ttc tgc cag      1061
Asp Gly Val Gln Gln Cys Pro Asp Gly Ser Asp Glu Asp Phe Cys Gln
330                     335                     340                     345

aat ctg ggc ctg gac cgc aag atg gta acc cac acg gca gct agt cct      1109
Asn Leu Gly Leu Asp Arg Lys Met Val Thr His Thr Ala Ala Ser Pro
                350                     355                     360

gcc ctg cca aga acc aca ggg ccg agt gaa gat gca ggg ggt gat tcc      1157
Ala Leu Pro Arg Thr Thr Gly Pro Ser Glu Asp Ala Gly Gly Asp Ser
            365                     370                     375

ttg gtg gaa aag tct cag aaa gcc act gcc cca aac aag cca cct gca      1205
Leu Val Glu Lys Ser Gln Lys Ala Thr Ala Pro Asn Lys Pro Pro Ala
        380                     385                     390

tta tca aac aca gag aag agg aat cat tcc gcc ttt tgg gga cca gag      1253
Leu Ser Asn Thr Glu Lys Arg Asn His Ser Ala Phe Trp Gly Pro Glu
    395                     400                     405

agt caa atc att cct gtg atg cca ggt gca gtg cta ccc ctg gcg ctg      1301
Ser Gln Ile Ile Pro Val Met Pro Gly Ala Val Leu Pro Leu Ala Leu
410                     415                     420                     425

ggt ttg gct atc act gct ctg ctg ctt ctc atg gtt gca tgc cga cta      1349
Gly Leu Ala Ile Thr Ala Leu Leu Leu Leu Met Val Ala Cys Arg Leu
                430                     435                     440

cga ctg gtg aaa cag aaa ctg aaa aaa gct cgt ccc att aca tct gag      1397
Arg Leu Val Lys Gln Lys Leu Lys Lys Ala Arg Pro Ile Thr Ser Glu
            445                     450                     455

gaa tcg gac tac ctc ata aat ggg atg tat cta tag taatgtaatt      1443
Glu Ser Asp Tyr Leu Ile Asn Gly Met Tyr Leu
        460                     465

tcaatacctt ggggcaggga catgttttgt ttataattta tacatctatt aagttctgga      1503

tatttacagc ttcttttgtt tttaattggg ccagaagatt ctgcaaatcc ca      1555

<210>  18
<211>  468
<212>  PRT
<213>  Homo sapiens

<400>  18

Met Ala Ser Val Ala Gln Glu Ser Ala Gly Ser Gln Arg Arg Leu Pro
1               5                   10                  15

Pro Arg His Gly Ala Leu Arg Gly Leu Leu Leu Leu Cys Leu Trp Leu
            20                  25                  30

Pro Ser Gly Arg Ala Ala Leu Pro Pro Ala Ala Pro Leu Ser Glu Leu
        35                  40                  45

His Ala Gln Leu Ser Gly Val Glu Gln Leu Leu Glu Glu Phe Arg Arg
    50                  55                  60

Gln Leu Gln Gln Glu Arg Pro Gln Glu Glu Leu Glu Leu Glu Leu Arg
65                  70                  75                  80

Ala Gly Gly Gly Pro Gln Glu Asp Cys Pro Gly Pro Gly Ser Gly Gly
                85                  90                  95

Tyr Ser Ala Met Pro Asp Ala Ile Ile Arg Thr Lys Asp Ser Leu Ala
            100                 105                 110

Ala Gly Ala Ser Phe Leu Arg Ala Pro Ala Ala Val Arg Gly Trp Arg
        115                 120                 125

Gln Cys Val Ala Ala Cys Cys Ser Glu Pro Arg Cys Ser Val Ala Val
    130                 135                 140

Val Glu Leu Pro Arg Arg Pro Ala Pro Pro Ala Ala Val Leu Gly Cys
145                 150                 155                 160

Tyr Leu Phe Asn Cys Thr Ala Arg Gly Arg Asn Val Cys Lys Phe Ala
                165                 170                 175

Leu His Ser Gly Tyr Ser Ser Tyr Ser Leu Ser Arg Ala Pro Asp Gly
            180                 185                 190

Ala Ala Leu Ala Thr Ala Arg Ala Ser Pro Arg Gln Glu Lys Asp Ala
        195                 200                 205

Pro Pro Leu Ser Lys Ala Gly Gln Asp Val Val Leu His Leu Pro Thr
    210                 215                 220

Asp Gly Val Val Leu Asp Gly Arg Glu Ser Thr Asp Asp His Ala Ile
225                 230                 235                 240

Val Gln Tyr Glu Trp Ala Leu Leu Gln Gly Asp Pro Ser Val Asp Met
                245                 250                 255

Lys Val Pro Gln Ser Gly Thr Leu Lys Leu Ser His Leu Gln Glu Gly
            260                 265                 270

Thr Tyr Thr Phe Gln Leu Thr Val Thr Asp Thr Ala Gly Gln Arg Ser
        275                 280                 285

Ser Asp Asn Val Ser Val Thr Val Leu Arg Ala Ala Tyr Ser Thr Gly
    290                 295                 300

Gly Cys Leu His Thr Cys Ser Arg Tyr His Phe Phe Cys Asp Asp Gly
305                 310                 315                 320

Cys Cys Ile Asp Ile Thr Leu Ala Cys Asp Gly Val Gln Gln Cys Pro
                325                 330                 335

Asp Gly Ser Asp Glu Asp Phe Cys Gln Asn Leu Gly Leu Asp Arg Lys
            340                 345                 350

Met Val Thr His Thr Ala Ala Ser Pro Ala Leu Pro Arg Thr Thr Gly
        355                 360                 365

Pro Ser Glu Asp Ala Gly Gly Asp Ser Leu Val Glu Lys Ser Gln Lys
    370                 375                 380

Ala Thr Ala Pro Asn Lys Pro Pro Ala Leu Ser Asn Thr Glu Lys Arg
385                 390                 395                 400

Asn His Ser Ala Phe Trp Gly Pro Glu Ser Gln Ile Ile Pro Val Met
                405                 410                 415

Pro Gly Ala Val Leu Pro Leu Ala Leu Gly Leu Ala Ile Thr Ala Leu
            420                 425                 430

Leu Leu Leu Met Val Ala Cys Arg Leu Arg Leu Val Lys Gln Lys Leu
        435                 440                 445

Lys Lys Ala Arg Pro Ile Thr Ser Glu Glu Ser Asp Tyr Leu Ile Asn
    450                 455                 460

Gly Met Tyr Leu
465

<210>  19
<211>  1501
<212>  DNA
<213>  Homo sapiens

<220>
<221>  misc_feature
<222>  (1)..(1)
<223>  LP273

<220>
<221>  CDS
<222>  (19)..(951)

<400>  19
aacttactgt gtggcaga atg aag cct ctg ctt gaa acc ctt tat ctt ttg        51
                    Met Lys Pro Leu Leu Glu Thr Leu Tyr Leu Leu
                    1               5                   10

ggg atg ctg gtt cct gga ggg ctg gga tat gat aga tcc tta gcc caa        99
Gly Met Leu Val Pro Gly Gly Leu Gly Tyr Asp Arg Ser Leu Ala Gln
            15                  20                  25

cac aga caa gag att gtg gac aag tca gtg agt cca tgg agc ctg gag       147
His Arg Gln Glu Ile Val Asp Lys Ser Val Ser Pro Trp Ser Leu Glu
        30                  35                  40

acg tat tcc tat aac ata tac cac ccc atg gga gag atc tat gag tgg       195
Thr Tyr Ser Tyr Asn Ile Tyr His Pro Met Gly Glu Ile Tyr Glu Trp
    45                  50                  55

atg aga gag atc agt gag aag tac aag gaa gtg gtg aca cag cat ttc       243
Met Arg Glu Ile Ser Glu Lys Tyr Lys Glu Val Val Thr Gln His Phe
60                  65                  70                  75

cta gga gtg acc tat gag acc cac ccc atg tat tat ctg aag atc agc       291
Leu Gly Val Thr Tyr Glu Thr His Pro Met Tyr Tyr Leu Lys Ile Ser
                80                  85                  90

caa cca tct ggt aat ccc aag aaa atc att tgg atg gac tgt gga att       339
Gln Pro Ser Gly Asn Pro Lys Lys Ile Ile Trp Met Asp Cys Gly Ile
            95                  100                 105

cac gcc aga gaa tgg att gct cct gct ttt tgc caa tgg ttc gtc aaa       387
His Ala Arg Glu Trp Ile Ala Pro Ala Phe Cys Gln Trp Phe Val Lys
        110                 115                 120

gaa att cta caa aac cat aaa gac aac tca agt ata cgc aag ctc ctt       435
Glu Ile Leu Gln Asn His Lys Asp Asn Ser Ser Ile Arg Lys Leu Leu
    125                 130                 135

agg aac ctg gac ttc tat gtc ctt cca gtt ctt aac ata gat ggt tat       483

Arg Asn Leu Asp Phe Tyr Val Leu Pro Val Leu Asn Ile Asp Gly Tyr
140                 145                 150                 155

atc tac act tgg aca act gat cgt ctt tgg agg aaa tcc cgt tca ccc       531
Ile Tyr Thr Trp Thr Thr Asp Arg Leu Trp Arg Lys Ser Arg Ser Pro
                160                 165                 170

cat aat aat ggc aca tgt ttt ggg acg gat ctc aat cga aat ttc aat       579
His Asn Asn Gly Thr Cys Phe Gly Thr Asp Leu Asn Arg Asn Phe Asn
            175                 180                 185

gca tct tgg tgt aag aga caa gaa gag aaa ctg aag aat aac aac aga       627
Ala Ser Trp Cys Lys Arg Gln Glu Glu Lys Leu Lys Asn Asn Asn Arg
        190                 195                 200

gat cta tca atg gtt cga atg aaa tcc atg ttt gct att ggc ttt tgt       675
Asp Leu Ser Met Val Arg Met Lys Ser Met Phe Ala Ile Gly Phe Cys
    205                 210                 215

ttt act gcc cta atg gga atg ttc aat tcc ata ttt gat ggt aga gtg       723
Phe Thr Ala Leu Met Gly Met Phe Asn Ser Ile Phe Asp Gly Arg Val
220                 225                 230                 235

gtg gca aag ctt cct ttt acc cct ctt tct tac atc caa gga ctg tct       771
Val Ala Lys Leu Pro Phe Thr Pro Leu Ser Tyr Ile Gln Gly Leu Ser
                240                 245                 250

cat cga aat ctg ctg gga gat gac acc aca gac tgt tcc ttc att ttc       819
His Arg Asn Leu Leu Gly Asp Asp Thr Thr Asp Cys Ser Phe Ile Phe
            255                 260                 265

ctg tat att ctc tgt act atg tcg att cga cag aac att cag aag att       867
Leu Tyr Ile Leu Cys Thr Met Ser Ile Arg Gln Asn Ile Gln Lys Ile
        270                 275                 280

ctc ggc ctt gcc cct tca cga gcc gcc acc aag cag gca ggt gga ttt       915
Leu Gly Leu Ala Pro Ser Arg Ala Ala Thr Lys Gln Ala Gly Gly Phe
    285                 290                 295

ctt ggc cca cca cct cct tct ggg aag ttc tct tga actcaagaac            961
Leu Gly Pro Pro Pro Pro Ser Gly Lys Phe Ser
300                 305                 310

tctttatttt ctatcattct ttctagacac acacacatca gactggcaac tgttttgtag    1021

caagagccat aggtagcctt actacttggg cctctttcta gttttgaatt atttctaagc    1081

cttttgggta tgattagagt gaaaatggca gccagcaaac ttgatagtgc ttttggtcct    1141

agatgatttt tatcaaataa gtggattgat tagttaagtt caggtaatgt ttatgtaatg    1201

aaaaacaaat agcatccttc ttgtttcatt tacataagta ttttctgtgg gaccgactct    1261

caaggcactg tgtatgccct gcaagttggc tgtctatgag catttagaga tttagaagaa    1321

aaatttagtt tgtttaaccc ttgtaactgt ttgttttgtt gttgtttttt tttcaagcca    1381

aatacatgac ataagatcaa taaagaggcc aaatttttag ctgttttatg tacaaaaaaa    1441

aaaaaaaagg gcggccgccg actagtgagc tcgtcgaccc gggaattaat tccggaccgg    1501

<210>  20
<211>  310
<212>  PRT
<213>  Homo sapiens

<400>  20

Met Lys Pro Leu Leu Glu Thr Leu Tyr Leu Leu Gly Met Leu Val Pro
1               5                   10                  15

Gly Gly Leu Gly Tyr Asp Arg Ser Leu Ala Gln His Arg Gln Glu Ile
            20                  25                  30

Val Asp Lys Ser Val Ser Pro Trp Ser Leu Glu Thr Tyr Ser Tyr Asn
        35                  40                  45

Ile Tyr His Pro Met Gly Glu Ile Tyr Glu Trp Met Arg Glu Ile Ser
    50                  55                  60

Glu Lys Tyr Lys Glu Val Val Thr Gln His Phe Leu Gly Val Thr Tyr
65                  70                  75                  80

Glu Thr His Pro Met Tyr Tyr Leu Lys Ile Ser Gln Pro Ser Gly Asn
                85                  90                  95

Pro Lys Lys Ile Ile Trp Met Asp Cys Gly Ile His Ala Arg Glu Trp
            100                 105                 110

Ile Ala Pro Ala Phe Cys Gln Trp Phe Val Lys Glu Ile Leu Gln Asn
        115                 120                 125

His Lys Asp Asn Ser Ser Ile Arg Lys Leu Leu Arg Asn Leu Asp Phe
    130                 135                 140

Tyr Val Leu Pro Val Leu Asn Ile Asp Gly Tyr Ile Tyr Thr Trp Thr
145                 150                 155                 160

Thr Asp Arg Leu Trp Arg Lys Ser Arg Ser Pro His Asn Asn Gly Thr
                165                 170                 175

Cys Phe Gly Thr Asp Leu Asn Arg Asn Phe Asn Ala Ser Trp Cys Lys
            180                 185                 190

Arg Gln Glu Glu Lys Leu Lys Asn Asn Asn Arg Asp Leu Ser Met Val
        195                 200                 205

Arg Met Lys Ser Met Phe Ala Ile Gly Phe Cys Phe Thr Ala Leu Met
    210                 215                 220

Gly Met Phe Asn Ser Ile Phe Asp Gly Arg Val Val Ala Lys Leu Pro
225                 230                 235                 240

Phe Thr Pro Leu Ser Tyr Ile Gln Gly Leu Ser His Arg Asn Leu Leu
                245                 250                 255

Gly Asp Asp Thr Thr Asp Cys Ser Phe Ile Phe Leu Tyr Ile Leu Cys
            260                 265                 270

Thr Met Ser Ile Arg Gln Asn Ile Gln Lys Ile Leu Gly Leu Ala Pro
        275                 280                 285

Ser Arg Ala Ala Thr Lys Gln Ala Gly Gly Phe Leu Gly Pro Pro Pro
    290                 295                 300

Pro Ser Gly Lys Phe Ser
305                 310

<210>  21
<211>  1770
<212>  DNA
<213>  Homo sapiens

<220>
<221> misc_feature
<222> (1)..(1)
<223> LP277

<220>
<221> CDS
<222> (76)..(642)

<400> 21
taggtgctgt gctgtcctga ggcctgggcc atggtgccca aggaaagccc ctgaagctca 60

ccaggaggaa gaagc atg cag ggc acc cct gga ggc ggg acg cgc cct ggg 111
Met Gln Gly Thr Pro Gly Gly Gly Thr Arg Pro Gly
1               5                   10

cca tcc ccc gtg gac agg cgg aca ctc ctg gtc ttc agc ttt atc ctg 159
Pro Ser Pro Val Asp Arg Arg Thr Leu Leu Val Phe Ser Phe Ile Leu
        15                  20                  25

gca gca gct ttg ggc caa atg aat ttc aca ggg gac cag gtt ctt cga 207
Ala Ala Ala Leu Gly Gln Met Asn Phe Thr Gly Asp Gln Val Leu Arg
    30                  35                  40

gtc ctg gcc aaa gat gag aag cag ctt tca ctt ctc ggg gat ctg gag 255
Val Leu Ala Lys Asp Glu Lys Gln Leu Ser Leu Leu Gly Asp Leu Glu
45                  50                  55                  60

ggc ctg aaa ccc cag aag gtg gac ttc tgg cgt ggc cca gcc agg ccc 303
Gly Leu Lys Pro Gln Lys Val Asp Phe Trp Arg Gly Pro Ala Arg Pro
                65                  70                  75

agc ctc cct gtg gat atg aga gtt cct ttc tct gaa ctg aaa gac atc 351
Ser Leu Pro Val Asp Met Arg Val Pro Phe Ser Glu Leu Lys Asp Ile
            80                  85                  90

aaa gct tat ctg gag tct cat gga ctt gct tac agc atc atg ata aag 399
Lys Ala Tyr Leu Glu Ser His Gly Leu Ala Tyr Ser Ile Met Ile Lys
        95                  100                 105

gac atc cag gtg ctg ctg gat gag gaa aga cag gcc atg gcg aaa tcc 447
Asp Ile Gln Val Leu Leu Asp Glu Glu Arg Gln Ala Met Ala Lys Ser
    110                 115                 120

cgc cgg ctg gag cgc agc acc aac agc ttc agt tac tca tca tac cac 495
Arg Arg Leu Glu Arg Ser Thr Asn Ser Phe Ser Tyr Ser Ser Tyr His
125                 130                 135                 140

acc ctg gag gag ata tat agc tgg att gac aac ttt gta atg gag cat 543
Thr Leu Glu Glu Ile Tyr Ser Trp Ile Asp Asn Phe Val Met Glu His
                145                 150                 155

tcc gat att gtc tca aaa att cag att ggc aac agc ttt gaa aac cag 591
Ser Asp Ile Val Ser Lys Ile Gln Ile Gly Asn Ser Phe Glu Asn Gln
            160                 165                 170

tcc att ctt gtc ctg aag ggg cca tgg tct tct ctc tct aat gtt gtc 639
Ser Ile Leu Val Leu Lys Gly Pro Trp Ser Ser Leu Ser Asn Val Val
        175                 180                 185

tga agccttgcta ttcaaagcac ggcccctgca tggcagcatc aacatcacct 692

gggagcttat tagaaatgcc aaacctcaga cccctcccca gacctactaa gtcagaatct 752

gcactggaat aagatcccca ggagactcat gtgcccatta gagtttgaga aaccctggtc 812

ttaagatact ttcaatcctg ggaaattctg tgcttctgga tggatcactt tgctcgccaa 872

tggcaccacc atattttcaa aggccctcac acgtgtgcac ttttgggcac atggacagtt 932

cagcactgga ggttctcggc acccagccat ctggatcgac actggaattc actcccggga 992

gtggatcacc catgccaccg gcatctggac tgccaataag attgtcagtg attatggcaa 1052

agaccgtgtc ctgacagaca tactgaatgc catggacatc ttcatagagc tcgtcacaaa 1112

ccctgatggg tttgctttta cccacagcat gaaccgctta tggcggaaga acaagtccat 1172

cagacctgga atcttctgca tcggcgtgga tctcaacagg aactggaagt cgggttttgg 1232

aggaaatggt tctaacagca acccctgctc agaaacttat cacgggccct cccctcagtc 1292

ggagccggag gtggctgcca tagtgaactt catcacagcc catggcaact tcaaggctct 1352

gatctccatc cacagctact ctcagatgct tatgtaccct tacggccgat tgctggagcc 1412

cgtttcaaat cagagggagt tgtacgatct tgccaaggat gcggtggagg ccttgtataa 1472

ggtccatggg atcgagtaca tttttggcag catcagcacc accctctatg tggccagtgg 1532

gatcaccgtc gactgggcct atgacagtgg catcaagtac gccttcagct ttgagctccg 1592

ggacactggg cagtatggct tcctgctgcc ggccacacag atcatcccca cggcccagga 1652

gacgtggatg gcgcttcgga ccatcatgga gcacaccctg aatcacccct actagcagca 1712

cgactgaggg caggaggctc catccttctc cccaaggtct gtggctaagg gcgaattc 1770

<210> 22
<211> 188
<212> PRT
<213> Homo sapiens

<400> 22

Met Gln Gly Thr Pro Gly Gly Gly Thr Arg Pro Gly Pro Ser Pro Val
1               5                   10                  15

Asp Arg Arg Thr Leu Leu Val Phe Ser Phe Ile Leu Ala Ala Ala Leu
            20                  25                  30

Gly Gln Met Asn Phe Thr Gly Asp Gln Val Leu Arg Val Leu Ala Lys
        35                  40                  45

Asp Glu Lys Gln Leu Ser Leu Leu Gly Asp Leu Glu Gly Leu Lys Pro
    50                  55                  60

Gln Lys Val Asp Phe Trp Arg Gly Pro Ala Arg Pro Ser Leu Pro Val
65                  70                  75                  80

Asp Met Arg Val Pro Phe Ser Glu Leu Lys Asp Ile Lys Ala Tyr Leu
                85                  90                  95

Glu Ser His Gly Leu Ala Tyr Ser Ile Met Ile Lys Asp Ile Gln Val
            100                 105                 110

Leu Leu Asp Glu Glu Arg Gln Ala Met Ala Lys Ser Arg Arg Leu Glu
        115                 120                 125

Arg Ser Thr Asn Ser Phe Ser Tyr Ser Ser Tyr His Thr Leu Glu Glu
    130                 135                 140

Ile Tyr Ser Trp Ile Asp Asn Phe Val Met Glu His Ser Asp Ile Val
145                 150                 155                 160

Ser Lys Ile Gln Ile Gly Asn Ser Phe Glu Asn Gln Ser Ile Leu Val
                165                 170                 175

Leu Lys Gly Pro Trp Ser Ser Leu Ser Asn Val Val
            180                 185

<210>  23
<211>  1136
<212>  DNA
<213>  Homo sapiens


<220>
<221>  misc_feature
<222>  (1)..(1)
<223>  LP287

<220>
<221>  CDS
<222>  (13)..(852)

<400>  23
ccgactggca ag atg gtg ccc agc tct ccg cgc gcg ctc ttc ctt ctg ctc      51
              Met Val Pro Ser Ser Pro Arg Ala Leu Phe Leu Leu Leu
              1               5                   10

ctg atc ctc gcc tgc ccc gag ccg cgg gct tcc cag aac tgt ctc agc      99
Leu Ile Leu Ala Cys Pro Glu Pro Arg Ala Ser Gln Asn Cys Leu Ser
    15                  20                  25

aaa cag cag ctc ctc tcg gcc atc cgc cag ctg cag cag ctg ctg aag      147
Lys Gln Gln Leu Leu Ser Ala Ile Arg Gln Leu Gln Gln Leu Leu Lys
30                  35                  40                  45

ggc cag gag aca cgc ttc gcc gag ggc atc cgc cac atg aag agc cgg      195
Gly Gln Glu Thr Arg Phe Ala Glu Gly Ile Arg His Met Lys Ser Arg
                50                  55                  60

ctg gcc gcg ctg cag aac tct gtg ggc agg gtg ggc cca gat gcc ctt      243
Leu Ala Ala Leu Gln Asn Ser Val Gly Arg Val Gly Pro Asp Ala Leu
            65                  70                  75

cca gtt tcc tgc ccg gct ctg aac acc ccc gca gac ggc aga aag ttt      291
Pro Val Ser Cys Pro Ala Leu Asn Thr Pro Ala Asp Gly Arg Lys Phe
        80                  85                  90

gga agc aag tac tta gtg gat cac gaa gtc cat ttt acc tgc aac cct      339
Gly Ser Lys Tyr Leu Val Asp His Glu Val His Phe Thr Cys Asn Pro
    95                  100                 105

ggg ttc cgg ctg gtc ggg ccc agc agc gtg gtg tgt ctt ccc aat ggc      387
Gly Phe Arg Leu Val Gly Pro Ser Ser Val Val Cys Leu Pro Asn Gly
110                 115                 120                 125

acc tgg aca ggg gag cag ccc cac tgt aga ggt atc agt gaa tgc tcc      435
Thr Trp Thr Gly Glu Gln Pro His Cys Arg Gly Ile Ser Glu Cys Ser
                130                 135                 140

agc cag cct tgt caa aat ggt ggt aca tgt gta gaa gga gtc aac cag      483
Ser Gln Pro Cys Gln Asn Gly Gly Thr Cys Val Glu Gly Val Asn Gln
            145                 150                 155

tac aga tgc att tgt cct cca gga agg act ggg aac cgc tgt cag cat      531
Tyr Arg Cys Ile Cys Pro Pro Gly Arg Thr Gly Asn Arg Cys Gln His
        160                 165                 170

cag gcc cag act gcc gcc ccc gag ggc agc gtg gcc ggc gac tcc gcc      579
Gln Ala Gln Thr Ala Ala Pro Glu Gly Ser Val Ala Gly Asp Ser Ala
    175                 180                 185

ttc agc cgc gcg ccg cgc tgt gcg cag gtg gag cgg gct cag cac tgc      627
Phe Ser Arg Ala Pro Arg Cys Ala Gln Val Glu Arg Ala Gln His Cys
190                 195                 200                 205

agc tgc gag gcc gga ttc cac ctg agc ggc gcc gcc ggc gac agc gtc      675
Ser Cys Glu Ala Gly Phe His Leu Ser Gly Ala Ala Gly Asp Ser Val
                210                 215                 220

tgc cag gat gtg gat gaa tgt gtg ggc ctg cag ccg gtg tgc ccc cag      723
Cys Gln Asp Val Asp Glu Cys Val Gly Leu Gln Pro Val Cys Pro Gln
            225                 230                 235

ggg acc aca tgc atc aac acc ggt gga agc ttc cag tgt gtc agc cct     771
Gly Thr Thr Cys Ile Asn Thr Gly Gly Ser Phe Gln Cys Val Ser Pro
        240                 245                 250

gag tgc ccc gag ggc agc ggc aat gtg agc tac gtg aag acg tct cca     819
Glu Cys Pro Glu Gly Ser Gly Asn Val Ser Tyr Val Lys Thr Ser Pro
    255                 260                 265

ttg tat ttt gga gag cca gtc ttc cag ctc tga gattccttct tcagcttggt     872
Leu Tyr Phe Gly Glu Pro Val Phe Gln Leu
270                 275

tgattctgct gataagactt gagactgcac tgtgaaattc ttctagtgtg ttcttcagct     932

ttatcagtgg cggaggaaga agaaaaatac ttctatttga gaaccctatt gaggttgcat     992

gccaacttct ggagatgttc agacaagaaa aaaagaaatg tggtaaattc taattttacc    1052

tttttatgtc tattttattt acatgacttc agtatccaaa acctaatata aaaaaaaaat    1112

cacataataa aaaaaaaaaa aaaa                                           1136

<210>  24
<211>  279
<212>  PRT
<213>  Homo sapiens

<400>  24

Met Val Pro Ser Ser Pro Arg Ala Leu Phe Leu Leu Leu Leu Ile Leu
1               5                   10                  15

Ala Cys Pro Glu Pro Arg Ala Ser Gln Asn Cys Leu Ser Lys Gln Gln
            20                  25                  30

Leu Leu Ser Ala Ile Arg Gln Leu Gln Gln Leu Leu Lys Gly Gln Glu
        35                  40                  45

Thr Arg Phe Ala Glu Gly Ile Arg His Met Lys Ser Arg Leu Ala Ala
    50                  55                  60

Leu Gln Asn Ser Val Gly Arg Val Gly Pro Asp Ala Leu Pro Val Ser
65                  70                  75                  80

Cys Pro Ala Leu Asn Thr Pro Ala Asp Gly Arg Lys Phe Gly Ser Lys
                85                  90                  95

Tyr Leu Val Asp His Glu Val His Phe Thr Cys Asn Pro Gly Phe Arg
            100                 105                 110

Leu Val Gly Pro Ser Ser Val Val Cys Leu Pro Asn Gly Thr Trp Thr
        115                 120                 125

Gly Glu Gln Pro His Cys Arg Gly Ile Ser Glu Cys Ser Ser Gln Pro
    130                 135                 140

Cys Gln Asn Gly Gly Thr Cys Val Glu Gly Val Asn Gln Tyr Arg Cys
145                 150                 155                 160

Ile Cys Pro Pro Gly Arg Thr Gly Asn Arg Cys Gln His Gln Ala Gln
                165                 170                 175

Thr Ala Ala Pro Glu Gly Ser Val Ala Gly Asp Ser Ala Phe Ser Arg
            180                 185                 190

Ala Pro Arg Cys Ala Gln Val Glu Arg Ala Gln His Cys Ser Cys Glu
        195                 200                 205

Ala Gly Phe His Leu Ser Gly Ala Ala Gly Asp Ser Val Cys Gln Asp
    210                 215                 220

Val Asp Glu Cys Val Gly Leu Gln Pro Val Cys Pro Gln Gly Thr Thr
225                 230                 235                 240

Cys Ile Asn Thr Gly Gly Ser Phe Gln Cys Val Ser Pro Glu Cys Pro
                245                 250                 255

Glu Gly Ser Gly Asn Val Ser Tyr Val Lys Thr Ser Pro Leu Tyr Phe
            260                 265                 270

Gly Glu Pro Val Phe Gln Leu
        275

<210>  25
<211>  2303
<212>  DNA
<213>  Homo sapiens

<220>
<221>  misc_feature
<222>  (1)..(1)
<223>  LP209

<220>
<221>  CDS
<222>  (257)..(1303)

<400>  25
tcagggcttc atacaggaaa tctattgctg tgtcaagttc cagagaaaag cttctgttcg      60
tccaagttac taaccaggct aaaccacata gacgtgaagg aaggggctag aaggaaggga     120
gtgccccact gttgatgggg taagaggatc ctgtactgag aagttgacca gagagggtct     180
caccatgcgc acagttcctt ctgtacctgt gtggaggaaa agtactgagt gaagggcaga     240
aaaagagaaa acagaa atg ctc tgc cct tgg aga act gct aac cta ggg cta     292
ctg ttg att ttg act atc ttc tta gtg gcc gaa gcg gag ggt gct gct       340
caa cca aac aac tca tta atg ctg caa act agc aag gag aat cat gct       388
tta gct tca agc agt tta tgt atg gat gaa aaa cag att aca cag aac       436
tac tcg aaa gta ctc gca gaa gtt aac act tca tgg cct gta aag atg       484
gct aca aat gct gtg ctt tgt tgc cct cct atc gca tta aga aat ttg       532
atc ata ata aca tgg gaa ata atc ctg aga ggc cag cct tcc tgc aca       580
aaa gcc tac agg aaa gaa aca aat gag acc aag gaa acc aac tgt act     628
gat gag aga ata acc tgg gtc tcc aga cct gat cag aat tcg gac ctt     676
cag att cgt cca gtg gcc atc act cat gac ggg tat tac aga tgc ata     724
atg gta aca cct gat ggg aat ttc cat cgt gga tat cac ctc caa gtg     772
tta gtt aca cct gaa gtg acc ctg ttt caa aac agg aat aga act gca     820
gta tgc aag gca gtt gca ggg aag cca gct gcg cag atc tcc tgg atc     868
cca gag ggc gat tgt gcc act aag caa gaa tac tgg agc aat ggc aca     916
gtg act gtt aag agt aca tgc cac tgg gag gtc cac aat gtg tct acc     964
gtg acc tgc cac gtc tcc cat ttg act ggc aac aag agt ctg tac ata     1012
gag cta ctt cct gtt cca ggt gcc aaa aaa tca gca aaa tta tat att     1060
cca tat atc atc ctt act att att att ttg acc atc gtg gga ttc att     1108
tgg ttg ttg aaa gtc aat ggc tgc aga aaa tat aaa ttg aat aaa aca     1156
gaa tct act cca gtt gtt gag gag gat gaa atg cag ccc tat gcc agc     1204
tac aca gag aag aac aat cct ctc tat gat act aca aac aag gtg aag     1252
gca tct cag gca tta caa agt gaa gtt gac aca gac ctc cat act tta     1300
taa gttgttggac tctagtacca agaaacaaca acaaacgaga tacattataa     1353
ttactgtctg attttcttac agttctagaa tgaagactta tattgaaatt aggttttcca     1413
aggttcttag aagacatttt aatggattct cattcatacc cttgtataat tggaattttt     1473
gattcttagc tgctaccagc tagttctctg aagaactgat gttattacaa agaaaataca     1533
tgcccatgac caaatattca aattgtgcag gacagtaaat aatgaaaacc aaatttcctc     1593
aagaaataac tgaagaagga gcaagtgtga acagtttctt gtgtatcctt tcagaatatt     1653
ttaatgtaca tatgacatgt gtatatgcct atggtatatg tgtcaattta tgtgtcccct     1713
tacatataca tgcacatatc tttgtcaagg caccagtggg aacaatacac tgcattactg     1773
ttctatacat atgaaaacct aataatataa gtcttagaga tcattttata tcatgacaag     1833
tagagctacc tcattctttt taatggttat ataaaattcc attgtatagt tatatcatta 1893
tttaattaaa aacaacccta atgatggata tttagattct tttaagtttt gtttatttct 1953
tttaagtttt gtttgtggta taaacaatac cacatagaat gtttcttgtt catatatctc 2013
tttgtttttg agtatatctg taggataact ttcttgagtg gaattgtcag gtcaaagggt 2073
ttgtgcattt tactattgat atatatgtta aattgtgtca aatatatatg tcaaattccc 2133
tccaacattg tttaaatgtg cctttcccta aatttctatt ttaataactg tactattcct 2193
gcttctacag ttgccacttt ctctttttaa tcaaccagat taaatatgat gtgagattat 2253
aataagaatt atactattta ataaaaatgg atttataaaa aaaaaaaaaa 2303

<210> 26
<211> 348
<212> PRT
<213> Homo sapiens

<400> 26

<210>  27
<211>  265
<212>  DNA
<213>  Homo sapiens

<220>
<221>  misc_feature
<222>  (1)..(1)
<223>  LP209b

<220>
<221>  CDS
<222>  (112)..(264)

<400>  27
ggatcctgta ctgagaagtt gaccagagag ggtctcacca tgcgcacagt tccttctgta      60

cctgtgtgga ggaaaagtac tgagtgaagg gcagaaaaag agaaaacaga a atg ctc      117
                                                         Met Leu
                                                         1

tgc cct tgg aga act gct aac cta ggg cta ctg ttg att ttg act atc      165
Cys Pro Trp Arg Thr Ala Asn Leu Gly Leu Leu Leu Ile Leu Thr Ile
        5                   10                  15

ttc tta gtg gcc gct tca agc agt tta tgt atg gat gaa aaa cag att      213
Phe Leu Val Ala Ala Ser Ser Ser Leu Cys Met Asp Glu Lys Gln Ile
    20                  25                  30

aca cag aac tac tcg aaa gta ctc gca gaa gtt aac act tca tgg cct      261
Thr Gln Asn Tyr Ser Lys Val Leu Ala Glu Val Asn Thr Ser Trp Pro
35                  40                  45                  50

gta a                                                                265
Val

<210>  28
<211>  51

<212>   PRT
<213>   Homo sapiens

<400>   28

Met Leu Cys Pro Trp Arg Thr Ala Asn Leu Gly Leu Leu Leu Ile Leu
1               5                   10                  15

Thr Ile Phe Leu Val Ala Ala Ser Ser Ser Leu Cys Met Asp Glu Lys
            20                  25                  30

Gln Ile Thr Gln Asn Tyr Ser Lys Val Leu Ala Glu Val Asn Thr Ser
        35                  40                  45

Trp Pro Val
    50

<210>   29
<211>   714
<212>   DNA
<213>   Homo sapiens

<220>
<221>   CDS
<222>   (1)..(714)

<400>   29
atg ctc tgc cct tgg aga act gct aac cta ggg cta ctg ttg att ttg       48
Met Leu Cys Pro Trp Arg Thr Ala Asn Leu Gly Leu Leu Leu Ile Leu
1               5                   10                  15

act atc ttc tta gtg gcc gct tca agc agt tta tgt atg gat gaa aaa       96
Thr Ile Phe Leu Val Ala Ala Ser Ser Ser Leu Cys Met Asp Glu Lys
            20                  25                  30

cag att aca cag aac tac tcg aaa gta ctc gca gaa gtt aac act tca      144
Gln Ile Thr Gln Asn Tyr Ser Lys Val Leu Ala Glu Val Asn Thr Ser
        35                  40                  45

tgg cct gta aag atg gct aca aat gct gtg ctt tgt tgc cct cct atc      192
Trp Pro Val Lys Met Ala Thr Asn Ala Val Leu Cys Cys Pro Pro Ile
    50                  55                  60

gca tta aga aat ttg atc ata ata aca tgg gaa ata atc ctg aga ggc      240
Ala Leu Arg Asn Leu Ile Ile Ile Thr Trp Glu Ile Ile Leu Arg Gly
65                  70                  75                  80

cag cct tcc tgc aca aaa gcc tac agg aaa gaa aca aat gag acc aag      288
Gln Pro Ser Cys Thr Lys Ala Tyr Arg Lys Glu Thr Asn Glu Thr Lys
                85                  90                  95

gaa acc aac tgt act gat gag aga ata acc tgg gtc tcc aga cct gat      336
Glu Thr Asn Cys Thr Asp Glu Arg Ile Thr Trp Val Ser Arg Pro Asp
            100                 105                 110

cag aat tcg gac ctt cag att cgt cca gtg gcc atc act cat gac ggg      384
Gln Asn Ser Asp Leu Gln Ile Arg Pro Val Ala Ile Thr His Asp Gly
        115                 120                 125

tat tac aga tgc ata atg gta aca cct gat ggg aat ttc cat cgt gga      432
Tyr Tyr Arg Cys Ile Met Val Thr Pro Asp Gly Asn Phe His Arg Gly
    130                 135                 140

tat cac ctc caa gtg tta gtt aca cct gaa gtg acc ctg ttt caa aac      480
Tyr His Leu Gln Val Leu Val Thr Pro Glu Val Thr Leu Phe Gln Asn
145                 150                 155                 160

agg aat aga act gca gta tgc aag gca gtt gca ggg aag cca gct gcg      528
Arg Asn Arg Thr Ala Val Cys Lys Ala Val Ala Gly Lys Pro Ala Ala
                165                 170                 175

cag atc tcc tgg atc cca gag ggc gat tgt gcc act aag caa gaa tac      576

Gln Ile Ser Trp Ile Pro Glu Gly Asp Cys Ala Thr Lys Gln Glu Tyr
            180                 185                 190

tgg agc aat ggc aca gtg act gtt aag agt aca tgc cac tgg gag gtc 624
Trp Ser Asn Gly Thr Val Thr Val Lys Ser Thr Cys His Trp Glu Val
        195                 200                 205

cac aat gtg tct acc gtg acc tgc cac gtc tcc cat ttg act ggc aac 672
His Asn Val Ser Thr Val Thr Cys His Val Ser His Leu Thr Gly Asn
    210                 215                 220

aag agt ctg tac ata gag cta ctt cct gaa aat ata aat tga 714
Lys Ser Leu Tyr Ile Glu Leu Leu Pro Glu Asn Ile Asn
225                 230                 235

<210> 30
<211> 237
<212> PRT
<213> Homo sapiens

<400> 30

Met Leu Cys Pro Trp Arg Thr Ala Asn Leu Gly Leu Leu Leu Ile Leu
1               5                   10                  15

Thr Ile Phe Leu Val Ala Ala Ser Ser Ser Leu Cys Met Asp Glu Lys
            20                  25                  30

Gln Ile Thr Gln Asn Tyr Ser Lys Val Leu Ala Glu Val Asn Thr Ser
        35                  40                  45

Trp Pro Val Lys Met Ala Thr Asn Ala Val Leu Cys Cys Pro Pro Ile
    50                  55                  60

Ala Leu Arg Asn Leu Ile Ile Ile Thr Trp Glu Ile Ile Leu Arg Gly
65                  70                  75                  80

Gln Pro Ser Cys Thr Lys Ala Tyr Arg Lys Glu Thr Asn Glu Thr Lys
                85                  90                  95

Glu Thr Asn Cys Thr Asp Glu Arg Ile Thr Trp Val Ser Arg Pro Asp
            100                 105                 110

Gln Asn Ser Asp Leu Gln Ile Arg Pro Val Ala Ile Thr His Asp Gly
        115                 120                 125

Tyr Tyr Arg Cys Ile Met Val Thr Pro Asp Gly Asn Phe His Arg Gly
    130                 135                 140

Tyr His Leu Gln Val Leu Val Thr Pro Glu Val Thr Leu Phe Gln Asn
145                 150                 155                 160

Arg Asn Arg Thr Ala Val Cys Lys Ala Val Ala Gly Lys Pro Ala Ala
                165                 170                 175

Gln Ile Ser Trp Ile Pro Glu Gly Asp Cys Ala Thr Lys Gln Glu Tyr
            180                 185                 190

Trp Ser Asn Gly Thr Val Thr Val Lys Ser Thr Cys His Trp Glu Val
        195                 200                 205

His Asn Val Ser Thr Val Thr Cys His Val Ser His Leu Thr Gly Asn
    210                 215                 220

Lys Ser Leu Tyr Ile Glu Leu Leu Pro Glu Asn Ile Asn
225                 230                 235

<210>  31
<211>  265
<212>  DNA
<213>  Homo sapiens

<220>
<221>  CDS
<222>  (112)..(264)

<400>  31
ggatcctgta ctgagaagtt gaccagagag ggtctcacca tgcgcacagt tccttctgta      60

cctgtgtgga ggaaaagtac tgagtgaagg gcagaaaaag agaaaacaga a atg ctc      117
                                                         Met Leu
                                                         1

tgc cct tgg aga act gct aac cta ggg cta ctg ttg att ttg act atc      165
Cys Pro Trp Arg Thr Ala Asn Leu Gly Leu Leu Leu Ile Leu Thr Ile
        5                   10                  15

ttc tta gtg gcc gct tca agc agt tta tgt atg gat gaa aaa cag att      213
Phe Leu Val Ala Ala Ser Ser Ser Leu Cys Met Asp Glu Lys Gln Ile
    20                  25                  30

aca cag aac tac tcg aaa gta ctc gca gaa gtt aac act tca tgg cct      261
Thr Gln Asn Tyr Ser Lys Val Leu Ala Glu Val Asn Thr Ser Trp Pro
35                  40                  45                  50

gta a                                                                265
Val

<210>  32
<211>  51
<212>  PRT
<213>  Homo sapiens

<400>  32

Met Leu Cys Pro Trp Arg Thr Ala Asn Leu Gly Leu Leu Leu Ile Leu
1               5                   10                  15

Thr Ile Phe Leu Val Ala Ala Ser Ser Ser Leu Cys Met Asp Glu Lys
            20                  25                  30

Gln Ile Thr Gln Asn Tyr Ser Lys Val Leu Ala Glu Val Asn Thr Ser
        35                  40                  45

Trp Pro Val
    50

<210>  33
<211>  1653
<212>  DNA
<213>  Homo sapiens

<220>
<221>  misc_feature
<222>  (1)..(1)
<223>  LP293

<220>
<221>  CDS
<222>  (185)..(751)

<400> 33
cggctcgagg cgccaggctg tgagggagct gaggacagac agaaggacca cggagggggt 60

gacgggctgg tgtgaggatt ggtgcccctg ggccaggact ctcctctctt ctccctgctg 120

gctccagacc agagtccaag ccctaggcag tgccaccctt acccagccca gccttgaaga 180

caga atg aga ggg gtt tcc tgt ctc cag gtc ctg ctc ctt ctg gtg ctg 229
     Met Arg Gly Val Ser Cys Leu Gln Val Leu Leu Leu Leu Val Leu
     1               5                   10                  15

gga gct gct ggg act cag gga agg aag tct gca gcc tgc ggg cag ccc 277
Gly Ala Ala Gly Thr Gln Gly Arg Lys Ser Ala Ala Cys Gly Gln Pro
                20                  25                  30

cgc atg tcc agt cgg atc gtt ggg ggc cgg gat ggc cgg gac gga gag 325
Arg Met Ser Ser Arg Ile Val Gly Gly Arg Asp Gly Arg Asp Gly Glu
            35                  40                  45

tgg ccg tgg cag gcg agc atc cag cat cgt ggg gca cac gtg tgc ggg 373
Trp Pro Trp Gln Ala Ser Ile Gln His Arg Gly Ala His Val Cys Gly
        50                  55                  60

ggg tcg ctc atc gcc ccc cag tgg gtg ctg aca gcg gcg cac tgc ttc 421
Gly Ser Leu Ile Ala Pro Gln Trp Val Leu Thr Ala Ala His Cys Phe
    65                  70                  75

ccc agt gcc cct ccc aga gtg gcg acc gct aca agg agt aag ggt gcc 469
Pro Ser Ala Pro Pro Arg Val Ala Thr Ala Thr Arg Ser Lys Gly Ala
80                  85                  90                  95

gct gct gga ctc gcg cac ctg cga cgg cct cta cca cgt ggg cgc gga 517
Ala Ala Gly Leu Ala His Leu Arg Arg Pro Leu Pro Arg Gly Arg Gly
                100                 105                 110

cgt gcc cag gct gag cgc att gtg ctg cct ggg agt ctg tgt gcc ggc 565
Arg Ala Gln Ala Glu Arg Ile Val Leu Pro Gly Ser Leu Cys Ala Gly
            115                 120                 125

tac ccc cag ggc cac aag gac gcc tgc cag gtg tgc acc cag cct ccc 613
Tyr Pro Gln Gly His Lys Asp Ala Cys Gln Val Cys Thr Gln Pro Pro
        130                 135                 140

cag cct ccg gag tcc cct ccc tgt gcc cag cac cct ccc tcc ctg aac 661
Gln Pro Pro Glu Ser Pro Pro Cys Ala Gln His Pro Pro Ser Leu Asn
    145                 150                 155

tcc agg acc cag gac atc cca act cag gct cag gat cct ggc ctc caa 709
Ser Arg Thr Gln Asp Ile Pro Thr Gln Ala Gln Asp Pro Gly Leu Gln
160                 165                 170                 175

cct aga ggc acc acg cca ggg gtc tgg aac cct gag aac tga 751
Pro Arg Gly Thr Thr Pro Gly Val Trp Asn Pro Glu Asn
                180                 185

agtcctggga gggctgggac ctaggctcct ctttctcctg cagggtgatt ctgggggacc 811

tctgacctgc ctgcagtctg ggagctgggt cctggtgggc gtggtgagct ggggcaaggg 871

ttgtgccctg cccaaccgtc caggggtcta caccagtgtg gccacatata gcccctggat 931

tcaggctcgc gtcagcttct aatgctagcc ggtgaggctg acctggagcc agctgctggg 991

gtccctcagc ctcctggttc atccaggcac ctgcctatac cccacatccc ttctgcctcg 1051

aggccaagat gcctaaaaaa gctaaaggcc accccacccc ccacccacca cctcctgcct 1111

cctctcctct ttggggatca ccagctctga ctccaccaac cctcatccag gaatctgcca 1171

tgagtcccag ggagtcacac tccccactcc cttcctggct tgtatttact tttcttggcc 1231

ctggccaggg ctgggcgcaa ggcacgcagt gatgggcaaa ccaattgctg cccatctggc 1291

ctgtgtgccc atctttttct ggagaaagtc agattcacag catgacagag atttgacacc 1351

agggagatcc tccatagctg gctttgagga cacggggacc acagccatga gcggcctcta 1411

agagctgaga gacagccggc agggaatcgg aaccctcaga cccacagccg caaggcactg 1471

gattctggca gcaccctgaa ggagctggga agtaagttct tccccagcct ccagataaga 1531

gccccgccgg ccaatccctt catttcaacc taaagagacc ctaagcagag aacctagctg 1591

agccactcct gacctacaaa gttgtgactt aataaatgtg tgctttaagg ctgcaaaaaa 1651

aa 1653

<210> 34
<211> 188
<212> PRT
<213> Homo sapiens

<400> 34

Met Arg Gly Val Ser Cys Leu Gln Val Leu Leu Leu Leu Val Leu Gly
1               5                   10                  15

Ala Ala Gly Thr Gln Gly Arg Lys Ser Ala Ala Cys Gly Gln Pro Arg
            20                  25                  30

Met Ser Ser Arg Ile Val Gly Gly Arg Asp Gly Arg Asp Gly Glu Trp
        35                  40                  45

Pro Trp Gln Ala Ser Ile Gln His Arg Gly Ala His Val Cys Gly Gly
    50                  55                  60

Ser Leu Ile Ala Pro Gln Trp Val Leu Thr Ala Ala His Cys Phe Pro
65                  70                  75                  80

Ser Ala Pro Pro Arg Val Ala Thr Ala Thr Arg Ser Lys Gly Ala Ala
                85                  90                  95

Ala Gly Leu Ala His Leu Arg Arg Pro Leu Pro Arg Gly Arg Gly Arg
            100                 105                 110

Ala Gln Ala Glu Arg Ile Val Leu Pro Gly Ser Leu Cys Ala Gly Tyr
        115                 120                 125

Pro Gln Gly His Lys Asp Ala Cys Gln Val Cys Thr Gln Pro Pro Gln
    130                 135                 140

Pro Pro Glu Ser Pro Pro Cys Ala Gln His Pro Pro Ser Leu Asn Ser
145                 150                 155                 160

Arg Thr Gln Asp Ile Pro Thr Gln Ala Gln Asp Pro Gly Leu Gln Pro
                165                 170                 175

Arg Gly Thr Thr Pro Gly Val Trp Asn Pro Glu Asn
            180                 185

<210> 35
<211> 1606
<212> DNA
<213> Homo sapiens

<220>
<221> misc_feature
<222> (1)..(1)
<223> LP294

<220>
<221>  CDS
<222>  (52)..(894)

<400>  35
gtccaagccc taggcagtgc cacccttacc cagcccagcc ttgaagacag a atg aga      57
                                                         Met Arg
                                                         1

ggg gtt tcc tgt ctc cag gtc ctg ctc ctt ctg gtg ctg gga gct gct      105
Gly Val Ser Cys Leu Gln Val Leu Leu Leu Leu Val Leu Gly Ala Ala
        5                   10                  15

ggg act cag gga agg aag tct gca gcc tgc ggg cag ccc cgc atg tcc      153
Gly Thr Gln Gly Arg Lys Ser Ala Ala Cys Gly Gln Pro Arg Met Ser
    20                  25                  30

agt cgg atc gtt ggg ggc cgg gat ggc cgg gac gga gag tgg ccg tgg      201
Ser Arg Ile Val Gly Gly Arg Asp Gly Arg Asp Gly Glu Trp Pro Trp
35                  40                  45                  50

cag gcg agc atc cag cat cgt ggg gca cac gtg tgc ggg ggg tcg ctc      249
Gln Ala Ser Ile Gln His Arg Gly Ala His Val Cys Gly Gly Ser Leu
                55                  60                  65

atc gcc ccc cag tgg gtg ctg aca gcg gcg cac tgc ttc ccc agg agg      297
Ile Ala Pro Gln Trp Val Leu Thr Ala Ala His Cys Phe Pro Arg Arg
            70                  75                  80

gca ctg cca gct gag tac cgc gtg cgc ctg ggg gcg ctg cgt ctg ggc      345
Ala Leu Pro Ala Glu Tyr Arg Val Arg Leu Gly Ala Leu Arg Leu Gly
        85                  90                  95

tcc acc tcg ccc cgc acg ctc tcg gtg ccc gtg cga cgg gtg ctg ctg      393
Ser Thr Ser Pro Arg Thr Leu Ser Val Pro Val Arg Arg Val Leu Leu
    100                 105                 110

ccc ccg gac tac tcc gag gac ggg gcc cgc ggc gac ctg gca ctg ctg      441
Pro Pro Asp Tyr Ser Glu Asp Gly Ala Arg Gly Asp Leu Ala Leu Leu
115                 120                 125                 130

cag ctg cgt cgc ccg gtg ccc ctg agc gct cgc gtc caa ccc gtc tgc      489
Gln Leu Arg Arg Pro Val Pro Leu Ser Ala Arg Val Gln Pro Val Cys
                135                 140                 145

ctg ccc gtg ccc ggc gcc cgc ccg ccg ccc ggc aca cca tgc cgg gtc      537
Leu Pro Val Pro Gly Ala Arg Pro Pro Pro Gly Thr Pro Cys Arg Val
            150                 155                 160

acc ggc tgg ggc agc ctc cgc cca gga gtg ccc ctc cca gag tgg cga      585
Thr Gly Trp Gly Ser Leu Arg Pro Gly Val Pro Leu Pro Glu Trp Arg
        165                 170                 175

ccg cta caa gga gta agg gtg ccg ctg ctg gac tcg cgc acc tgc gac      633
Pro Leu Gln Gly Val Arg Val Pro Leu Leu Asp Ser Arg Thr Cys Asp
    180                 185                 190

ggc ctc tac cac gtg ggc gcg gac gtg ccc cag gct gag cgc att gtg      681
Gly Leu Tyr His Val Gly Ala Asp Val Pro Gln Ala Glu Arg Ile Val
195                 200                 205                 210

ctg cct ggg agt ctg tgt gcc ggc tac ccc cag ggc cac aag gac gcc      729
Leu Pro Gly Ser Leu Cys Ala Gly Tyr Pro Gln Gly His Lys Asp Ala
                215                 220                 225

tgc cag ggt gat tct ggg gga cct ctg acc tgc ctg cag tct ggg agc      777
Cys Gln Gly Asp Ser Gly Gly Pro Leu Thr Cys Leu Gln Ser Gly Ser
            230                 235                 240

tgg gtc ctg gtg ggc gtg gtg agc tgg ggc aag ggt tgt gcc ctg ccc      825
Trp Val Leu Val Gly Val Val Ser Trp Gly Lys Gly Cys Ala Leu Pro
        245                 250                 255

aat cgt cca ggg gtc tac acc agt gtg gcc aca tat agc ccc tgg att      873
Asn Arg Pro Gly Val Tyr Thr Ser Val Ala Thr Tyr Ser Pro Trp Ile
    260                 265                 270

cag gct cgc gtc agc ttc taa tgctagccgg tgaggctgac ctggagccag 924
Gln Ala Arg Val Ser Phe
275                 280

ctgctggggt ccctcagcct cctggttcat ccaggcacct gcctataccc cacatccctt 984

ctgcctcgag gccaagatgc ctaaaaaagc taaaggccac cccacccccc acccaccacc 1044

tcctgcctcc tctcctcttt ggggatcacc agctctgact ccaccaaccc tcatccagga 1104

atctgccatg agtcccaggg agtcacactc cccactccct tcctggcttg tatttacttt 1164

tcttggccct ggccagggct gggcgcaagg cacgcagtga tgggcaaacc aattgctgcc 1224

catctggcct gtgtgcccat ctttttctgg agaaagtcag attcacagca tgacagagat 1284

ttgacaccag ggagatcctc catagctggc tttgaggaca cggggaccac agccatgagc 1344

ggcctctaag agctgagaga cagccggcag ggaatcggaa ccctcagacc cacagccgca 1404

aggcactgga ttctggcagc accctgaagg agctgggaag taagttcttc cccagcctcc 1464

agataagagc cccgccggcc aatcccttca tttcaaccta aagagaccct aagcagagaa 1524

cctagctgag ccactcctga cctacaaagt tgtgacttaa taaatgtgtg ctttaagctg 1584

caaaaaaaaa aaaaaaaagg gc 1606

<210> 36
<211> 280
<212> PRT
<213> Homo sapiens

<400> 36

Met Arg Gly Val Ser Cys Leu Gln Val Leu Leu Leu Leu Val Leu Gly
1               5                   10                  15

Ala Ala Gly Thr Gln Gly Arg Lys Ser Ala Ala Cys Gly Gln Pro Arg
            20                  25                  30

Met Ser Ser Arg Ile Val Gly Gly Arg Asp Gly Arg Asp Gly Glu Trp
        35                  40                  45

Pro Trp Gln Ala Ser Ile Gln His Arg Gly Ala His Val Cys Gly Gly
    50                  55                  60

Ser Leu Ile Ala Pro Gln Trp Val Leu Thr Ala Ala His Cys Phe Pro
65                  70                  75                  80

Arg Arg Ala Leu Pro Ala Glu Tyr Arg Val Arg Leu Gly Ala Leu Arg
                85                  90                  95

Leu Gly Ser Thr Ser Pro Arg Thr Leu Ser Val Pro Val Arg Arg Val
            100                 105                 110

Leu Leu Pro Pro Asp Tyr Ser Glu Asp Gly Ala Arg Gly Asp Leu Ala
        115                 120                 125

Leu Leu Gln Leu Arg Arg Pro Val Pro Leu Ser Ala Arg Val Gln Pro
    130                 135                 140

Val Cys Leu Pro Val Pro Gly Ala Arg Pro Pro Pro Gly Thr Pro Cys
145                 150                 155                 160

Arg Val Thr Gly Trp Gly Ser Leu Arg Pro Gly Val Pro Leu Pro Glu
                165                 170                 175

Trp Arg Pro Leu Gln Gly Val Arg Val Pro Leu Leu Asp Ser Arg Thr
            180                 185                 190

Cys Asp Gly Leu Tyr His Val Gly Ala Asp Val Pro Gln Ala Glu Arg
        195                 200                 205

Ile Val Leu Pro Gly Ser Leu Cys Ala Gly Tyr Pro Gln Gly His Lys
    210                 215                 220

Asp Ala Cys Gln Gly Asp Ser Gly Gly Pro Leu Thr Cys Leu Gln Ser
225                 230                 235                 240

Gly Ser Trp Val Leu Val Gly Val Val Ser Trp Gly Lys Gly Cys Ala
                245                 250                 255

Leu Pro Asn Arg Pro Gly Val Tyr Thr Ser Val Ala Thr Tyr Ser Pro
            260                 265                 270

Trp Ile Gln Ala Arg Val Ser Phe
        275                 280

<210>  37
<211>  1725
<212>  DNA
<213>  Homo sapiens

<220>
<221>  misc_feature
<222>  (1)..(1)
<223>  LP295

<220>
<221>  CDS
<222>  (147)..(1388)

<400>  37
cacacaggta cacacaataa aactcacatg cacacacata cacacacaac acaccaggga      60

agtgacaacc ctgaacatca ggtgtcagca aacacagagg ctggcagctg gctggtatca     120

gctctgcagc caggaggaca gagaag atg gcc ttc att gca gct ttg ggg ctc      173
                             Met Ala Phe Ile Ala Ala Leu Gly Leu
                             1               5

ttg atg gca ggg atc tgc cct gct gtc ctc tgt gat ggc aca ctg gga      221
Leu Met Ala Gly Ile Cys Pro Ala Val Leu Cys Asp Gly Thr Leu Gly
10                  15                  20                  25

agg gac act cta tcc cat gaa gac cac ggc aag ggg aga caa ctg cac      269
Arg Asp Thr Leu Ser His Glu Asp His Gly Lys Gly Arg Gln Leu His
                30                  35                  40

agt ctc aca ttg gct tcc agc aac act gac ttc gca ttg agc ctc tac      317
Ser Leu Thr Leu Ala Ser Ser Asn Thr Asp Phe Ala Leu Ser Leu Tyr
            45                  50                  55

aag aag ctg gct ttg agg aat cca gat aaa aat gtt gtc ttc tcc cct      365
Lys Lys Leu Ala Leu Arg Asn Pro Asp Lys Asn Val Val Phe Ser Pro
        60                  65                  70

ctt agc atc tca gct gcc ttg acc att ttg tct ctg gga gca aaa gac      413
Leu Ser Ile Ser Ala Ala Leu Thr Ile Leu Ser Leu Gly Ala Lys Asp
    75                  80                  85

agc acc atg gaa gag att cta gaa ggt ctc aag ttc aat ctc aca gag      461

Ser Thr Met Glu Glu Ile Leu Glu Gly Leu Lys Phe Asn Leu Thr Glu
90                  95                  100                 105

atc act gag gaa gaa atc cac cag ggc ttt ggg cac ctc cta cag agg     509
Ile Thr Glu Glu Glu Ile His Gln Gly Phe Gly His Leu Leu Gln Arg
                110                 115                 120

ctc agc cag cca gag gac cag gta gag atc aat aca ggt agt gcc ctg     557
Leu Ser Gln Pro Glu Asp Gln Val Glu Ile Asn Thr Gly Ser Ala Leu
            125                 130                 135

ttt att gac aaa gag cag ccg ata ctg tca gaa ttc cag gag aag aca     605
Phe Ile Asp Lys Glu Gln Pro Ile Leu Ser Glu Phe Gln Glu Lys Thr
        140                 145                 150

agg gct ctg tac cag gct gag gcc ttc gta gct gac ttc aag cag ccc     653
Arg Ala Leu Tyr Gln Ala Glu Ala Phe Val Ala Asp Phe Lys Gln Pro
    155                 160                 165

aat gag gcc aaa aag ctc atc aat gac tat gtg agc aat cag acc cag     701
Asn Glu Ala Lys Lys Leu Ile Asn Asp Tyr Val Ser Asn Gln Thr Gln
170                 175                 180                 185

ggg aag atc gca gaa ctg ttc tca gac ctg gag gag agg aca tcc atg     749
Gly Lys Ile Ala Glu Leu Phe Ser Asp Leu Glu Glu Arg Thr Ser Met
                190                 195                 200

gtg ctg gtg aac tat ctc cta ttt aaa ggc aaa tgg aag gcc ccc ttt     797
Val Leu Val Asn Tyr Leu Leu Phe Lys Gly Lys Trp Lys Ala Pro Phe
            205                 210                 215

aac ccc aat gac aca att gag tct gag ttc tac ttg gat gag aag agg     845
Asn Pro Asn Asp Thr Ile Glu Ser Glu Phe Tyr Leu Asp Glu Lys Arg
        220                 225                 230

tct gtg aag gtg ccc atg atg aaa att aag gaa gta acc aca ccc tat     893
Ser Val Lys Val Pro Met Met Lys Ile Lys Glu Val Thr Thr Pro Tyr
    235                 240                 245

gtc cgg gat gag gaa ctc tcc tgc tct gtg ttg gag ctg aag tac aca     941
Val Arg Asp Glu Glu Leu Ser Cys Ser Val Leu Glu Leu Lys Tyr Thr
250                 255                 260                 265

gga aat gcc agc gcc ctg ttt atc ctc cct gac caa ggc aag atg cag     989
Gly Asn Ala Ser Ala Leu Phe Ile Leu Pro Asp Gln Gly Lys Met Gln
                270                 275                 280

cag gtg gaa tcc agc ttg caa cca gag acc ctg aag aag tgg aag gac     1037
Gln Val Glu Ser Ser Leu Gln Pro Glu Thr Leu Lys Lys Trp Lys Asp
            285                 290                 295

tct ttg atc cct agg att ata aat gat ctc cgc atg ccc aag ttc tcc     1085
Ser Leu Ile Pro Arg Ile Ile Asn Asp Leu Arg Met Pro Lys Phe Ser
        300                 305                 310

atc tcc aca gac tac agt cta aaa gag gtc ctt cca gag ctg ggc att     1133
Ile Ser Thr Asp Tyr Ser Leu Lys Glu Val Leu Pro Glu Leu Gly Ile
    315                 320                 325

aag aaa gtc ttc tcc cag caa gct gat ctg agt agg atc aca ggg acc     1181
Lys Lys Val Phe Ser Gln Gln Ala Asp Leu Ser Arg Ile Thr Gly Thr
330                 335                 340                 345

aag gat ctg tat gtc tct caa gtg gtc cac aag gct gtg ctg gat gtg     1229
Lys Asp Leu Tyr Val Ser Gln Val Val His Lys Ala Val Leu Asp Val
                350                 355                 360

gat gag aca ggc aca gaa gca act gct gct aca ggg gtc gct aca gtc     1277
Asp Glu Thr Gly Thr Glu Ala Thr Ala Ala Thr Gly Val Ala Thr Val
            365                 370                 375

ata cgt aga caa cct cga act ctg aat ttc aac cgg cca ttc atg gtg     1325
Ile Arg Arg Gln Pro Arg Thr Leu Asn Phe Asn Arg Pro Phe Met Val
        380                 385                 390

gtt atc act gac atg gat agt cag tct atc ctc ttt gtt gcc aaa atc     1373
Val Ile Thr Asp Met Asp Ser Gln Ser Ile Leu Phe Val Ala Lys Ile

395                 400                 405

act aac ccc aag taa gcccaaagct ccccaagatc tgacagttct gcccaggacc      1428
Thr Asn Pro Lys
410

ctgggactga gcctgcatgt ggatctctgt atatgtcctg acattcatgc tctgattggc    1488

tgtttcaaag ttgtcttaga cagtgacatc gactatctct atggccccca tgtgcacagg    1548

agcctttaga ctgtcagtgt caggctctta ggccccttgg gagcatctac ccatgtttct    1608

gaacctggaa tctatcttta ttcttcccta ggtgactcct ctttctgtgt tcatacccca    1668

aacctagtta ttggcaagta catccagtcc caataaatgc tttgagcaca aaaaaaa       1725

<210>  38
<211>  413
<212>  PRT
<213>  Homo sapiens

<400>  38

Met Ala Phe Ile Ala Ala Leu Gly Leu Leu Met Ala Gly Ile Cys Pro
1               5                   10                  15

Ala Val Leu Cys Asp Gly Thr Leu Gly Arg Asp Thr Leu Ser His Glu
            20                  25                  30

Asp His Gly Lys Gly Arg Gln Leu His Ser Leu Thr Leu Ala Ser Ser
        35                  40                  45

Asn Thr Asp Phe Ala Leu Ser Leu Tyr Lys Lys Leu Ala Leu Arg Asn
    50                  55                  60

Pro Asp Lys Asn Val Val Phe Ser Pro Leu Ser Ile Ser Ala Ala Leu
65                  70                  75                  80

Thr Ile Leu Ser Leu Gly Ala Lys Asp Ser Thr Met Glu Glu Ile Leu
                85                  90                  95

Glu Gly Leu Lys Phe Asn Leu Thr Glu Ile Thr Glu Glu Glu Ile His
            100                 105                 110

Gln Gly Phe Gly His Leu Leu Gln Arg Leu Ser Gln Pro Glu Asp Gln
        115                 120                 125

Val Glu Ile Asn Thr Gly Ser Ala Leu Phe Ile Asp Lys Glu Gln Pro
    130                 135                 140

Ile Leu Ser Glu Phe Gln Glu Lys Thr Arg Ala Leu Tyr Gln Ala Glu
145                 150                 155                 160

Ala Phe Val Ala Asp Phe Lys Gln Pro Asn Glu Ala Lys Lys Leu Ile
                165                 170                 175

Asn Asp Tyr Val Ser Asn Gln Thr Gln Gly Lys Ile Ala Glu Leu Phe
            180                 185                 190

Ser Asp Leu Glu Glu Arg Thr Ser Met Val Leu Val Asn Tyr Leu Leu
        195                 200                 205

Phe Lys Gly Lys Trp Lys Ala Pro Phe Asn Pro Asn Asp Thr Ile Glu

210                 215                 220

Ser Glu Phe Tyr Leu Asp Glu Lys Arg Ser Val Lys Val Pro Met Met
225                 230                 235                 240

Lys Ile Lys Glu Val Thr Thr Pro Tyr Val Arg Asp Glu Glu Leu Ser
                245                 250                 255

Cys Ser Val Leu Glu Leu Lys Tyr Thr Gly Asn Ala Ser Ala Leu Phe
            260                 265                 270

Ile Leu Pro Asp Gln Gly Lys Met Gln Gln Val Glu Ser Ser Leu Gln
        275                 280                 285

Pro Glu Thr Leu Lys Lys Trp Lys Asp Ser Leu Ile Pro Arg Ile Ile
    290                 295                 300

Asn Asp Leu Arg Met Pro Lys Phe Ser Ile Ser Thr Asp Tyr Ser Leu
305                 310                 315                 320

Lys Glu Val Leu Pro Glu Leu Gly Ile Lys Lys Val Phe Ser Gln Gln
                325                 330                 335

Ala Asp Leu Ser Arg Ile Thr Gly Thr Lys Asp Leu Tyr Val Ser Gln
            340                 345                 350

Val Val His Lys Ala Val Leu Asp Val Asp Glu Thr Gly Thr Glu Ala
        355                 360                 365

Thr Ala Ala Thr Gly Val Ala Thr Val Ile Arg Arg Gln Pro Arg Thr
    370                 375                 380

Leu Asn Phe Asn Arg Pro Phe Met Val Val Ile Thr Asp Met Asp Ser
385                 390                 395                 400

Gln Ser Ile Leu Phe Val Ala Lys Ile Thr Asn Pro Lys
                405                 410

<210>  39
<211>  86
<212>  DNA
<213>  Artificial Sequence

<220>
<223>  synthetic construct

<400>  39
gcgcctcgag atttccccga aatctagatt tccccgaaat gatttccccg aaatgatttc      60

cccgaaatat ctgccatctc aattag                                           86

<210>  40
<211>  27
<212>  DNA
<213>  Artificial Sequence

<220>
<223>  synthetic construct

<400>  40
   gcggcaagct ttttgcaaag cctaggc                                           27

<210>  41
<211>  270
<212>  DNA
<213>  Artificial Sequence

<220>
<223>  synthetic construct

<400>  41
ctcgagattt ccccgaaatc tagatttccc cgaaatgatt ccccgaaatg atttccccga      60

aatatctgcc atctcaatta gtcagcaacc atagtcccgc ccctaactcc gcccatcccg     120

cccctaactc cgcccagttc cgcccattct ccgccccatg gctgactaat tttttttatt     180

tatgcagagg ccgaggccgc ctcggcctct gagctattcc agaagtagtg aggaggcttt     240

tttggaggcc taggcttttg caaaaagctt                                      270


<210>  42
<211>  32
<212>  DNA
<213>  artificial sequence

<220>
<223>  synthetic construct

<400>  42
gcgctcgagg gatgacagcg atagaacccc gg                                    32


<210>  43
<211>  31
<212>  DNA
<213>  Artificial Sequence

<220>
<223>  synthetic construct

<400>  43
gcgaagcttc gcgactcccc ggatccgcct c                                     31


<210>  44
<211>  82
<212>  DNA
<213>  Artificial Sequence

<220>
<223>  synthetic construct

<400>  44
ggggactttc ccgcggcctc gaggggactt tcccggggac tttccgggga tccgggactt      60

tccatcctgc catctcaatt ag                                               82


<210>  45
<211>  70
<212>  DNA
<213>  Artificial Sequence

<220>
<223>  synthetic construct

<400>  45
gcggcctcga ggggactttc ccggggactt tccggggatc cgggactttc catcctgcca      60

tctcaattag                                                             70


<210>  46
<211>  27
<212>  DNA
<213>  Artificial Sequence

<220>
<223>  synthetic construct

<400>  46
gcggcaagct ttttgcaaag cctaggc                                          27


<210>  47
<211>  256
<212>  DNA
<213>  Artificial Sequence

<220>
<223>  synthetic construct

<400>  47
ctcgagggga ctttcccggg gactttccgg ggactttccg ggactttcca tctgccatct      60

caattagtca gcaaccatag tcccgcccct aactccgccc atcccgcccc taactccgcc     120

cagttccgcc cattctccgc cccatggctg actaattttt tttatttatg cagaggccga     180

ggccgcctcg gcctctgagc tattccagaa gtagtgagga ggcttttttg gaggcctagg     240

cttttgcaaa aagctt                                                     256